# EUROPEAN PATENT SPECIFICATION

(11) **EP 1 383 731 B1**
(45) Date of publication and mention of the grant of the patent: **05.08.2009**
(21) Application number: 01981772.5
(22) Date of filing: 22.10.2001
(51) Int. Cl.: C07C 229/38, C07C 317/22, C07C 63/04, C07C 257/00, C07D 333/22, C07D 307/02, C07D 277/30, C07D 207/30, C07D 207/08, C07D 211/70, C07D 311/78, A61K 31/192, A61K 31/341, A61P 7/00

(54) **BIARYL COMPOUNDS AS SERINE PROTEASE INHIBITORS**
BIARYLVERBINDUNGEN ALS SERINPROTEASEINHIBITOREN
COMPOSES BIARYLE UTILISES COMME INHIBITEURS DE SERINE PROTEASE

(30) Priority: 06.04.2001 US 281735 P
(43) Date of publication of application: 28.01.2004
(73) Proprietor: BIOCRYST PHARMACEUTICALS, INC., Birmingham, Alabama 35244 (US)
(72) Inventor: BABU, Yarlagadda, S., Birmingham, AL 35226 (US); ROWLAND, Scott, R., Hoover, AL 35226 (US); CHAND, Pooran, Birmingham, AL 35226 (US); KOTIAN, Pravin, L., Birmingham, AL 35226 (US); EL-KATTAN, Yahya, Hoover, AL 35216 (US); NIWAS, Shri, Birmingham, AL 35244 (US)
(74) Representative: Zanoli, Enrico
(86) International application number: PCT/US2001/032582
(87) International publication number: WO 2002/034711

(56) References cited:
- WO-A1-99/41231
- US-A- 4 551 279
- KOHRT J T ET AL: "An efficient synthesis of 2-(3-(4-amidinophenylcarbamoyl)naphthalen- 2 yl)-5-((2,2-methylpropyl)carbamo yl)benzoic acid: a factor VIIa inhibitor discovered by the Ono Pharmaceutical Company" TETRAHEDRON LETTERS, ELSEVIER SCIENCE PUBLISHERS, AMSTERDAM, NL, vol. 41, no. 32, 5 August 2000 (2000-08-05), pages 6041-6044, XP004243509 ISSN: 0040-4039
- PRYOR K.E. ET AL.: 'The activated core approach to combinatorial chemistry: A selection of new core molecules' TETRAHEDRON vol. 54, 1998, pages 4107 - 4124, XP004162184

## Description

### Technical Field

The present invention relates to the identification, through synthesis and testing, of heretofore unreported compounds which, in appropriate pharmaceutical compositions, exert a therapeutic effect through reversible inhibition of serine proteases.

### Background of Invention

Serine proteases make up the largest and most extensively studied group of proteolytic enzymes. Their critical roles in physiological processes extend over such diverse areas as blood coagulation, fibrinolysis, complement activation, reproduction, digestion, and the release of physiologically active peptides. Many of these vital processes begin with cleavage of a single peptide bond or a few peptide bonds in precursor protein or peptides. Sequential limited proteolytic reactions or cascades are involved in blood clotting, fibrinolysis, and complement activation. The biological signals to start these cascades can be controlled and amplified as well. Similarly, controlled proteolysis can shut down or inactivate proteins or peptides through single bond cleavages.

While serine proteases are physiologically vital, they also can be hazardous. Their proteolytic action, if uncontrolled, can destroy cells and tissues through degradation of proteins. As a natural safeguard in normal plasma, 10% of the protein matter is composed of protease inhibitors. The major natural plasma inhibitors are specific for serine proteinases. Diseases (associated protease given in the parentheses) such as pulmonary emphysema (cathepsin G), adult respiratory distress syndrome (chymases), and pancreatitis (trypsin, chymotrypsin, and others) are characterized by uncontrolled serine proteases. Other proteases appear to be involved in tumor invasion (plasmin, plasminogen activator), viral transformation, and inflammation (kallikrein). Thus the design and synthesis of specific inhibitors for this class of proteinases could offer major therapeutic benefits.

Thrombus formation, that is blood coagulation, is normally initiated by tissue injury; its normal purpose is to slow or prevent blood loss and facilitate wound healing. There are other conditions, however, not directly connected with tissue injury that may promote the coagulation process and lead instead to harmful consequences; examples of such conditions are atherosclerosis and inflammation.

The complex pathways of blood coagulation involve a series of enzyme reactions in which plasma coagulation factors, actually enzyme precursors or zymogens, are sequentially activated by limited proteolysis. Blood coagulation, or the coagulation cascade, is viewed mechanistically as two pathways, the extrinsic and the intrinsic (Fig. 1). Each pathway proceeds through a sequence of the Roman-numeral-designated factors until they converge at the activation of factor X after merger of the pathways. Thrombin generation proceeds stepwise through a common pathway. Thrombin then acts on the solution plasma protein, fibrinogen, to convert it to stable insoluble fibrin clots, thus completing the coagulation cascade.

The extrinsic pathway is vital to the initiation phase of blood coagulation while the intrinsic pathway provides necessary factors in the maintenance and growth of fibrin. The initiation of the coagulation cascade involves the release of tissue factor (TF) from injured vessel endothelial cells and subendothelium. TF then acts upon factor VII to form the TF/FVIIa complex (where VIIa designates the activated factor rather than the zymogen form). This complex initiates coagulation by activating factors IX and X. The resulting factor Xa forms a prothrombinase complex that activates prothrombin to produce the thrombin that converts fibrinogen to insoluble fibrin. In contrast, the intrinsic system is activated in vivo when certain coagulation proteins contact subendothelial connective tissue. In the sequence that follows, contact factors XII and XI are activated. The resulting factor XIa activates factor IX; then factor IXa activates factor X thereby intersecting with the extrinsic pathway.

With time, the TF/FVIIIa complex (of the extrinsic pathway) loses activity due to the action of tissue factor pathway inhibitor (TFPI), a Kunitz-type protease inhibitor protein which, when complexed with factor Xa, can inhibit the proteolytic activity of TF/FVIIa. If the extrinsic system is inhibited, additional factor Xa is produced through the thrombin-mediated action in the intrinsic pathway. Thrombin, therefore, exerts a dual catalytic role in (a) the conversion of fibrinogen to fibrin and (b) mediating its own production. The autocatalytic aspect of thrombin production affords an important safeguard against excessive blood loss, and, assuming presence of a threshold level of prothrombinase, ensures that the blood coagulation process will go to completion.

While the ability to form blood clots is vital to survival, there are disease states wherein the formation of blood clots within the circulatory system can cause death. When patients are afflicted with such disease states, it is not desirable to completely inhibit the clotting system because life-threatening hemorrhage would follow. Thus, it is highly desirable to develop agents that inhibit coagulation by inhibition of factor VIIa without directly inhibiting thrombin.

Kohrt J.T. et al, Tetrahedron Letters, vol. 41, No.32, 5 August 2000, pages 6041-6044, report a small molecule factor VIIa inhibitior and a synthesis thereof. The molecule comprises a napthyl phenyl moiety and the synthesis relies upon a palladium catalyzed Stille coupling reaction.

Need for the prevention of intravascular blood clots or for anti-coagulant treatment in many clinical situations is well known. Drugs in use today are often not satisfactory. A high percentage of patients who suffer internal injuries or undergo certain surgical procedures develop intravascular blood clots which, if unchecked, cause death. In total hip replacement surgery, for example, it is reported that 50% of the patients develop deep vein thrombosis (DVT). Current approved therapies involve administration of heparin in various forms, but results are not entirely satisfactory; 10-20% of patients suffer DVT and 5-10% have bleeding complications. Along these lines, see International Publication No. WO 00/15658.

Other examples of clinical situations for which better anticoagulants would be of great value are when patients undergo transluminal coronary angioplasty and treatment for myocardial infarction or crescendo angina. The present therapy for these conditions is administration of heparin and aspirin, but this treatment is associated with a 6-8% abrupt vessel closure rate within 24 hours of the procedure. Transfusion therapy due to bleeding complications is required in approximately 7% of cases following the use of heparin. Occurrences of delayed vessel closures are also significant, but administration of heparin after termination of the procedure affords little beneficial effect and can be detrimental.

Heparin and certain derivatives thereof are the most commonly used anti-clotting agents. These substances exert their effects mainly through inactivation of thrombin, which is inactivated 100 times faster than factor Xa. Two other thrombin-specific anticoagulants, hirudin and hirulog, are in clinical trials (as of September 1999). However, bleeding complications are associated with these agents.

In preclinical studies in baboons and dogs, the targeting of enzymes involved in earlier stages of the coagulation cascade, such as factor VIIa or factor Xa, prevents clot formation and does not produce bleeding side effects observed with direct thrombin inhibitors.

Several preclinical studies reveal that inhibition of TF/FVIIa offers the widest window of therapeutic effectiveness and safety with respect to bleeding risk of any anticoagulant approach tested including thrombin, platelet, and factor Xa inhibition.

A specific inhibitor of factor VIIa would provide clinicians with a valuable and needed agent that would be safe and effective in situations where the present drugs of choice, heparin and related sulfated polysaccharides, are no better than marginally effective.

There exists a need for a low molecular weight specific serine protease inhibitors specific toward various enzymes, particularly for factor VIIa that does not cause unwanted side effects.

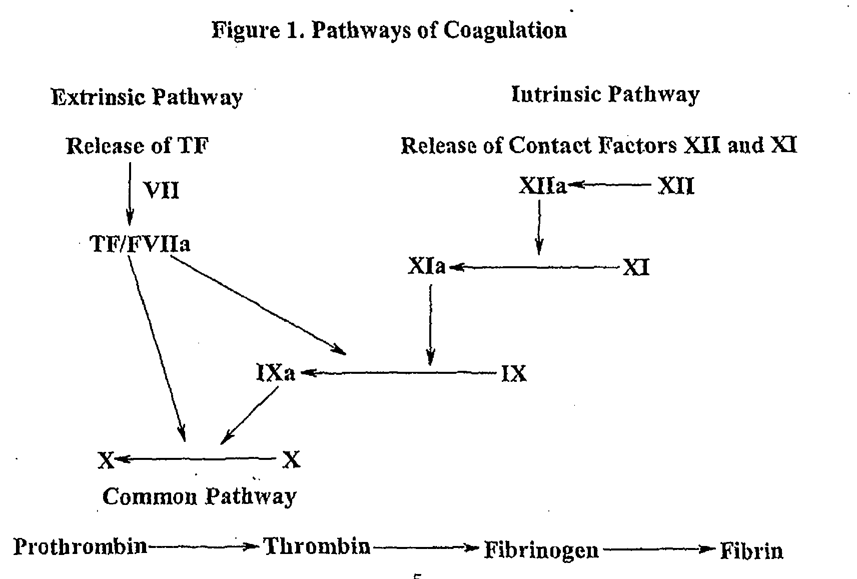

The figure illustrates the extrinsic and intrinsic pathways of blood coagulation.

### Summary of Invention

An aspect of the present invention relates to compounds represented by the formulae as claimed.

Still other objects and advantages of the present invention will become readily apparent by those skilled in the art from the following detailed description, wherein it is shown and described preferred embodiments of the invention, simply by way of illustration of the best mode contemplated of carrying out the invention. As will be realized the invention is capable of other and different embodiments, and its several details are capable of modifications in various obvious respects, without departing from the invention. Accordingly, the description is to be regarded as illustrative in nature and not as restrictive.

### Best and Various Modes for Carrying Out Invention

Listed below are definitions of various terms used to describe this invention. These definitions apply to the terms as they are used throughout this specification, unless otherwise limited in specific instances, either individually or as part of a larger group.

The term "alkyl" refers to straight or branched chain unsubstituted hydrocarbon groups of 1 to 20 carbon atoms, preferably 1 to 8 carbon atoms.

Preferred alkyl groups are lower alkyl groups containing 1 to about 8 carbon, and more preferably 1 to about 5 carbon atoms, and can be straight or branched-chain saturated aliphatic hydrocarbon groups.

Examples of suitable alkyl groups include methyl, ethyl and propyl. Examples of branched alkyl groups include isopropyl and t-butyl.

Pharmaceutically acceptable salts of the compounds of the present invention include those derived from pharmaceutically acceptable, inorganic and organic acids and bases. Examples of suitable acids include hydrochloric, hydrobromic, sulphuric, nitric, perchloric, fumaric, maleic, phosphoric, glycollic, lactic, salicyclic, succinic, toluene-p-sulphonic, tartaric, acetic, citric, methanesulphonic, formic, benzoic, malonic, naphthalene-2-sulphonic, trifluoroacetic and benzenesulphonic acids.

Salts derived from appropriate bases include alkali such as sodium and ammonia.

It is of course understood that the compounds of the present invention relate to all optical isomers and stereo-isomers at the various possible atoms of the molecule.

The synthetic routes leading to the compounds claimed are described in the following schemes.

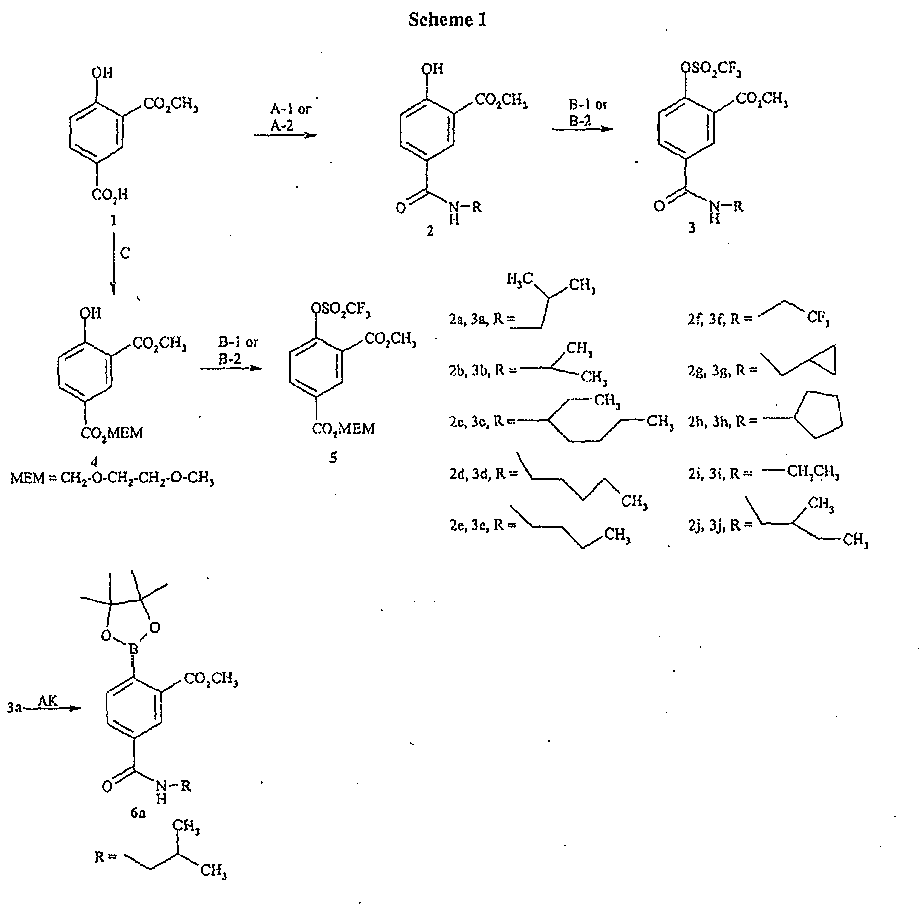

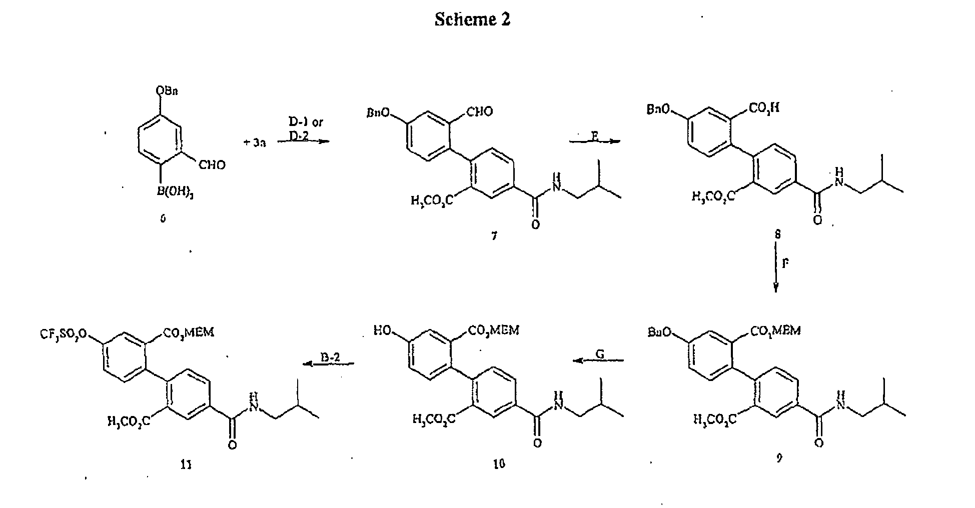

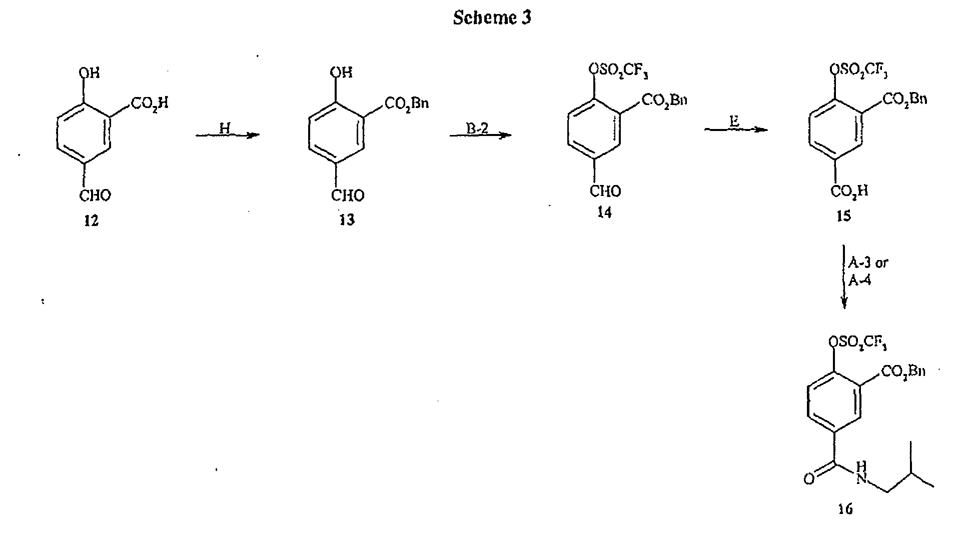

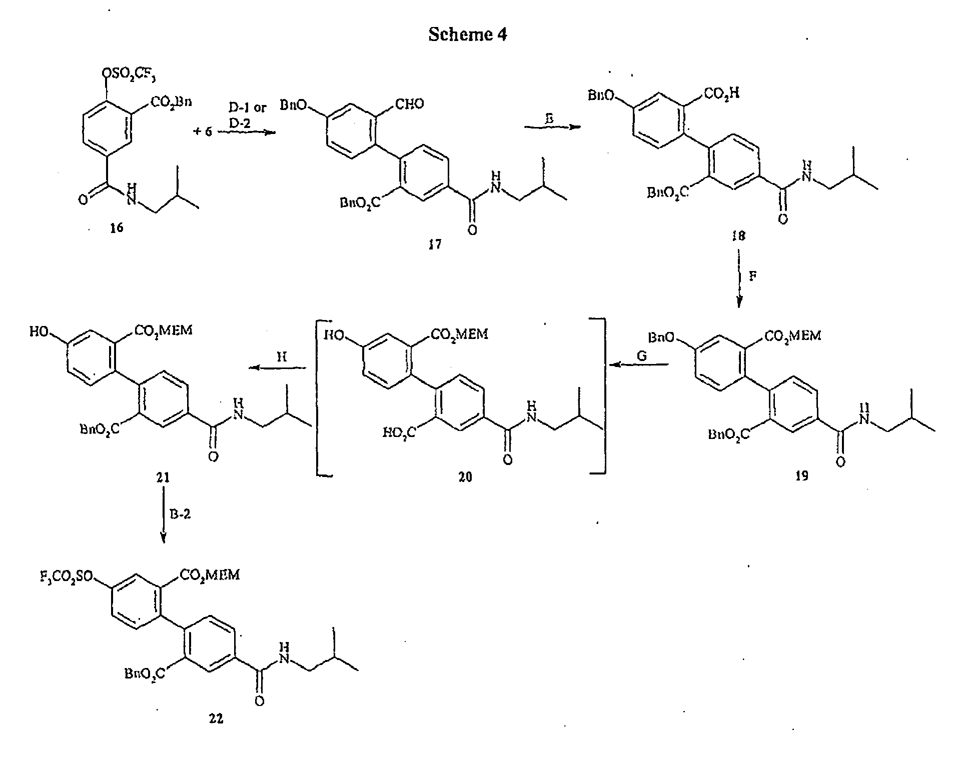

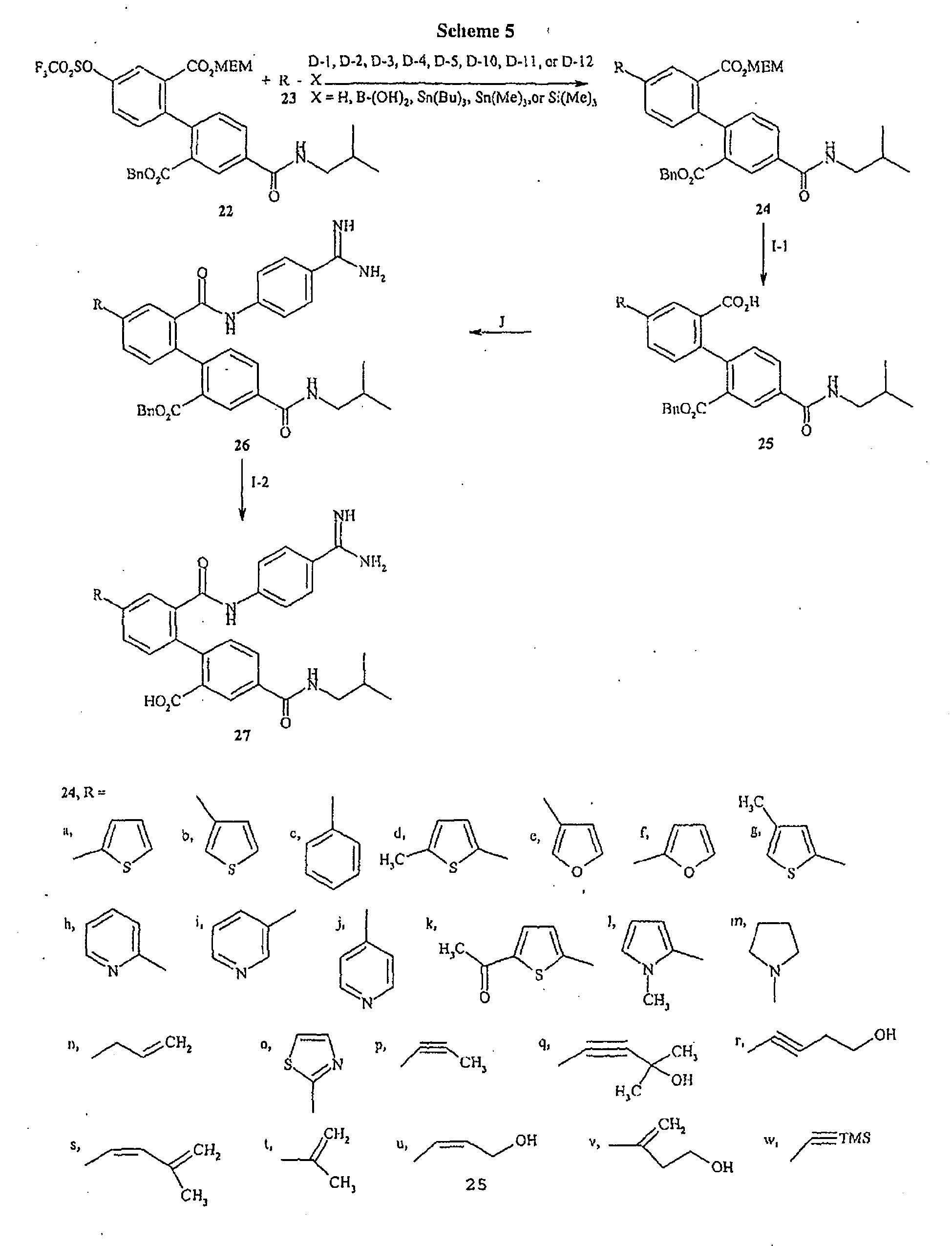

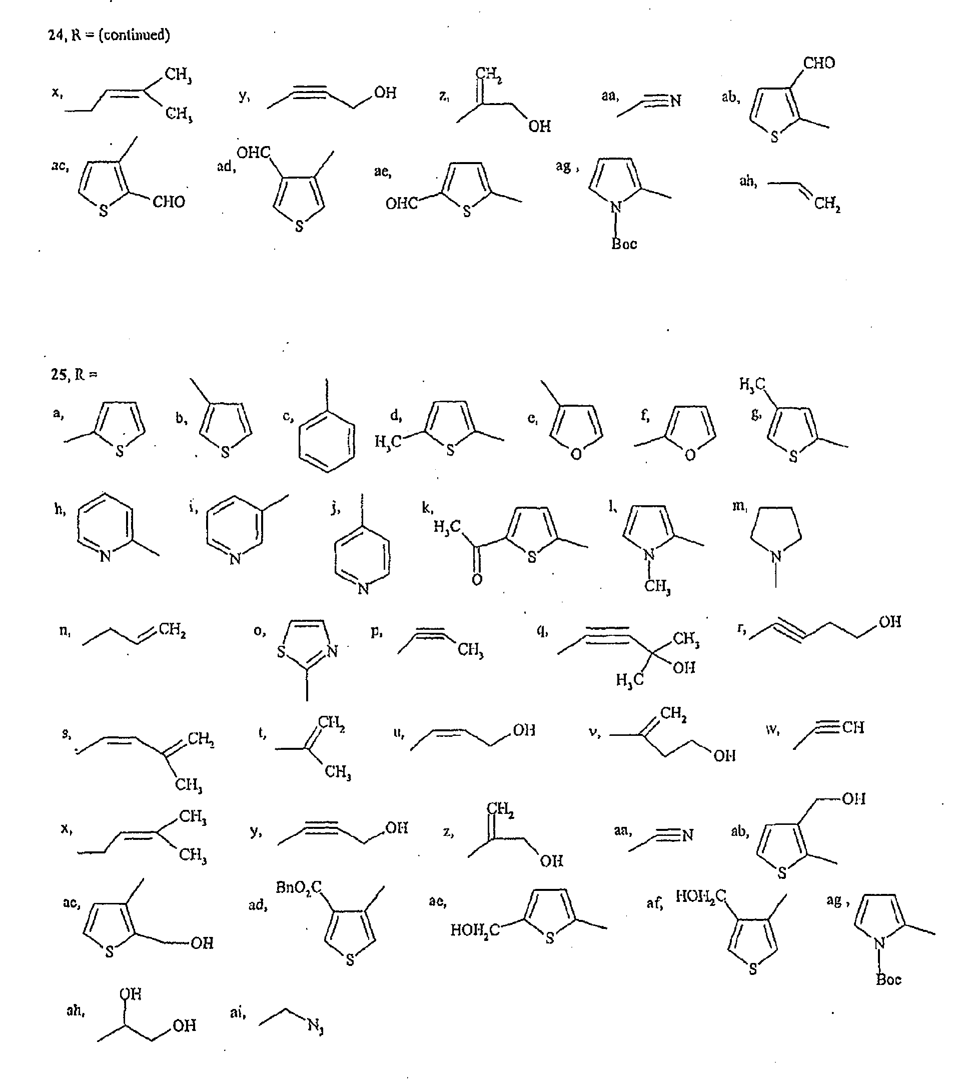

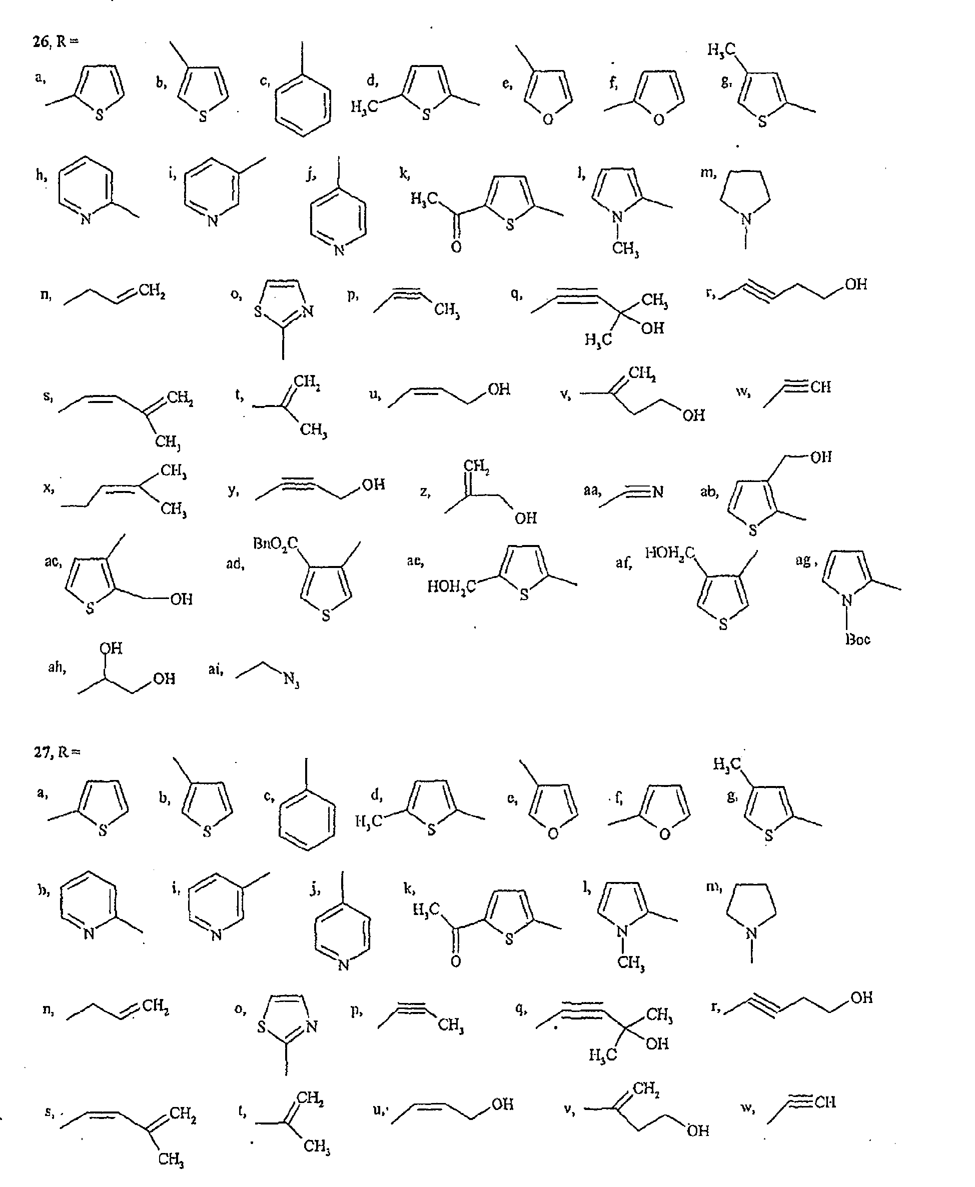

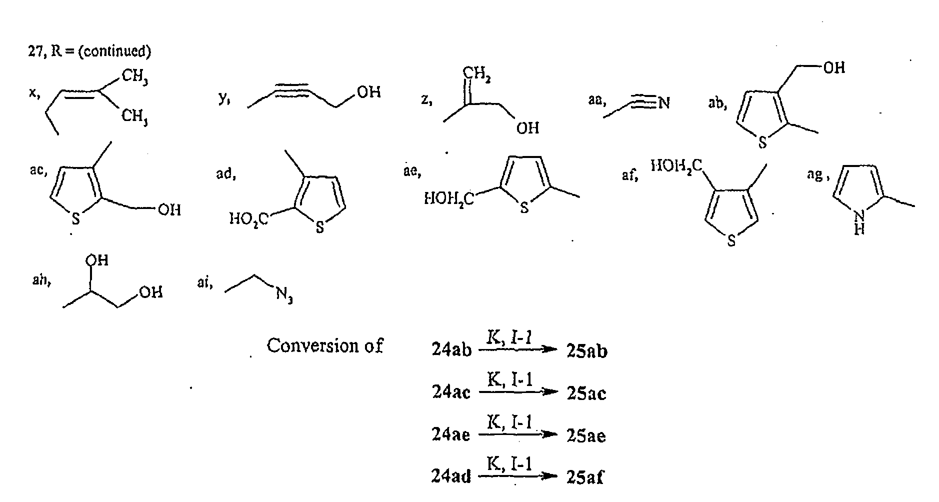

The reduction of the formyl group of **24ab, 24ac, 24ae,** and **24ad** was accomplished with NaBH₄ to give corresponding alcohols **24ab-i, 24ac-i, 24ae-i**, and **24ad-i,** respectively. Later, the MEM group was removed under acidic conditions to give **25ab, 25ac, 25ae,** and **25af,** respectively.

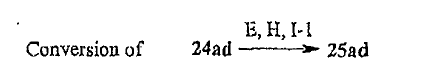

The aldehyde **24ad** was oxidized to acid **24ad-i** which was protected as benzyl ester to give **24ad-ii.** MEM deprotection under acidic conditions produced **25ad.**

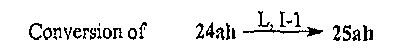

The vinyl compound **24ah** was oxidized with OsO₄ to give diol **24ah-i,** followed by acidic hydrolysis of the MEM group to produce **25ah.**

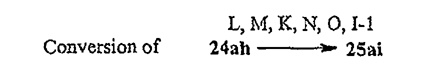

The vinyl compound **24ah** on dihydroxylation with OsO₄ gave diol **24ah-i.** Oxidative cleavage of the diol with NaIO4 produced aldehyde **24ah-ii.** The aldehyde on reduction gave alcohol **24ah-iii,** which on further reaction with methane sulfonyl chloride yielded mesylate **24ah-iv.** The mesylate on further reaction with sodium azide gave the corresponding azide **24ah-v,** which on acidic hydrolysis produced **25ai.** 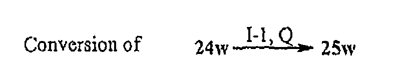 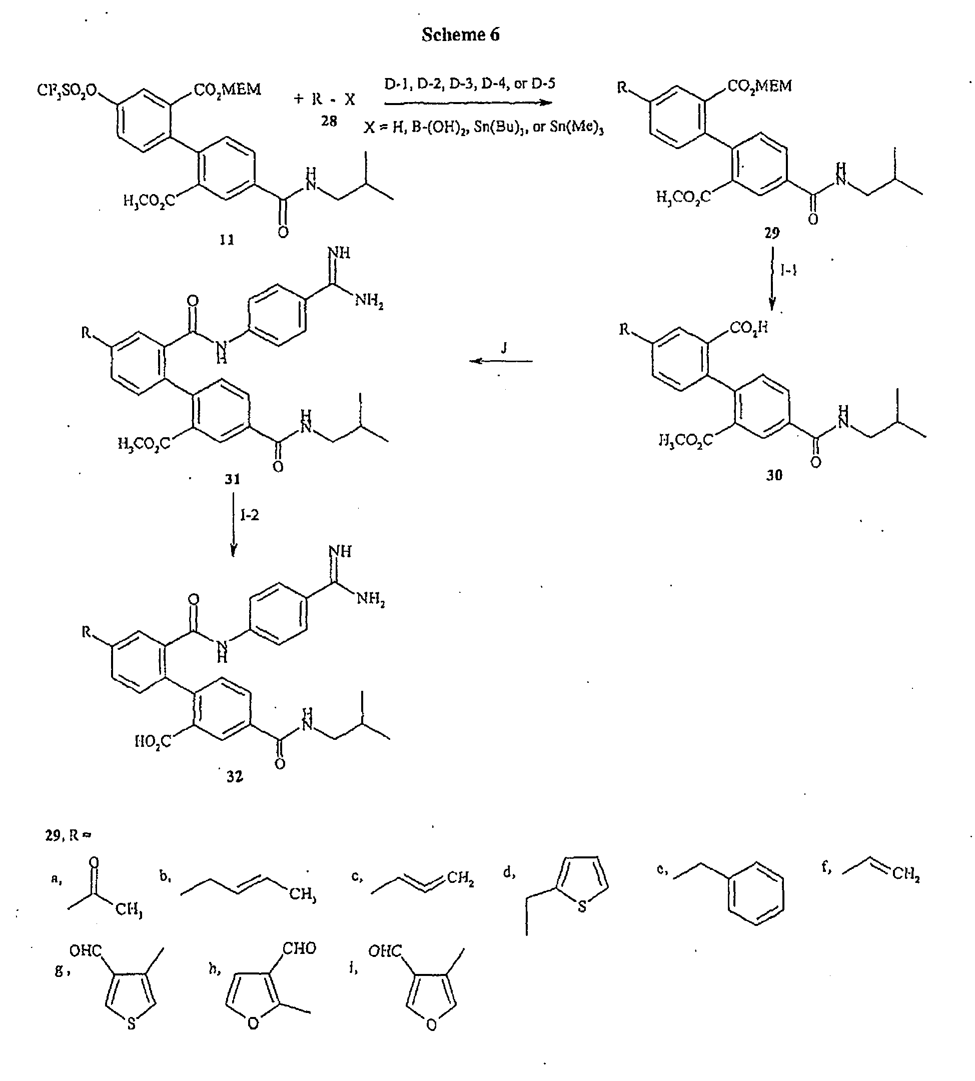 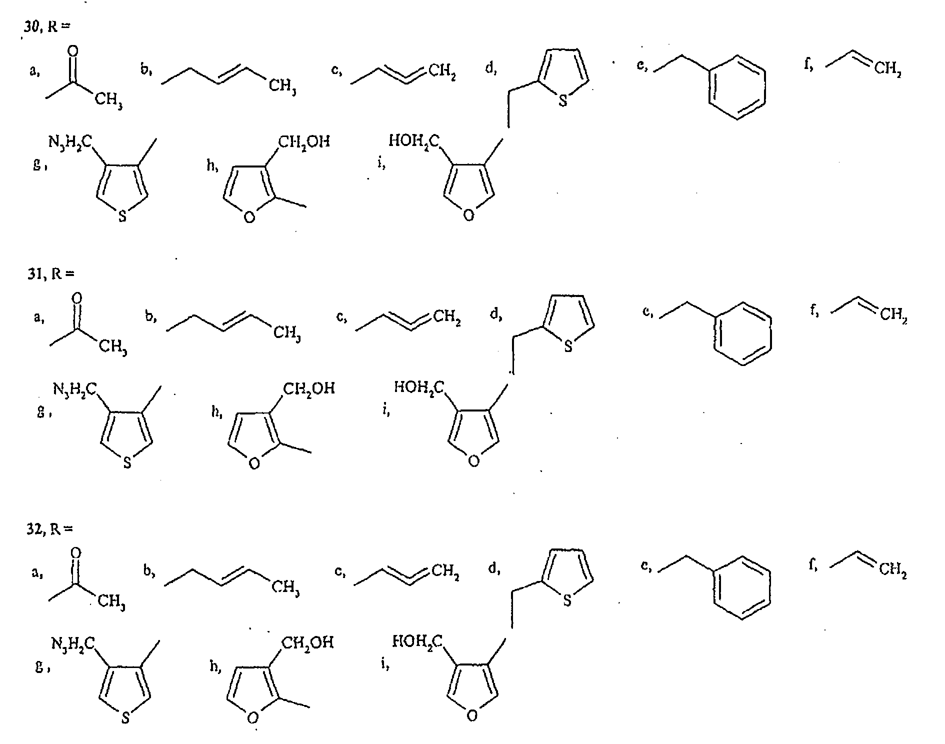 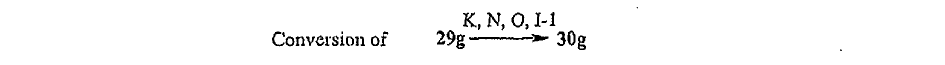 Aldehyde **29g** was converted to alcohol **29g-i** by reduction with NaBH₄, followed by the reaction of methanesulfonyl chloride to give mesylate **29g-ii.** The mesyl group was displaced with azide to give **29g-iii** and finally, the MEM group was removed under acidic conditions to give **30g.**

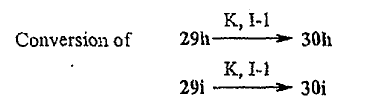

The reduction of the formyl group of **29h** and **29i** was accomplished with NaBH₄ to give corresponding alcohols **29h-i** and **29i-i,** respectively. Later, the MEM group was removed under acidic conditions to give **30h** and **30i,** respectively.

Compounds of the type **23** and **28,** where X = -Sn(Bu)₃, are prepared using the methods AG-1 or AG-2

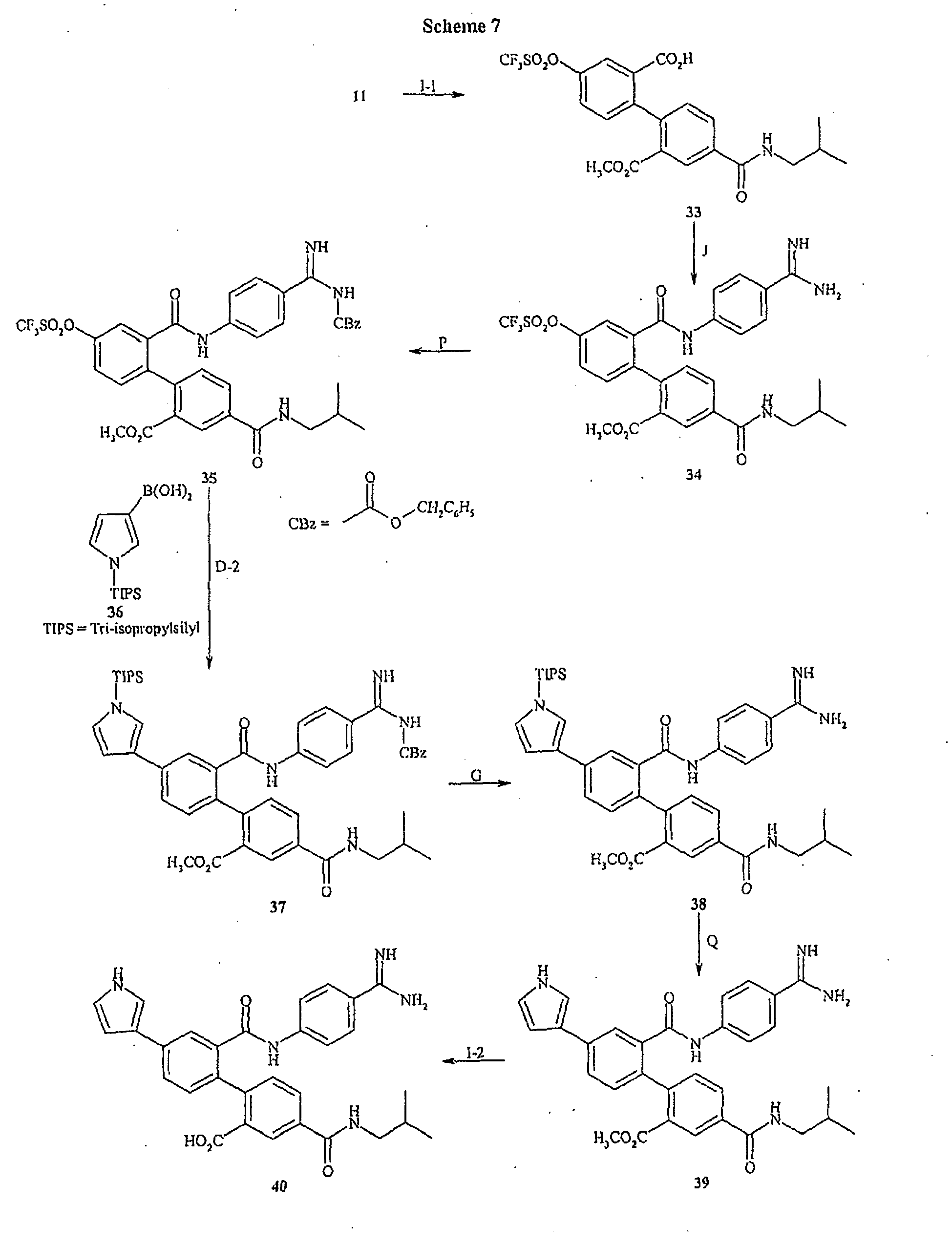

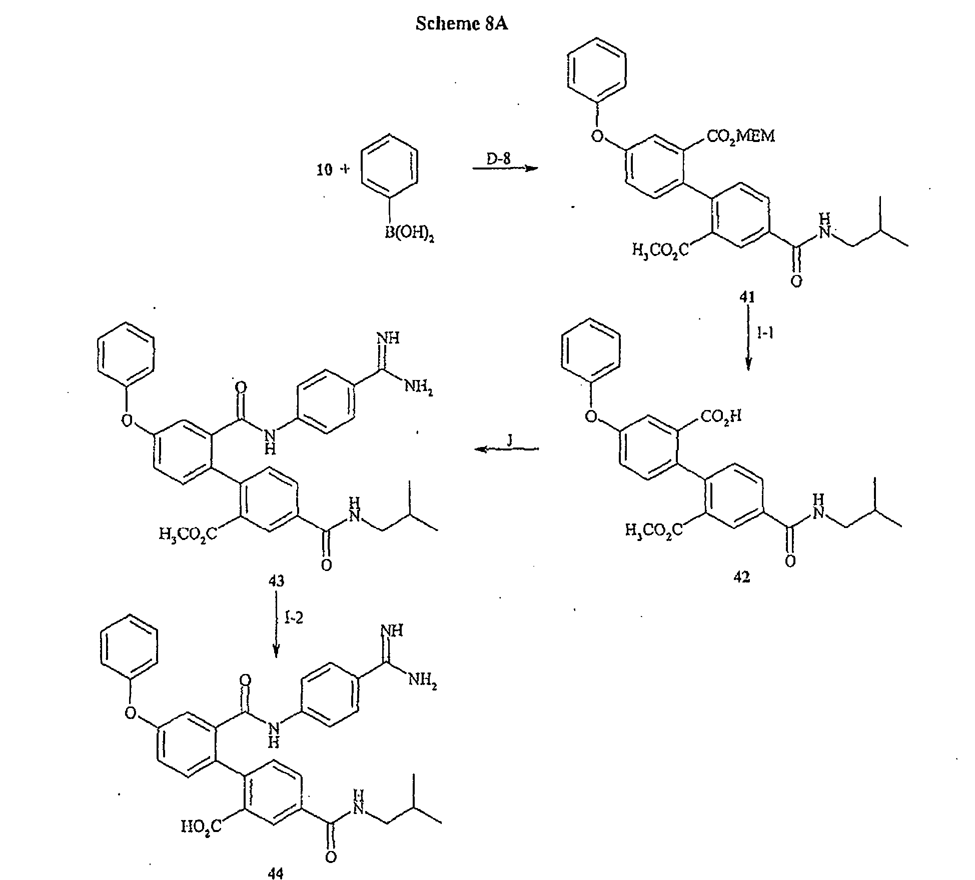

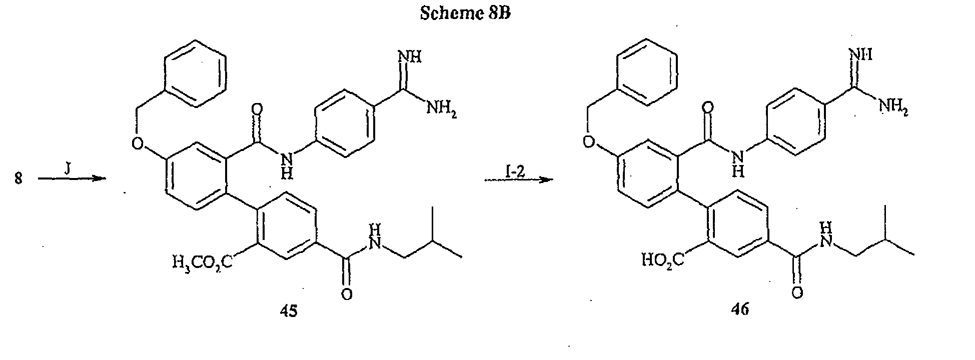

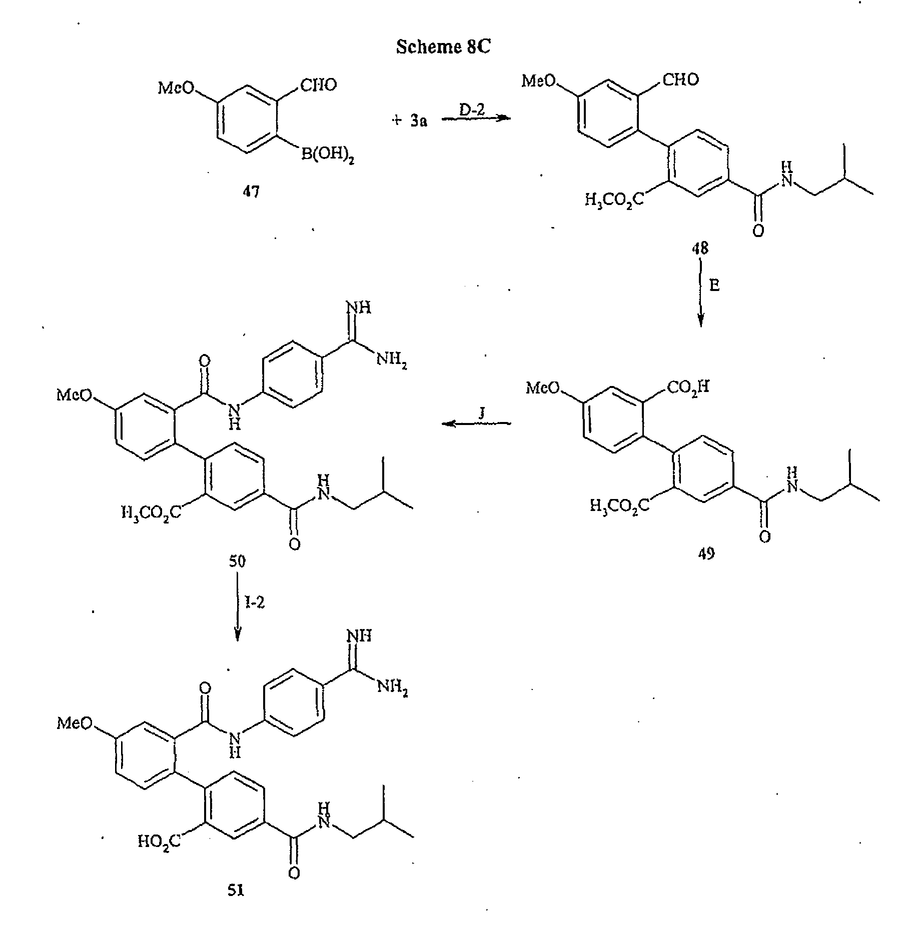

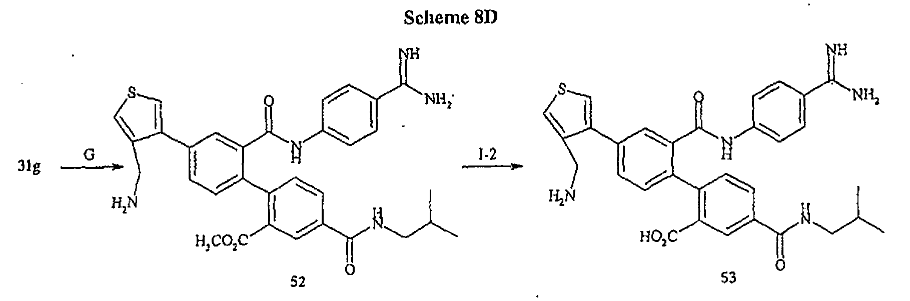

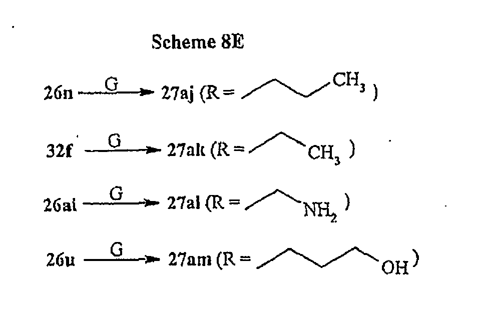

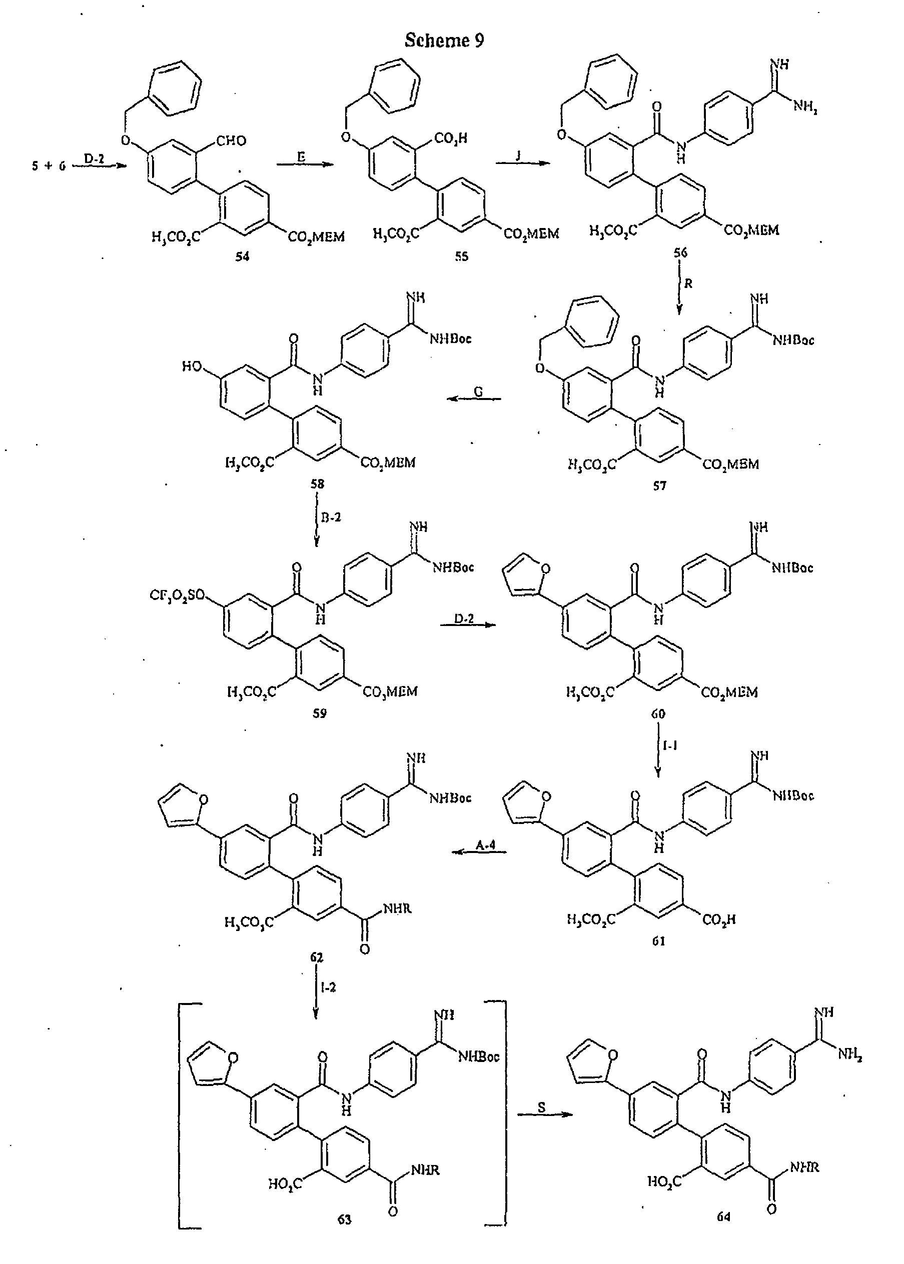

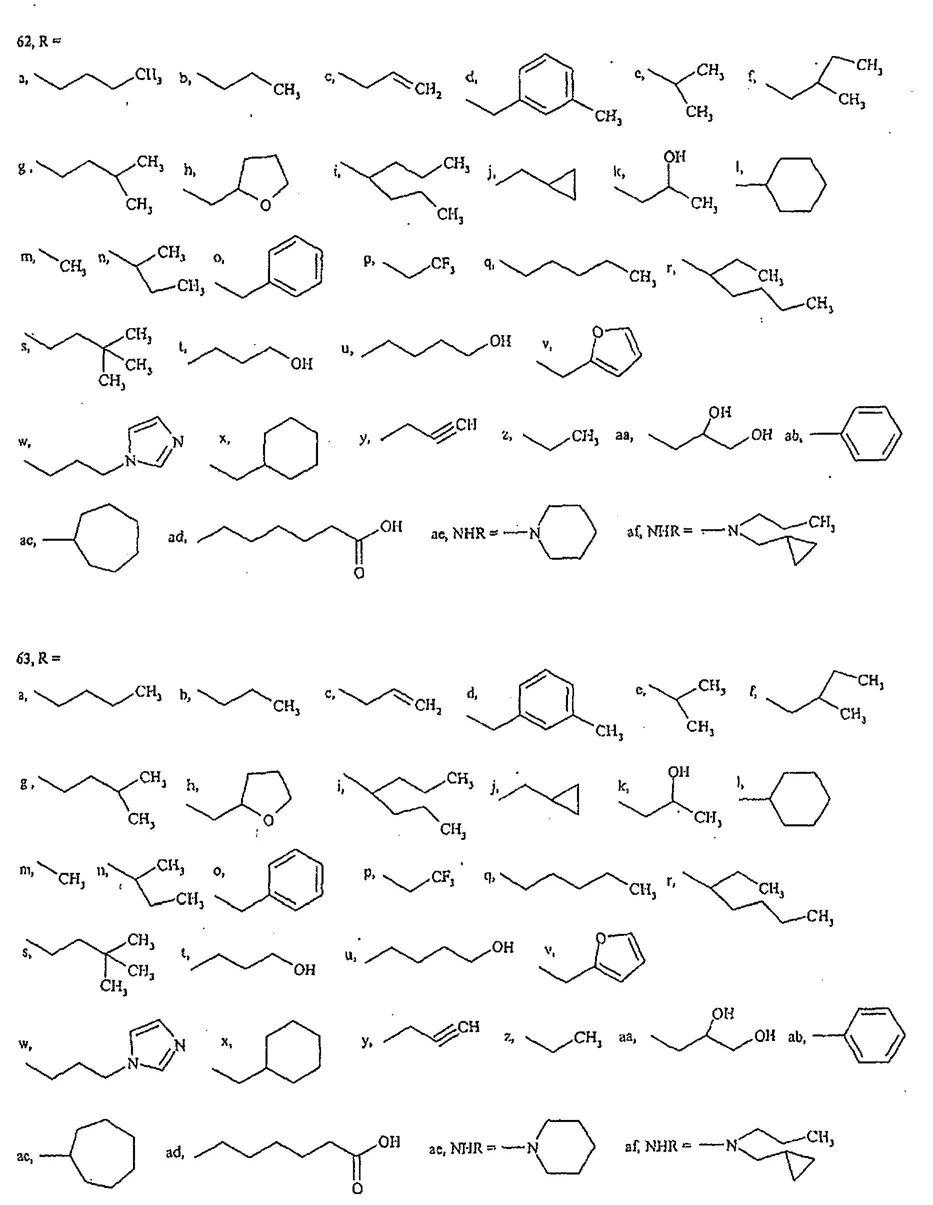

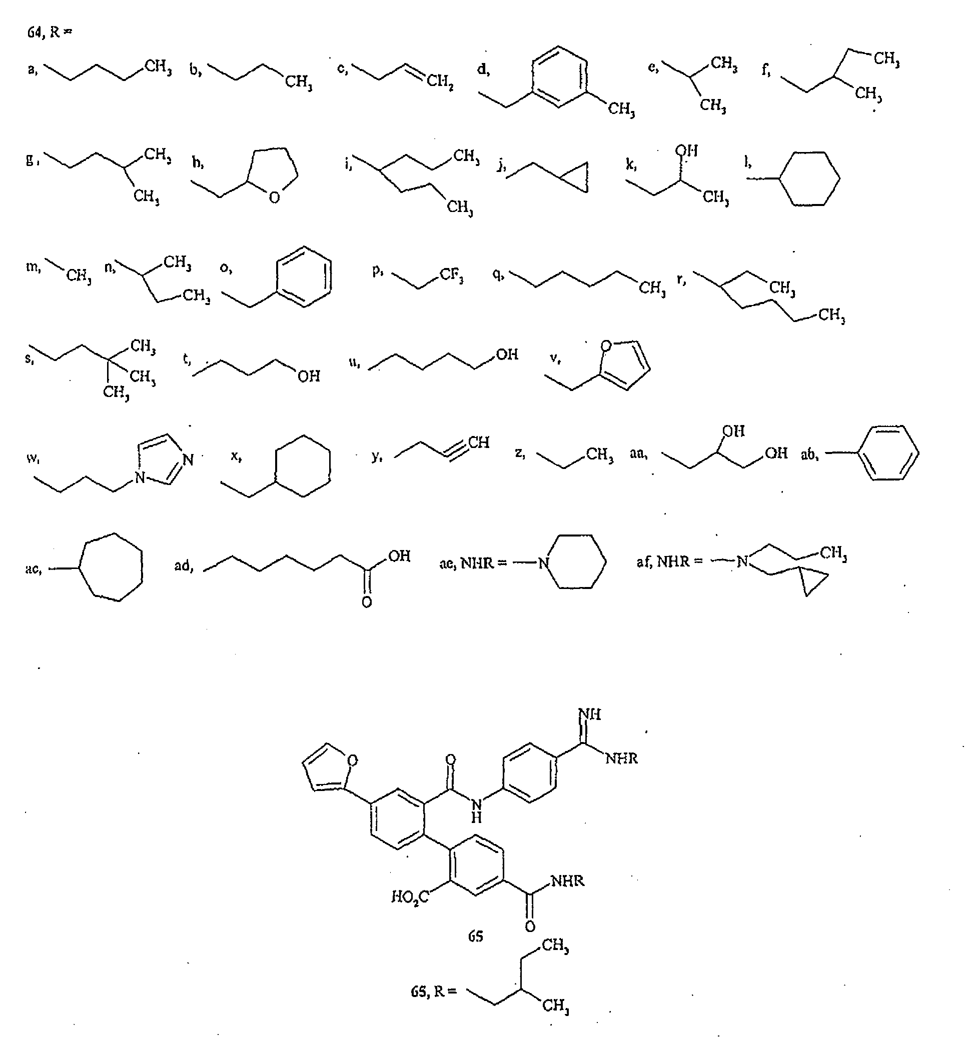

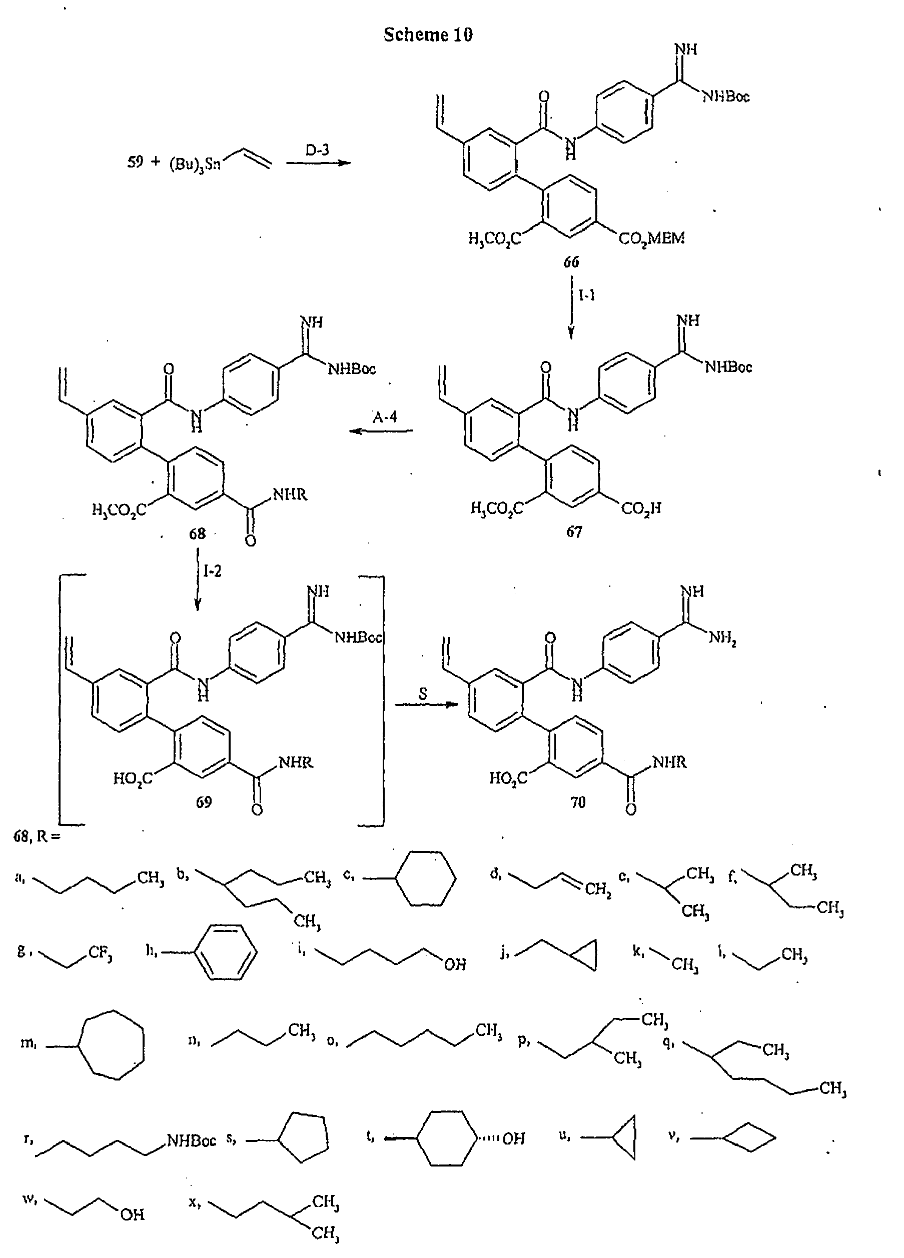

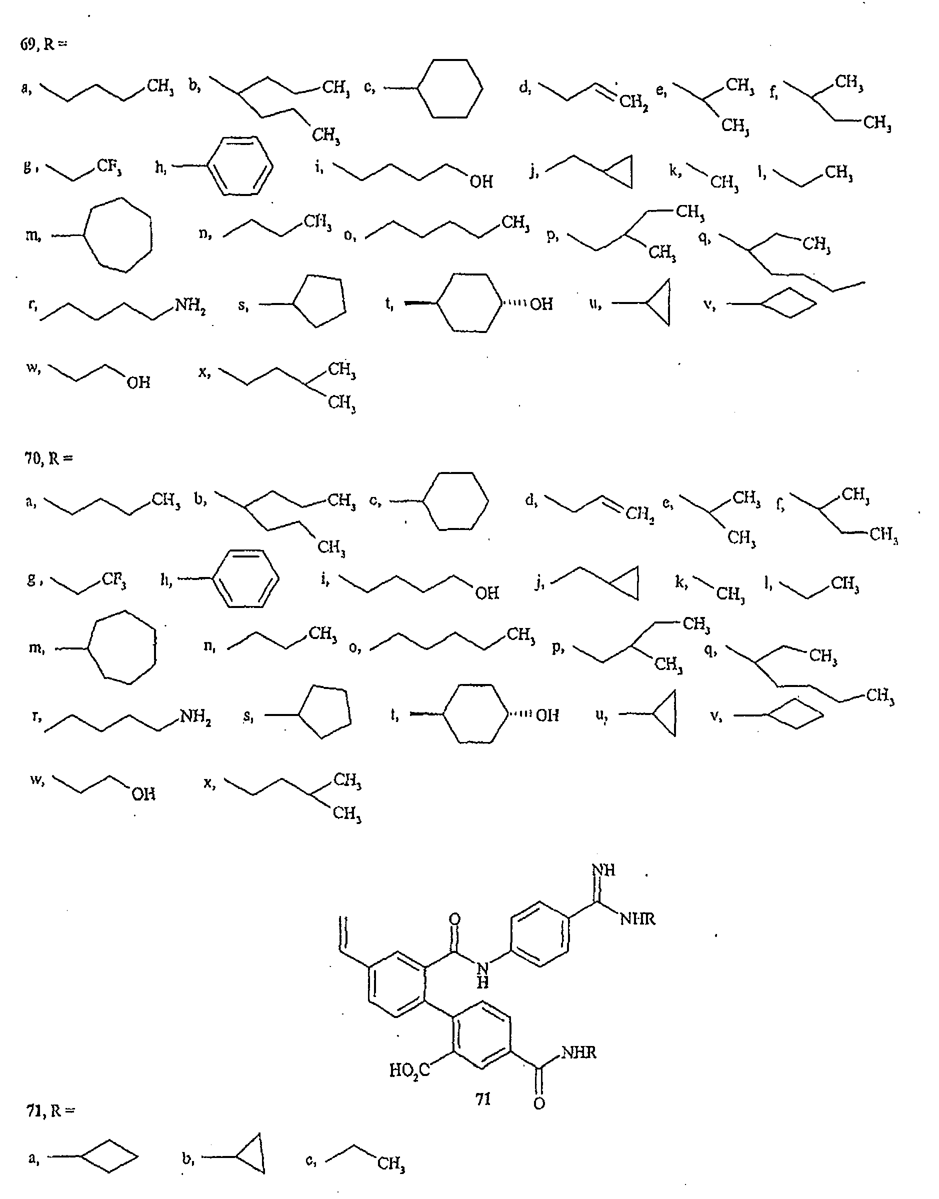

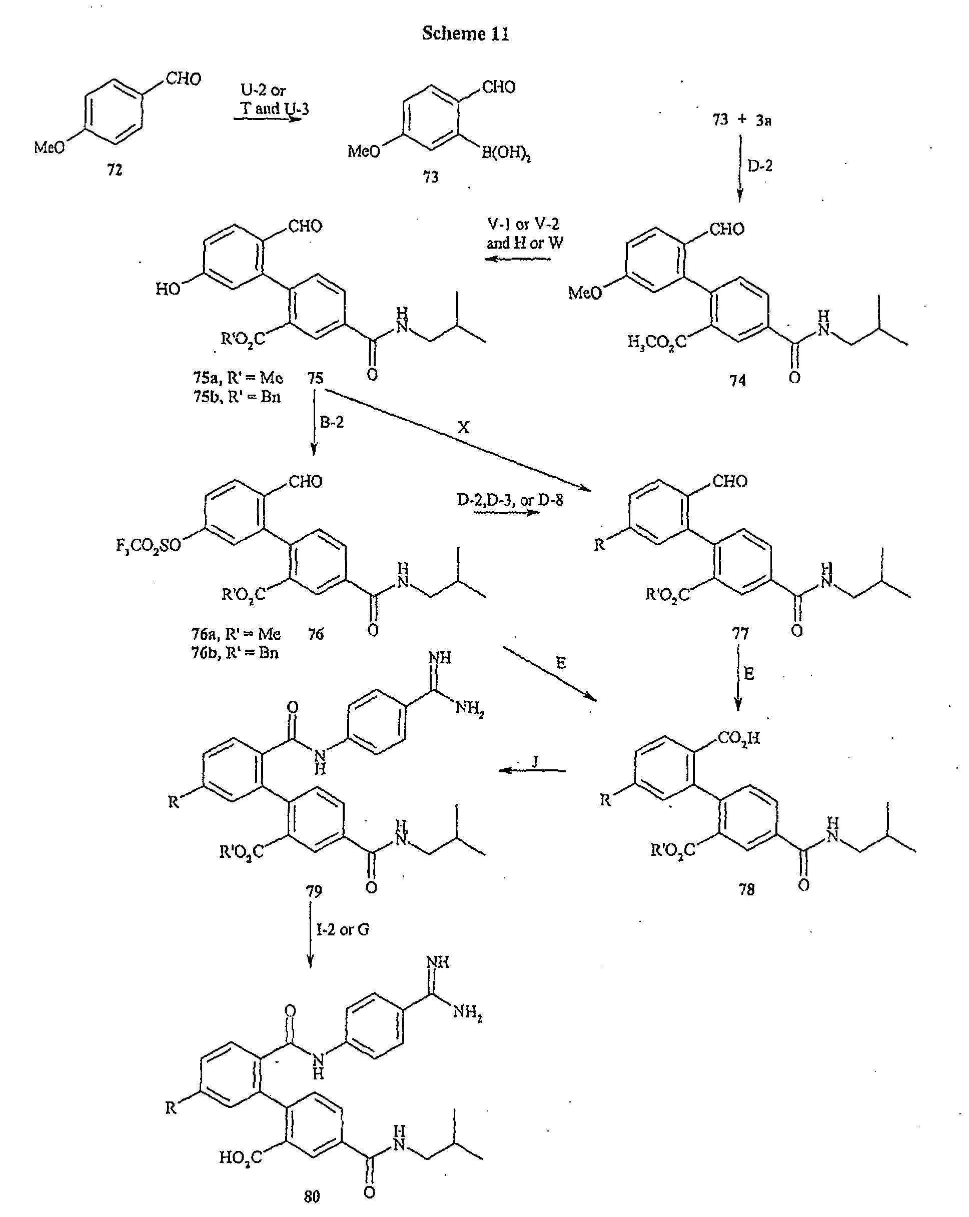

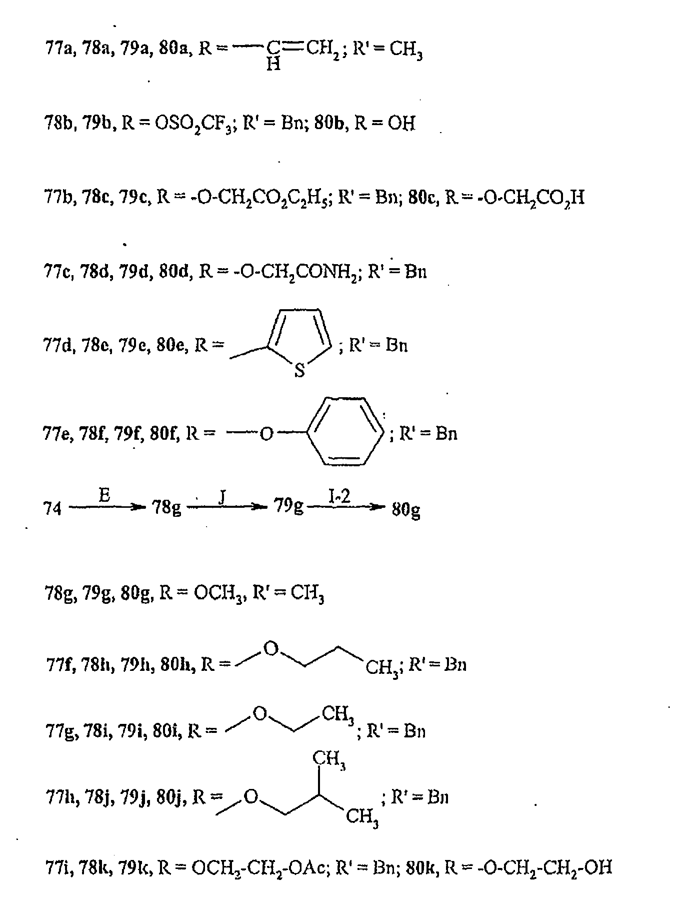

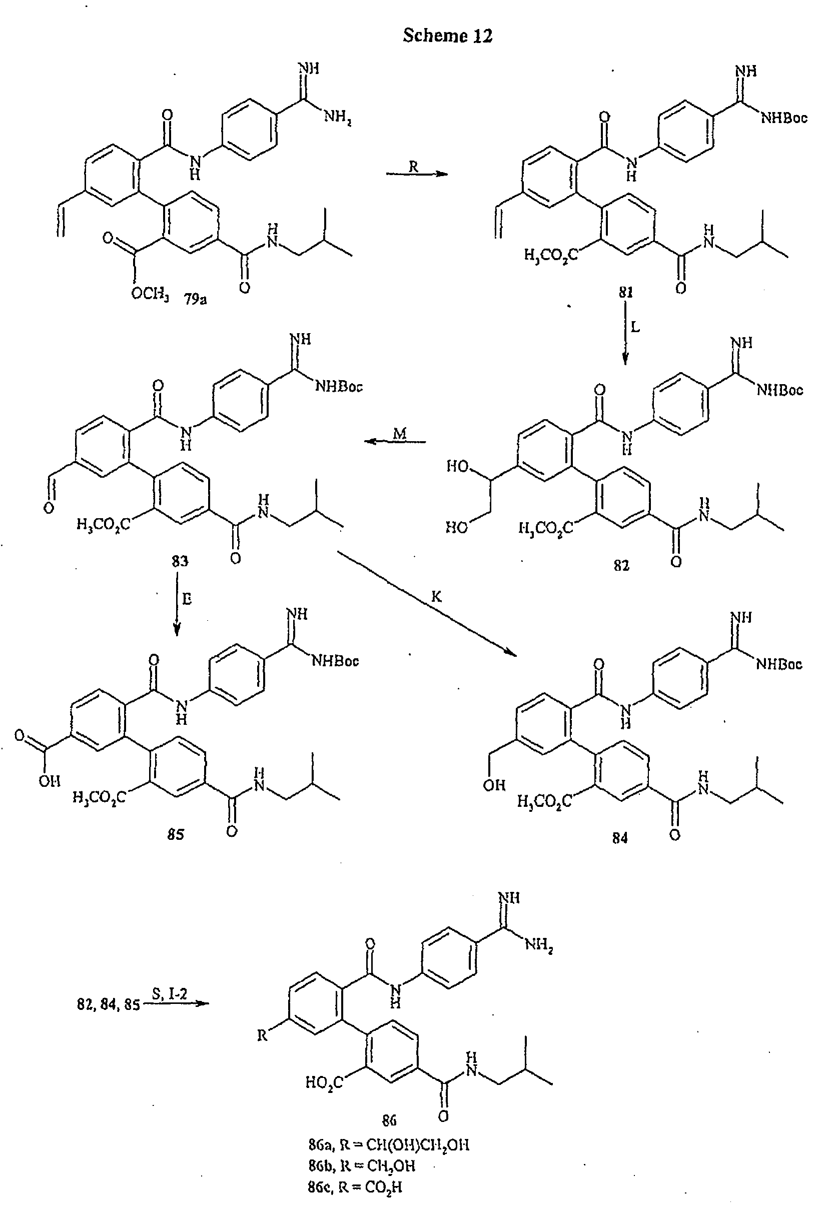

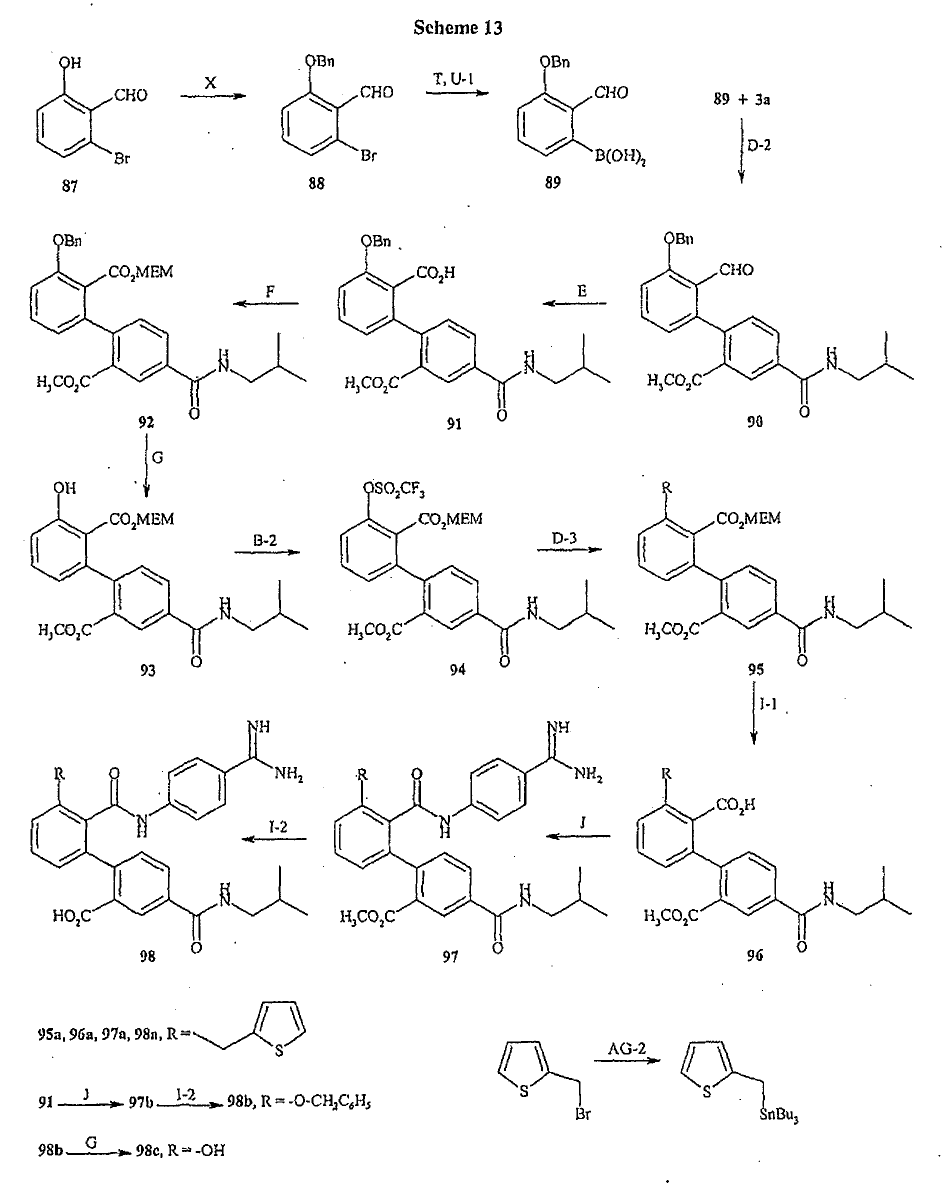

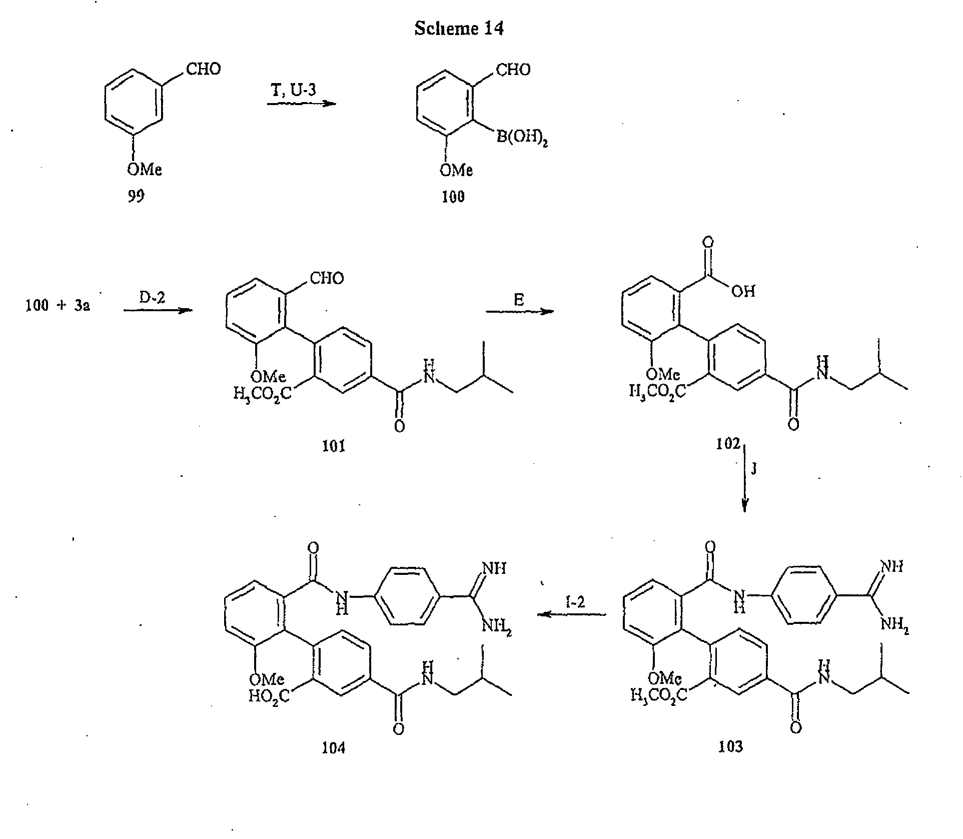

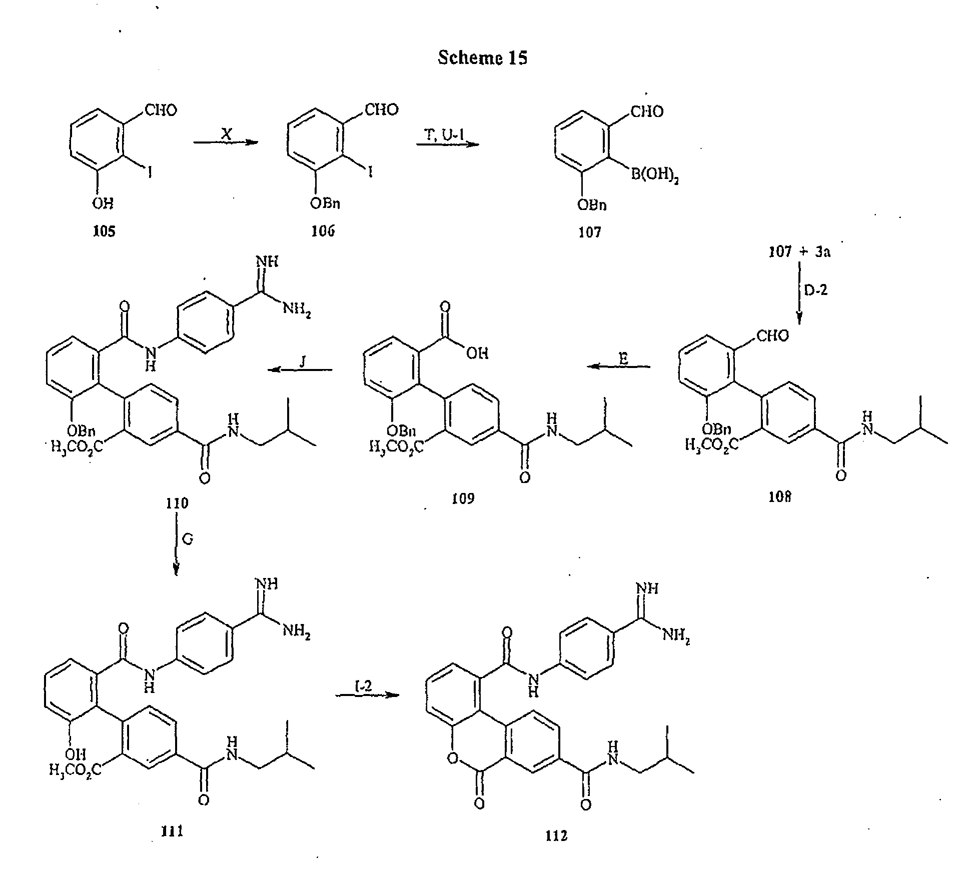

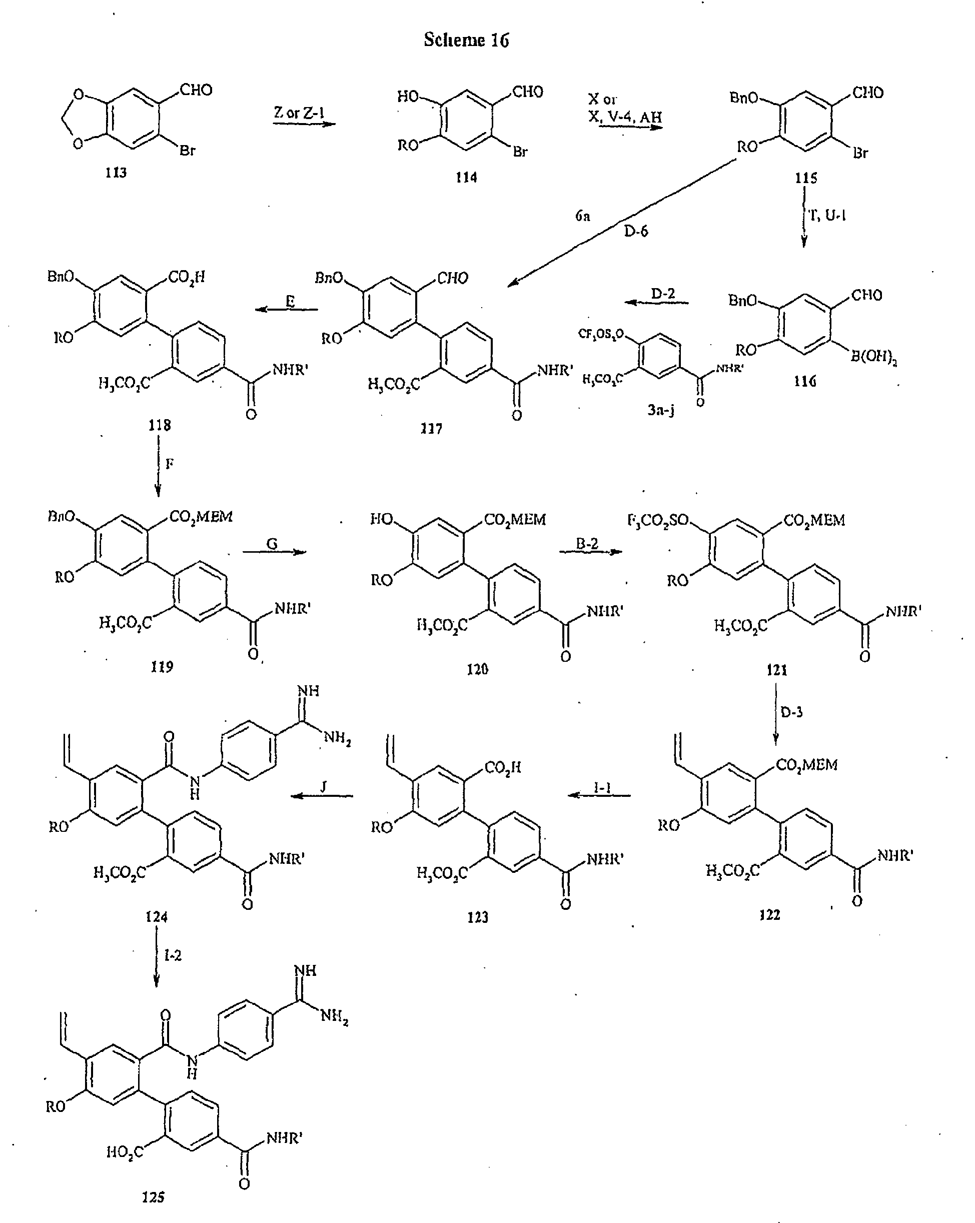

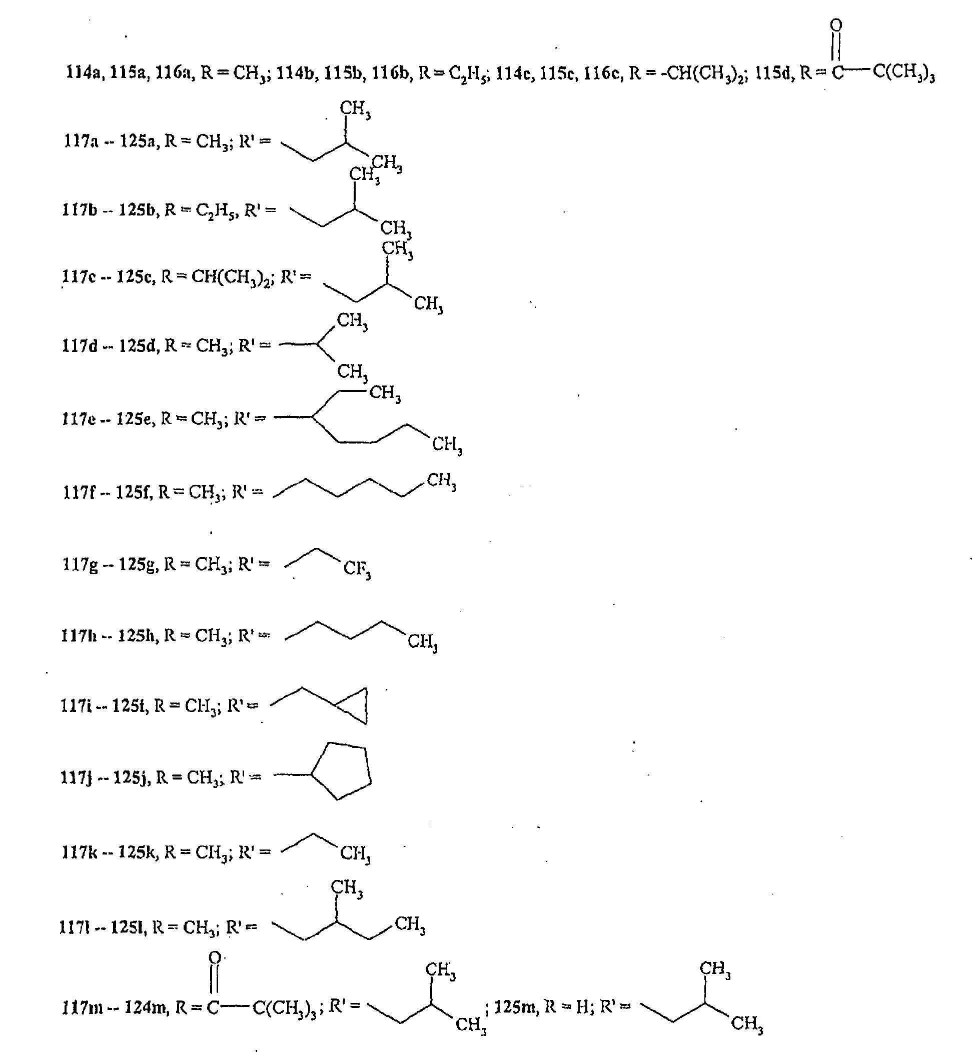

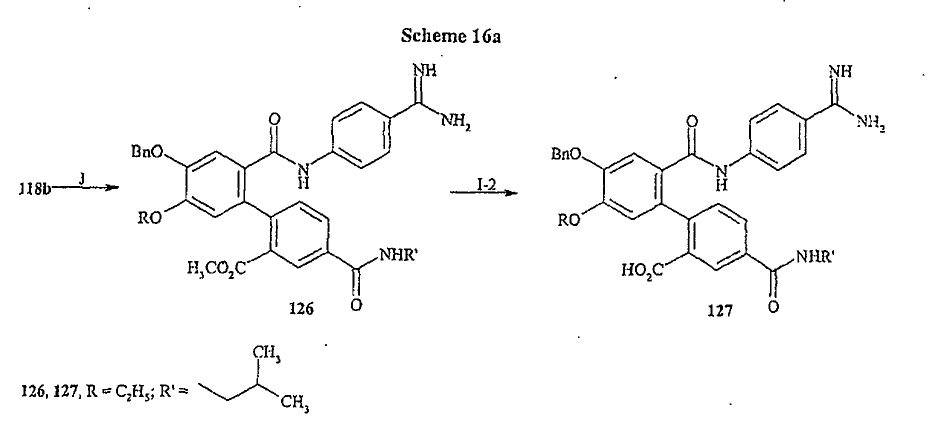

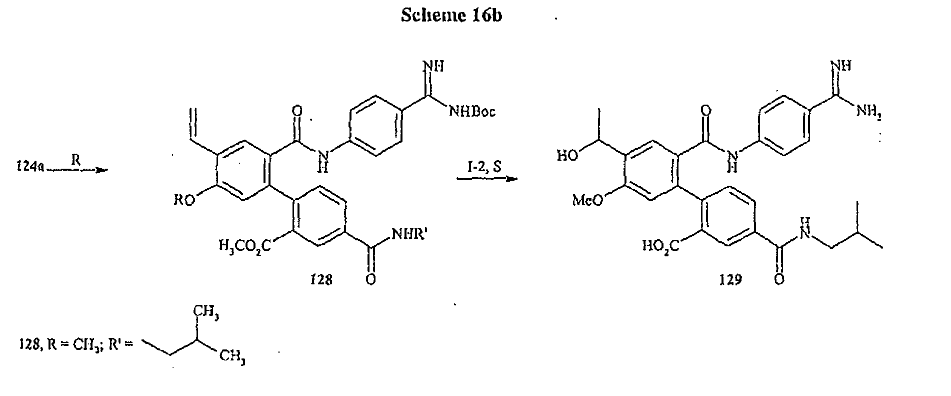

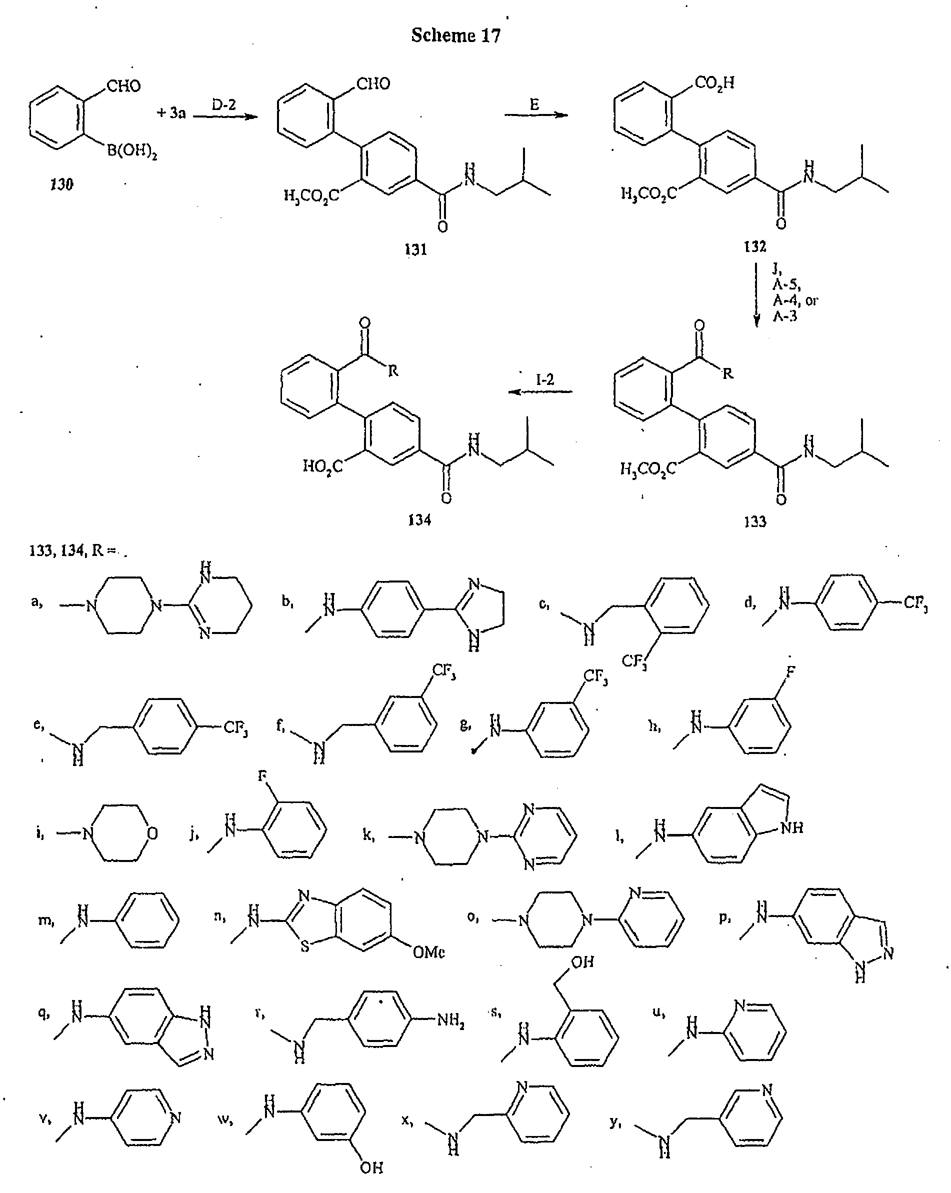

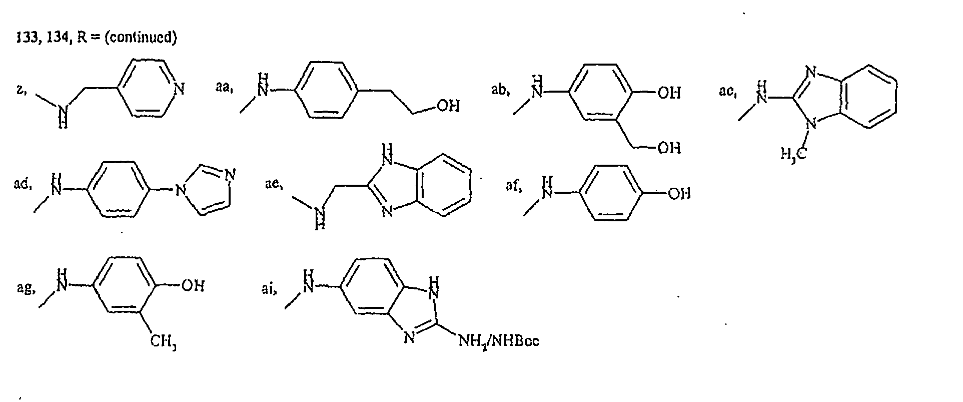

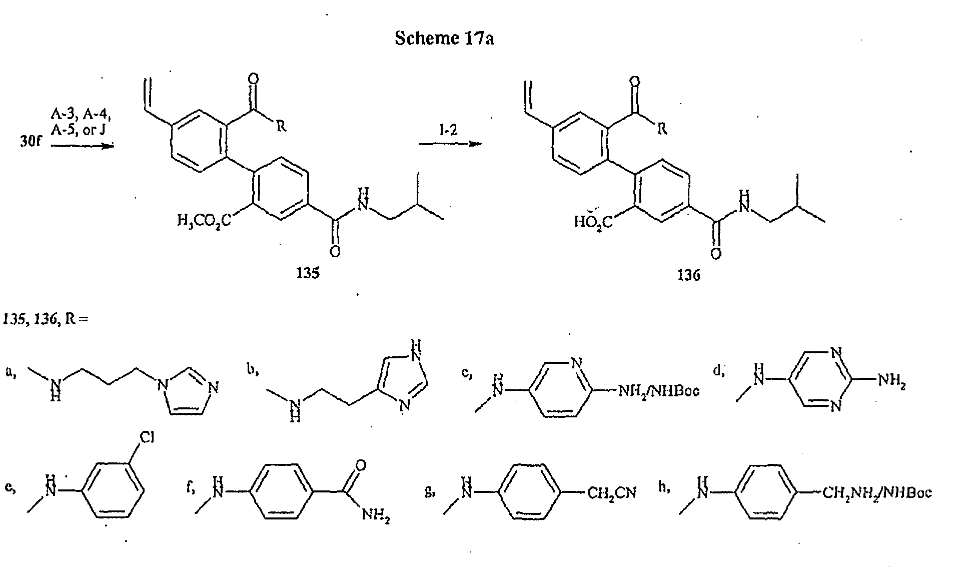

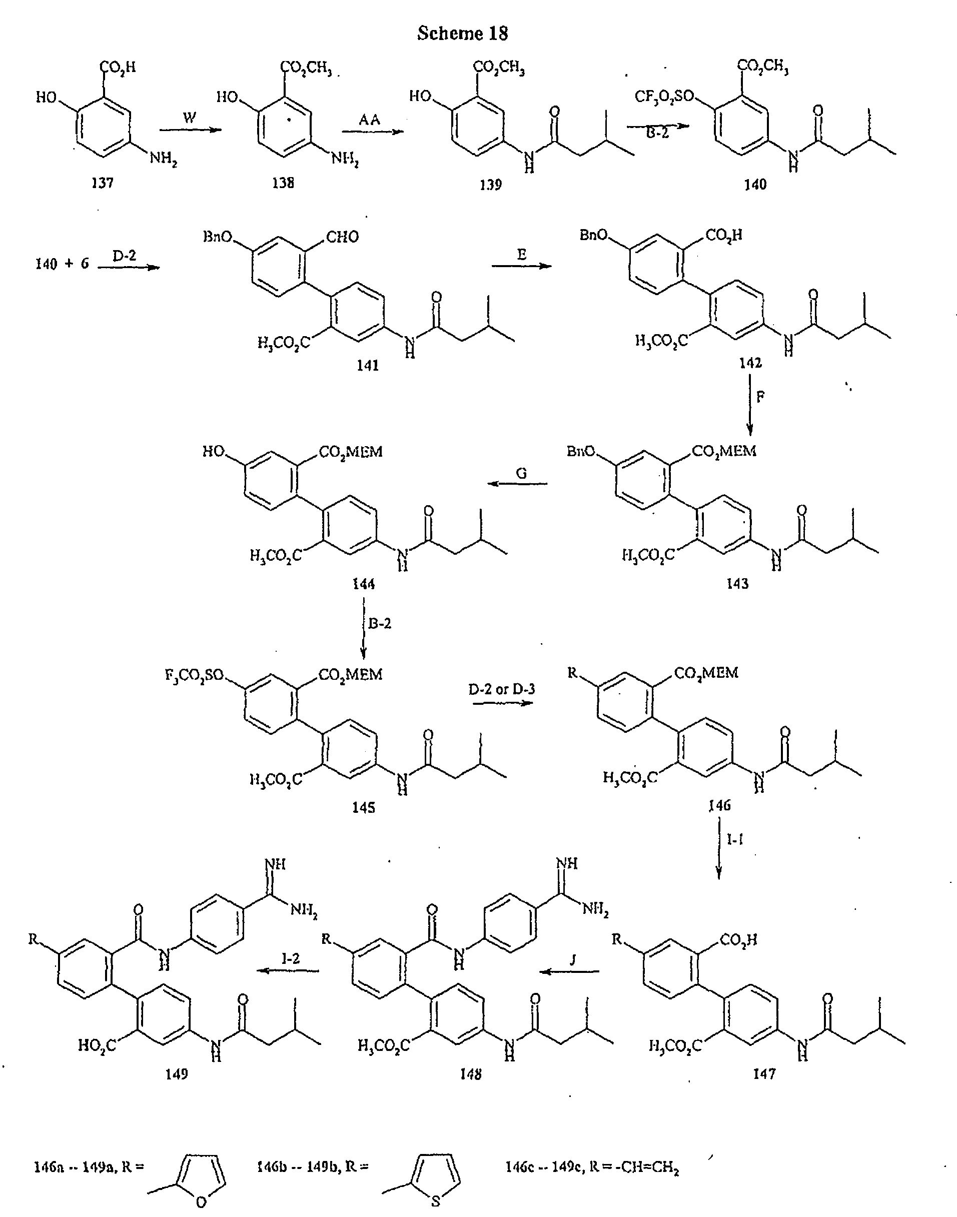

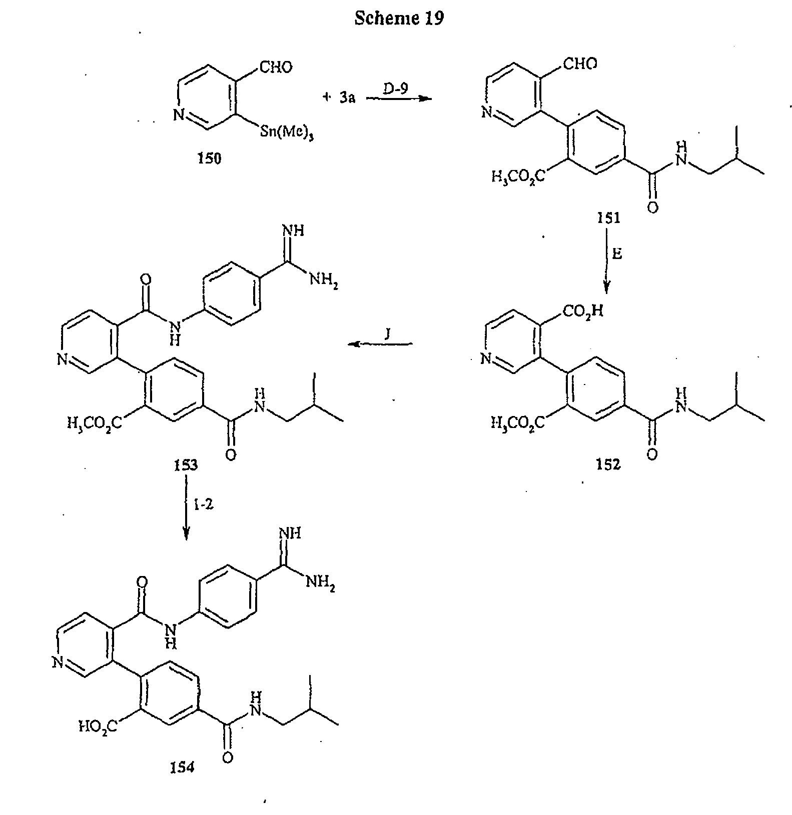

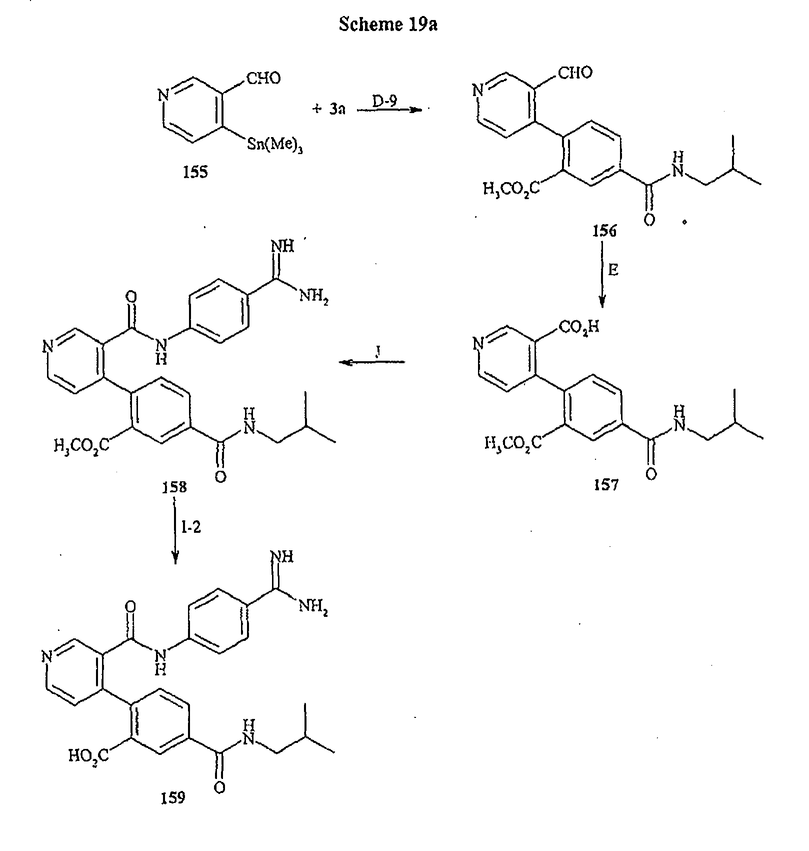

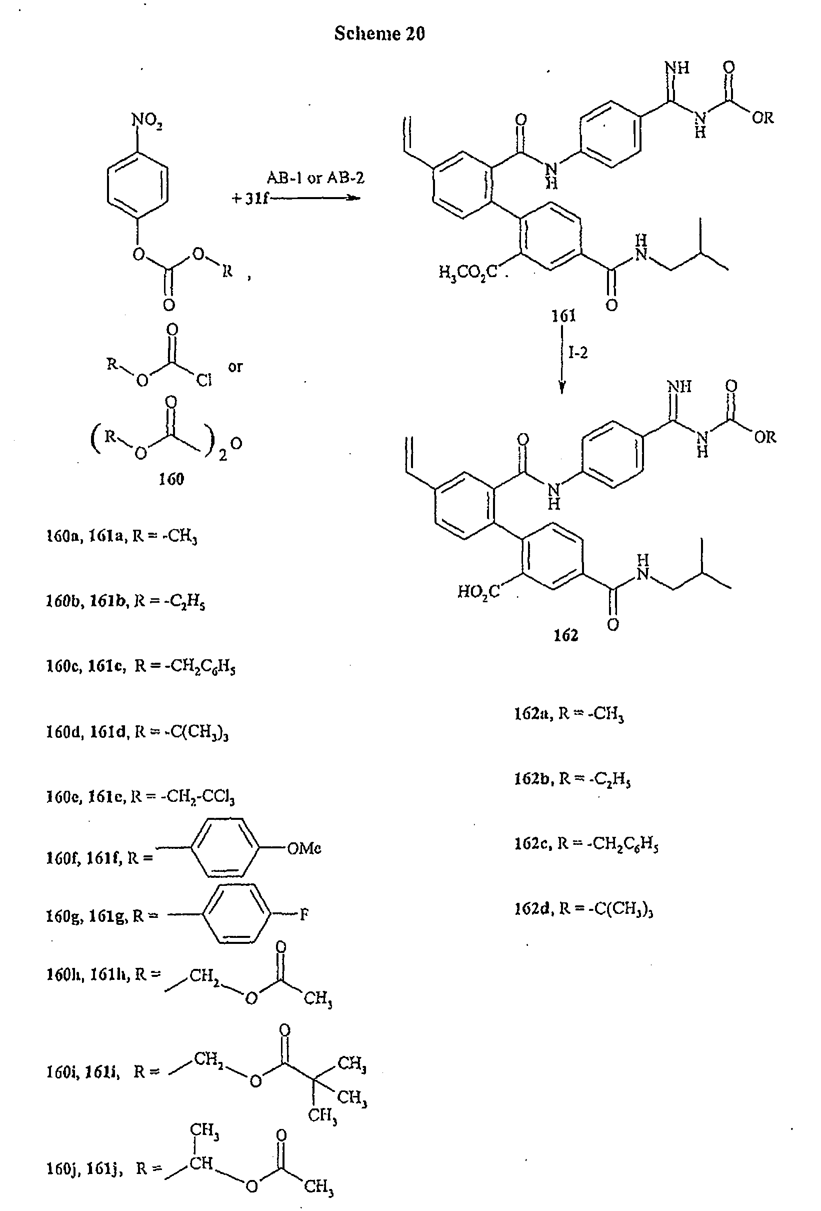

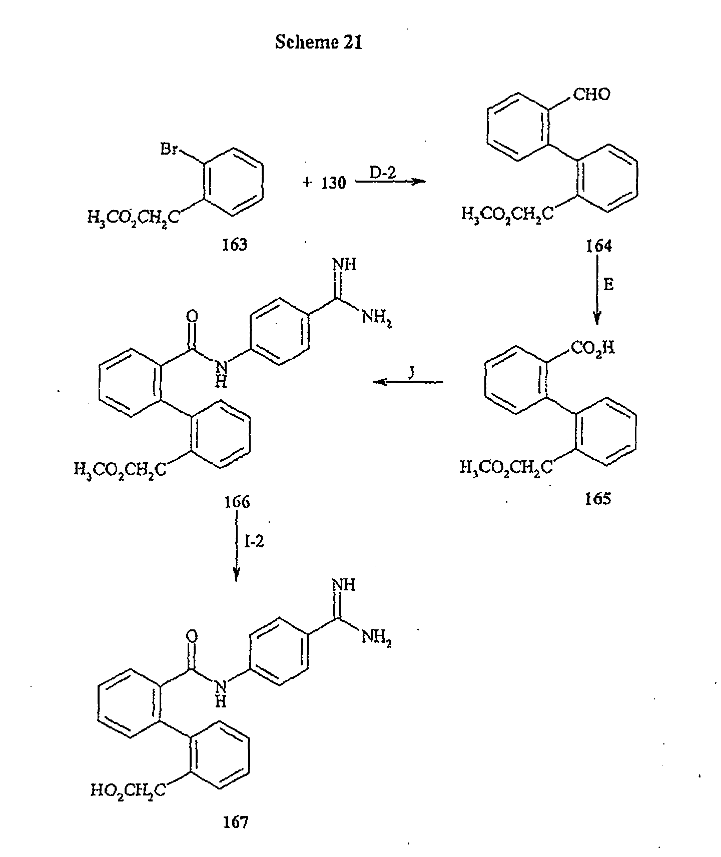

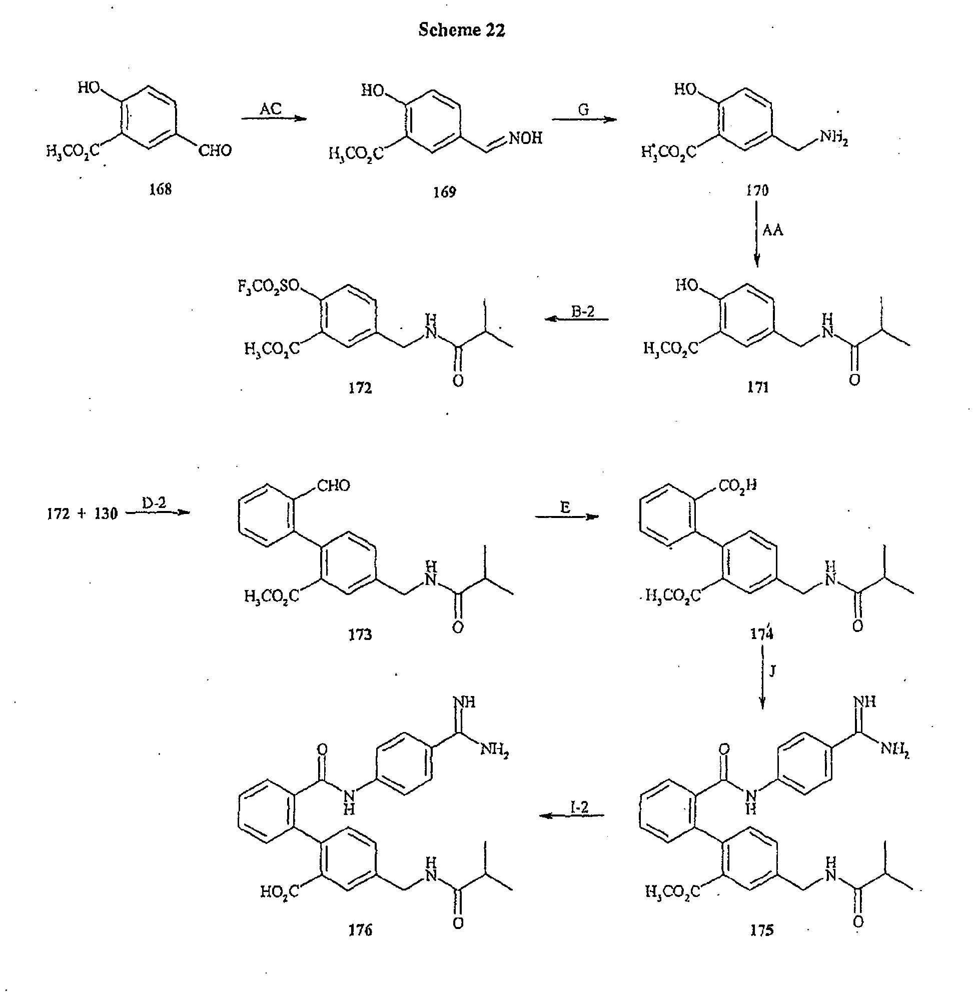

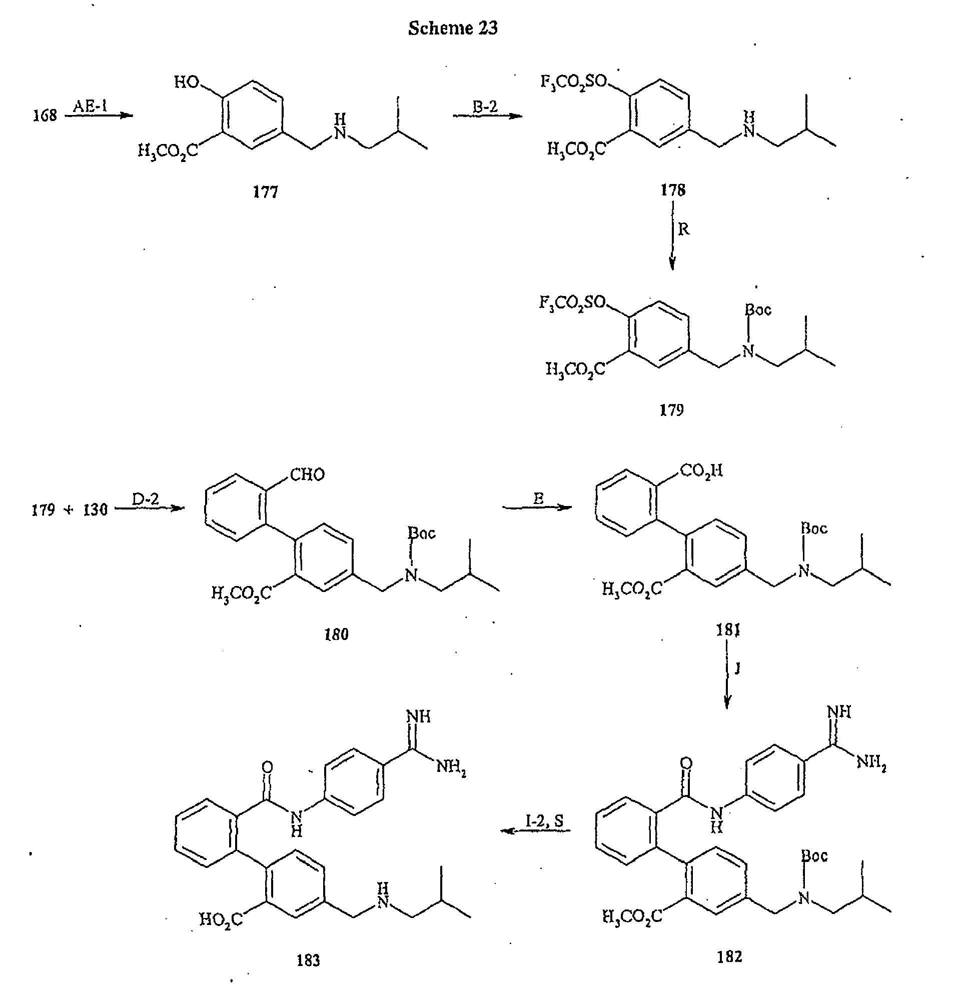

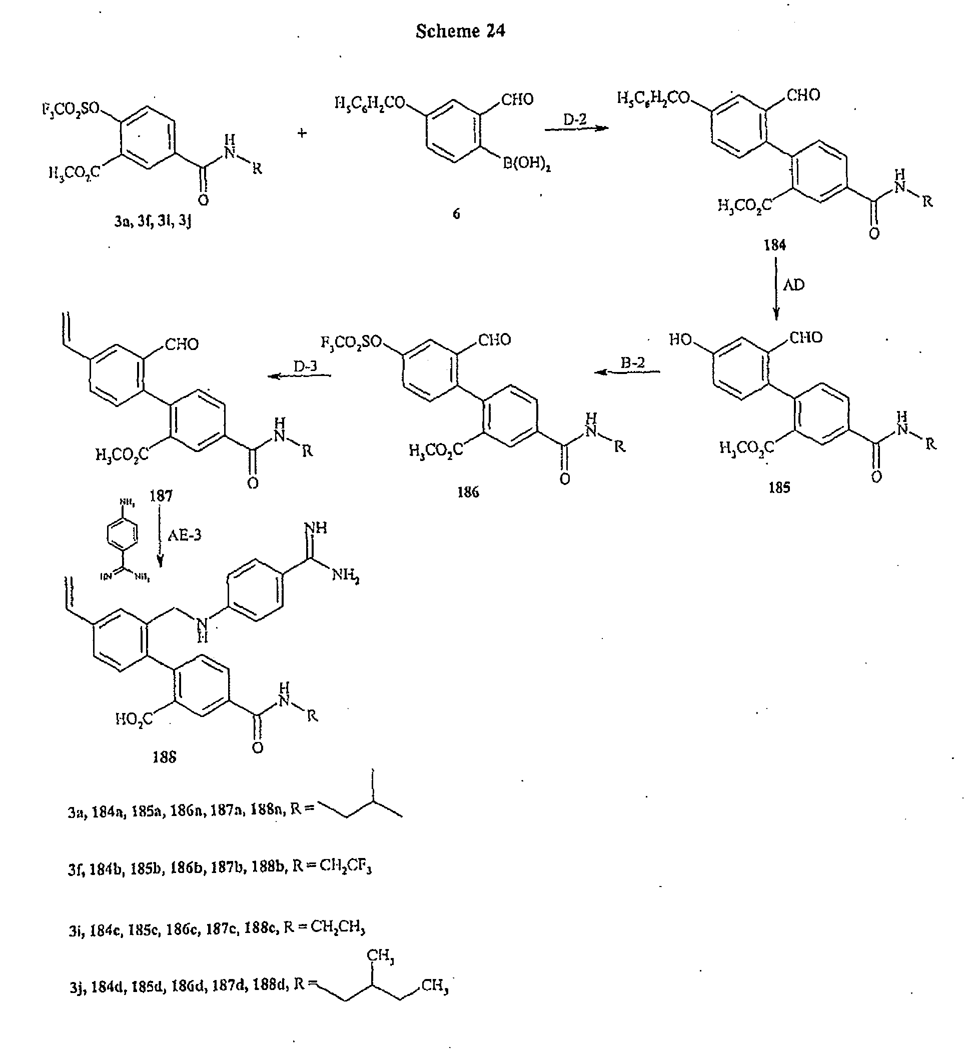

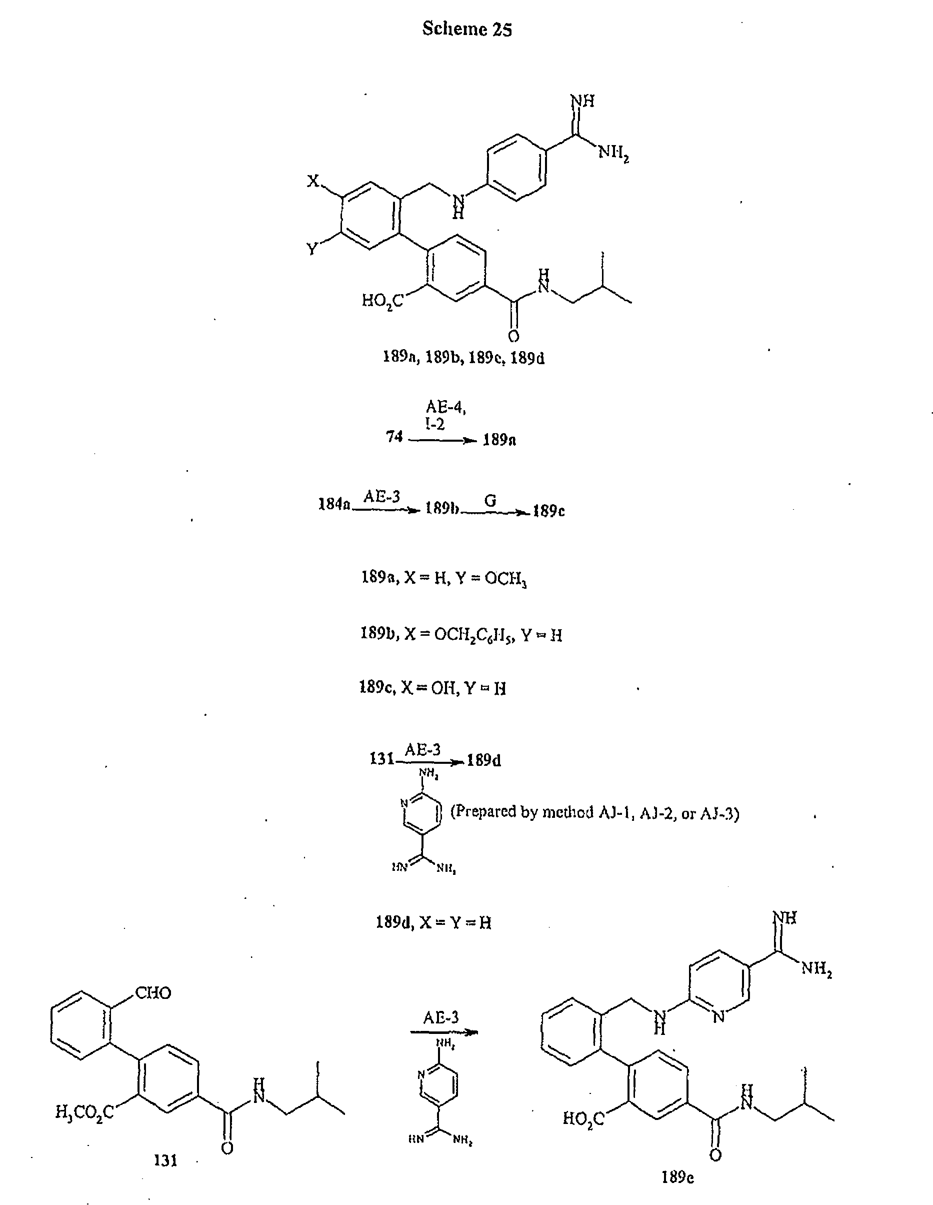

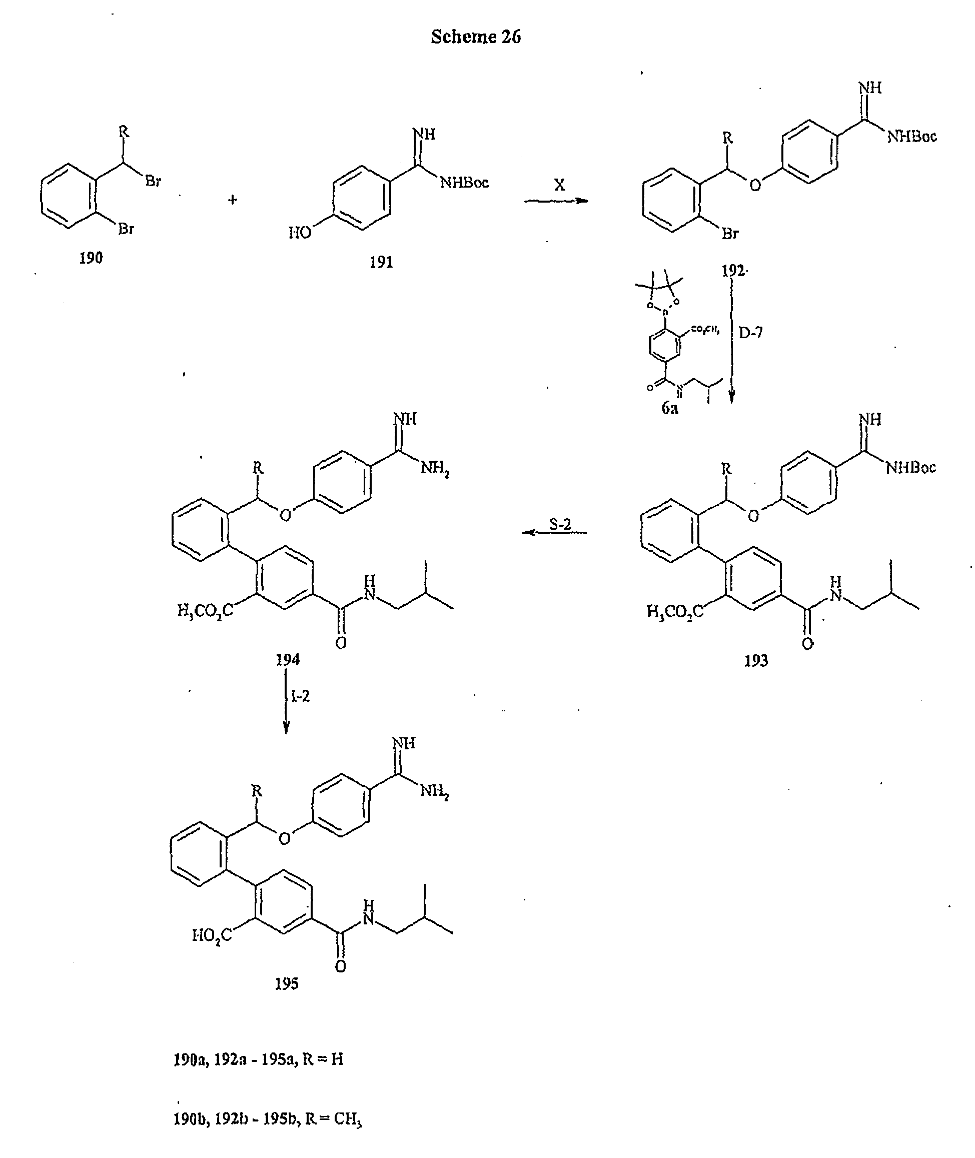

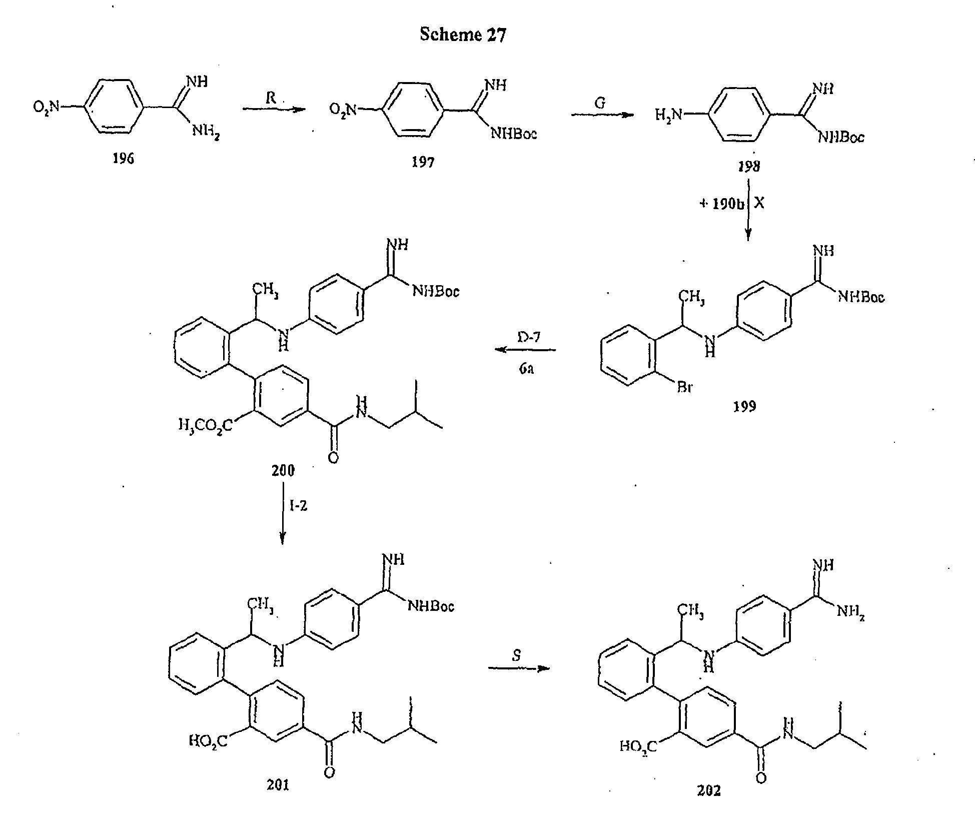

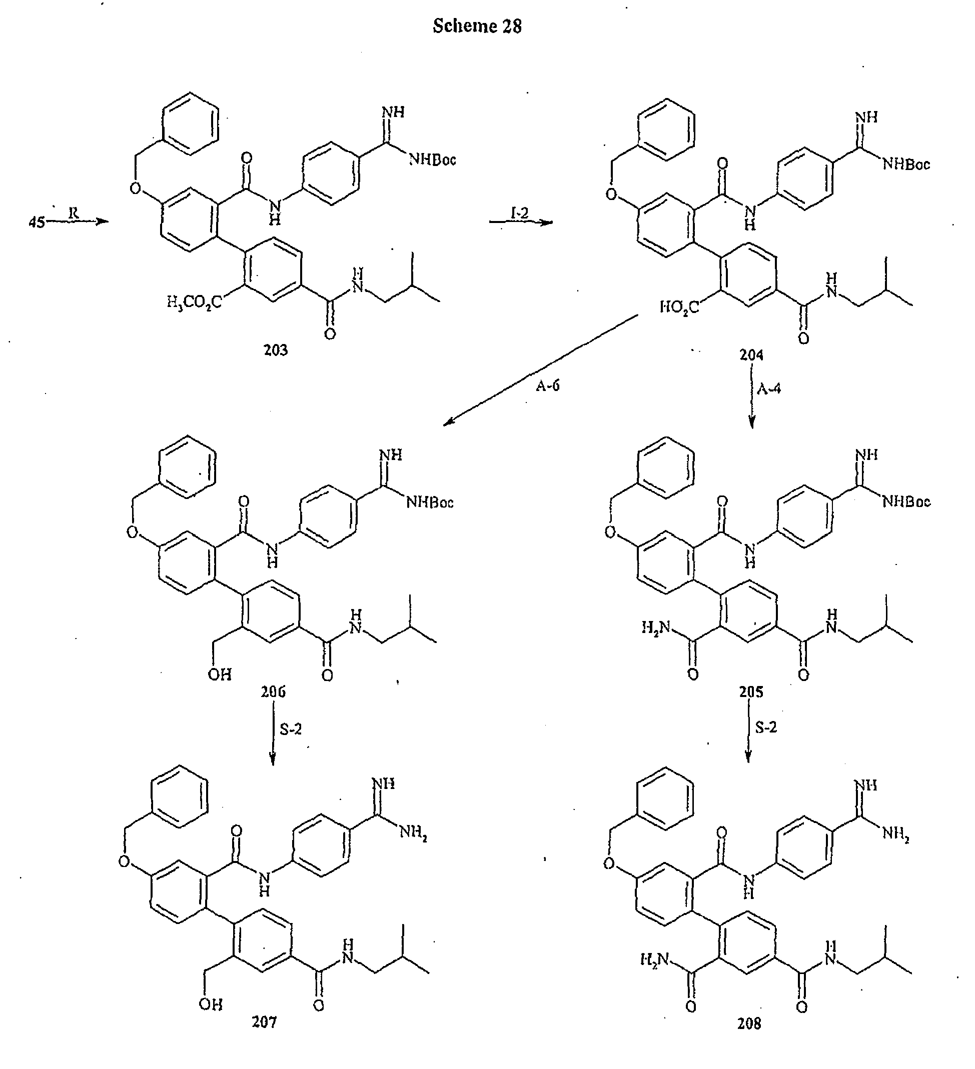

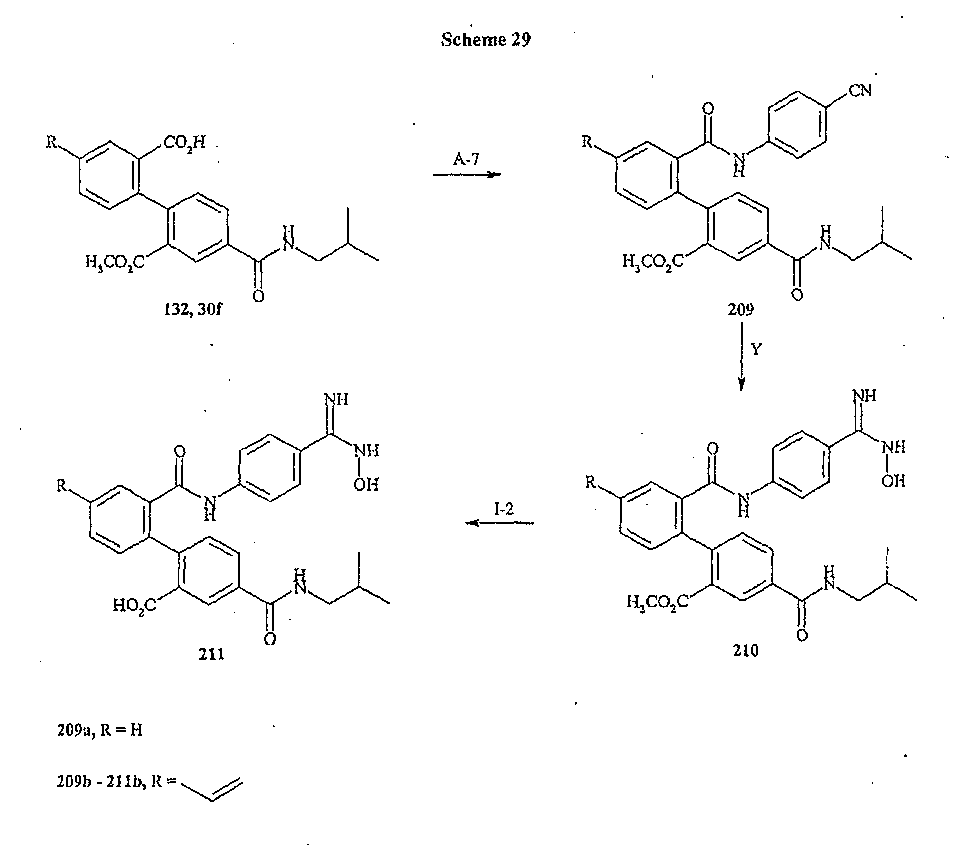

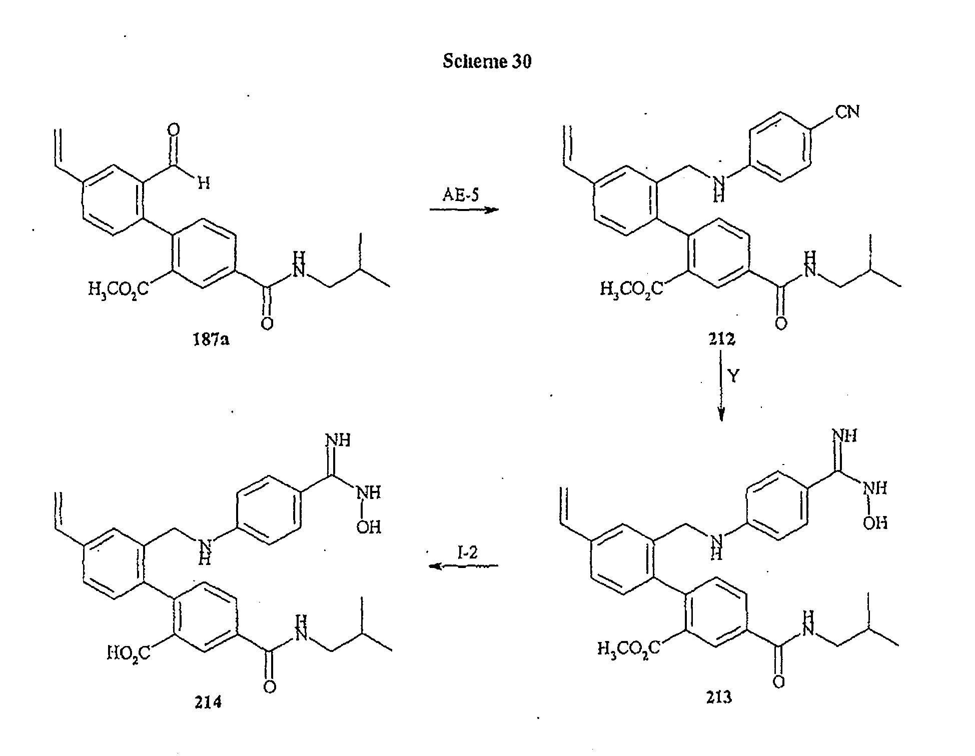

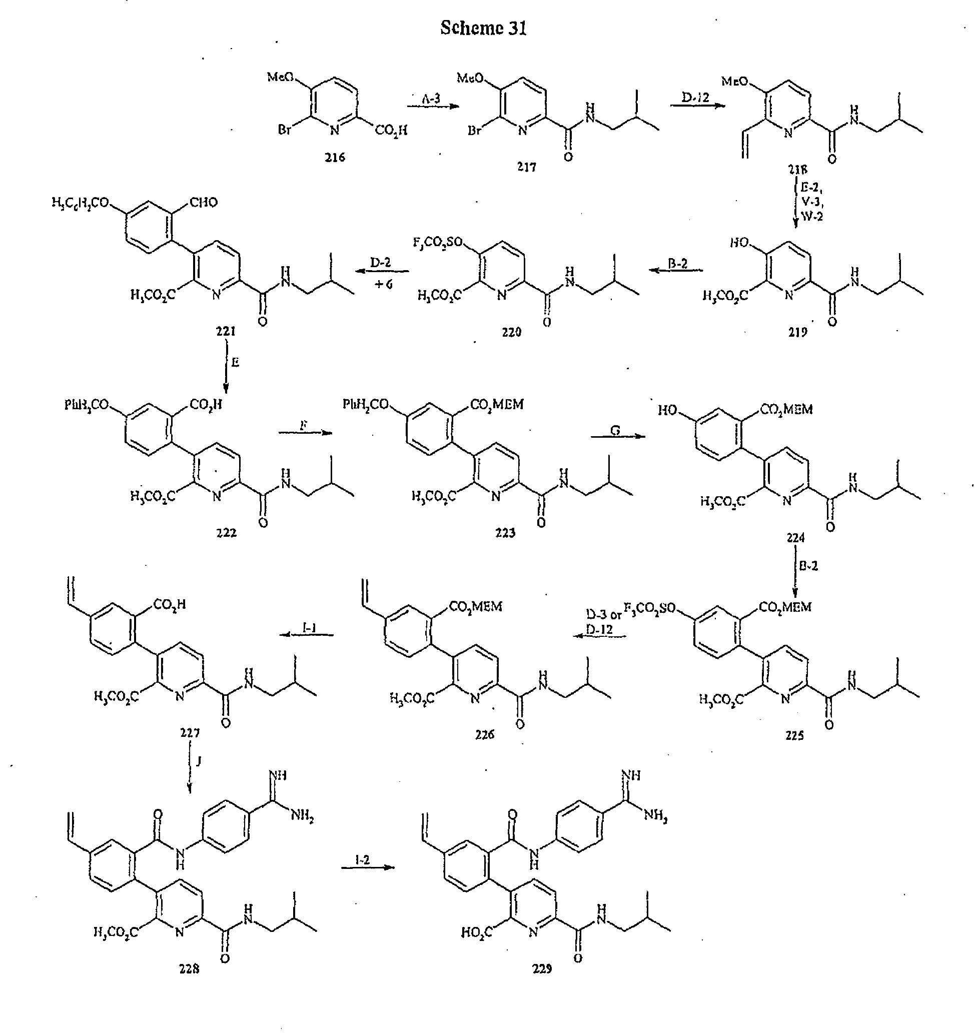

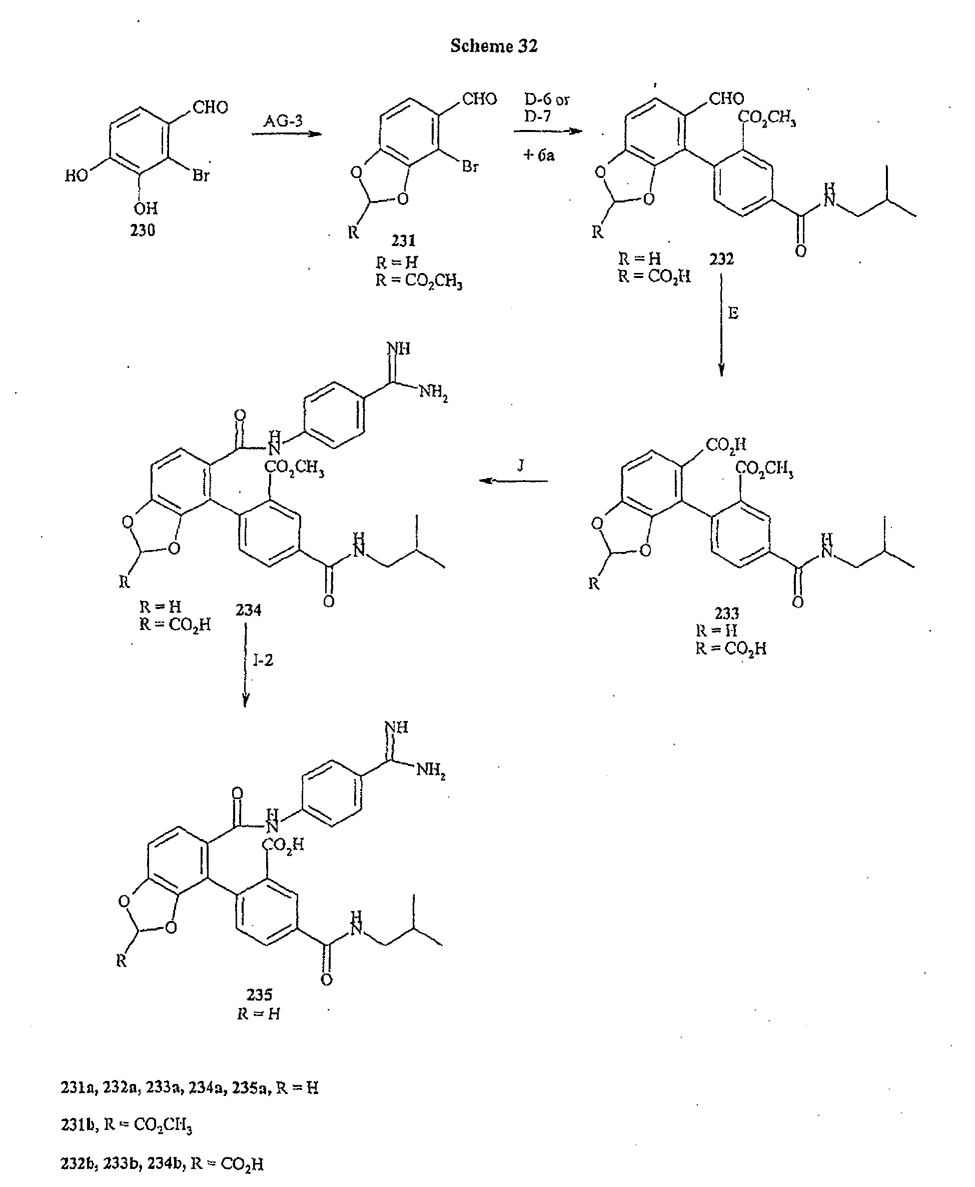

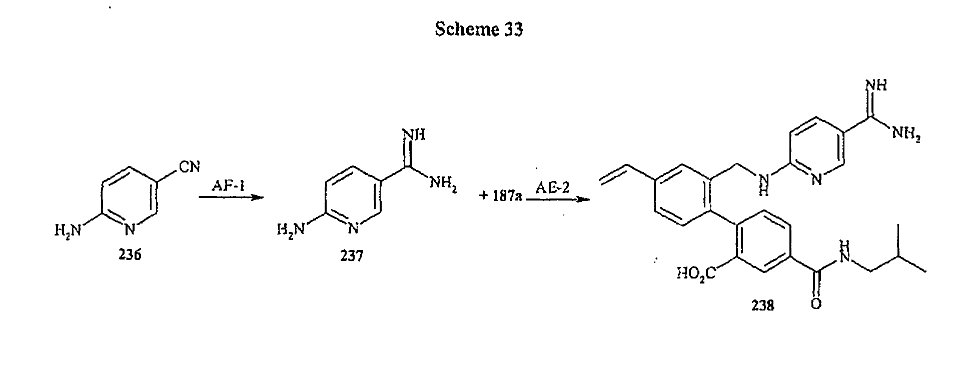

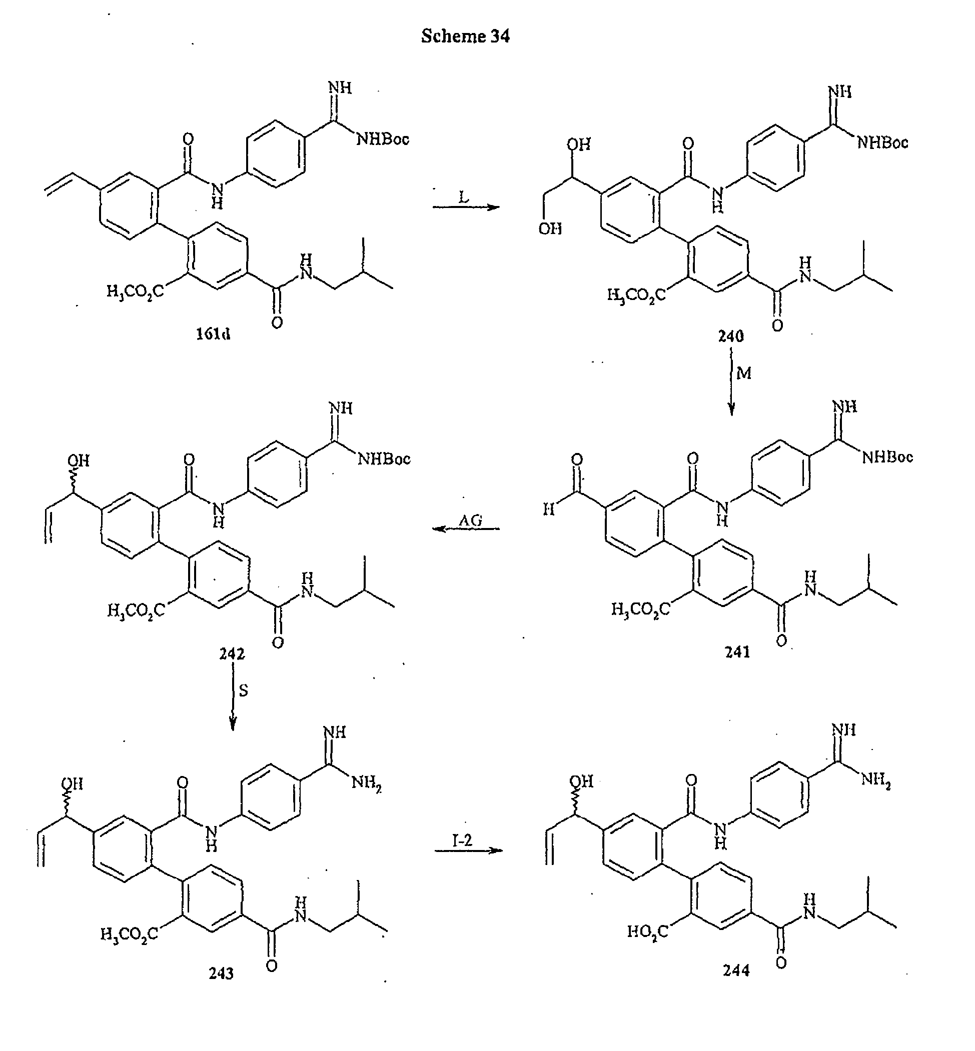

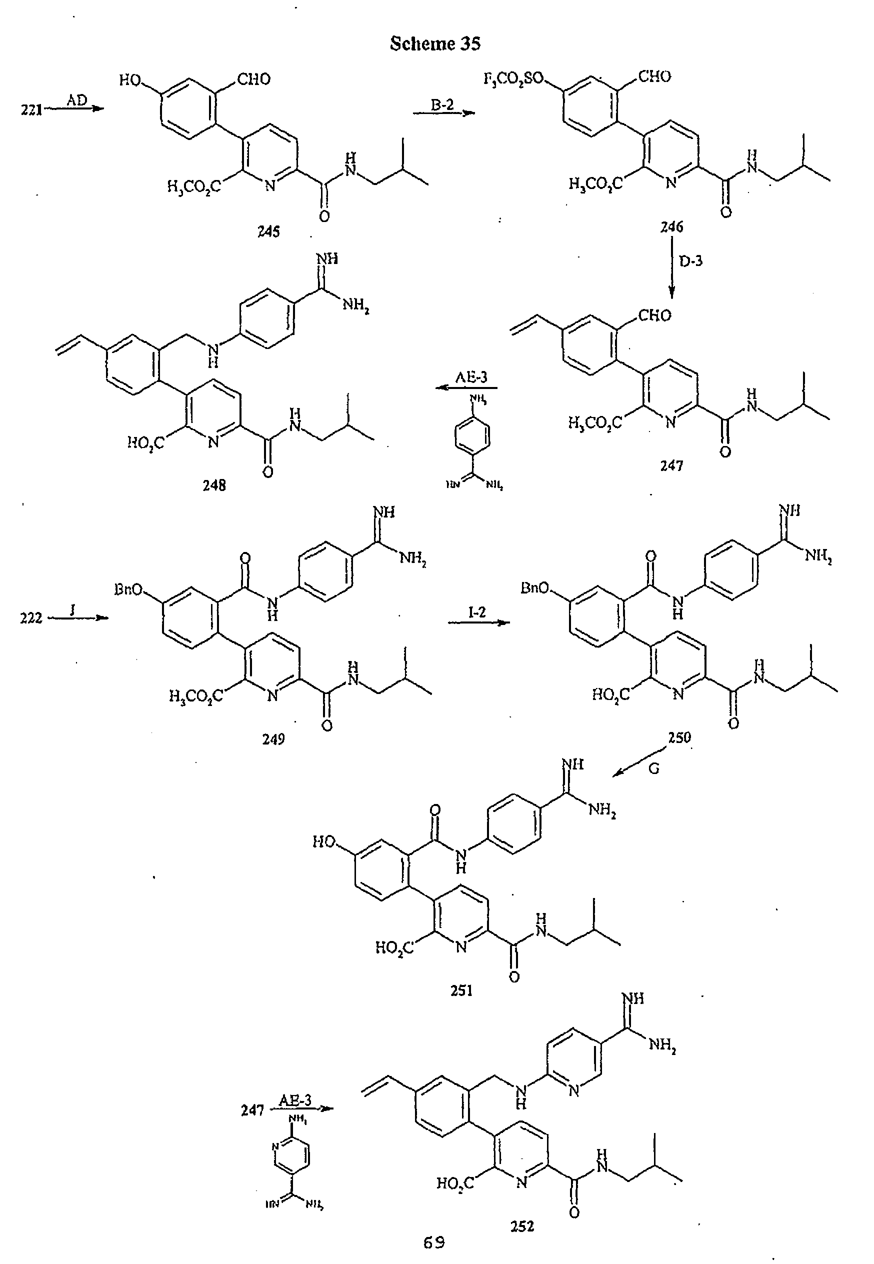

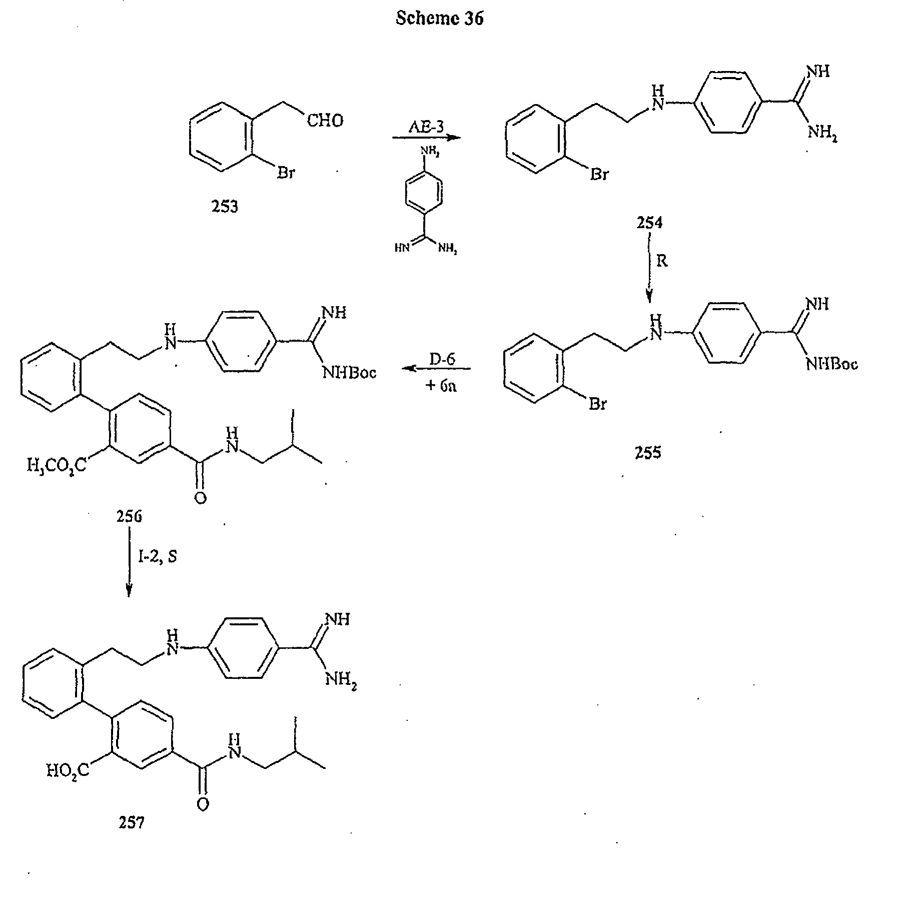

### General Methods of Reparation

The following abbreviations have been used:
THF: Tetrahydrofuran; DMF: Dimethylformamide
DME: 1,2-Dimethoxyethane; MAP; 4-(Dimethylamino)pyridine
Boc anhydride: Di-tert-butyl dicarbonate; TIPS: Triisopropylsilyl
MEM: Methoxyethoxymethyl; Bn: Phenylmethyl or Benzyl

The organic extracts were dried over sodium sulfate or magnesium sulfate.

The general methods for the preparation of the compounds of formula (I) are given below:

### A-1: Conversion of acid to amide

To derivative (1 mmol), was added thionyl chloride (12.6 mmol) and a few drops of DMF. The reaction mixture was refluxed for 2 h and concentrated in vacuo to obtain an oily residue. The residue was dissolved in dichloromethane (3 mL); cooled with ice water and amine (5 mmol) was added. The reaction mixture was stirred at room temperature overnight, washed with 1N HCl, saturated sodium hydrogen carbonate, water, brine, dried and concentrated in vacuo. The product obtained was purified by crystallization or flash column chromatography to furnish the desired amide.

### A-2: Conversion of acid to amide

To a solution of acid derivative (1 mmol) in dichloromethane (10 mL) at 0 °C was added triethylamine (3 mmol) and ethyl chloroformate (3 mmol). The reaction mixture was stirred at the same temperature for 30 min and the corresponding amine (6 mmol) was added. The reaction mixture was stirred at room temperature overnight and quenched with IN HCl. The organic layer was separated, washed with water, brine, dried and concentrated in vacuo. The product obtained was purified by crystallization or flash column chromatography to furnish the desired amide.

### A-3; Conversion of acid to amide

To a solution of acid (1 mmol) in dichloromethane (5 mL) was added 2M oxalyl chloride in dichloromethane (2.5 mmol), followed by a drop of DMF. The reaction mixture was stirred for 2h at room temperature and concentrated in vacuo. The residue was co-evaporated once with dichloromethane (5 mL) and then dried in vacuo. To the residue in dichloromethane (10 mL) were further added triethylamine (3 mmol) and the corresponding amine (1.2 mmol). The reaction mixture was stirred for 16 h and washed with water, brine, dried and concentrated in vacuo. The product obtained was purified by crystallization or flash column chromatography to furnish the desired amide.

### A-4: Conversion of acid to amide

To a solution of acid (1 mmol) in dichloromethane or THF (10 mL) cooled with an ice bath was added triethylamine (1.2 mmol) and ethyl chloroformate or isobutyl chloroformate (1.2 mmol). The reaction mixture was stirred at 0°C for 30 min and the corresponding amine (2.5 mmol) was added. The reaction mixture was stirred at room temperature overnight and quenched with 1N HCl. The organic layer was separated, washed with water, brine, dried and concentrated in vacuo. The product obtained was purified by crystallization or flash column chromatography to furnish the desired amide.

### A-5: Conversion of acid to amide

A mixture of carboxylic acid (1 mmol), amine (1.1 mmol), 1-hydroxybenzotriazole (1 mmol) and 1-(3-dimethylaminopropyl)-3-ethylcarbodiimide methiodide (1.1 mmol) in pyridine (10 mL) was stirred overnight at room temperature and was concentrated *in vacuo* to dryness, The residue obtained was purified by column chromatography or used as such for the next step.

### A-6: Reduction of acid to alcohol

To a solution of acid (1 mmol) in dichloromethane or THF (10 mL) at 0 °C was added triethylamine (1.2 mmol) and ethyl chloroformate or isobutyl chloroformate (1.2 mmol). The reaction mixture was stirred at 0 °C for 30 min and sodium borohydride (1.25 mmol) was added. The reaction mixture was stirred at room temperature overnight and quenched with IN HCl. The reaction mixture was extracted with ethyl acetate. The organic layers were combined, washed with water, brine, dried and concentrated in vacuo to furnish the desired alcohol. This can be purified further, if needed, by crystallization or column chromatography.

### A-7: Conversion of acid to amide

A mixture of carboxylic acid (1 mmol), amine (1 mmol), and 4-dimethylaminopyridie (0.12 mmol) in xylene (10 mL) was stirred at 80 °C for 10 min. Phosphorus trichloride (1 mmol) was added and the reaction mixture was heated with stirring at 150°C for 2 hr. After cooling, the product was extracted with EtOAc. The organic layers were combined, washed with water, brine, dried and concentrated *in vacuo.* The product obtained was purified by flash column chromatography to furnish the desired amide.

### B-1: Conversion of phenolic hydroxyl to triflate

To a phenol (1 mmol) in dichloromethane (2.5 mL) was added pyridine (5 mmol) under a nitrogen atmosphere and cooled to -10 C. To the cold reaction mixture was added dropwise triflic anhydride (2 mmol) in dichloromethane (2.5 mL) over a period of 10 mins and allowed to warm to room temperature and stirred for 16 h. The reaction mixture was quenched with saturated aqueous sodium hydrogen carbonate solution and the organic layer was separated. The organic layer was washed with IN HCl, saturated sodium hydrogen carbonate, water, brine, dried and concentrated in vacuo. The product obtained was purified by crystallization or flash column chromatography to furnish the desired triflate.

### B-2: Conversion of phenolic hydroxyl to triflate

To a solution of substituted phenol (1 mmol) in DMF (10 mL) was added N-phenylbis(trifluoromethanesulphonimide) (1.1 mmol), and triethylamine (2 mmol) and stirred at room temperature overnight. The reaction mixture was quenched with ice water and extracted twice with ether. The organic layers were combined, washed with brine, dried and concentrated *in vacuo* to furnish the desired triflate.

### C: Conversion of acid to MEM ester

To a solution of acid derivative (1 mmol) in DMF (10 mL) was added sodium bicarbonate (1.05 mmol), and MEM-Cl (1.05 mmol) and was stirred at room temperature for 24 h. The reaction mixture was quenched with ice water and extracted twice with ether. The organic layers were combined, washed with brine, dried and concentrated *in vacuo* to furnish crude product. Purification by flash column chromatography or crystallization gave the desired MEM ester.

### D-1: Coupling of boronic acid with triflate

A mixture of triflate (1 mmol), aryl boronic acid (1.5 mmol), potassium phosphate (3 mmol), potassium bromide (2.4 mmol) and tetrakis(triphenylphosphine)palladium (0.05 mmol) in dioxane (10 mL) was heated at reflux overnight under an argon atmosphere. The reaction mixture was cooled, quenched with water and was extracted with ethyl acetate. The organic layers were combined, dried and concentrated *in vacuo.* Purification by flash column chromatography or crystallization gave the coupled product.

### D-2: Coupling of boronic acid with triflate

A mixture of triflate (1 mmol), aryl boronic acid (2 mmol), sodium hydrogen carbonate (3 mmol) and tetrakis(triphenylphosphine)palladium (0.05 mmol) or bis(triphenylphosphine)palladium(II)chloride (0.05 mmol) in DME/water (9:1, 10 mL) was heated at reflux overnight. The reaction mixture was cooled, quenched with water and extracted with ethyl acetate. The organic layer was dried and concentrated *in vacuo.* Purification by flash column chromatography or crystallization gave the coupled product.

### D-3: Coupling of tributyltin derivative with triflate

A mixture of triflate (1 mmol), tributyltin derivative (3 mmol), tetraethylammonium chloride (6 mmol), and bis(tripheuylphosphine)pailadium(II)-chloride (0.05 mmol) in DMF (10 mL) was heated at 70 °C overnight under an argon atmosphere. The reaction mixture was cooled, quenched with water (20 mL) and extracted with ethyl acetate (2 X 10 mL). The organic layers were combined, dried and concentrated in *vacuo.* Purification by flash column chromatography or crystallization gave the coupled product.

### D-4: Coupling of trimethyltin derivative with triflate

A mixture of triflate (1 mmol), trimethyltin derivative (3 mmol), and bis(triphenylphosphine)palladium(II)chloride (0.05 mmol) in THF (10 mL) was heated at 70 °C overnight under an argon atmosphere. The reaction mixture was cooled, quenched with water and extracted with ethyl acetate (2 X 10 mL). The organic layers were combined, dried and concentrated *in vacuo.* Purification by flash column chromatography or crystallization gave the coupled product.

### D-5: Coupling of alkyne with triflate

A mixture of triflate (1 mmol), triethylamine (4.5 mmnol), substituted alkyne (3.5 mmol), and bis(triphenylphosphine)palladium(II)chloride (0.05 mmol) in DMF (10 mL) was heated at 70 °C overnight under an argon atmosphere. The reaction mixture was cooled, quenched with water (20 mL) and extracted with ethyl acetate (2 X 10 mL). The organic layers were combined, dried and concentrated *in vacuo.* Purification by flash column chromatography or crystallization gave the coupled product.

### D-6: Coupling of boronate ester with aryl bromides

A mixture of boronate ester (2 mmol), aryl bromide (1 mmol), potassium phosphate (3 mmol) and bis(diphonylphosphinoferrocene)palladium(II)chloride (0.05 mmol) in DMF (10 mL) was heated at 100 °C for overnight under an argon atmosphere. The reaction mixture was cooled, quenched with water (20 mL) and extracted with ethyl acetate (2 X 10 mL). The organic layers were combined, dried and concentrated *in vacuo.* Purification by flash column chromatography or crystallization gave the desired product.

### D-7: Coupling of boronate ester with aryl bromides

A mixture of boronate ester (2 mmol), aryl bromide (1 mmol), sodium hydrogen carbonate (3 mmol) and bis(diphenylphosphosphinoferrocene)palladium(II)chloride (0.05 mmol) in DME/water (9:1, 10 mL) was heated at 50-70 °C for overnight under an argon atmosphere. The reaction mixture was cooled, quenched with water (20 mL) and was extracted with ethyl acetate (2 X 10 mL). The organic layers were combined, dried and concentrated *in vacuo.* Purification by flash column chromatography or crystallization gave the coupled product.

### D-8: Coupling of phenol with boronic acid

A mixture of phenol (1 mmol), aryl boronic acid (3 mmol), molecular sieves (4A°), pyridine (5 mmol), copper(II)acetate (1 mmol) and bis(triphenylphosphine)-palladium(II)chloride (0.05 mmol) in dichloromethane (10 mL) was stirred at room temperature overnight under an argon atmosphere. The reaction mixture was cooled, filtered through a pad of Celite and concentrated *in vacuo.* Purification of the crude by flash column chromatography gave the coupled aryl ether.

### D-9: Coupling of trimethyltin derivative with triflate

To a solution of triflate (1 mmol), LiCl (4 mmol), PPh₃ (0.15 mmol), CuBr (0.2 mmol), and bis(triphenylphosphine)palladimn(II)chloride (0.07 g) in DMF (10 mL) under an atmosphere of argon was added trimethylstannyl compound (0.8 mmol) and a crystal of 2,6-di-*t*-butyl-4-methylphenol. After the mixture was stirred at 90 °C for 3 h, a second portion of aryl-trimethylstannyl compound (0.5 mmol) was added. The reaction mixture was stirred at 90 °C overnight. Water was added and extracted with ethyl acetate. The organic layer was dried (MgSO₄), concentrated and purified by flash column chromatography or crystallization to furnish the desired coupled product.

### D-10: Coupling of amine with triflate

A mixture of triflate (0.75 mmol), amine (0.9 mmol), potassium phosphate (1.1 mmol), 2-(di-t-butylphosphino)biphenyl (0.015 mmol) and tris(dibenzylideneacetone) dipalladitun(0) (10 mg) in DME (10 mL) was heated at reflux overnight under an argon atmosphere. The reaction mixture was concentrated in *vacuo* and the residue was purified by flash column chromatography to furnish the desired coupled product.

### D-11: Conversion of triflate to cyano compound

To a solution of triflate (0.84 mmol), zinc cyanide (0.54 mmol), Palladium acetate (0.016 mmol), 2-(di-*tert*-butylphosphine)biphenyl ( 0.016 mmol) and N-methyl pyrrolidine (10 mL) was heated under argon at 160 °C for 48 h. The reaction mixture was cooled to room temperature and quenched with water (50 mL). The reaction mixture was extracted with ethyl acetate (2 X 25 mL). The organic layers were combined, dried, filtered and concentrated *in vacuo.* The residue obtained was purified by flash column chromatography to furnish the desired cyano compound.

### D-12: Coupling of tetravinyltin with triflate or halide

To a solution of aryl triflate or bromide (1 mmol) in DMF (5 mL) were added LiCl (5 mmol), tetravinyltin (2 mol), and dichlorbis(triphenylphosphine)palladium (II) (0.01 mmol). The reaction mixture was stirred at 70 °C under nitrogen for 5 h and then diluted with ethyl acetate and filtered. The organic layer was washed with water and brine and dried (MgSO₄). After evaporating the solvent *in vacuo*, the compound was purified by flash-column chromatography to give the desired product.

### E: Oxidation of aryl aldehyde to acid

A mixture of aldehyde (1 mmol), *tert*-butanol (5 mL), water (2 mL) and acetonitrile (1 mL, additional amount may be added until the reaction mixture was homogenous) was stirred at room temperature. The solution was cooled in ice-bath and 2-methyl-2-butene (1 mL), sodium chlorite (6 mmol) and sodium dihydrogenphosphate (1.6 mmol) were added. The reaction mixture was stirred at room temperature for 2 h. If the solid separated out, the mixture was filtered to collect the solid, the desired product. If no solid separated out, then the reaction mixture was concentrated in vacuo to remove acetonitrile, diluted with water (10 mL) and extracted with ethyl acetate (2 X 10 mL). The organic layers were combined, washed with water, brine, dried and concentrated in vacuo to furnish crude acid. Purification was achieved, if needed, by crystallization or using flash column chromatography to obtain pure acid.

### E-2: Oxidation of vinyl compound to acid

To a solution of vinyl compound (1 mmol) in acetone (5 mL) was added KMnO₄ (4 mmol). The reaction mixture was stirred for 3 h (the reaction is exothermic, and refluxed on its own during the addition of KMnO₄). The reaction mixture was diluted with methanol and water and filtered. The organic solvents were evaporated *in vacuo.* and the aqueous layer was acidified to pH 1 and extracted several times with ethyl acetate/DME. The combined organic layers were dried (MgSO₄) to furnish the desired acid.

### F: Conversion of aromatic acid to MEM ester

To a solution of aromatic acid (1 mmol) in THF (10 mL) was added diisopropylethylamine (2 mmol) and 2-methoxyethoxymethylchloride (1.1 mmol). The reaction mixture was stirred a room temperature for 3 h and diluted with ether (25 mL). The reaction mixture was washed with water (10 mL), brine (10 mL), dried and concentrated *in vacuo* to obtain product as colorless oil. The product was purified by flash column chromatography to furnish desired product.

### G: Conversion of aromatic benzyl ether to aromatic phenol, benzyl ester to acid, benzyl carbamate to amine, alkene to alkane, azide to amine, nitro to amine, and oxime to amine

To a solution of appropriate substrate (1 mmol) in ethanol (10 mL) was added 10% palladium on carbon (10-wt%). The reaction mixture was hydrogenated at 50 psi for 2 to 24 h (until all starting material disappeared as confirmed by MS and TLC analysis). The catalyst was removed by filtration through a pad of Celite under nitrogen. The filtrate was concentrated *in vacuo* to furnish the product, which was purified by flash column chromatography or crystallization.

### H: Conversion of aromatic acid to benzyl ester

To a solution of aromatic acid (1 mmol) in DMF (10 mL) was added sodium bicarbonate (1.05 mmol), and benzyl bromide (1.05 mmol) and stirred at room temperature for 24 h. The reaction mixture was quenched with ice water and extracted twice with ethyl acetate. The organic layers were combined, washed with water and brine, dried and concentrated *in vacuo* to furnish crude product. Purification by crystallization or flash column chromatography gave the desired ester.

### I-1: Hydrolysis of MEM ester to acid

To a solution of MEM ester (1 mmol) in DME (8 mL) was added 6 N HCl (2 mL) and stirred at room temperature overnight. The reaction mixture was neutralized with solid sodium hydrogen carbonate (18 mmol) and concentrated *in vacuo*. The reaction mixture was acidified with 0.5 N HCl (20 mL) and extracted with ethyl acetate (2 X 20 mL). The organic layers were combined, washed with brine (20 mL), dried and concentrated *in vacuo* to furnish crude product. Purification of the crude by flash column chromatography gave the product. Alternatively the crude reaction mixture was diluted with water (10 mL) and concentrated in vacuo to remove DME. The solid obtained was collected by filtration and dried in vacuo to furnish pure acid.

### I-2: Hydrolysis of ester to acid

To a solution of ester (1 mmol) in MeOH (10 mL) was added 1 N NaOH (10 mmol). The reaction mixture was stirred at room temperature for 2-3 h, filtered through a plug of cotton, and concentrated *in vacuo* to remove MeOH. The pH of the aqueous layer was adjusted to below 7. The solid that separated, was collected by filtration, washed with water and dried *in vacuo* to furnish the desired acid.

### J: Coupling of acid with amino compounds

To a solution of acid (1 mmol) in DMF (5 mL) was added corresponding amine (1.1 mmol) and stirred at room temperature until homogenous. Pyridine (5 mL) was added to the reaction mixture followed by 1,3-dicyclohexylcarbodiimide (1.2 mmol) and stirred overnight at room temperature. The mixture was quenched with 6 N HCl (10 mL), diluted with ice cold water (10 mL) and extracted with chloroform (2 X 10 mL). The organic layers were combined washed with brine (10 mL), dried and filtered. Purification of the crude by flash column chromatography gave the product as a solid. If the product was soluble in water, then the reaction mixture was concentrated in vacuo to remove pyridine and DMF and purified by flash column chromatography.

### K: Reduction of aldehyde to alcohol

To a solution of aldehyde (1 mmol) in THF (10 mL) was added sodium borohydride (0.4 mmol), The reaction mixture was stirred for 30 mins and quenched with glacial acetic acid (0.3 mL). The reaction mixture was diluted with water (10 mL) and extracted with ethyl acetate (2 X 10 mL). The organic layers were combined and washed with brine (10 mL), dried, filtered and concentrated in vacuo to obtain crude product which was purified by flash column chromatography.

### L: Conversion of vinyl group to diol

To a solution of vinyl compound (1 mmol) in THF/*tert*-butanol (1:1,10 mL) and water (2 mL) was added 4-methylmorpholine N-oxide (2.5 mmol) and osmium tetraoxide (1 mL, 2.5 wt% in *tert*-butanol, 0.1 mmol). The reaction mixture was stirred at room temperature for 2 h and quenched with saturated aqueous solution of sodium sulfite (5 mL). The reaction was stirred at room temperature for 30 mins and diluted with brine (10 mL) and ethyl acetate (10 mL). The organic layer was separated and the aqueous layer was extracted with ethyl acetate (10 mL). The organic layers were combined and washed with brine (10 mL), dried, filtered and concentrated in vacuo. The crude product was purified by flash column chromatography to furnish the desired diol.

### M: Conversion of diol to aldehyde

To a solution of diol (1 mmol) in DME/water (9:1, 10 mL) was added sodium metaperiodate (3 mmol) and stirred at room temperature for 30 min. The reaction mixture was quenched with water (10 mL) and extracted with ethyl acetate (2 X 10 mL). The organic layers were combined and washed with brine (10 mL), died, filtered and concentrated in vacuo. The crude product was purified by flash column chromatography to furnish the desired aldehyde.

### N: Conversion of alcohol to mesylate

To a solution of alcohol (1 mmol) in DME (10 mL) was added dimethylaminopyridine (0.1 mmol), methane sulfonyl chloride (3 mmol) and diisopropylethylamine or triethylamine (5 mmol). The reaction mixture was stirred at room temperature overnight. The reaction mixture was diluted with water (10 mL) and extracted with ethyl acetate (2 X 10 mL). The combined organic layers were washed with brine, dried, filtered and concentrated *in vacuo.* The residue obtained, was purified by column chromatography to furnish the desired mesylate.

### O: Conversion of mesylate to azide

To a solution of mesylate (1 mmol) in DMSO (10 mL) was added sodium azide (25 mmol) and heated at 100 °C overnight. The reaction mixture was cooled and diluted with cold water (25 mL). The reaction mixture was extracted with ethyl acetate (2 X 115 mL). The combined organic layers were washed with water (10 mL), brine (10 mL), dried, filtered and concentrated *in vacuo* The residue obtained was purified by column chromatography to furnish the desired azido compound.

### P: Protection of amine as benzyl carbamate

A mixture of amino compound (1 mmol), benzyl chloroformate (2 mmol) and triethylamine (10 mL) in pyridine (10 mL) was stirred at room temperature overnight. The reaction mixture was concentrated *in vacuo* to remove organic solvents and diluted with 0.1 N HCl (10 mL). The product was extracted with chloroform (2 X 10 mL), dried, filtered and concentrated *in vacuo.* The residue obtained was purified by column chromatography to furnish the desired carbamate.

### Q: Conversion of silyl protected amine to amine

A mixture of silyl protected amine (1 mmol), tetrabutylammonium fluoride (1.0 M in THF, 2 mmol) in THF (10 mL) was stirred at room temperature for 1.5 h. The reaction mixture was concentrated in vacuo and purified by column chromatography to obtain the desired product.

### R: Protection of amine as tert-butyl carbamate

To a solution of amino compound (1 mmol) in acetonitrile (5 mL) was added triethylamine (2 mmol) and BOC anhydride (1.2 mmol). The reaction mixture was stirred for 2 h and concentrated *in vacuo.* Water was added to the residue and extracted with ethyl acetate. The organic layer was washed with brine, dried (MgSO₄), and the solvent was evaporated *in vacuo* to furnish tert-butyl carbamate. If needed, the product was purified by crystallization or column chromatography.

### S: Conversion of tert-butyl carbamate to amine

To a solution of *tert*-butyl carbamate (1 mmol) in dichloromethane (10 mL) was added trifluoroacetic acid (2 mL). The solution was stirred at room temperature for 4 h and concentrated *in vacuo.* The residue was purified by column chromatography or crystallization to give the desired amine.

### S-2: Conversion of tert-butyl carbamate to amine

To a solution of *tert*-butyl carbamate (1 mmol) in methanol (13 mL) was added 6 N HCl (8.75 mL, 52 mmol) and water (4.25 mL). The reaction mixture was stirred at room temperature for 2 days. The pH was adjusted to 7 using conc. ammonium hydroxide and the solid that separated out, was collected by filtration, washed with ether, dried in vacuo to furnish the desired product. If no solid separated out, the product was isolated by extraction with chloroform and evaporating the organic layer.

### T: Protection of aldehyde as acetal

To a solution of aldehyde (1 mmol) in ethanol (5 mL) was added triethyl orthoformate (1.4 mmol), ammonium nitrate (0.2 mmol) and stirred at room temperature overnight (if reaction was not complete by TLC and NMR analysis of an aliquot, the reaction mixture was heated at 50 °C until complete). After completion of the reaction, the mixture was quenched with triethylamine (0.2 mmol) and concentrated *in vacuo* to remove ethanol. The residue was dissolved in ether, filtered to remove any insoluble inorganic impurities, and evaporated to dryness. The product obtained was used as such without further purification.

### U-1: Conversion of bromide to boronic acid

To a mixture of bromo compound (1 mmol) in ether (10 mL), cooled to -78 °C, n-butyl lithium (1.2 mmol) was added dropwise and the reaction mixture was stirred for 30 mins after the addition was completed. Tributyl borate (1.3 mmol) in ether (10 mL) was added to the reaction and stirred at -78 °C for 2 h. The reaction mixture was allowed to warm to 0 °C and quenched with 2 M HCl (10 mL). The reaction mixture was stirred at room temperature for 1h and cooled with ice. The aqueous layer was separated and the organic layer was extracted twice with 1N NaOH (2 X 10 mL). The basic extracts were combined and washed with ether (10 mL). The basic layer was acidified to pH 4 using 6 N HCl and the solid that separated out was collected by filtration, washed with water and hexane and dried *in vacuo* to furnish boronic acid as a solid. If no solid product is obtained then the basic layer was extracted with ether (2 X 10 mL). The organic layers were combined, dried and concentrated *in vacuo* to furnish boronic acid.

### U-2: Synthesis of boronic acid by ortho lithiation of aryl aldehyde

To a solution of N,N,N'-trimethylethylenediamine (1 mmol) in THF/ether (10 mL, 1:1) cooled to -20 °C was added dropwise, over a period of 15 mins, n-butyl lithium (1 mmol) and stirred at -24 °C for 15 mins. Aldehyde (1 mmol) at -20 °C was added dropwise over a period of 10 mins to this mixture. The reaction mixture was further stirred for 15 mins at -20 °C followed by the addition of n-butyl lithium (2.8 mmol) dropwise over a period of 15 mins and stirred at 4 °C overnight. The reaction mixture was cooled to -40 °C and tributyl borate (5.6 mmol) in ether (20 mL) was added to the reaction and stirred at 4 °C for 12 h. The reaction mixture was allowed to warm to 0 °C and quenched with 2 M HCl (3 mmol) and heated at reflux for 2 h and added to ice water (25 mL). The aqueous layer was separated and the organic layer extracted twice with IN NaOH (2 X 10 mL). The basic extracts were combined and washed with ether (10 mL). The basic layer was acidified to pH 3 using 6 N HCl and the solid that separated out was collected by filtration, washed with water and hexane and dried *in vacuo* to furnish boronic acid as a solid. If no solid product was obtained, then the basic layer was extracted with ether (2 X 10 mL). The organic layers were combined, dried and concentrated *in vacuo* to furnish boronic acid.

### U-3: Synthesis of boronic acid by ortho lithiation of aryl acetal

To a solution of aryl acetal compound (1 mmol) in ether (10 mL) at -78 °C, *tert-*butyl lithium (1.1 mmol) was added dropwise and the reaction mixture was stirred for 3 h at -20 °C after the addition was completed. Tributyl borate (1.2 mmol) in ether (10 mL) was added to the reaction and stirred at -20 °C for 1 h. The reaction mixture was allowed to warm to 0 °C and quenched with 2 M HCl (10 mL). The reaction mixture was stirred at room temperature for 1h. The aqueous layer was separated and the organic layer was extracted twice with IN NaOH (2 X 10 mL). The basic extracts were combined and washed with ether (10 mL). The basic layer was acidified to pH 4 using 6 N HCl and the solid that separated out was collected by filtration, washed with water and hexane and dried *in vacuo* to furnish boronic acid as a solid. If no solid product was obtained then the mixture was extracted with ether (2 X 10 mL). The organic layers were combined, dried and concentrated *in vacuo* to furnish boronic acid.

### V-1: Demethylation of aryl methyl ether to phenol

In a round bottom flask (50 mL), pyridine hydrochloride (10g) was heated in an oil bath at 180 °C. After the entire solid had melted, the corresponding aryl methyl ether (1 mmol) was added in small portions over a period of 20 min. The reaction mixture was heated at 180 °C for 4 h, cooled and quenched with water (100 mL). The reaction mixture was extracted with ethyl acetate (3 X 10mL). The combined organic layers were washed with brine, dried over MgSO₄, concentrated to give phenol. This can be further purified if needed by crystallization or column chromatography.

### V-2: Demethylation of aryl methyl ether to phenol

To a solution of aryl ether (1 mmol) in dichloromethane (10 mL) cooled to -78 °C was added boron tribromide (3 mmol). The reaction mixture was allowed to warm to room temperature overnight and quenched with water (10 mL). The solid obtained was collected by filtration to give the desired product. More product was obtained after evaporation of the organic layer and washing the residue with water. Alternatively, if a homogenous biphasic mixture was obtained on addition of water, the organic layer was separated, washed with brine, dried over MgSO₄, and concentrated to give the desired phenol. This can be further purified if needed by crystallization or column chromatography.

### V-3: Demethylation of aryl methyl ether to phenol

To a solution of aryl methyl ether (1 mmol) in dichloromethane (5 mL) was added AlCl₃ (8.5 mmol). The reaction mixture was heated to reflux for 12 h under nitrogen. To this mixture was added 12 mL of 1 N HCl slowly and the organic layer was separated. The aqueous layer was re-extracted several times with ethyl acetate/DME. The combined organic layers were washed with brine, dried (MgSO₄), and evaporated *in vacuo* to furnish the desired phenol, which was purified by column chromatography.

### V-4: Demethylation of aryl methyl ether to phenol

To a stirred slurry of NaH (2 mmol) in anhydrous toluene (5 mL) under nitrogen atmosphere was added para-thiocresol (2 mmol) dissolved in toluene (40 mL). The mixture was stirred at room temperature for 30 min and hexamethylphosphoric triamide (2 mmol) in toluene (5 mL) was added dropwise over a period of 30 min. A solution of aryl ether (1 mmol) in toluene (5 mL) was added in one portion. The reaction mixture was stirred at reflux for 9.5 h, cooled to room temperature and diluted with ethyl acetate (40 mL). The organic layer was extracted with 1 N aqueous NaOH solution (2 X 20 mL). The basic layer was acidified to pH 5 and extracted with ethyl acetate (2 X 20 mL). The organic layers were combined, washed with water, dried (MgSO₄) and concentrated in vacuo. The residue obtained was purified by flash column chromatography to afford the desired phenol compound.

### W: Conversion of acid to methyl ester

A mixture of acid (1 mmol), conc. H₂SO₄ or conc HCl (0.5 mL) and methanol (10 mL) was heated at reflux for 16 h. The mixture was concentrated to half of its volume and the residue poured into a saturated sodium bicarbonate solution. The precipitate was collected by filtration, washed with water and dried to give the desired ester. If the ester did not come as solid, it was extracted with ethyl acetate. The organic layer was dried, filtered and concentrated to give the desired ester.

### W-2: Conversion of acid to ester

A solution of methanolic HCl or ethanolic HCl was prepared by the addition of acetyl chloride (1 mL) to methanol/ethanol (9 mL) at 0 °C and stirred for 30 mins. To the solution of anhydrous methanolic HCl was added acid (1 mmol) and stirred at room temperature (or reflux if needed) overnight. The reaction mixture was concentrated to dryness *in vacuo* and the residue was purified by column chromatography or crystallization to furnish the desired ester.

### X: Conversion of phenol to alkyl aryl ethers or alkylation of amines

To a solution phenol or amine (1 mmol) in DMF (10 mL) was added cesium carbonate (1.25 mmol) and corresponding bromide (1.1 mmol). The reaction mixture was stirred at room temperature overnight and quenched with water (25 mL). The product was extracted with ether (2 X 25 mL), the organic layers were combined and washed with water (25 mL), brine (25 mL), dried and concentrated *in vacuo* to furnish crude product. The crude was purified by crystallization or flash column chromatography.

### Y: Conversion of nitrile to hydroxycarbamimidoyl

To a solution ofnitrile compound (1 mmol) in ethyl alcohol (10 mL) was added hydroxylamine (50% aqueous solution, 5 mmol). The mixture was stirred at reflux for 2-5 h. The reaction mixture was concentrated in vacuo to furnish the desired hydroxycarbamimidoyl compound.

### Z: Opening of aromatic methylene dioxy compound with alcohol

A solution of potassium tert-butoxide (2.25 mmol) in DMSO (1.25 mL) was heated at 50 °C for 30 min. Methanol (1.25 mL) was added to it and continued heating at 50 °C for 30 min. To the reaction mixture was added 1,2-methylenedioxy aromatic compound (1 mmol) and continued heating at 50 °C for 30 min. The reaction mixture was cooled to room temperature and quenched with water (10 mL) and 1 N sodium hydroxide (16 mL). The reaction m mixture was washed with ether (2 X 10 mL) and acidified to pH 4 using conc HCl. The solid obtained was collected by filtration to furnish the desired product.

### Z-1: Opening of aromatic methylene dioxy compound with alcohol

To a mixture of methylene dioxy compound (1 mmol) in HMPA (2.5 mL) were added sodium methoxide (2.5 mmol) and heated with stirring at 150 °C for 12 min. The mixture was cooled and poured into ice water (20 mL), NaOH (30 mg) and stirred for 10 min. It was then extracted with ether and the aqueous layer was acidified to pH 4 with HCl and extracted with ether. The later ethereal extracts were combined, dried and concentrated. The residue was purified by crystallization or column chromatography.

### AA: Conversion of amine to amide in the presence of a phenol

To a solution of amino compound (1 mmol) in pyridine (5 mL) was added, dropwise, acid chloride (2 mmol) at 0 °C under N₂. The mixture was stirred for 45 min and was then poured into ice water and acidified with 1 N HCl. The precipitated solid was collected by filtration, washed with IN HCl, hexane, and then dried *in vacuo* to give crude product. The crude product was added to freshly prepared sodium methoxide solution (0.1 M, 10 mL) and stirred for 30 min at room temperature. The reaction mixture was quenched with acetic acid (1 mmol) and concentrated *in vacuo*. The residue was dissolved in ethyl acetate and washed with water. The water layer was extracted with ethyl acetate, and the combined organic layers were washed with brine, dried (MgSO₄) and evaporated to yield a solid. The solid was washed with hexane and dried *in vacuo* to furnish the desired amide.

### AB-1: Conversion of amino of amidine to amino carbamate

To amidine compound (1 mmol) was added 0.1N NaOH (10 mL) and stirred at room temperature for 5 min. The reaction mixture was concentrated *in vacuo* and to the residue was added alkyl or aryl 4-nitrophenyl carbonate (2 mmol) in 20 mL of hexamethylphosphoramide and stirred at 45 °C for 24 h. The reaction was quenched with water (100 mL) and extracted with ethyl acetate (2 X 100 mL). The combined extracts were washed with water (100 mL) and brine (100 mL), dried over anhydrous magnesium sulfate, filtered and concentrated *in vacuo*. The residue obtained was purified by flash column chromatography to furnish the desired product.

### AB-2: Conversion of amino of amidine to amino carbamate

To a solution of amidine compound (1 mmol) in acetonitrile (25 mL) was added triethylamine (5 mL) and aryl/alkyl chloroformate (2 mmol) or dialkyl/aryl carbonate. The reaction mixture was stirred at room temperature for 16 h and quenched with water (100 mL). The reaction mixture was extracted with ethyl acetate (2 X 100 mL). The combined extracts were washed with brine (100 mL), dried over anhydrous magnesium sulfate, filtered and concentrated *in vacuo*. The residue obtained was purified by flash column chromatography to furnish the desired product.

### AC: Conversion of aldehyde to oxime

To a stirred solution of aldehyde (1 mmol) in ethanol (10 mL) was added pyridine (10 mL) and hydroxylamine hydrochloride (1.25 mmol). The reaction mixture was stirred overnight at room temperature under nitrogen and then concentrated *in vacuo* to one third of its original volume. Water (10 mL) was added and the precipitated solid was collected by filtration and dried *in vacuo*. The product was used as such for next step without further purification.

### AD: Debenzylation in the presence of aldehyde

To a solution of phenyl methoxyaryl aldehyde (1 mmol) in dichloromethane (10 mL) cooled to -78 °C was added dropwise under a nitrogen atmosphere boron tribromide (1M solution in dichloromethane, 1.2 mmol). The reaction mixture was allowed to warm to room temperature and stirred at room temperature overnight. The reaction mixture was quenched with water (10 mL) and the layers were separated. The aqueous layer was extracted with chloroform (10 mL). The organic layers were combined, washed with brine (10 mL), dried, filtered and concentrated in vacuo to furnish crude product. Purification of the crude by flash column chromatography furnished the desired phenolic aldehyde

### AE-1: Reductive amination of aldehyde

To a stirred solution of aldehyde (1 mmol) in methanol (40 mL) was added amine (3.3 mmol) followed by the addition of glacial acetic acid (0.3 mL). The reaction mixture was stirred for 30 min under nitrogen at room temperature, and then sodium cyanoborohydride (1.5 mmol) was added. After stirring for 20 min, the solvent was evaporated *in vacuo*, and the residue was taken in ethyl acetate. The organic layer was washed with water, and the insoluble material was removed from the organic layer by filtration. The pH of the aqueous phase was adjusted to 7 with IN NaOH and was extracted twice with ethyl acetate. The combined organic layers were washed with brine and dried (MgSO₄). The solvent was evaporated *in vacuo* to furnish crude product. The crude product was purified by crystallization or flash column chromatography.

### AE-2: Reductive amination of aldehyde

To a mixture of aminoarylamidine (1.2 mmol), 4A° molecular sieves, and sodium hydroxide (1 N solution in anhydrous methanol, 1.2 mL, 1.2 mmol) in methanol (10 mL) was added a solution of aldehyde (1 mmol) in THF (10 mL). The reaction mixture was heated for 15 mins at reflux temperature and was cooled to room temperature. Acetic acid (1 %) and sodium cyanoborohydride (1 M solution in THF, 5 mmol) was added to the reaction mixture and stirred at room temperature overnight. The reaction mixture was quenched with 1 N NaOH (30 mmol) and stirred for additional 2 h and concentrated in vacuo to remove methanol. The mixture was diluted with water (15 mL) and washed with ether (2 x 10 mL). The aqueous layer was acidified to pH 2 using 6 N HCl and the solid that separated out was collected by filtration, washed with ether, dried in vacuo to furnish product, which was purified by flash column chromatography, if needed.

### AE-3: Reductive amination of aldehyde

A mixture of aminoarylamidine (2 mmol), 4A° molecular sieves, pyridine (6 mL) in methanol (9 mL) was heated at 50 °C for one hour. A solution of aldehyde (1 mmol) in methanol (7.5 mL) containing acetic acid (1 %) was added and continued heating for 4 h to 12 h. The reaction mixture was cooled and sodium cyanoborohydride (1 M solution in THF, 5 mmol) was added to the reaction mixture and stirred at room temperature overnight. The reaction mixture was quenched with 5 N NaOH (30 mmol) and stirred for additional 2 h. The reaction mixture was filtered through Celite (to remove molecular sieves) and concentrated to remove methanol. The mixture was diluted with water (15 mL) and washed with ether (2 X 10 mL). The aqueous layer was filtered and solid obtained was kept aside (mainly product). The aqueous layer was acidified to pH 2 using 6 N HCl and the solid that separated out was collected by filtration. The combined solid materials were purified, if needed, by flash column chromatography.

### AE-4: Reductive amination of aldehyde

To a mixture of aldehyde (1 mmol) and aminoarylamidine (1.1 mmol) in MeOH at room temperature was added triethyl amine (2.75 mmol), sodium cyanoborohydride (0.83 mmol) and zinc chloride (0.9 mmol). The reaction mixture was stirred at room temperature overnight and concentrated to remove methanol. The reaction mixture was quenched with 1 N NaOH (10 mL), diluted with water (10 mL), and extracted with EtOAc (5 X 20 mL). The combined organic extracts were washed with brine (15 mL), dried (MgSO4), filtered through Celite and concentrated to give the product. Purification of the crude by flash column chromatography gave the desired product.

### AE-5: Reductive amination of aldehyde

To a solution of amine (1.2 mmol) in MeOH (10 mL) was added aldehyde (1 mmol) in THF (10 mL) containing acetic acid (0.1 mL) drop-wise. The mixture was stirred at 50 °C for 4-12 h and then cooled to room temperature. Sodium cyanoborohydride (1.5 mmol) was added to the reaction mixture and stirred at room temperature overnight. Water was added and pH of the solution was adjusted to 7. The solution was extracted with ethyl acetate. The organic layer was dried (MgSO₄) and evaporated *in vacuo*. The residue was purifeid by flash column chromatography to furnish the desired amine.

### AF-1: Synthesis of amidine from nitrile

Acetyl chloride (5 mL) was added to methanol (5 mL) at 0 °C drop-wise and stirred at room temperature for 15 mins. To this solution of methanolic HCl was added nitrile compound (1 mmol) and stirred at room temperature overnight. The reaction mixture was concentrated in vacuo and dried. The residue obtained of the resulting methyl imidate was dissolved in methanol (10 mL). Dry ammonia gas was bubbled into the reaction mixture at reflux temperature for 5 h. The reaction mixture was concentrated to furnish the required amidine.

### AG: Addition of Grignard reagent to aryl aldehyde

To a solution of aryl aldehyde (1 mmol) in THF (15 mL) cooled to -78 °C was added drop wise under a nitrogen atmosphere, vinyl magnesium bromide (1 M solution in THF, 5 mmol). The reaction mixture was allowed to warm to room temperature and stirred for 48 h. The reaction was quenched carefully with saturated aqueous ammonium chloride solution (10 mL) and extracted with ethyl acetate (2 X 10 mL). The organic layers were combined, washed with brine (10 mL), dried and concentrated *in vacuo*. The residue obtained was purified by flash column chromatography to obtain the desired addition product.

### AG-1: Synthesis of tributylvinyltin compounds from vinyl bromide containing hydroxyl

To a solution of vinyl bromide with hydroxyl (1 mmol) in dichloromethane (20 mL) was added *tert*-butyldimethylsilyl chloride (1.5 mmol) and DMAP (1.5 mmol) and stirred at room temperature overnight. The reaction mixture was quenched with water (20 mL) and the aqueous layer separated. The organic layer was washed with 0.1 N aqueous HCl (10 mL), brine (20 mL), dried and concentrated in vacuo to furnish corresponding *tert*-butyldimethylsilyloxy compound as an oil which was used as such for the next step.

To a solution of the above oily residue (1 mmol) in diethyl ether (20 mL) cooled to -78 °C was added dropwise *tert*-butyllithium (1.7 M in pentane, 2 mmol) over a period of 15 mins. The reaction mixture was stirred at -78 °C for 3 h and quenched at -78 °C with 2 N aqueous sulfuric acid (2 mL) and water (18 mL). The reaction mixture was neutralized using 2 N NaOH and the organic layer was separated. The organic layer was washed with water (20 mL), brine (20 mL), dried and concentrated in vacuo. Purification of the crude residue obtained by flash column chromatography furnished the desired tributyltin compound.

### AG2: Synthesis of tributylmethyltin compounds from arylmethyl bromides or allyl bromides

To lithium clippings (10 mmol) in THF (10 mL) cooled to -40 °C was added dropwise tributyltin chloride (0.27 mL, I mmol) in THF (5 mL) over a period of 15 min. The reaction mixture was allowed to warm to room temperature and stirred for 16 h. The reaction mixture was filtered through glass wool to remove insoluble impurities and cooled to -40 °C. A freshly prepared solution of arylmethyl bromide or allyl bromide (1 mmol) was added dropwise over a period of 10 mins and stirred at room temperature overnight. The reaction mixture was quenched with saturated aqueous ammonium chloride solution (10 mL) and extracted with ether (2 X 10 mL). The organic layers were combined, washed with brine (10 mL), dried, filtered and concentrated in vacuo to furnish desired tributyltinalkyl and was used as such without further purification.

### AG-3: 4-Bromo-5-formyl-benzo[1,3]dioxole-2-carboxylic acid methyl ester

To a mixture of 2-bromo-3,4-dihydroxy-benzaldehyde (2.17 g, 10.0 mmol) and K₂CO₃ (5.56 g, 40.2 mmol) in *n*-propanol (25 mL) was added dibromoacetic acid (2.18, 10.0 mmol) and the mixture was heated at reflux temperature for 24 h. After cooling to room temperature, another portion of dibromoacetic acid (1.75 g, 8.0 mmol) was added. The mixture was stirred at reflux for 46 h. *n*-Propanol was evaporated and water (30 mL) was added. The resulting aqueous solution was acidified to pH 2 by adding 1 N HCl and extracted with ethyl acetate (3 X 100 mL). The combined organic layers were dried (MgSO₄) and evaporated *in vacuo* to afford crude 4-bromo-5-formyl-benzo[1,3]dioxole-2-carboxylic acid (1.34 g) as a brownish solid. This crude product was dissolved in anhydrous methanol (50 mL) and conc. H₂SO₄ (5 mL) was added drop by drop. The resulting mixture was refluxed overnight and cooled to room temperature. Water (50 mL) was added and the resulting aqueous solution was extracted with ethyl acetate (100 mL) X 3). The combined organic layers were dried (MgSO₄) and evaporated *in vacuo*. The residue was purified by flash column chromatography (ethyl acetate:hexane = 5:95) to furnish 4-bromo-5-formyl-benzo[1,3]dioxole-2-carboxylic acid methyl ester as a white solid.

### AH: Synthesis of tert-butyl ester of phenol

To a solution of phenol (1 mmol) in pyridine (10 mL) was added 2,2-dimethyl-propionyl chloride (1.2 mmol) dropwise. The mixture was stirred at room temperature for overnight and diluted with water (100 mL). The reaction mixture was extracted with ethyl acetate (3 X 50 mL). The organic layers were combined and washed with aqueous 0.5 N HCl (100 mL), water, brine, dried (MgSO₄) and concentrated *in vacuo*. The crude residue was purified by flash column chromatography to furnish the desired ester.

### AI: Preparation of 2-bromo-5-hydroxy benzaldehyde

To a solution 3-hydroxybenzaldehyde (Aldrich, 101.39 g, 805 mmol) in chloroform (1000 mL), was added bromine (45 mL, 845 mmol) in chloroform (200 mL) drop wise over a period of 2 h at room temperature. The reaction mixture was stirred at room temperature overnight and filtered to collect crude 2-bromo-5-hydroxy benzaldehyde (32 g) as a dark brown solid. The filtrate was concentrated to 200 mL, filtered through a pad of Celite and silica gel (40 g) and washed with ether (1000 mL). The filtrate was concentrated in vacuo to give a second crop of the crude desired aldehyde (60 g) as a dark brown solid. The above solids were combined and dissolved in glacial acetic acid (360 mL) by heating. Water (840 mL) was added and the solution was filtered hot. The solution was allowed to attain room temperature and kept in a refrigerator overnight. The crystals obtained were collected by filtration and washed with water, dried overnight in vacuo to furnish (60 g, 37%) of the desired product as a purplish brown crystalline solid, mp: 135 °C.

### AJ-1: Amidine from nitrile

A mixture of nitrile (1 mmol) and hydroxylamine (aqueous 50%, 1.8 mL) in EtOH (15 mL) was refluxed for 3 h and concentrated *in vacuo*. To the residue obtained was added EtOH (20 mL), acetic acid (2 mL) and a small amount of Raney nickel. The reaction mixture was hydrogenated (50 psi) for 14-24 h, filtered and concentrated *in vacuo*. The residue obtained, was purified by flash column chromatography to obtain the corresponding amidine.

### AJ-2: Amidine from nitrile

A mixture of nitrile (1 mmol) and saturated methanolic HCl solution (freshly prepared by bubbling HCl gas or prepared in-situ by premixing methanol and acetyl chloride at ice cold temperature) was stirred at room temperature overnight. The reaction mixture was concentrated *in vacuo* to furnish methyl imidate. To the residue of methyl imidate was added MeOH (40 mL) and ammonia gas was bubbled at reflux temperature for 16 h or till the reaction was complete. The reaction mixture was concentrated *in vacuo* and dried to furnish the desired amidine. Alternatively, the methyl imidate was dissolved in methanol and ammonium acetate (10 mmol) was added. The reaction mixture was concentrated in vacuo and purified by flash column chromatography to obtain the corresponding amidine.

### AJ-3: Amidine from nitrile

To a solution of nitrile (1 mmol) dissolved in methanol (5 mL) was added N-acetyl cystein (0.1 or 1 mmol) and ammonium acetate (5 mmol) and heated at reflux till the reaction was complete. The reaction mixture was concentrated in vacuo and purified by flash column chromatography to obtain the corresponding amidine.

### AK: Conversion of aryl triflates or halides to boronate ester

To dichloro[1,1'-bis(diphenylphosphino)ferrocene]palladium (II) dichloromethane adduct (0.75 mmol) under argon in dioxane (100 mL) was added aryl triflate (25 mmol), pinacolborane (31.5 mmol) and triethylamine (75 mmol). The reaction mixture was heated under argon at 100 °C for 3h or until complete as evidenced from TLC analysis. The reaction mixture was concentrated *in vacuo*. The residue obtained was purified by flash column chromatography to furnish the desired boronate ester. Alternatively, the following method can be used.

To dichloro[1,1'-bis(diphenylphosphino)ferrocene]palladium (II) dichloromethane adduct (0.03 mmol), 1,1'-bis(diphenylphosphino)ferrocene (0.03 mmol) under argon in dioxane (100 mL) was added aryl triflate (1 mmol), bis(pinacolata)diboron (1.1 mmol) and potassium acetate (3 mmol). The reaction mixture was heated under argon at 100 °C for 3h or until complete as evidenced from TLC analysis. The reaction mixture was concentrated *in vacuo*. The residue obtained was purified by flash column chromatography to furnish the desired boronate ester.

The examples of the compounds prepared are given in the following tables. The tables describe the compounds, their method of preparation, the starting material, and the analytical data. In some cases, where analytical data have not been given, those compounds were characterized at the later step in the synthesis.

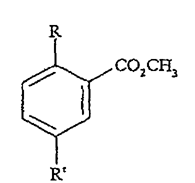

| **Cpd. No.** | **-R** | **-R'** | **Starting From** | **Method Used** | **Analytical Data** |
|---|---|---|---|---|---|
| **2a** | -OH | 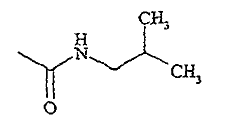 | **1** | A-1 or A-2 | ¹H NMR (DMSO-d₆): δ 10.26 (s, 1 H), 9.84 (s, 1 H), 8.15 (d, *J* = 3.0 Hz, 1 H), 7.64 (dd, *J* = 2.0 Hz and 8.9 Hz, 1 H), 6.94 (d, *J* = 8.9 Hz, 1 H), 3.90 (s, 3 H), 2.15 (d, *J* = 6.9 Hz, 2 H), 2.06 (m, *J* = 6.9 Hz, 1 H), 0.93 (d, *J* = 6.9 Hz, 1 H), 0.93 (d, J = 6 Hz, 6H); MS (ES⁺): 252.12 |
| **2b** | -OH | 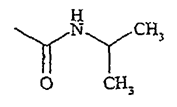 | **1** | A-1 or A-2 | Characterized in the next step |
| **2c** | -OH | 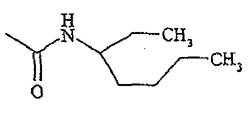 | **1** | A-1 or A-2 | MS (ES⁺): 294.54 |
| **2d** | -OH | 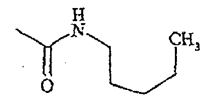 | **1** | A-1 or A-2 | MS (ES⁺): 288.49 (M+Na)⁺ |
| **2e** | -OH | 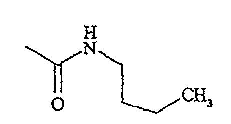 | **1** | A-1 or A-2 | Characterized in the next step |
| **2f** | -OH | 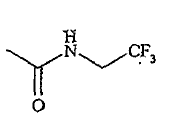 | **1** | A-1 or A-2 | MS (ES⁺): 300.40 (M+Na)⁺ |
| **2g** | -OH | 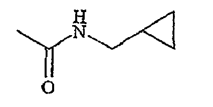 | **1** | A-1 or A-2 | MS (ES⁺): 272.48 (M+Na)⁺; MS (ES⁻): 248.66 |
| **2h** | -OH | 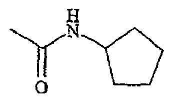 | **1** | A-1 or A-2 | MS (ES⁺): 286.48 (M+Na)⁺ |
| **2i** | -OH | 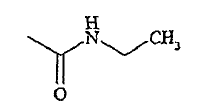 | **1** | A-1 or A-2 | MS (ES⁺): 224.54 |
| **2j** | -OH | 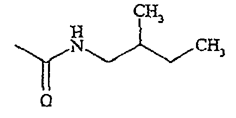 | **1** | A-1 or A-2 | Characterized in the next step |
| **3a** | -OSO₂CF₃ | 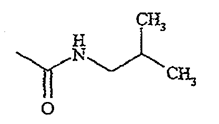 | **2a** | B-1 or B-2 | MS (ES⁺): 384.37 |
| **3b** | -OSO₂CF₃ | 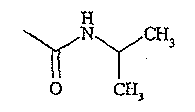 | **2b** | B-1 or B-2 | MS (ES⁺): 370.36 |
| **3c** | -OSO₂CF₃ | 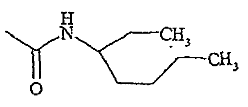 | **2c** | B-1 or B-2 | MS (ES⁺): 426.37 |
| **3d** | -OSO₂CF₃ | 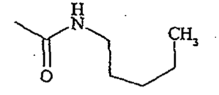 | **2d** | B-1 or B-2 | Characterized in the next step |
| **3e** | -OSO₂CF₃ | 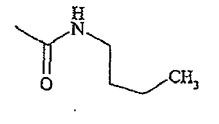 | **2e** | B-1 or B-2 | ¹H NMR (CDCl₃): δ 8.41 (d, *J* = 2.3 Hz, 1 H), 8.10 (dd, *J* = 8.5, 2.4 Hz, 1 H), 7.37 (d, *J* = 8.5 Hz, 1 H), 6.48 (broad, 1 H), 3.98 (s, 3 H), 3.46 (q, *J* = 7.2 Hz, 2 H), 1.62 (m, 2 H), 1.42 (m, 2H), 0.96 (t, *J =* 7.2 Hz, 3 H); MS (ES⁺): 384.1 |
| **3f** | -OSO₂CF₃ | 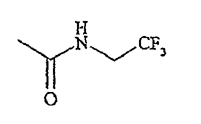 | **2f** | B-1 or B-2 | ¹H NMR (CDCl₃): δ 8.45 (d, *J* = 2.4 Hz, 1 H), 8.14 (dd, *J* = 8.7, 2.4 Hz, 1 H), 7.42 (d, *J* = 8.7 Hz, 1 H), 6.52 (broad, 1 H), 4.14 (m, 2 H), 4.00 (s, 3 H); MS (ES⁺): 410.2 |
| **3g** | -OSO₂CF₃ | 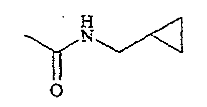 | **2g** | B-1 or B-2 | ¹H NMR (CDCl₃): δ 8.42 (d, *J* = 2.3 Hz, 1 H), 8.12 (dd, *J* = 8.5, 2.3 Hz, 1 H), 7.39 (d, *J* = 8.7 Hz, 1 H), 6.31 (broad, 1 H), 4.00 (s, 3 H), 3.34 (dd, *J* = 7.2, 5.5 Hz, 2 H), 1.07 (m, 1 H), 0.59 (m, 2 H), 0.30 (m, 2 H); MS (ES⁺): 382.2 |
| **3h** | -OSO₂CF₃ | 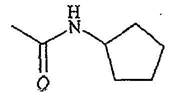 | **2h** | B-1 or B-2 | MS (ES⁺): 396.36 |
| **3i** | -OSO₂CF₃ | 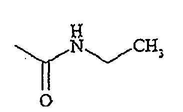 | **2i** | B-1 or B-2 | ¹H NMR (DMSO-*d₆*): δ 8.85 (t, *J* = 5.5 Hz, 1 H), 8.49 (d, *J* = 2.3 Hz, 1 H), 8.23 (dd, *J* = 8.7, 2.3 Hz, 1 H), 7.70 (d, *J* = 8.7 Hz, 1 H), 3.92 (s, 3 H), 3.31 (m, 2 H), 1.14 (t, *J* = 7.2 Hz, 3 H); MS (ES⁺): 356.1 |
| **3j** | -OSO₂CF₃ | 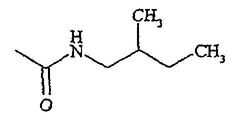 | **2j** | B-1 or B-2 | ¹H NMR (DMSO-*d₆*): δ 8.81 (t, *J* = 6.0 Hz, 1 H), 8.49 (d, *J* = 2.3 Hz, 1 H), 8.24 (dd, *J* = 8.7, 2.4 Hz, 1 H), 7.71 (d, *J* = 8.7 Hz, 1 H), 3.92 (s, 3 H), 3.15 (m, 2 H), 1.64 (m, 1 H), 1.41 (m, 1 H), 1.12 (m, 1 H), 0.88 (m, 6 H); MS (ES⁺): 398.2 |
| **5** | -OSO₂CF₃ | -CO₂MEM | **4** | B-2 | ¹H NMR (DMSO-d₆): δ 8.52 (d, *J* = 2.0 Hz, 1 H), 8.32 (dd, *J* = 2.0 and 8.9 Hz, 1 H), 7.72 (d, *J* = 7.9 Hz, 1 H), 5.50 (s, 2 H), 3.88 (s, 3 H), 3.78 (t, *J* = 4.9 Hz, 2 H), 3.44 (d, *J* = 4.9 Hz, 2 H), 3.17 (s, 3 H); MS (ES⁺): 439.1 (M+Na)⁺ |
| **6a** | 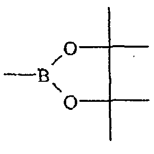 | 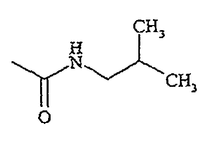 | **3a** | AK | ¹H NMR (CDCl₃): δ 8.29 (d, J = 1.6 Hz, 1 H), 7.96 (dd, J = 7.5 & 1.6 Hz, 1 H), 7.58 (d, J = 7.5 Hz, 1 H), 6.24 (bs, 1 H), 3.94 (s, 3 H), 3.30 (t, J = 6.5 Hz, 2 H), 1.92 (m, 1 H), 1.43 (s, 12 H), 0.99 (d, J = 6.5 Hz, 6 H); MS (ES+) 362.2 |
| **139** | -OH | 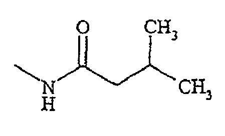 | **138** | AA | ¹H NMR (DMSO-d₆): δ 10.26 (s, 1 H), 9.84 (s, 1 H), 8.15 (d, *J* = 3.0 Hz, 1 H), 7.64 (dd, *J* = 2.0 Hz and 8.9 Hz, 1 H), 6.94 (d, *J* = 8.9 Hz, 1 H), 3.90 (s, 3 H), 2.15 (d, *J* = 6.9 Hz, 2 H), 2.06 (m, *J* = 6.9 Hz, 1 H), 0.93 (d, *J* = 6.9 Hz, 6 H); MS (ES⁺): 252.12 |
| **140** | -OSO₂CF₃ | 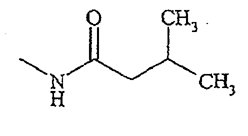 | **139** | B-2 | ¹H NMR (DMSO-d₆): δ 10.38 (s, 1 H), 8.36 (d, *J* = 2.8 Hz, 1 H), 7.99 (dd, *J* = 2.6 and 8.9 Hz, 1 H), 7.52 (d, *J* = 9.0 Hz, 1 H), 3.89 (s, 3 H), 2.23 (d, *J* = 7.0 Hz, 2 H), 2.09 (m, *J* = 6.6 Hz, 1 H), 0.94 (d, *J* = 6.6 Hz, 6 H); MS (ES⁺): 384.0 |
| **169** | -OH | 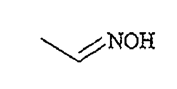 | **168** | AC | ¹H NMR (CDCl₃): δ 8.08 (s, 1 H), 8.00 (d, *J* = 2.3 Hz, 1 H), 7.75 (dd, *J* = 2.3 and 8.7 Hz, 1 H), 7.01 (d, *J* = 8.7 Hz, 1 H), 3.97 (s, 3 H), 3.50 (s, 1 H); MS (ES⁺): 196.1 |
| **170** | -OH | -CH₂NH₂ | **169** | G | ¹H NMR (DMSO-*d₆*): δ 7.79 (d, *J* = 2.0 Hz, 1 H), 7.5 (dd, *J* = 2.3 and 8.5 Hz, 1 H), 6.95 (d, *J* = 8.5 Hz, 1 H), 7.0 (d, *J* = 8.7 Hz, 1 H), 3.90 (s, 3 H), 3.72 (s, 2 H), 3.50 (bs, 2H); MS (ES⁺): 182.12 |
| **171** | -OH | 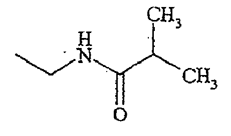 | **170** | AA | MS (ES⁻): 250.50; MS (ES⁺): 274.50 (M+Na)⁺ |
| **172** | -OSO₂CF₃ | 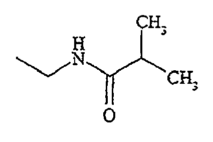 | **171** | B-2 | ¹H NMR (CDCl₃): δ 7.96 (d, *J* = 2.3 Hz, 1 H), 7.55 (d, *J* = 2.3 and 8.3 Hz, 1 H), 7.26 (d, *J* = 8.3 Hz, 1 H), 5.90 (br s, 1 H), 4.50 (d, *J* = 4.1 Hz, 2 H), 3.97 (s, 3 H), 2.44 (sep, *J* = 7.0 Hz, 1 H), 1.20 (d, *J* = 7.0 Hz, 6 H); MS (ES⁺): 384.1 |
| **177** | -OH | 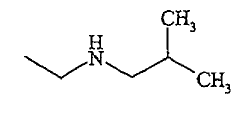 | **168** | AE-1 | ¹H NMR (DMSO-d₆): δ 10.62 (s, 1 H), 8.88 (m, 2 H), 7.99 (d, *J* = 2.3 Hz, 1 H), 7.70 (dd, *J* = 2.3 and 8.5 Hz, 1 H), 7.06 (d, *J* = 8.7 Hz, 1 H), 4.09 (m, 2 H), 3.91 (s, 3 H), 2.70 (m, 2 H), 1.98 (m, 1 H, *J* = 6.8 Hz), 0.93 (d, *J* = 6.8 Hz, 6 H); MS (ES⁺): 238.1 |
| **178** | -OSO₂CF₃ | 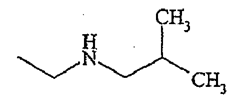 | **177** | B-2 | ¹H NMR (CDCl₃): δ 8.05 (d, *J* = 2.3 Hz, 1 H), 7.63 (dd, *J* = 2.3 and 8.3 Hz, 1 H), 7.25 (d, *J* = 8.3 Hz, 1 H), 3.96 (s, 3 H), 3.85 (s, 2 H), 2.43 (d, *J* = 6.8 Hz, 2 H), 1.77 (m, *J* = 6.6 Hz, 1 H), 0.93 (d, *J* = 6.6 Hz, 1 H); MS (ES⁺): 370.2 |
| **179** | -OSO₂CF₃ | 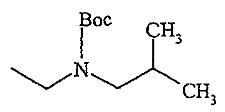 | **178** | R | ¹H NMR (DMSO-d₆): δ 7.93 (m, 1 H), 7.47 (m, 1 H), 7.26 (m, 1 H), 4.48 (m, 2 H), 3.96 (s, 3 H), 3.03 (m, 2 H), 1.91 (m, 1 H), 1.52 (m, 9 H), 0.89 (d, *J* = 6.6 Hz, 6 H); MS (ES⁺): 492.2 (M+Na)⁺ |

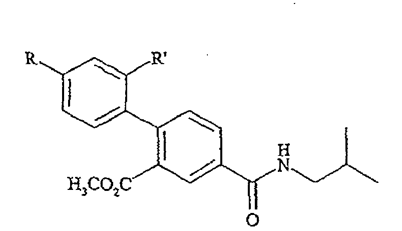

| **Cpd. No.** | **-R** | **-R'** | **Starting From** | **Method Used** | **Analytical Data** |
|---|---|---|---|---|---|
| **7** | -OBn | -CHO | **6 + 3a** | D-2 | ¹H NMR (DMSO-d6): δ9.78 (s, 1H), 8.85 (t, J = 5.7 Hz, 1H), 8.50 (d, J = 2.0 Hz, 1H), 8.20 (dd, J = 8.2, 1.9 Hz, 1H), 7.55 (m, 9H), 5.35 (s, 2H), 3.69 (s, 3H), 3.23 (t, J = 6.5 Hz, 2H), 1.98 (m, 1H), 1.02 (d, J = 6.8 Hz, 6H); MS (ES+): 446.3 |
| **8** | -OBn | -CO₂H | **7** | E | MS (ES⁺): 484.33 (M+Na)⁺ |
| **9** | -OBn | -CO₂MEM | **8** | F | MS (ES⁺): 572.2 (M+Na)⁺ |
| **10** | -OH | -CO₂MEM | **9** | G | MS (ES⁺): 482.33 [(M-MEM) = Na]⁺ |
| **11** | -OSO₂CF₃ | -CO₂MEM | **10** | B-2 | ¹H NMR (DMSO-d6): δ8.75 (t, J = 5.6 Hz, 1 H), 8.44 (d, J =1.6 Hz, 1H), 8.11 (dd, J = 8.0, 1.9 Hz, 1H), 8.01 (d, J = 2.9 Hz, 1H), 7.84 (dd, J = 8.4, 2.6 Hz, 1H), 7.47 (d, J = 8.5 Hz, 1H), 7.41 (d, J = 8.0 Hz, 1H), 5.23 (q, AB system, 2H), 3.59 (s, 3H), 3.44 (m, 2H), 3.30 (m, 2H), 3.18 (s, 3H), 3.13(t, J =6.6 Hz, 2H), 1.88 (m, 1H), 0.91 (d, J = 6.7 Hz, 6H); MS (ES+): 614.3 (M+Na)⁺ |
| **29a** | 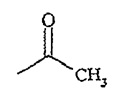 | -CO₂MEM | 11 | D-3 | Characterized in the next step |
| **29b** | 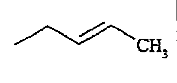 | -CO₂MEM | **11** | D-3 | MS (ES⁺): 520.2 (M+Na)⁺ |
| **29c** | 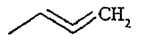 | -CO₂MEM | **11** | D-3 | MS (ES⁺): 482.3 |
| **29d** | 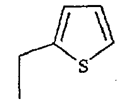 | -CO₂MEM | **11** | D-3 | MS (ES⁺): 562.3 (M+Na)⁺ |
| **29e** | 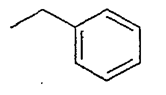 | -CO₂MEM | **11** | D-3 | MS (ES⁺): 556.4 (M+Na)⁺ |
| **29f** | 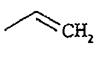 | -CO₂MEM | **11** | D-3 | ¹H NMR (DMSO-d6): δ8.50 (t, J = 5.6 Hz, 1 H), 8.18 (d, J = 1.9 Hz, 1H), 7.86 (dd, J = 7.9, 1.9 Hz, 1H), 7.78 (d, J =1.7 Hz, 1H), 7.56 (dd, J = 8.0, 1.8 Hz, 1H), 7.13 (d, J = 8.0 Hz, 1H), 7.00 (d, J = 7.9 Hz, 1H), 6.67 (dd, J =17.6, 11.1 Hz, 1H), 5.76 (d, J = 17.6 Hz, 1H), 5.19 (d, J = 11.1 Hz, 1H), 4.99 (q, AB system, 2H), 3.37 (s, 3H), 3.20 (m, 2H), 3.11 (m, 2H), 2.97 (s, 3H), 2.91 (t, J = 6.7 Hz, 2H), 1.67 (m, 1H), 0.70 (d, J = 6.6 Hz, 6H); MS (ES+): 492.3 (M+Na)⁺ |
| **29g** | 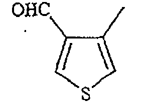 | -CO₂MEM | **11** | D-2 | MS (ES⁺): 576.2 (M+Na)⁺; MS (ES⁻): 552.2 |
| **29h** | 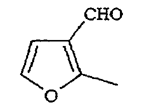 | -CO₂MEM | **11** | D-2 | MS (ES⁺): 538.2 |
| **29i** | 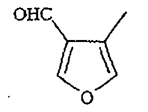 | -CO₂MEM | **11** | D-2 | MS (ES⁺): 560.4 (M+Na)⁺ |
| **30a** | 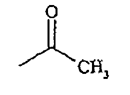 | -CO₂H | **29a** | I-1 | MS (ES⁺): 398.3 ; MS (ES⁻) 396.3 |
| **30b** | 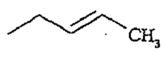 | -CO₂H | **29b** | I-1 | Characterized in the next step |
| **30c** | 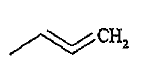 | -CO₂H | **29c** | I-1 | MS (ES⁻): 392.1 |
| **30d** | 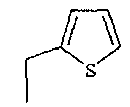 | -CO₂H | **29d** | I-1 | MS (ES⁺): 452.1 |
| **30e** | 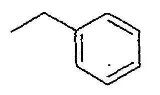 | -CO₂H | **29e** | I-1 | MS (ES⁺): 446.2 |
| **30f** | 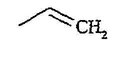 | -CO₂H | **29f** | I-1 | MS (ES⁻): 380.1 |
| **30g** | 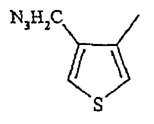 | -CO₂H | **29g** | K, N, O, I-1 | MS (ES⁺): 515.3 (M+Na)⁺; MS (ES⁻): 491.2 |
| **30h** | 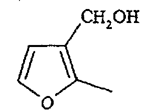 | -CO₂H | **29h** | K, I-1 | MS (ES⁻): 450.1 |
| **30i** | 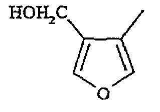 | -CO₂H | **29i** | K, I-1 | MS (ES⁻): 450.3 |
| **33** | -OSO₂CF₃ | -CO₂H | **11** | I-1 | Characterized in the next step |
| **41** | 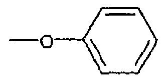 | -CO₂MEM | **10** | D-8 | MS (ES⁻): 534.30 |
| **42** | 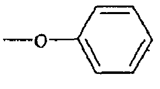 | -CO₂H | **41** | I-1 | MS (ES⁻): 446.30 |
| **48** | -OCH₃ | -CHO | **47 + 3a** | D-2 | MS (ES⁺): 392.2 (M+Na)^{÷} |
| **49** | -OCH₃ | -CO₂H | **48** | E | MS (ES⁺): 386.1; 408.1 (M+Na)^{÷} |

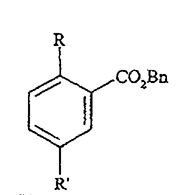

| **Cpd. No.** | **-R** | **-R'** | **Starting From** | **Method Used** | **Analytical Data** |
|---|---|---|---|---|---|
| **14** | -OSO₂CF₃ | -CHO | **13** | B-2 | Characterized in the next step |
| **15** | -OSO₂CF₃ | -CO₂H | **14** | E | MS (ES⁻): 403.58 |
| **16** | -OSO₂CF₃ | 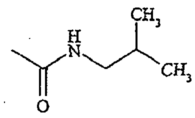 | **15** | A-3 or A-4 | ¹HN^{M}R (DMSO-d₆): δ 8.83 (t, J = 6 Hz, 1 H), 8.49 (d, J = 2.6 Hz, 1 H), 8.23 (dd, J = 8.6 Hz, 1 H), 7.72 (d, J = 8.6 Hz, 1 H), 7.49 (m, 2 H), 7.41 (m, 3 H), 5.43 (s, 2 H), 3.1 (t, J = 6.9 Hz, 2 H), 2.29 (m, 1 H), 0.89 (d, J = 6.9 Hz, 6 H). |

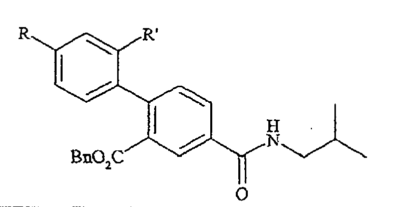

| **Cpd. No.** | **-R** | **-R'** | **Starting From** | **Method Used** | **Analytical Data** |
|---|---|---|---|---|---|
| **17** | -OBn | -CHO | **16+6** | D-2 | ¹HNMR (DMSO-d₆): δ 0.88 (d, J = 6.0 Hz, 6 H), 1.85 (m, 1 H), 3.1 (t, J = 6.0 Hz, 2 H), 5.02 (q, J = 13 and 2.5 Hz, 2 H), 5.18 (s, 2 H), 6.88 (m, 2 H), 7.17 (d, J = 8.6 Hz, 1 H), 7.26 (m, 4 H), 7.35 (m, 1 H), 7.40 (m, 4 H), 7.49 (d, J = 7.7 Hz, 2 H), 8.07 (dd, J = 7.7 and 1.7 Hz, 1 H), 8.38 (d, J = 1.7 Hz, 1 H), 8.72 (t, J = 6 Hz, 1 H), 9.63 (s, 1 H); MS (ES⁺):522.89 |
| **18** | -OBn | -CO₂H | **17** | E | ¹HNMR (DMSO-d₆): δ 0.86 (d, J = 6.9 Hz, 6 H), 1.85 (m, 1 H), 3.09 (t, J = 6.9 Hz, 2 H), 5.01 (d, J = 5.01 Hz, 2 H), 5.14 (s, 2 H), 7.08 (m, 3 H), 7.14 (dd, J = 8.6 and 2.6 Hz, 1 H), 7.27 (m, 4 H), 7.34 (m, 1 H), 7.41 (m, 3 H), 7.48 (m, 2 H), 7.99 (dd, J = 6.9 and 1.8 Hz, 1 H), 8.32 (s. 1 H), 8.64 (t, J = 6 Hz, 1 H), 12.57 (s, 1 H); MS (ES+):538.86 |
| **19** | -OBn | -CO₂MEM | **18** | F | ¹HNMR (DMSO-d₆): δ 0.90 (d, J = 6.8 Hz, 6 H), 1.86 (m, 1 H), 3.10 (t, J = 6.5 Hz, 2 H), 3.16 (s, 3 H), 3.28 (dd, J = 3 and 6 Hz, 2 H), 3.36 (dd, J = 3 and 6 Hz, 2 H), 5.02 (d, J = 3.8 Hz, 2 H), 5.12 (d, J = 15 Hz, 2 H), 5.64 (s, 2 H), 7.11 (m, 3 H), 7.24 (dd, J = 8.25 and 2.75 Hz, 1 H), 7.29 (m, 4 H), 7.35 (m, 1 H), 7.42 (m, 3 H), 7.49 (m, 2 H), 8.02 (dd, J = 1.7 and 8.2 Hz, 1 H), 8.36 (d, 1.7 Hz, 1 H), 8.68 (t, J = 6 Hz, 1 H); MS (ES+): 626.44 |
| **21** | -OH | -CO₂MEM | **19** | G, H | ¹HNMR (DMSO-d₆): δ 0.88 (d, J = 6 Hz, 6 H), 1.85 (m, 1 H) 3.10 (t, J = 6 Hz, 2 H) 3.16 (s, 3 H), 3.28 (m 2 H), 3.35 (m, 2 H), 5.04 (d, J = 3.5 Hz, 2H) 5.11 (d, J = 14 Hz, 2 H), 6.98 (m, 2 H), 7.11 m, 2 H), 7.29 (m, 5 H), 8.03 (dd, J = 8 and 2 Hz, 1 H), 8.32 (d, J = 2 Hz, 1 H), 8.67 (t, J = 6 Hz, 1 H), 9.9 (s, 1 H); MS (ES+) 536.30 (100%_{:} M⁺¹) |
| **22** | -OSO₂CF₃ | -CO₂MEM | **21** | B-2 | ¹HNMR (DMSO-d₆): δ 0.89 (d, J = 6.8 Hz, 6 H), 1.86 (m, 1 H), 3.12 (t, J = 6.5 Hz, 2 H), 3.16 (s, 3 H), 3.29 (m, 2 H), 3.40 (m, 2 H), 5.04 (s, 2 H), 5.16 (dd, J = 18 and 6 Hz, 2 H), 7.15 (m, 2 H), 7.31 (m, 3 H), 7.36 (d, J = 8.5 Hz, 1 H), 7.41 (d, J = 8.5 Hz, 1 H), 7.73 (dd, J = 8.6 and 2.6 Hz, 1 H), 7.85 (d, J = 2.6 Hz, 1 H), 8.07 (dd, J = 7.7and 1.7 Hz, 1 H), 8.45 (d, J = 1.7 Hz, 1 H), 8.73 (t, J = 6 Hz, 1 H); MS (ES+) 668.15 |
| **24a** | 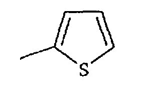 | -CO₂MEM | **22 + 23** | D-1 | ¹HNMR (DMSO-d₆): δ 0.89 (d, J = 6.8 Hz, 6 H), 1.87 (m, 1 H), 3.12 (t, J = 6 Hz, 2 H), 3.16 (s, 3 H), 3.29 (m, 2 H), 3.39 (m, 2 H), 5.05 (d, J = 2.6 Hz, 2 H), 5.16 (d, J = 17 Hz, 2 H), 7.08 (m, 2 H), 7.21 (m, 4 H), 7.24 (d, J = 7.7 Hz, 1 H), 7.35 (d, J = 7.7 Hz, 1 H), 7.62 (d, J = 3.5 Hz, 1 H), 7.64 (d, J = 5 Hz, 1 H), 7.86 (d, J = 8.6 Hz, 1 H), 8.06 (m, 2 H), 8.42 (s, 1 H), 8.73 (t, J = 6 Hz, 1 H); MS (ES+) 602.52 |
| **24b** | 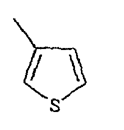 | -CO₂MEM | **22 + 23** | D-1 | ¹HNMR (DMSO-d₆): δ 0.89 (d, J = 6.8 Hz, 6 H), 1.87 (m, 1 H), 3.12 (t, J = 6 and 6.8 Hz, 2 H), 3.16 (s, 3 H), 3.30 (m, 2 H), 3.39 (dd, J = 5.2 and 3.4 Hz, 2 H), 5.04 (d, J = 4.3 Hz, 2 H), 5.16 (d, J = 16 Hz, 2 H), 7.08 (m, 2 H), 7.20 (m, 3 H), 7.24 (d, J = 8.6 Hz, 1 H), 7.35 (d, J = 8.6 Hz, 1 H), 7.61 (d, J = 5 Hz, 1H), 7.71 (dd, J = 4.8 and 3 Hz, 1 H), 7.91 (dd, J = 1.7and 7.7 Hz, 1 H), 8.00 (m, 1 H), 8.06 (dd, J = 2 and 8 Hz, 1 H), 8.14 (d, J = 1.7 Hz, 1 H), 8.41 (d, J = 1.7 Hz, 1 H), 8.68 (t, J = 6 Hz, 1 H); MS (ES+) 602.27 |
| **24c** | 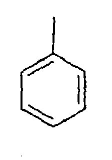 | -CO₂MEM | **22 + 23** | D-1 | ¹HNMR (DMSO-d₆): δ 0.89 (d, J = 6.8 Hz, 6 H), 1.87 (m, 1 H), 3.12 (t, J = 6 and 6.8 Hz, 2 H), 3.16 (s, 3 H), 3.30 (m, 2 H), 3.40 (m, 2 H), 5.05 (d, J = 5 Hz, 2 H), 5.17 (d, J = 17 Hz, 2 H), 7.09 (m, 2 H), 7.21 (m, 3 H), 7.30 (d, J = 7.7 Hz, 1 H), 7.37 (d, J = 7.7 Hz, 1 H), 7.44 (m, 1 H), 7.54 (t, J = 7.7 Hz, 2 H), 7.73 (d, J = 6.8 Hz, 2 H), 7.88 (dd, J =1.7 and 7.7 Hz, 1 H), 8.07 (dd, J = 7.7 and 1.7 Hz, 1 H), 8.11 (d, J = 1.7 Hz, 1 H), 8.42 (d, J = 1.7 Hz, 1 H), 8.72 (t, J = 6 Hz, 1 H); MS (ES+) 596.45 |
| **24d** | 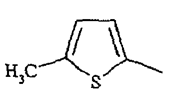 | -CO₂MEM | **22 + 23** | D-1 | MS (ES+) 616 |
| **24e** | 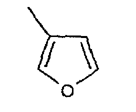 | -CO₂MEM | **22 + 23** | D-1 | MS (ES+) 586.4 |
| **24f** | 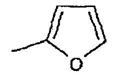 | -CO₂MEM | **22 + 23** | D-1 | MS (ES⁺): 586.39 |
| **24g** | 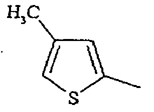 | -CO₂MEM -CO₂MEM | **22 + 23** | D-1 | MS (ES⁺): 616.63 |
| **24h** | 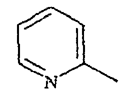 | -CO₂MEM | **22 + 23** | D-1 | MS (BS⁺): 597.25 |
| **24i** | 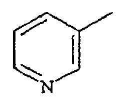 | -CO₂MEM | **22 + 23** | D-1 | MS (ES⁺): 597.4 |
| **24j** | 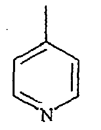 | -CO₂MEM | **22 + 23** | D-1 | MS (ES⁺): 597.4 |
| **24k** | 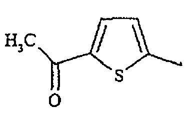 | -CO₂MEM | **22 + 23** | D-1 | MS (ES⁺): 644.3 |
| **241** | 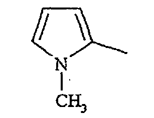 | -CO₂MEM | **22 + 23** | D-3 | Characterized at the next step |
| **24m** | 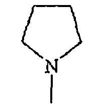 | -CO₂MEM | **22+23** | D-10 | Characterized at the next step |
| **24n** | 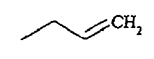 | -CO₂MEM | **22 + 23** | D-3 | MS (ES⁺): 560.74 |
| **24o** | 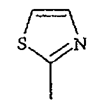 | -CO₂MEM | **22 + 23** | D-4 | MS (ES⁺): 603.72 |
| **24p** | 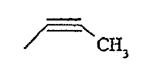 | -CO₂MEM | **22 + 23** | D-5 | MS (ES⁺): 558.3 |
| **24q** | 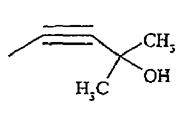 | -CO₂MEM | **22+23** | D-5 | Characterized in the next step |
| **24r** | 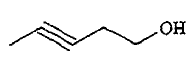 | -CO₂MEM | **22 + 23** | D-5 | MS (ES⁺): 610.4 (M+Na)⁺ |
| **24s** | 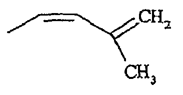 | -CO₂MEM | **22 + 3** | D-3 | Characterized in the next step |
| **24t** | 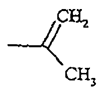 | -CO₂MEM | **22 + 23** | D-3 | Characterized in the next step |
| **24u** | 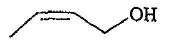 | -CO₂MEM | **22 + 23** | D-3 | MS (ES⁺): 598.4 (M+Na)⁺ |
| **24v** | 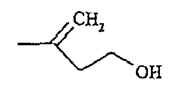 | -CO₂MEM | **22 + 23** | D-3 | MS (ES⁺): 500.4 [(M-MEM)-1]⁻ |
| **24w** | 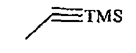 | -CO₂MEM | **22 + 23** | D-5 | Characterized in the next step |
| **24x** | 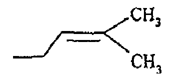 | -CO₂MEM | **22 + 23** | D-3 | MS (ES⁺): 610.5 (M+Na)⁺ |
| **24y** | 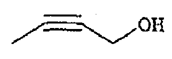 | -CO₂MEM | **22 + 23** | D-5 | MS (ES⁺): 596.4 (M+Na)⁺ |
| **24z** | 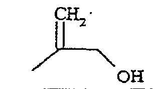 | -CO₂MEM | **22 + 23** | D-3 | MS (ES⁺): 576.3 (M+Na)⁺ |
| **24aa** | 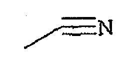 | -CO₂MEM | **22 + 23** | D-11 | Characterized in the next step |
| **24ab** | 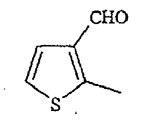 | -CO₂MEM | **22 + 23** | D-2 | MS (ES⁺): 630.55 |
| **24ac** | 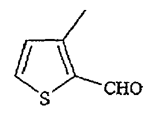 | -CO₂MEM | **22 + 23** | D-2 | MS (ES⁺): 630.74 |
| **24ad** | 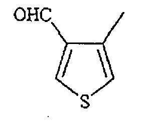 | -CO₂MEM | **22 + 23** | D-2 | MS (ES⁺): 652.3 |
| **24ae** | 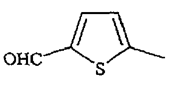 | -CO₂MEM | **22 + 23** | D-2 | Characterized in the next step |
| **24ag** | 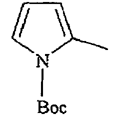 | -CO₂MEM | **22 + 23** | D-1 | MS (ES⁺): 685.01 |
| **24ah** | 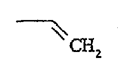 | -CO₂MEM | **22 + 23** | D-3 | MS (ES⁺): 546.49 |
| **25a** | 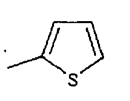 | CO₂H | **24a** | I-1 | ¹HNMR (DMSO-d₆): δ 0.91 (d, J = 6.9 Hz, 6 H), 1.88 (m, 1 H), 3.13 (t, J = 6.9 and 6 Hz, 2 H), 5.07 (d, J = 11.2 Hz, 2 H), 7.09 (m, 2 H), 7.22 (m, 5 H), 7.35 (d, 7.7 Hz, 1 H), 7.63 (d, 2.6 Hz, 1 H), 7.65 (d, J = 5.2 Hz, 1 H), 7.82 (dd, J = 7.7 and 1.7 Hz, 1 H), 8.05 (d, J = 1.7 Hz, 1 H), 8.07 (s, 1 H), 8.40 (s, 1 H), 8.72 (t, J = 6 Hz, 1 H), 12.77 (bis, 1 H); MS (ES+) 514.19 |
| **25b** | 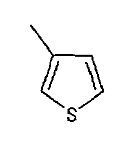 | CO₂H | **24b** | I-1 | ¹HNMR (DMSO-d₆). δ 0.92 (d, J = 6.9 Hz, 6 H), 1.88 (m, 1 H), 3.12 (t, J = 6.9 and 6 Hz, 2 H), 5.07 (d, J = 13 Hz, 2 H), 7.09 (m, 2 H), 7.22 (m, 4 H), 7.35 (d, J = 8.6 Hz, 1 H), 7.63 (d, J = 5.2 Hz, 1 H), 7.70 (dd, J = 2.6 and 4.3 Hz, 1 H), 7.88 (dd, J = 7.2 and 1.7 Hz, 1 H), 8.02 (d, J = 1.7 Hz, 1 H), 8.07 (dd, J =1.7 and 7.7 Hz, 1 H), 8.15 (m, 1 H), 8.39 (d, J = 1.7 Hz, 1 H), 8.72 (t, J = 6 Hz, 1 H), 12.70 (brs, 1 H); MS (ES+) 514.06 |
| **25c** | 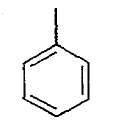 | CO₂H | **24c** | I-1 | ¹HNMR (DMSO-d₆): δ 12.73 (bs, 1 H), 8.73 (t, J = 6 Hz, 1 H), 8.41 (d, J = 1.7 Hz, 1 H), 8.12 (d, J = 1.7 Hz, 1 H), 8.07 (dd, J = 7.7 & 1.7 Hz, 1 H), 7.83 (dd, J = 7.7 & 1.7 Hz, 1 H), 7.72 (d, J = 6.9 Hz, 2 H), 7.54 (t, J = 7.7, 2 H), 7.44 (t, J = 7.7 Hz, 1 H), 7.37 (d, J = 7.7 Hz, 1 H), 7.28 (d, J = 7.7 Hz, 1 H), 7.21 (m, 3 H), 7.09 (m, 2 H), 5.08 (d, J = 14 Hz, 2 H), 3.13 (t, J = 6.5 Hz, 2 H), 1.88 (m, 1 H), 0.91 (d, 6.8 Hz, 6 H); MS (ES+) 507.93 |
| **25d** | 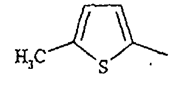 | CO₂H | **24d** | I-1 | ¹HNMR (DMSO-d₆): δ 12.75 (bs, 1 H), 8.71 (t, J = 6 Hz, 1 H), 8.39 (d, J = 1.7 Hz, 1 H), 8.05 (dd, J = 1.7 & 7.7 Hz, 1 H), 8.01 (d, J = 2.5 Hz, 1 H), 7.75 (dd, J = 2.5 & 7.7 Hz, 1 H), 7.42 (d, 3.4 Hz, 1 H), 7.34 (d, J = 7.7 Hz, 1 H), 7.22 (m, 3 H), 7.19 (d, J = 8.6 Hz, 1 H), 7.09 (m, 2 H), 6.95 (d, J = 3.4 Hz, 1 H), 5.06 (d, J = 11 Hz, 2 H), 3.12 (t, J = 6.5 Hz, 2 H), 2.52 (s, 3 H), 1.89 (m, 1 H), 0.81 (d, 6.8 Hz, 6 H); MS (ES+) 528.51 |
| **25e** | 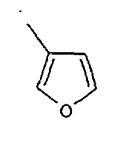 | CO₂H | **24e** | I-1 | ¹HNMR (DMSO-d₆): δ 0.89 (d, J = 6 Hz, 6 H), 1.86 (m, 1 H), 3.12 (t, J = 6.8 and 6.0 Hz, 2 H), 5.03 (d, J = 10 Hz, 2 H), 7.02 (s, 1 H), 7.06 (m, 2 H), 7.16 (d, J = 8.6 Hz, 1 H), 7.21 (m, 3 H), 7.31 (d, J = 7.7 Hz, 1 H), 7.75 (dd, J = 8.5 and 1.7 Hz, 1 H), 7.78 (t, J = 1.7 Hz, 1 H), 8.04 (m, 2 H), 8.29 (s, 1 H), 8.36 (d, J = 1.7 Hz, 1 H), 8.66 (t, J = 6 and 5.2 Hz, 1 H), 12.58 (bs, 1 H); MS (ES+) 498.49 |
| **25f** | 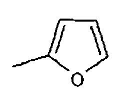 | CO₂H | **24f** | I-1 | MS (ES⁺): 498.36 |
| **25g** | 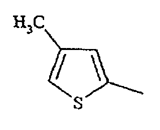 | CO₂H | **24g** | I-1 | ¹HNMR (DMSO-d₆): δ 12.72 (bs, 1 H), 8.69 (t, J = 6 Hz, 1 H), 8.39 (d, J = 1.7 Hz, 1 H), 8.06 (m, 2 H), 7.79 (dd, J = 1.7 & 7.7 Hz, 1 H), 7.45 (s, 1 H), 7.35 (d, J = 7.7 Hz, 1 H), 7.21 (m, 5 H), 7.1 (m, 2 H), 5.07 (d, J = 8.6 Hz, 2 H), 3.12 (t, J = 6.5 Hz, 2 H), 2.29 (s, 3 H), 1.89 (m, 1 H), 0.91 (d, 6.8 Hz, 6 H); MS (ES+) 528.38 |
| **25h** | 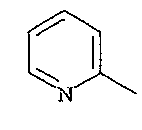 | CO₂H | **24h** | I-1 | ¹HNMR (DMSO-d₆): δ 12.74 (bs, 1 H), 8.73 (m, 2 H), 8.63 (d, J = 1.7 Hz, 1 H), 8.41 (d, J = 1.7 Hz, 1 H), 8.23 (dd, J = 1.7 and 7.7 Hz 1 H), 8.08 (dd, J = 1.7 & 7.7 Hz, 1 H), 8.05 (d, J = 7.7 Hz, 1 H), 7.96 (dt, J = 7.7 & 1.7 Hz, 1 H), 7.43 (dd, J = 6 & 7 Hz, 1 H), 7.37 (d, J = 7.7 Hz, 1 H), 7.29 (d, J = 8.6 Hz, 1 H), 7.18 (m, 3 H), 7.08 (m, 2 H), 5.01 (q, J = 10 & 25 Hz, 2 H), 3.13 (t, J = 6.9 and 6 Hz, 2 H), 1.89 (m, 1 H), 0.92 (d, J = 6.9 Hz, 6 H); MS (ES+) 509.58 |
| **25i** | 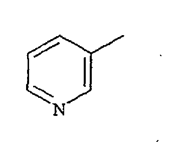 | CO₂H | **24i** | I-1 | ¹HNMR (DMSO-d₆): δ 12.70 (bs, 1 H), 8.91 (d, J = 2.6 Hz, 1 H), 8.68 (t, J = 6 & Hz, 1 H), 8.62 (d, J = 2 Hz, 1 H), 8.4 (d, J = 1.7 Hz, 1 H), 8.12 (m, 2 H), 8.05 (dd, J = 8.6 & 1.7 Hz, 1 H), 7.88 (d, 8.5 & 1.7 Hz, 1 H), 7.53 (dd, J = 8.6 & 5.2 Hz, 1 H), 7.34 (d, J = 7.7 Hz, 1 H), 7.28 (d, J = 8.6 Hz, 1 H), 7.18 (m, 3 H), 7.08 (m, 2 H), 5.04 (d, J = 12 Hz, 2 H), 3.11 (t, J = 6.5 Hz, 2 H), 1.87 (m, 1 H), 0.9 (d, 6.8 Hz, 6 H); MS (ES⁺) 509.11 |
| **25j** | 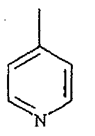 | CO₂H | **24j** | I-1 | ¹HNMR (DMSO-d₆): δ 0.90 (d, J = 6.9 Hz, 6 H), 1.88 (m, 1 H), 3.11 (t, J = 6.9 and 6 Hz, 2 H), 5.03 (s, 2 H), 7.06 (m, 2H), 7.18 (m, 3 H), 7.33 (d, 8.4 Hz, 1 H), 7.30 (d, J = 8.4 Hz, 1 H), 7.75 (d, J = 6.2 Hz, 2 H), 7.85 (m, 1 H), 8.05 (dd, J = 7.6 and 1.7 Hz, 1 H), 8.18 (s, 1 H), 8.40 (d, J = 2 Hz, 1 H), 8.71 (m, 4 H); MS (ES+) 509.49 |
| **25k** | 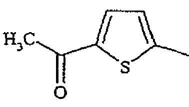 | CO₂H | **24K** | I-1 | Characterized in the next step |
| **25l** | 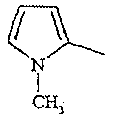 | CO₂H | **241** | I-1 | MS (FS⁺): 511.54 |
| **25m** | 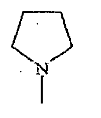 | CO₂H | **24m** | I-1 | MS (ES⁺): 501.66 |
| **25n** | 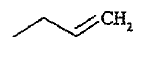 | CO₂H | **24n** | I-1 | MS (ES⁺): 472.4 |
| **25o** | 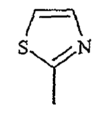 | CO₂H | **24o** | I-1 | MS (ES⁺): 515.65 |
| **25p** | 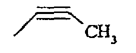 | CO₂H | **24p** | I-1 | Characterized in the next step |
| **25q** | 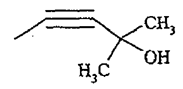 | CO₂H | **24q** | I-1 | MS (ES⁺): 536.3 (M+Na)⁺ |
| **25r** | 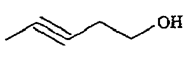 | CO₂H | **24r** | I-1 | MS (ES⁻): 500.4 |
| **25s** | 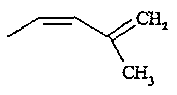 | CO₂H | **24s** | I-1 | Characterized in the next step |
| **25t** | 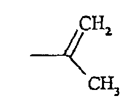 | CO₂H | **24t** | I-1 | Characterized in the next step |
| **25u** | 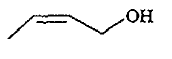 | CO₂H | **24u** | I-1 | MS (ES⁻): 486.4 |
| **25v** | 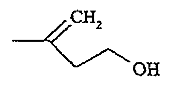 | CO₂H | **24v** | I-1 | MS (ES⁺): 524.3 (M+Na)⁺ |
| **25w** | 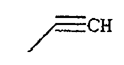 | CO₂H | **24w** | I-1, Q | Characterized in the next step |
| **25x** | 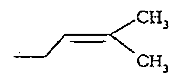 | CO₂H | **24x** | I-1 | MS (ES⁻): 498.3 |
| **25y** | 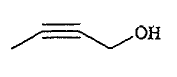 | CO₂H | **24y** | I-1 | MS (ES⁻): 484.3 |
| **25z** | 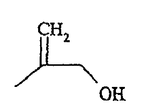 | CO₂H | **24z** | I-1 | MS (ES⁺): 488.3 |
| **25aa** | 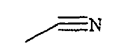 | CO₂H | **24aa** | I-1 | Characterized in the next step |
| **25ab** | 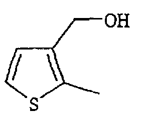 | CO₂H | **24ab** | K, I-1 | MS (ES⁻): 544.27 |
| **25ac** | 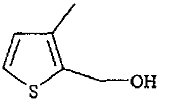 | CO₂H | **24ac** | K, I-1 | MS (ES⁺): 544.2 |
| **25ad** | 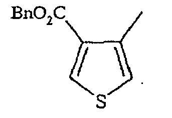 | CO₂H | **24ad** | E, H, I-1 | MS (ES⁺): 670.3 (M+Na)⁺ |
| **25ae** | 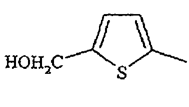 | CO₂H | **24ae** | K, I-1 | ¹HNMR (DMSO-d₆): δ 9.1 (bs, 2 H), 8.8 (bs, 2 H), 8.5 (t, J = 6 Hz, 1 H), 8.02 (s, 1 H), 7.68 (s, 1 H), 7.62 (m, 6 H), 7.53 (d, J = 5.8 Hz, 1 H), 7.15 (d, J = 6 Hz, 1 H),), 7.13 (m, 1 H), 7.01 (s, 1 H), 5.5 (t, J = 5 Hz, 1 H), 4.7 (d, J = 5 Hz, 2 H), 3.01 (m, 2 H), 1.8 (m, 1 H), 0.85 (d, J = 6.8 Hz, 6 H) |
| **25af** | 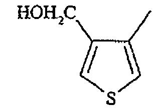 | CO₂H | **24ad** | K, I-1 | MS (ES⁺): 566.2 (M+Na)⁺ |
| **25ag** | 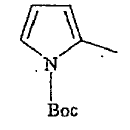 | CO₂H | **24ag** | I-1 | MS (ES⁺): 597.7 |
| **25ah** | 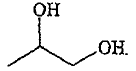 | CO₂H | **24ah** | L, I-1 | MS (ES⁺): 492.54 |
| **25ai** | 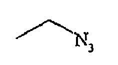 | CO₂H | **24ai** | L, M, K, N, O, I-1 | Characterized in the next step |

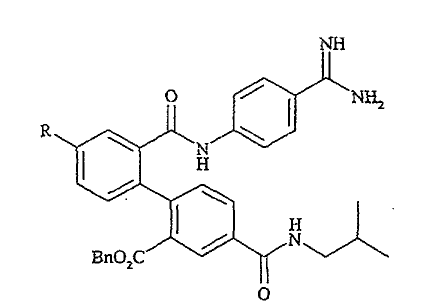

| **Cpd. No.** | **-R** | **Starting From** | **Method Used** | **Analytical Data** |
|---|---|---|---|---|
| **26a** | 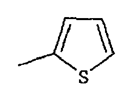 | **25a** | J | ¹HNMR (DMSO-d₆): δ 0.88 (d, J = 6.9 Hz, 6 H), 1.84 (m, 1 H), 3.07 (t, J = 6.9 and 6.0 Hz, 2 H), 5.05 (s, 2 H), 7.04 (d, J = 6.9 Hz, 2 H), 7.20 (m, 4 H), 7.35 (d, J = 7.7 Hz, 1 H), 7.43 (d, J = 7.7 Hz, H), 7.66 (d, J = 5.2 Hz, 1 H), 7.70 (d, J = 4.3 Hz, 1 H), 7.75 (m, 4 H), 7.82 (dd, J = 7.7 and 1.7 Hz, 1 H), 7.94 (d, J = 1.7 Hz, 1 H), 8.03 (dd, J = 7.7 and 1.7 Hz, 1 H), 8.26 (dd, J = 7.7. and 1.7 Hz, 1 H), 8.69 (t, J = 6 Hz, 1 H), 8.80 (s, 2 H), 9.17 7 (s, 2 H), 10.76 (s, 1 H); MS (ES+) 631.05 |
| **26b** | 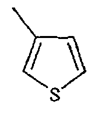 | **25b** | J | ¹HNMR (DMSO-d₆): δ 0.88 (d, J = 6.9 Hz, 6 H), 1.84 (m, 1 H), 3.07 (t, J= 6.8 and 6.0 Hz, 2 H), 5.04 (s, 2 H), 7.02 (d, J = 6.8 Hz, 2 H), 7.20 (m, 3 H), 7.34 (d, J = 7.7 Hz, 1 H), 7.43 (d, J = 8.6 Hz, 1 H), 7.72 (m, 6 H), 7.90 (dd, J =1.7 and 7.7 Hz, 1 H), 8.05 (m, 3 H), 8.23 (d, J = 1.7 Hz, 1 H), 8.68 (t, J = 6 and 5.2 Hz, 1 H), 8.82 (s, 2 H), 9.17 (s, 2 H), 10.73 (s, 1 H); MS (ES+) 631.82 |
| **26c** | 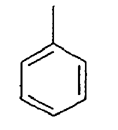 | **25c** | J | ¹HNMR (DMSO-d₆): δ 10.75 (s, 1 H), 9.19 (s, 2 H), 8.89 (s, 2 H), 8.69 (t, J = 6 Hz, 1 H), 8.29 (d, J = 1.7 Hz, 1 H), 8.07 (dd, J = 7.7 & 1.7 Hz, 1 H), 7.99 (d, J =1.7 Hz, 1 H), 7.87 (dd, J = 7.7 & 1.7 Hz, 1 H), 7.83 (d, J = 7.7 Hz, 2 H), 7.77 (m 5 H), 7.54 (t, J = 7.7, 2 H), 7.43 (m, 3 H), 7.19 (m, 3 H), 7.03 (d, J = 6.9 Hz, 2 H), 5.04 (bs, 2 H), 3.09 (t, J = 6.5 Hz, 2 H), 1.84 (m, 1 H), 0.89 (d, 6.8 Hz, 6 H); MS (ES+) 625.81 |
| **26d** | 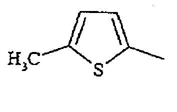 | **25d** | J | ¹HNMR (DMSO-d₆): δ 10.7 (s, 1 H), 9.14 (s, 2 H), 8.82 (s, 2 H), 8.64 (t, J = 6 Hz, 1 H), 8.21 (s, 1 H), 7.98 (dd, J = 7.8 & 2 Hz, 1 H), 7.8 (d, J = 2 Hz, 1 H), 7.7 (m, 4 H), 7.68 (dd, J = 2 & 7.8 Hz, 1 H), 7.44 (d, J = 3 Hz, 1 H), 7.37 (d, 7.8 Hz, 1 H), 7.27 (d, J = 7.7 Hz, 1 H), 7.16 (m, 3 H), 7.0 (s, 1 H), 6.99 (s, 1 H), 6.86 (d, J = 3 Hz, 1 H), 5.0 (s, 2 H), 3.03 (t, J = 6.5 Hz, 2 H), 2.46 (s, 3 H), 1.78 (m, 1 H), 0.83 (d, 6.8 Hz, 6 H); MS (ES+) 645.77 |
| **26e** | 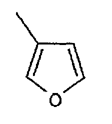 | **25e** | J | ¹HNMR (DMSO-d₆): δ 0.87 (d, J = 6.2 Hz, 6 H), 1.73 (m, 1 H), 3.07 (t, J = 6.7 and 6.2 Hz, 2 H), 5.05 (s, 2 H), 7.03 (dd, J = 1.7 and 8 Hz, 2 H), 7.11 (d, J 1.7 Hz, 1 H), 7.21 (m, 3 H), 7.31 (d, J = 8 Hz, 1 H), 7.42 (d, J = 8 Hz, 1 H), 7.78 (m, 5 H), 7.92 (d, J = 1.7 Hz, 1 H), 8.02 (dd, J = 8 and 1.7 Hz, 1 H), 8.25 (d, J = 1.9 Hz, 1 H), 8.33 (s, 1 H), 8.63 (t, J = 6 and 5 Hz, 1 H), 8.80 (bs, 2 H), 9.14 (bs, 2 H), 10.67 (s, 1 H); MS (ES+) 615.75 |
| **26f** | 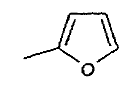 | **25f** | J | ¹HNMR (DMSO-d₆): δ 0.87 (d, J = 6.7 Hz, 6 H), 1.83 (m, 1 H), 3.06 (t, J = 6.7 and 6.2 Hz, 2 H), 5.04 (s, 2 H), 6.67 (m, 1 H), 7.03 (m, 2 H), 7.16 (m, 3 H), 7.35 (d, J = 8.6 Hz, 1 H), 7.42 (d, J = 8 Hz, 1 H), 7.74 (m, 4 H), 7.85 (m, 2 H), 7.98 (d, J = 1.2 Hz, 1 H), 8.03 (dd, J = 1.7 and 8 Hz, 1 H), 8.25 (d, J= 1.8 Hz, 1 H), 8.67 (t, J = 6.2 and 5.5 Hz, 1 H), 8.88 (bs, 2 H), 9.12 (bs, 2 H), 10.772 (bs, 1 H); MS (ES+) 615.75 |
| **26g** | 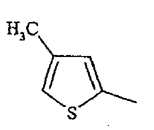 | **25g** | J | ¹HNMR (DMSO-d₆): δ 10.67 (s, 1 H), 9.12 (s, 2 H), 8.78 (s, 2 H), 8.61 (t, J = 6 Hz, 1 H), 8.21 (s, 1 H), 7.98 (dd, J = 7.8 & 2 Hz, 1 H), 7.84 (d, J = 2 Hz, 1 H), 7.7 (m, 5 H), 7.46 (s, 1 H), 7.39 (d, 7.8 Hz, 1 H), 7.29(d, J = 7.7 Hz, 1 H), 7.16 (m, 4H), 7.01 (s, 1 H), 6.99 (s, 1 H), 5.0 (s, 2 H), 3.03 (t, J = 6.5 Hz, 2 H), 2.23 (s, 3 H), 1.79 (m, 1 H), 0.83 (d, 6.8 Hz, 6 H); MS (ES+) 645.77 |
| **26h** | 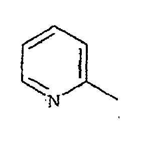 | **25h** | J | ¹HNMR (DMSO-d₆): δ 10.77 (bs, 1 H), 8.95 (bs, 4 H), 8.76 (d, J =4.3 Hz, 1 H), 8.69 (t, J = 6 Hz, 1 H), 8.4 (s, 1 H), 8.29 (m, 2 H), 8.15 (d, J = 7.7 Hz, 1 H), 8.07 (dd, J = 1.7 and 7.7 Hz, 1 H), 7.99 (dt, J = 1.7 & 7.7 Hz, 1 H), 7.76 (m, 4 H), 7.46 (m, 2 H), 7.18 (m, 3 H), 7.05 (s, 1 H), 7.03 (s, 1 H), 5.06 (s, 2 H), 3.10 (t, J = 6.9 and 6 Hz, 2 H), 1.86 (m, 1 H), 0.89 (d, J = 6.9 Hz, 6 H); MS (ES+) 626.69 |
| **26i** | 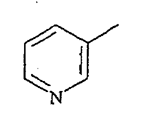 | **25i** | J | ¹HNMR (DMSO-d₆): δ 10.73 (bs, 1 H), 9.16 (6s, 2 H), 9.05 (d, J =1.9 Hz, 1 H), 8.79 (s, 2 H), 8.69 (1, J = 6 & Hz, 1 H), 8.64 (dd, J = 1.2 & 5 Hz, 1 H), 8.29 (d, J = 1.7 Hz, 1 H), 8.24 (d, J = 8 Hz, 1 H), 8.05 (m, 2 H), 7.93 (dd, 8 & 1.8 Hz, 1 H), 7.76 (m, 5 H), 7.56 (dd, J = 8 & 4.3 Hz, 1 H), 7.44 (d, J = 7.4 Hz, 2 H), 7.18 (m, 3 H), 7.0 (m, 2 H), 5.0 (s, 2 H), 3.08 (t, J = 6.5 Hz, 2 H), 1.82 (m, 1 H), 0.88 (d, 6.8 Hz, 6 H);; MS (ES+) 626.44 |
| **26j** | 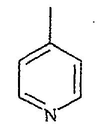 | **25j** | J | ¹HNMR (DMSO-d₆): δ 0.87 (d, J = 6.9 Hz, 6 H), 1.75 (m, 1 H), 3.08 (t, J = 6.9 and 6.0 Hz, 2 H), 5.03 (s, 2 H), 7.03 (m, 1 H), 7.18 (m, 3 H), 7.45 (t, J = 7.8 and 7 Hz, 2 H), 7.76 (s, 4 H), 7.87 (d, J = 6 Hz, 2 H), 7.94 (dd, J = 8 and 2 Hz, 1 H), 8.05 (dd, J = 8 and 2 Hz, 1 H), 8.08 (d, J = 2 Hz, 1 H), 8.29 (d, J = 2 Hz, 1 H), 8.70 (m, 3 H), 8.84 (s, 2 H), 9.11 (s, 2 H), 10.76 (s, 1 H); MS (ES+) 626.76 |
| **26k** | 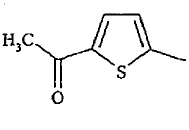 | **25k** | J | ¹HNMR (DMSO-d₆): δ 10.72 (bs, 1 H), 9.15 (bs, 2 H), 8.81 (bs, 2 H), 8.86 (t, J = 6 Hz, 1 H), 8.28 (s, 1 H), 8.03 (m, 3 H), 7.91 (d, J = 7.9 Hz, 1 H), 7.81 (d, J = 4 Hz, 1 H), 7.74 (s, 4 H), 7.42 (d, J = 7.9 Hz, 1 H), 7.38 (d, J = 7.9 Hz, 1 H), 7.18 (m, 3 H), 7.04 (m, 2 H), 5.04 (bs, 2 H), 3.07 (t, J = 6 Hz, 2 H), 2.57 (s, 3 H), 1.83 (m, 1 H), 0.87 (d, J = 6.8 Hz, 6 H); MS (ES+) 673.7 |
| **26l** | 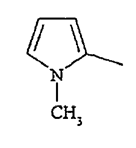 | **25l** | J | ¹HNMR (DMSO-d₆): δ 10.66 (s, 1 H), 9.20 (s, 2 H), 8.86 (s, 2 H), 8.66 (t, J = 6 Hz, 1 H), 8.24 (d, J = 2 Hz, 1 H), 8.15 (dd, J = 7.8 & 2 Hz, 1 H), 7.69 (m, 4 H), 7.68 (d, J = Hz, 1 H), 7.63 (d, J = 7.9 Hz, 1 H), 7.43 (d, J = 7.9 Hz, 1 H), 7.37 (d, J = 7.9 Hz, 1 H), 7.24 (m, 3 H), 7.09 (m, 2 H), 6.92 (s, 1 H), 6.40 (s, 1 H), 6.17 (t, J = 4 Hz, 1 H), 5.10 (bs, 2 H), 3.74 (s, 3 H), 3.09 (t, J = 6 Hz, 2 H), 1.83 (m, 1 H), 0.88 (d, J = 6.8 Hz, 6 H); MS (ES+) 628.65 |
| **26m** | 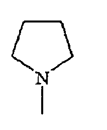 | **25m** | J | MS (ES+): 618.91 |
| **26n** | 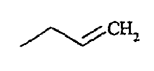 | **25n** | J | ¹HNMR (DMSO-d₆): δ 10.56 (s, 1 H), 9.15 (bs, 2 H), 8.84 (bs, 2 H), 8.64 (t, J = 6 Hz, 1 H), 8.19 (d, J = 2 Hz, 1 H), 7.99 (d, J = 7 Hz, 1 H), 7.70 (m, 4 H), 7.46 (s, 1 H), 7.36 (m, 2 H), 7.24 (m, 3 H), 7.05 (s, 1 H), 7.00 (s, 1 H), 6.0 (m, 1 H), 5.18 (d, J = 16 Hz, 1 H), 5.10 (d, J = 11 Hz, 1 H), 5.0 (s, 2 H), 3.47 (d, J = 6 Hz, 1 H), 3.43 (t, J = 6 Hz, 2 H), 1.79 (m, 1 H), 0.83 (d, J = 6.8 Hz, 6 H); MS (ES+) 589.5 |
| **26o** | 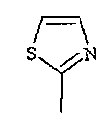 | **25o** | J | ¹HNMR (DMSO-d₆): δ 10.84 (s, 1 H), 9.16 (s, 2 H), 8.78 (s, 2 H), 8.69 (t, J = 6 Hz, 1 H), 8.27 (d, J = 2 Hz, 1 H), 8.19 (s, 1 H), 8.09 (dd, J = 2 & 7.7 Hz, 1 H), 8.04 (dd, J = 2 & 7.7 Hz, 1 H), 8.01 (d, J = 4 Hz, 1 H), 7.89 (d, J = 3 Hz, 1 H), 7.73 (m, 4 H), 7.44 (dd, J = 3 & 7.8 Hz, 2 H), 7.16 (m, 3 H), 7.30 (s, H), 7.05 (s, 1 H), 5.03 (bs, 2 H), 3.06 (t, J = 6.5 Hz, 2 H), 1.82 (m, 1 H), 0.86 (d, 6.8 Hz, 6 H); MS (ES+) 632.4 |
| **26p** | 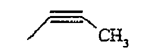 | **25p** | J | MS (ES⁺): 609.3 (M+Na)⁺ |
| **26q** | 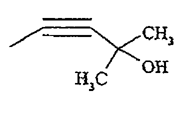 | **25q** | J | MS (ES⁺) 631.5 |
| **26r** | 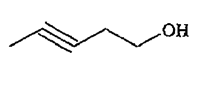 | **25r** | J | ¹HNMR (DMSO-d₆): δ 10.71 (s, 1 H), 9.16 (s, 2 H), 8.81 (s, 2 H), 8.68 (t, J = 6 Hz, 1 H), 8.25 (s, 1 H), 8.03 (d, J = 7.8 Hz, 1 H), 7.73 (m, 5 H), 7.69 (s, 1 H), 7.55 (d, J = 7.8 Hz, 1 H), 7.39 (d, J = 8.9 Hz, 1 H), 7.26 (m, 3 H), 7.03 (m, 2 H), 5.02 (bs, 2 H), 4.95 (t, J = 5 Hz, 1 H), 3.62 (q, J = 6 & 12.8 Hz, 2 H), 3.07 (t, J = 6 Hz, 2 H), 2.62 (t, J = 6 Hz, 2 H), 1.83 (m, 1 H), 0.88 (d, J = 6.8 Hz, 6 H); MS (ES+) 617.4 |
| **26s** | 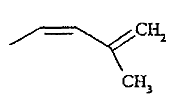 | **25s** | J | ¹HNMR (DMSO-d₆): δ 0.89 (d, J = 6.8 Hz, 6 H), 1.84 (m, 1 H), 1.99 (s, 3 H), 3.09 (t, J = 6 Hz, 2 H), 5.04 (s, 2 H), 5.18 (s, 1 H), 5.28 (s, 1 H), 6.73 (d, J = 16 Hz, 1 H), 7.04 (d, J = 6 Hz, 2 H), 7.23 (m, 5 H), 7.42 (d, J = 9 Hz, 1 H), 7.73 (m, 5 H), 7.85 (s, 1 H), 8.03 (dd, J = 9 and 2 Hz, 1 H), 8.26 (d, J = 2 Hz, 1 H), 8.69 (t, J = 6 Hz, 1 H), 8.87 (bs, 4 H), 10.91 (s, 1 H); MS (ES+) 615.4 |
| **26t** | 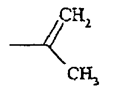 | **25t** | J | ¹HNMR (DMSO-d₆): δ 10.8 (br s, 1 H), 9.1 and 8.9 (2 br s, 4 H), 8.6 (m, 1 H), 8.2 (s, 1 H), 8.0 (m, 1 H), 7.8-7.6 (m, 6 H), 7.40 (, J = 6.9 Hz, 1 H ), 7.3 (m, 4 H), 7.0 (d, 1 H), 5.6 (m, 1 H), 5.2 (m, 1 H), 5.0 (br s, 1 H), 3.1 (t, J = 6.8 Hz, 2 H), 2.2 ( s, 3 H), 1.8 (m, 1 H), 0.95 (d, 6 H); MS (ES+) 589.4, MS (ES-) 587.5 |
| **26u** | 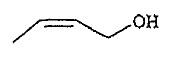 | **25u** | J | ¹HNMR (DMSO-d₆): δ 0.88 (d, J = 6.8 Hz, 6 H), 1.84 (m, 1 H), 3.09 (t, J = 6 Hz, 2 H), 4.33 (t, J = 5.5 Hz, 2 H), 5.02 (s, 2 H), 5.01 (t, J = 5.5 Hz, 1 H), 5.95 (m, 1 H), 6.57 (d, J = 11.5 Hz, 1 H), 7.04 (d, J = 6.7 Hz, 2 H), 7.25 (m, 3 H), 7.31 (d, J = 7.8 Hz, 1 H), 7.43 (m, 2 H), 7.54 (s, 1 H), 7.74 (s, 4 H), 8.05 (dd, J = 7.8 and 2 Hz, 1 H), 8.23 (d, J = 2 Hz, 1 H), 8.69 (t, J = 6 Hz, 1 H), 8.83 (bs, 2 H), 9.18 (bs, 2 H), 10.66 (s, 1 H); MS (ES+) 605.3 |
| **26v** | 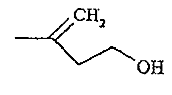 | **25v** | J | ¹HNMR (DMSO-d₆): δ 0.88 (d, J = 6.8 Hz, 6 H), 1.84 (m, 1 H), 2.75 (t, J = 7 Hz, 2 H), 3.09 (t, J = 6 Hz, 2 H), 3.60 (m, 2 H), 4.65 (t, J = 5 Hz, 1 H), 5.05 (s, 2 H), 7.05 (d, J = 7 Hz, 2 H), 7.29 (m, 5 H), 7.42 (d, J = 7.8 Hz, 1 H), 7.66 (dd, J = 7.8 and 2 Hz, 1 H), 7.75 (m, 6 H), 8.03 (dd, J = 7.8 and 2 Hz, 1 H), 8.25 (s, 1 H), 8.68 (t, J = 6 Hz, 1 H), 8.82 (bs, 2 H), 9.18 (bs, 2 H), 10.68 (s, 1 H); MS (ES+) 619.4 |
| **26w** | 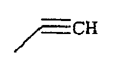 | **25w** | J | ¹HNMR (DMSO-d₆): δ 0.88 (d, J = 6.8 Hz, 6 H), 1.84 (m, 1 H), 3.09 (t, J = 6 Hz, 2 H), 4.41 (s, 1 H), 5.04 (d, J = 11 Hz, 2 H), 7.05 (d, J = 5.5 Hz, 2 H), 7.29 (m, 3 H), 7.34 (d, J = 8 Hz, 1 H), 7.40 (d, J = 8 Hz, 1 H), 7.65 (dd, J = 8 and 2 Hz, 1 H), 7.75 (s, 4 H), 7.79 (s, 1 H), 8.05 (dd, J = 8 and 2 Hz, 1 H), 8.28 (d, J = 2 Hz, 1 H), 8.71 (t J = 6 Hz, 1 H), 8.82 (bs, 2 H), 9.17 (bs, 2 H), 10.73 (s, 1 H); MS (ES+) 573.3 |
| **26x** | 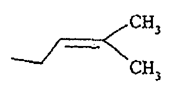 | **25x** | J | ¹HNMR (DMSO-d₆): δ 0.86 (d, J = 6.8 Hz, 6 H), 1.47 (s, 3 H), 1.74 (s, 3 H), 1.85 (m, 1 H), 3.06 (t, J = 6 Hz, 2 H), 3.43 (d, J = 8 Hz, 1 H), 5.04 (s, 2 H), 5.1 (m, 1 H), 7.03 (m, 2 H), 7.23 (m, 5 H), 7.52 (m, 2 H), 7.72 (m, 5 H), 8.02 (m, 1 H), 8.21 (s, 1 H), 8.66 (t, J = 6 Hz, 1 H), 8.81 (bs, 2 H), 9.23 (bs, 2 H), 10.52 (s, 1 H); MS (ES+) 617.6 |
| **26y** | 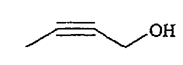 | **25y** | J | ¹HNMR (DMSO-d₆): δ 0.87 (d, J = 6.8 Hz, 6 H), 1.72 (m, 1 H), 3.07 (t, J = 6 Hz, 2 H), 4.36 (d, J = 6 Hz, 2 H), 5.0 (m, 2 H), 5.42 (t, J = 6 Hz, 1 H), 7.03 (d, J = 7 Hz, 2 H), 7.25 (m, 3 H), 7.31 (d, J = 8 Hz, 1 H), 7.39 (d, J = 8 Hz, 1 H), 7.58 (d, J = 8 Hz, 1 H), 7.73 (m, 5 H), 8.02 (dd, J = 10 and 2 Hz, 1 H), 8.23 (s, 1 H), 8.68 (t, J = 6 Hz, 1 H), 8.76 (bs, 2 H), 9.15 (bs, 2 H), 10.71 (s, 1 H); MS (ES+) 603.4 |
| **26z** | 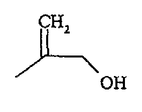 | **25z** | J | ¹HNMR (DMSO-d₆): δ 10.6 (s, 1 H), 9.17 (s, 1 H), 8.85 (s, 1 H), 8.68 (d, J = 5.9 Hz, 2 H), 8.25 (d, 1.98 Hz, 1 H), 8.05 (d, J = 1.96 Hz, 1 H), 8.03 (d, J = 1.9 Hz, 1 H), 7.75 (m, 4 H), 7.65 (m, 4 H), 7.41 (d, J = 7.87 Hz, 4 H), 7.25 (m, 1 H) 5.4 (s, 1 H), 5.2 (d, J = 5.9 Hz, 2 H), 4.44 (d, J = 5.9 Hz, 1 H), 3.09 (d, J = 6.89 Hz, 2 H), 1.89 (d, J = 6.89 Hz, 2 H) 0.88 (d, J = 5.9 Hz, 6 H); MS (ES+) 605.69 |
| **26aa** | 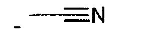 | **25aa** | J | Characterized in the next step |
| **26ab** | 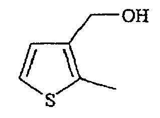 | **25ab** | J | ¹HNMR (DMSO-d₆): δ 10.70 (s, 1 H) 9.15 (bs, 2 H), 8.77 (bs, 2 H), 8.67 (t, J = 6 Hz, 1 H), 8,25 (s, 1 H), 8.04 (d, J = 7 Hz, 1 H), 7.77 (d, J = 2 Hz, 1 H), 7.71 (m 4 H), 7.70 (d, J = 2 Hz, 1 H), 7.59 (d, J = 6 Hz, 1 H), 7.46 (d, J = 8 Hz, 1 H), 7.41 (d, J = 8 Hz, 1 H), 7.22 (m, 3 H), 7.05 (s, 1 H), 7.03 (d, J = 2 Hz, 1 H), 5.31 (t, J = 6 Hz, 1 H), 5.04 (bs, 2 H), 4.51 (d, J = 6 Hz, 2 H), 3.07 (t, J = 6 Hz, 2 H), 1.82 (m, 1 H), 0.86 (d, J = 6.8 Hz, 6 H); MS (ES+) 661.74 |
| **26ac** | 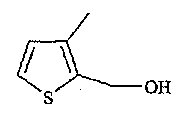 | **25ac** | J | ¹HNMR (DMSO-d₆): δ 0.87 (d, J = 6.8 Hz, 6 H), 1.83 (m, 1 H), 3.07 (t, J = 6 Hz, 2 H), 4.71 (d, J = 5 Hz, 2 H), 5.04 (bs, 2 H), 5.69 (t, J = 5 Hz, 1 H), 7.03 (d, J = 5.8 Hz, 2 H), 7.21 (m, 3 H), 7.35 (d, J = 5 Hz, 1 H), 7.38 (d, J = 8 Hz, 1 H), 7.44 (m, d, J = 8 Hz, 1 H), 7.58 (d, J = 5 Hz, 1 H), 7.74 (m, 6 H), 8.03 (d, J = 8 Hz, 1 H), 8,24 (s, 1 H), 8.67 (1, J = 6 Hz, 1 H), 8.79 (bs, 2 H), 9.14 (bs, 2 H), 10.64 (s, 1 H); MS (ES+) 661.74 |
| **26ad** | 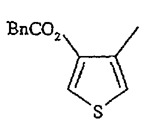 | **25ad** | J | ¹HNMR (DMSO-d₆): δ 9.65 (s, 1 H), 8.71 (t, J = 5.15 Hz, 1 H) 8.39 (d, J = 2.57 Hz, 4 H), 8.09 (d, J = 1.79 Hz, 4 H), 8.05 (d, J = 1.79 Hz, 4 H), 7.43 (d, J = 7.77 Hz, 2 H), 7.29 (s, 2 H), 7.19 (m, 2 H), 7.08 (m, 2 H), 5.03 (d, J = 2.58 Hz, 2 H) 3.29 (m, 2 H), 3.12 (s, 4 H), 2.49 (m, 2 H), 1.87 (m, 2 H), 0.90 (d, J = 6.87 Hz, 6 H); MS (ES+) 765.4 |
| **26ae** | 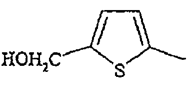 | **25ae** | J | ¹HNMR (DMSO-d₆): δ 9.1 (bs, 2 H), 8.8 (bs, 2 H), 8.5 (t, J = 6 Hz, 1 H), 8.02 (s, 1 H), 7.68 (s, 1 H), 7.62 (m, 6 H), 7.53 (d, J = 5.8 Hz, 1 H), 7.15 (d, J = 6 Hz, 1 H), ), 7.13 (m, 1 H), 7.01 (s, 1 H), 5.5 (t, J = 5 Hz, 1 H), 4.7 (d, J = 5 Hz, 2 H), 3.01 (m, 2 H), 1.8 (m, 1 H), 0.85 (d, J = 6.8 Hz, 6 H); MS (ES+) 571.2 |
| **26af** | 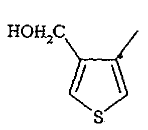 | **25af** | J | ¹HNMR (DMSO-d₆): δ 10.6 (s, 1 H), 9.17 (s, H), 8.85 (s, 1 H), 8.68 (d, J = 5.9 Hz, 2 H), 8.25 (d, 1.98 Hz, 1 H), 7.75 (m, 4 H), 7.65 (m, 4 H), 7.41 (d, J = 7.87 Hz, 4 H), 7.25 (m, 4 H), 5.4 (s, 1 H), 5.2 (d, J = 5.9 Hz, 2 H), 4.44 (d, J = 5.9 Hz, 1 H), 3.09 (d, J = 6.89 Hz, 2 H), 1.89 (d, J = 6.89 Hz, 2 H), 0.88 (d, J = 5.9 Hz, 6 H). |
| **26ag** | 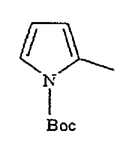 | **25ag** | J | ¹HNMR (DMSO-d₆): δ 0.90 (d, J = 6.9 Hz, 6 H), 1.41 (s, 9 H), 1.87 (m, 1 H), 3.11 (t, J = 6.9 and 6 Hz, 2 H), 5.07 (s, 2 H), 6.37 (t, J = 3.4 Hz, 1 H), 6.51 (s, 1 H), 7.11 (m, 2 H), 7.26 (m, 3 H), 7.33 (d, 7.7 Hz, 1 H), 7.41 (d, J = 8.6 Hz, 1 H), 7.45 (d, J = 1.7 Hz, 1 H), 7.61 (dd, J = 1.7 and 7.7,1 H), 7.74 (m, 5 H), 8.05 (dd, J = 8.6 and 1.7 Hz, 1 H), 8.26 (d, J = 1.7 Hz, 1 H), 8.66 (t, J = 5 and 6 Hz, 1 H), 8.77 (bs, 2 H), 9.15 (bs, 2 H), 10.58 (s, 1 H); MS (ES+) 714.78 |
| **26ah** | 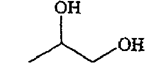 | **25ah** | J | MS (ES⁺): 609.6 |
| **26ai** | 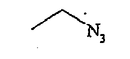 | **25ai** | J | ¹HNMR (DMSO-d₆): δ 10.8 (s, 1 H), 6.2 and 8.9 (2 br s, 2 H each, 4H), 8.7 (t, 1 H), 8.2 (s, 1 H), 8.0 (d, J = 6 Hz, 1 H), 7.7 (m, 5 H), 7.6 (d, J = 5 Hz, 1 H), 7.4 (d, J = 5.8 Hz, 1 H), 7.35 (d, J = 6.9 Hz, 1 H), 7.29 (m, 3 H), 7.0 (m, 2 H), 5.0 (m, 2 H), 4.6 (s, 2 H), 3.01 (t, J = 6.8 Hz, 2 H), 1.81 (m, 1 H), 0.95 (d, J = 6.8 Hz, 6 H); MS (ES+) 604.3 |

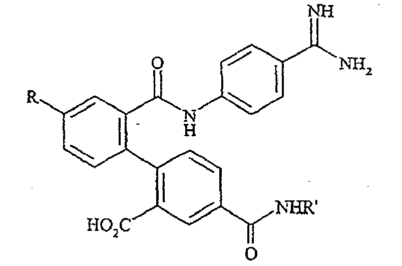

| **Cpd. No.** | **-R** | **-R'** | **Starting From** | **Method Used** | **Analytical Data** |
|---|---|---|---|---|---|
| **27a** | 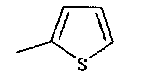 | 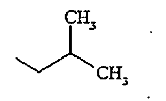 | **26a** | I-2 | ¹H NMR (DMSO-d₆): δ 14.95 (s, 1 H), 8.97 (s, 4 H), 8.5 (t, J = 6 Hz, 1 H), 7.97 (d, J = 2 Hz, 1 H), 7.80 (d, J = 2 Hz, 1 H), 7.73 (dd, J = 7.9 and 2 Hz, 1 H), 7.61 (m, 7 H), 7.18 (t, J = 3.9 Hz, 1 H), 7.05 (d, J = 7.9 Hz, 1 H), 6.93 (d, J = 7.9 Hz, 1 H), 3.01 (t, J = 6.9 and 6.0 Hz, 2 H), 1.81 (m, 1 H), 0.84 (d, J = 6.9 Hz, 6 H); MS (ES⁺): 541.17 |
| **27b** | 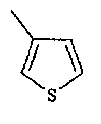 | 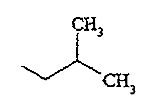 | **26b** | 1-2 | ¹H NMR (DMSO-d₆): δ 13.24 (s, 1 H), 9.05 (s, 2 H), 8.9 (s, 2 H), 8.49 (t, J = 6 and 5.2 Hz, 1 H), 7.97 (s, 1 H), 7.99 (s, 1 H), 7.87 (s, 1H), 7.75 (d, J = 7.7 Hz, 1 H), 7.65 (m, 1 H), 7.62 (m, 6 H), 7.05 (d, J = 7.7 Hz, 1 H), 6.93 (d, J = 7.7 Hz, 1 H), 3.01 (t, J = 6.9 and 6.0 Hz, 2 H), 1.81 (m, 1 H), 0.85 (d, J = 6.9 Hz, 6 H); MS (ES⁺): 541.42 |
| **27c** | 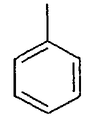 | 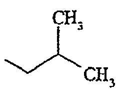 | **26c** | I-2 | ¹H NMR (DMSO-d₆): δ 13.28 (s, 1 H), 9.04 (s, 4 H), 8.5 (t, J = 6 Hz, 1 H), 7.97 (s, 1 H), 7.82 (s, 1 H), 7.74 (m, 3 H), 7.62 (m, 5 H), 7.5 (t, J = 7.7 Hz, 2 H), 7.4 (t, J = 7,7, 1 H), 7.1 (d, J = 7.7 Hz, 2 H), 6.97 (d, J = 7.7. Hz, 1 H), 3.01 (t, J = 6.5 Hz, 2 H), 1.8 (m, 1 H), 0.85 (d, 6.8 Hz, 6 H); MS (ES⁺): 535.48 |
| **27d** | 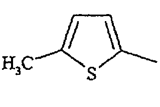 | 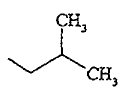 | **26d** | I-2 | ¹H NMR (DMSO-d₆): δ 9.03 (s, 2 H), 8.89 (s, 2 H), 8.49 (t, J = 6 Hz, 1 H), 7.99 (s, 1 H), 7.65 (m, 8 H), 7.37 (d, J = 3 Hz, 1 H), 7.04 (d, J = 7.7 Hz, 1 H), 6.98 (s, 1 H), 6.82 (d, J = 3 Hz, 1 H), 2.98 (t, J = 6.5 Hz, 2 H), 2.46 (s, 3 H), 1.76 (m, 1 H), 0.81 (d, 6.8 Hz, 6 H); MS (ES⁺):555.61 |
| **27e** | 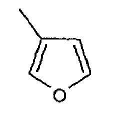 | 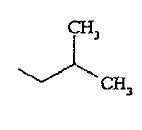 | **26e** | I-2 | ¹H NMR (DMSO-d₆): δ 14.10 (s, 1 H), 9.05 (bs, 2 H), 8.79 (bs, 2 H), 8.47 (t, J = 5.6 Hz, 1 H), 8.3 (s, 1 H), 7.96 (d, J = 2 Hz, 1 H), 7.78 (m, 1 H), 7.63 (m, 7 H), 7.05 (m, 1 H), 7.01 (d, J = 7.7 Hz, 1 H), 6.92 (d, J = 7.7 Hz, 1 H), 3.02 (t, J = 4.9 Hz, 2 H), 1.81 (m, 1 H), 0.85 (d, J = 6.3 Hz, 6 H); MS (ES⁺): 525.36 |
| **27f** | 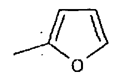 | 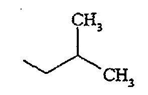 | **26f** | I-2 | ¹H NMR (DMSO-d₆): δ 9.07 (s, 2 H), 8.86 (s, 2 H), 8.53 (t, J = 5 Hz, 1 H), 8.03 (s, 1 H), 7.89 (d, J = 1.4 Hz, 1 H), 7.78 (m, 2 H), 7.65 (m, 6 H), 7.1 (m, 2 H), 7.08 (d, J = 7 Hz, 1 H), 6.64 (dd, J = 3.5 and 2 Hz, 1 H), 3.03 (t, J = 6.9 and 6.0 Hz, 2 H), 1.81 (m, 1 H), 0.86 (d, J = 6.9 Hz, 6 H); MS (ES⁺): 525.43 |
| **27g** | 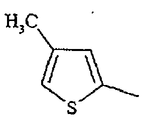 | 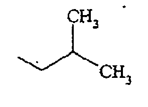 | **26g** | I-2 | ¹H NMR (DMSO-d₆): δ 13.81 (s, 1 H), 8.74 (bs, 4 H), 8.43 (t, J = 6 Hz, 1 H), 7.92 (d, J = 2 Hz, 1 H), 7.69 (d, J = 2 Hz, 1 H), 7.62 (dd, J = 7.7 & 2 Hz, 1 H), 7,54 (m, 5 H), 7.38 (s, 1 H), 7.15 (s, 1 H), 6.99 (d, J = 7.8 Hz, 1 H), 6.89 (d, J = 6.8 Hz, 1 H), 2.97 (t, J = 6.5 Hz, 2 H), 2.20 (s, 3 H), 1.76 (m, 1 H), 0.8 (d, 6.8 Hz, 6 H); MS (ES⁺): 555.67 |
| **27h** | 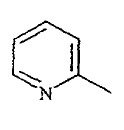 | 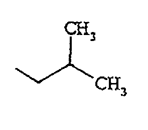 | **26h** | I-2 | ¹H NMR (DMSO-d₆): δ 13.95 (bs, 1 H), 8.99 (bs, 2 H), 8.79 (bs, 2 H), 8.65 (d, J = 5 Hz, 1 H), 8.43 (t, J = 6 Hz, 1 H), 8.25 (s, 1 H), 8.09 (d, J = 7.8 Hz, 1 H), 8.00 (d, J = 7.8 Hz, 1 H), 7.94 (s, 1 H), 7.87 (t, J = 7.8 Hz, 1 H), 7.58 (m, 5 H), 7.34 (dd, J = 7.8 & 5 Hz, 1 H), 7.09 (dd, J = 7.7 Hz, 1 H), 6.90 (d, J = 7.8 Hz, 1 H), 2.97 (t, J = 5 Hz, 2 H), 1.76 (m, 1 H), 0.81 (d, 6.8 Hz, 6 H); MS (ES⁺): 268.64 (m/2) |
| **27i** | 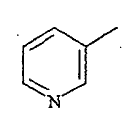 | 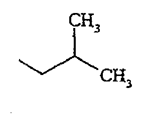 | **26i** | I-2 | ¹H NMR (DMSO-d₆): δ 9.05 (bs, 2 H), 8.95 (d, J = 2.1 Hz, 1 H), 8.75 (s, 2 H), 8.65 (dd, J = 5 & 1.4 Hz, 1 H), 8.5 (t, J = 5.6 Hz, 1 H), 8.2 (dt, J = 1.8 & 7.7 Hz, 1 H), 7.99 (d, J = 2.1 Hz, 1 H), 7.9 (d, J = 2.1 Hz, 1 H), 7.85 (dd, J = 7.7 &2.2 Hz, 2 H), 7.65 (m, 5 H), 7.55 (dd, J = 7.7 & 4.5 Hz, 1 H), 7.15 (d, J = 7.7 Hz, 1 H), 6.95 (d, J = 7.7 Hz, 1 H), 3.08 (t, J = 5 Hz, 2 H), 1.82 (m, 1 H), 0.9 (d, 6.8 Hz, 6 H); MS (ES⁺): 268.85 (m/ 2) |
| **27j** | 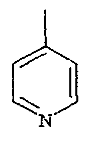 | 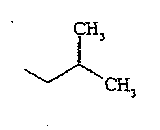 | **26j** | I-2 | ¹H MMR (DMSO-d₆): δ 14.19 (s, 1 H), 9.06 (bs, 2 H), 8.67 (bs, 2 H), 8.67 (d, J = 6 Hz, 2 H), 8.50 (t, J = 6 Hz, 1 H), 7.97 (m, 2 H), 7.91 (dd, J = 7.7 and 2 Hz, 1 H), 7.80 (d, J = 6 Hz, 2 H), 7.64 (m, 6 H), 7.18 (d, J = 7.7 Hz, 1 H), 6.95 (d, J = 7.7 Hz, 1 H), 3.02 (t, J = 5.0 Hz, 2 H), 1.82 (m, 1 H), 0.80 (d, J = 6.9 Hz, 6 H); MS (ES⁺): 536.43 |
| **27k** | 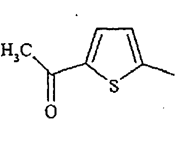 | 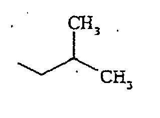 | **26k** | I-2 | ¹H NMR (DMSO-d₆): δ 9.04 (bs, 2 H), 8.78 (bs, 2 H), 8.55 (t, J = 6 Hz, 1 H), 8.1 (s, 1 H), 7.98 (d, J = 4 Hz, 1 H), 7.95 (s, 1 H), 7.87 (d, J = 7.9 Hz, 1 H), 7.75 (d, J = 6.9 Hz, 1 H), 7.66 (m, 4 H), 7.2 (m, 2 H), 7.09 (s, 1 H), 3.03 (t, J = 6 Hz, 2 H), 2.55 (s, 3 H), 1.81 (m, 1 H), 0.85 (d, J = 6.8 Hz, 6 H); MS (ES⁺): 583.59 |
| **271** | 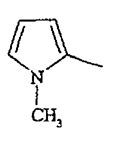 | 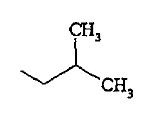 | **26l** | I-2 | ¹H NMR (DMSO-d₆): δ 9.1 (s, 2 H), 8.84 (s, 2 H), 8.56 (t, J = 6 Hz, 1 H), 8.08 (bs, 1 H), 7.67 (m, J = 7 H), 7.58 (d, J = 7.9 Hz, 1 H), 7.11 (m, 2 H), 6.91 (bs, 1 H), 6.31 (bs, 1 H), 6.11 (t, J = 3 Hz, 1 H), 3.74 (s, 3 H), 3.05 (t, J = 6 Hz, 2 H), 1.83 (m, 1 H), 0.88 (d, J = 6.8 Hz, 6 H); MS (ES⁺): 538.64 |
| **27m** | 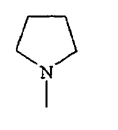 | 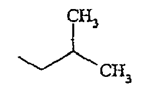 | **26m** | I-2 | ¹H NMR (DMSO-d₆): δ 9.04 (s, 2 H), 8.94 (s, 2 H), 8.46 (t, J = 6 Hz, 1 H), 7.96 (s, 1 H), 7.63 (m, 6 H), 6.94 (s, 1 H), 6.83 (d, J = 7.7 Hz, 1 H), 6.7 (d, J = 2, 1 H), 6.62 (dd, J = 7.7 and 2 Hz, 1 H), 3.28 (m, 4 H), 3.02 (t, J = 6.5 Hz, 2 H), 1.98 (m, 4 H), 1.82 (m,1H), 0.82 (d, 6.8 Hz, 6 H); MS (ES⁺): 528.76 |
| **27n** | 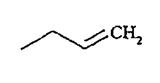 | 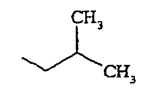 | **26n** | I-2 | ¹H NMR (DMSO-d₆): δ 13.96 (s, 1 H), 9.02 (s, 2 H), 8.85 (s, 2 H), 8.46 (t, J = 6 Hz, 1 H), 7.91 (s, 1 H), 7.58 (m, 4 H), 7.39 (s, 1 H), 7.25 (d, J = 7.8 Hz, 1 H), 6.92 (d, J = 7.7, 1 H), 6.87 (d, J = 7.7 Hz, 1 H), 6.01 (m, 1 H), 5.17 (d, J = 16.7 Hz, 1 H), 5.08 (d, J =10 Hz, 1 H), 3.45 (d, J = 6 Hz, 2H), 2.99 (t, J = 6 Hz, 2 H), 1.78 (m, 1 H), 0.83 (d, J = 6.8 Hz, 6 H); MS (ES⁺): 499.3 |
| **27o** | 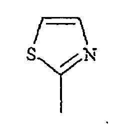 | 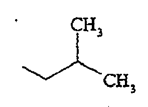 | **26o** | I-2 | ¹H NMR (DMSO-d₆): δ 14.08 (bs, 1 H), 9.06 (s, 2 H), 8.79 (s, 2 H), 8.51 (t, J = 6 Hz, 1 H), 8.11 (d, J = 2 Hz, 1 H), 8.01 (m, 3 H), 7.85 (d, J = 3 Hz, 1 H), 7.63 (m, 6 H), 7.17 (d, J = 7.8 Hz, 1 H), 6.97 (d, J = 7.8 Hz, 1 H), 3.02 (t, J = 6.5 Hz, 2 H), 1.81 (m, 1 H), 0.86 (d, 6.8 Hz, 6 H); MS (ES⁺): 542.2) |
| **27p** | 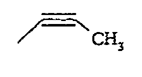 | 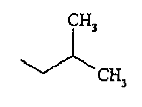 | **26p** | I-2 | ¹H NMR (DMSO-d₆): δ 9.1 and 9.2 (2 br s, 4 H, NH proton), 8.6 (m, 1 H), 8.3 (m, 1 H), 8.0-7.6 (m, 8 H, aromatic proton), 7.3 (m, 2 H), 3.1 (t, 2 H), 2.2 (s, 3 H), 1.8 (m, 1 H), 0.9 (2s, 6 H); IR (KBr Pellets) 2957, 1676, 1480, 1324, 844 cm⁻¹. MS (ES+) : 497 |
| **27q** | 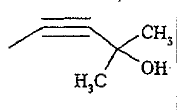 | 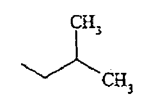 | **26q** | I-2 | ¹H NMR (DMSO-d₆): δ 9.06 (s, 2 H), 8.77 (s, 2 H), 8.53 (t, J = 6 Hz, 1 H), 8.03 (m, 1 H), 7.64 (m, 6 H), 7.46 (d, J = 6.9 Hz, 1 H), 7.05 (s, 2 H), 6.96 (s, 1 H), 5.52 (s, 1 H), 3.02 (t, J = 6.8 Hz, 2 H), 1.8 (m, 1 H), 1.48 (s, 6 H),0.85 (d, J= 6.8 Hz, 6 H); MS (ES⁻): 539.4 |
| **27r** | 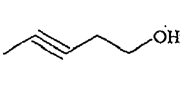 | 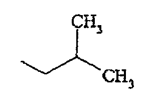 | **26r** | I-2 | ¹H NMR (DMSO-d₆): δ 9.06 (s, 2 H), 8.78 (s, 2 H), 8.52 (t, J = 6 Hz, 1 H), 8.01 (d, J = 6.8 Hz, 1 H), 7.62 (m, 7 H), 7.46 (d, J = 6.8 Hz, 1 H), 7.0 (m, 2 H), 4.94 (t, J = 6 Hz, 1 H), 3.60 (q, J = 6 & 12.8 Hz, 2 H), 3.01 (t, J = 6 Hz, 2 H), 2.58 (t, J = 6 Hz, 2 H), 1.82 (m, 1 H), 0.85 (d, J = 6.8 Hz, 6 H); MS (ES⁻): 525.4 |
| **27s** | 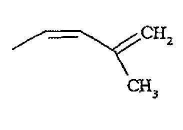 | 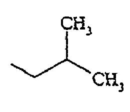 | **26s** | I-2 | ¹H NMR (DMSO-d₆): δ 9.01 (s, 2 H), 8.88 (s, 2 H), 8.5 (t, J = 6 Hz, 1 H), 8.07 (m, 1 H), 7.73 (m, 1 H), 7.63 (m, 7 H), 7.11 (d, J = 17 Hz, 1 H), 7.01 (d, J = 17 Hz, 1 H), 6.97 (m, 1 H), 6.69 (d, J = 17 Hz, 1 H), 5.24 (s, 1H), 5.14 (s, 1H), 3.03 (t, J = 6.9 and 6.0 Hz, 2 H), 1.92 (s, 3 H), 1.81 (m, 1 H), 0.84 (d, J = 6.9 Hz, 6 H); MS (ES⁺): 525.4 |
| **27t** | 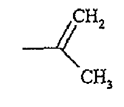 | 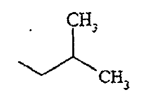 | **26t** | I-2 | ¹H NMR (DMSO-d₆): δ 9.08 (s, 2 H), 8.82 (s, 2 H), 8.53 (t, J = 6 Hz, 1 H), 8.04 (m, 1 H), 7.67 (m, 7 H), 7.04 (m, 2 H), 5.55 (s, 1H), 5.20 (s, 1H), 3.04 (t, J = 6.9 and 6.0 Hz, 2 H), 2.19 (s, 3 H), 1.81 (m, 1 H), 0.87 (d, J = 6.9 Hz, 6 H); MS (ES⁺): 499.4 |
| **27u** | 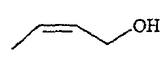 | 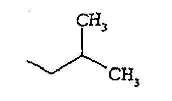 | **26u** | I-2 | ¹H NMR (DMSO-d₆): δ 9.11 (s, 2 H), 8.86 (s, 2 H), 8.57 (t, J = 6 Hz, 1 H), 8.13 (m, 1 H), 7.53 (m, 2 H), 7.74 (m, 6 H), 7.37 (d, J = 7 Hz, 1 H), 7.17 (m, 2 H), 6.54 (d, J = 12 Hz, 1 H), 5.91 (m, 1 H), 4.99 (m, 1 H), 4.31 (m, 2 H), 3.06 (t, J = 6.9 and 6.0 Hz, 2 H), 1.83 (m, 1 H), 0.87 (d, J = 6.9 Hz, 6 H); MS (ES⁺): 515.4 |
| **27v** | 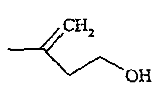 | 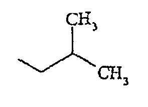 | **26v** | I-2 | ¹H NMR (DMSO-d₆): δ 9.08 (s, 2 H), 8.82 (s, 2 H), 8.54 (t, J = 6 Hz, 1 H), 8.05 (m, 1 H), 7.63 (m, 8 H), 7.06 (m, 2 H), 5.52 (s, 1 H), 5.2 (s, 1 H), 4.63 (t, J = 5 Hz, 1 H), 3.56 (m, 2 H), 3.05 (t, J = 6.9 and 6.0 Hz, 2 H), 2.71 (t, J = 7 Hz, 2 H), 1.82 (m, 1 H), 0.87 (d, J = 6.9 Hz, 6 H); MS (ES⁺): 529.4 |
| **27w** | 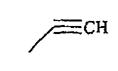 | 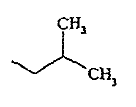 | **26w** | I-2 | ¹H NMR (DMSO-d₆): δ 9.08 (s, 2 H), 8.86 (s, 2 H), 8.54 (t, J = 6 Hz, 1 H), 8.03 (m, 1 H), 7.62 (m, 7 H), 7.08 (d, J = 7.5 Hz, 1 H), 6.99 (m, 1 H), 4.32 (s, 1 H), 3.03 (t, J = 6.9 and 6.0 Hz, 2 H), 2.71 (t, J = 7 Hz, 2 H), 1.82 (m, 1 H), 0.87 (d, J = 6.9 Hz, 6 H); MS (ES⁺): 483.3 |
| **27x** | 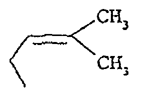 | 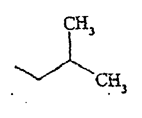 | **26x** | I-2 | ¹H NMR (DMSO-d₆): δ 13.8 (s, 1 H), 9.04 (s, 2 H), 8.96 (s, 2 H), 8.47 (t, J = 6 Hz, 1 H), 7.93 (s, 1 H), 7.61 (m, 6 H), 7.42 (m, 1 H), 6.91 (m, 2 H), 6.07 (dd, J = 17 and 9 Hz, 1 H), 5.35 (m, 1 H), 5.09 (dd, J = 17 and 11 H, 1 H), 3.38 (d, J = 6.5 Hz, 1 H), 3.0 (t, J = 7 Hz, 2 H), 1.78 (m, 1 H), 1.72 (s, 3 H), 1.41 (s, 3 H), 0.84 (d, J = 6.9 Hz, 6 H); MS (ES⁺): 527.5 |
| **27y** | 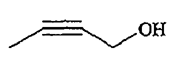 | 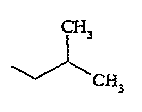 | **26y** | I-2 | ¹H NMR (DMSO-d₆): δ 8.99 (s, 2 H), 8.86 (s, 2 H), 8.52 (t, J = 6 Hz, 1 H), 8.03 (m, 1 H), 7.63 (m, 6 H), 7.50 (d, J = 7 Hz, 1 H), 7.07 (d, J = 7 Hz, 1 H), 7.12 (m, 1 H), 5.40 (t, J = 6 Hz, 1 H), 4.33 (d, J = 6.0 Hz, 2 H), 3.01 (t, J = 7 Hz, 2 H), 1.80 (m, 1 H), 0.84 (d, J = 6.9 Hz, 6 H); MS (ES⁺): 513.4 |
| **27z** | 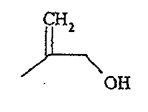 | 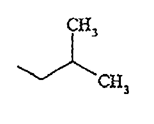 | **26z** | I-2 | ¹H NMF (DMSO-d₆): δ 9.50 (bs, 1 H), 8.77 (bs, 2 H), 8.49 (t, J = 6 Hz, 1 H), 7.98 (m, 1 H), 7.63 (m, 6 H), 7.55 (d, J = 6.9 Hz, 1 H), 7.01 (d, J = 7.9 Hz, 1 H), 6.99 (m, 1 H), 5.55 (s, 1 H), 5.38 (s, 1 H), 5.13 (t, J = 5 Hz, 1 H), 4.39 (d, J = 5 Hz, 2 H), 3.02 (t, J = 6.9 and 6.0 Hz, 2 H), 1.81 (m, 1 H), 0.86 (d, J = 6.9 Hz, 6 H); MS (ES⁺): 515.4 |
| **27aa** | 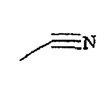 | 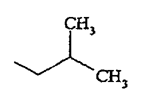 | **26aa** | I-2 | ¹H NMR (DMSO-d₆): δ 9.08 (s, 2 H), 8.73 (s, 2 H), 8.53 (t, J = 6 Hz, 1 H), 8.06 (s, 1 H), 8.02 (bs, 1 H), 7.94 (d, J = 7.8 Hz, 1 H), 7.62 (m, 6 H), 7.24 (d, J = 7.8 Hz, 1 H), 6.95 (d, J = 7.8 Hz, 1 H), 3.03 (t, J = 6 Hz, 2 H), 1.82 (m, 1 H), 0.87 (d, J = 6.8 Hz, 6 H); MS (ES⁺): 484.3 |
| **27ab** | 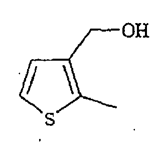 | 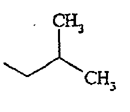 | **26ab** | I-2 | ¹H NMR (DMSO-d₆): δ 9.05 (bs, 2 H), 8.81 (bs, 2 H), 8.49 (t, J = 6 Hz, 1 H), 8.02 (s, 1 H), 7.68 (s, 1 H), 7.62 (m, 6 H), 7.53 (d, J = 6 Hz, 1 H), 7.21 (d, J = 6 Hz, 1 H), 7.13 (d, J = 7 Hz, 1 H), 7.01 (s, 1 H), 5.25 (t, J = 5 Hz, 1 H), 4.51 (d, J = 5 Hz, 2 H), 3.01 (t, J = 6 Hz, 2 H), 1.81 (m, 1 H), 0.85 (d, J = 6.8 Hz, 6 H); MS (ES⁺): 571.64 |
| **27ac** | 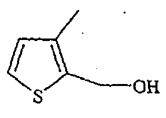 | 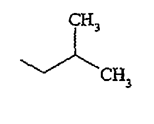 | **26ac** | I-2 | ¹H NMR (DMSO-d₆): δ 9.05 (bs, 2 H), 8.78 (s, 2 H), 8.52 (t, J = 6 Hz, 1 H), 8.02 (bs, 1 H), 7.65 (m, 6 H), 7.53 (d, J = 5 Hz, 1 H), 7.54 (d, J = 5 Hz, 1 H), 7.26 (d, J = 5 Hz, 1 H), 7.10 (m, 1 H), 6.99 (m, 1 H), 5.64 (t, J = 5 Hz, 1H), 4.71 (d, J = 5 Hz, 2H), 3.07 (t, J = 6.9 and 6.0 Hz, 2 H), 1.73 (m, 1 H), 0.84 (d, J = 6.9 Hz, 6 H); MS (ES⁺): 571.56 |
| **27ad** | 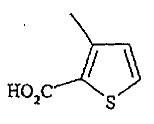 | 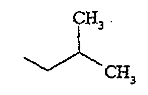 | **26ad** | I-2 | MS (ES⁺): 585.4 |
| **27ae** | 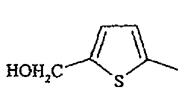 | 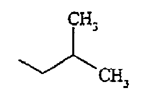 | **26ae** | I-2 | ¹H NMR (DMSO-d₆): δ 14.11 (bs, 1 H), 9.05 (bs, 2 H), 8.75 (bs, 2 H), 8.5 (m, 1 H), 8.0 (s, 1 H), 7.8-7.6 (m, 8 H), 7.49 (d, J = 3 Hz, 1 H), 7.1 (d, J = 6.9 Hz, 1 H), 7.0 (m, 1 H), 5.5 (m,1 H), 4.7 (m, 2 H), 3.09 (m, 2 H), 1.74 (m, 1 H) 0.86 (d, J = 6.9 Hz, 6 H); MS (ES+) 571.2 |
| **27af** | 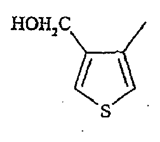 | 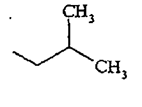 | **26af** | I-2 | ¹H NMR (DMSO-d₆): δ 14.11 (bs, 1 H), 9.05 (bs, 2 H), 8.75 (bs, 2 H), 8.49 (t, J = 6 Hz, 1 H), 7.97 (s, 1 H), 7.67 (d, J = 3 Hz, 1 H), 7.61 (m, 7 H), 7.54 (d, J = 3 Hz, 1 H), 7.06 (d, J = 6.9 Hz, 1 H), 6.89 (d, J = 6.9 Hz, 1 H), 5.23 (t, J = 5 Hz, 1 H), 5.42 (d, J = 5 Hz, 2 H), 3.09 (t, J = 6.9 and 6.0 Hz, 2 H), 1.74 (m, 1 H) 0.86 (d, J = 6.9 Hz, 6 H); MS (ES⁺): 571.3 |
| **27ag** | 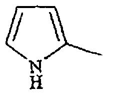 | 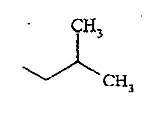 | **26ag** | I-2 | ¹H NMR (DMSO-d₆): δ 11.45 (s, 1 H), 9.08 (bs, 2 H), 8.88 (bs, 2 H), 8.75 (t, J = 6 Hz, 1 H), 8.04 (bs, 1 H), 7.88 (m, 1 H), 7.7 (m, 7 H), 7.03 (m, 2 H), 6.9 (m, 1 H), 6.62 (m, 1 H), 6.17 (m, 1 H), 3.07 (t, J = 6.9 and 6.0 Hz, 2 H), 1.84 (m, 1 H), 0.86 (d, J = 6.9 Hz, 6 H); MS (ES⁺): 524.65 |
| **27ah** | 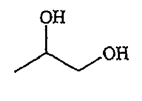 | 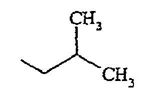 | **26ah** | I-2 | ¹H NMR (DMSO-d₆): δ 13.83 (s, 1 H), 8.9 (bs, 4 H), 8.47 (t, J = 6 Hz, 1 H), 7.95 (s, 1 H), 5.3 (s, 1 H), 7.61 (m, 6 H), 7.4 (m, 1 H), 6.95 (d, J = 7.7 Hz, 1 H), 6.85 (d, J = 7.7 Hz, 1 H), 6.64 (d, J = 9 Hz, 1 H), 6.22 (s, 1 H), 4.6 (t, J = 5.1 Hz, 1 H), 3.51 (d, J = 5.6 Hz, 2 H), 3.01 (t, J = 7 Hz, 2 H), 1.8 (m, 1 H), 0.85 (d, J = 6.9 Hz, 6 H); MS (ES⁺): 519.52 |
| **27ai** | 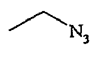 | 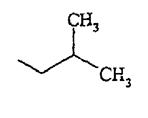 | **26ai** | I-2 | MS (ES+) 514.25 |
| **27aj** | 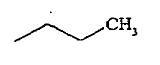 | 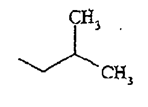 | **26n** | G | ¹H NMR (DMSO-d₆): δ 9.05 (s, 2 H), 8.67 (s, 2 H), 8.47 (t, J = 6 and 5 Hz, 1 H), 7.95 (m, 1 H), 7.95 (m, 1 H), 7.63 (m, 5H), 7.40 (s, 1 H), 7.38 (d, J = 7.7 Hz, 1 H), 6.92 (m, 2 H), 3.02 (t, J = 6.8 Hz, 2 H), 2.64 (m, 2 H), 1.80 (m, 1 H), 1.66 (m, 2 H), 0.96 (t, J = 8 and 6.5 Hz, 3 H), 0.85 (d, J = 6.8 Hz, 6 H); MS (ES-) 499.31 |
| **27ak** | 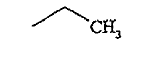 | 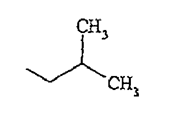 | **32f** | G | ¹H NMR (DMSO-d₆): δ 14.3 (bs, 1 H), 9.05 (bs, 2 H), 8.75 (bs, 2 H), 8.5 (m, 1 H), 8.0 (s, 1 H), 7.8-7.6 (m, 8 H), 7.49 (d, J = 3 Hz, 1 H), 7.1 (d, J = 6.9 Hz, 1 H), 7.0 (m, 1 H), 5.5 (m, 1 H), 4.7 (m, 2 H), 3.09 (m, 2 H), 1.74 (m, 1 H), 0.86 (d, J = 6.9 Hz, 6 H); MS (ES+) 487.2 |
| **27al** | 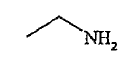 | 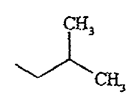 | **26ai** | G | MS (ES+) 488.3 (100%: M⁺¹) |
| **27am** | 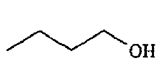 | 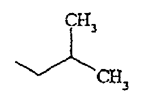 | **26u** | G | ¹H NMR (DMSO-d₆): δ 13.9 (bs, 1 H), 9.05 (2 bs, 4 H), 8.5 (m, 1 H), 7.9 (s, 1 H), 7.7-7.5 (m, 8 H), 7.3 (d, J = 3 Hz, 1 H), 6.9 (m, 2 H), 4.6 (m, 1H), 3.5 (m, 2 H), 3.09 (m, 2 H), 2.6 (m, 2 H), 1.8 (m, 1 H) 0.85 (d, J = 6.9 Hz, 6 H); MS (ES+) 517.3 |
| **32a** | 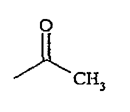 | 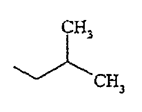 | **31a** | I-2 | ¹H NMR (DMSO-d₆): δ 9.84 (bs, 1 H), 9.07 (bs, 2 H), 8.87 (bs, 2 H), 8.51 (t, J = 6 and 5 Hz, 1 H), 8.13 (m, 1 H), 8.03 (m, 2 H), 7.65 (m, 5 H), 7.20 (d, J = 7.7 Hz, 1 H), 6.94 (d, J = 7.7.Hz, 1 H), 3.04 (t, J = 6.8 Hz, 2 H), 2.66 (s, 3 H), 1.83 (m, 1 H), 0.86 (d, J = 6.8 Hz, 6 H); MS (ES-) 499.4, (ES+) 501.4 |
| **32b** | 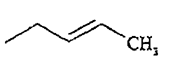 | 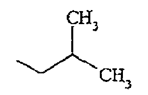 | **31b** | I-2 | Characterized in the next step |
| **32c** | 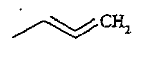 | 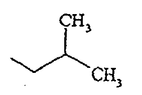 | **31c** | I-2 | ¹H NMR (DMSO-d₆): δ 14.24 (s, 1 H), 9.29 (bs, 2 H), 9.01 (bs, 2 H), 8.73 (t, J = 6 Hz, 1 H), 8.2 (d, J = 2 Hz, 1 H), 7.85 (m, 5 H), 7.74 (d, 2 Hz, 1 H), 7.4 (d, J = 8 Hz, 1 H), 7.22 (d, J = 7.4 Hz, 1 H), 7.13 (d, J = 7.5, 1 H), 6.73 (t, J = 6.8 Hz, 1 H), 5.59 (d, J = 6.8 Hz, 2 H), 3.25 (t, J = 6.8 Hz, 2 H), 2.04 (m, 1 H), 1.08 (d, J = 6.8 Hz, 6 H); MS (ES-) 495.1, (ES+) : 497.2 |
| **32d** | 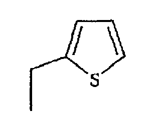 | 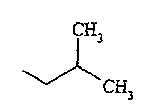 | **31d** | I-2 | MS (ES⁻) : 553.3 |
| **32e** | 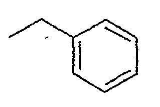 | 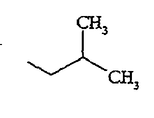 | **31e** | I-2 | ¹H NMR (DMSO-d₆): δ 13.642 (bs, 1 H), 9.06 (s, 2 H), 8.89 (s, 2 H), 8.50 (1, J = 6 and 5 Hz, 1 H), 7.98 (s, 1 H), 7.62 (m, 7 H), 7.43 (s, 1 H), 7.33 (m, 4 H), 6.95 (m, 2 H), 4.04 (s, 2 H), 3.02 (t, J = 6.8 Hz, 2 H), 1.80 (m, 1 H), 0.86 (d, J = 6.8 Hz, 6 H); MS (ES⁻) : 547.4 |
| **32f** | 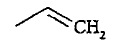 | 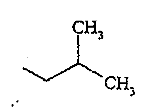 | **31f** | I-2 | ¹H NMR (DMSO-d₆): δ 0.85 (d, J = 6.9 Hz, 6 H), 1.81 (m, 1 H), 3.03 (t, J = 7 Hz, 2 H), 5.35 (d, J = 11 Hz, 1 H), 5.94 (d, J = 17 Hz, 1 H), 6.84 (dd, J = 17 and 11 Hz, 2 H), 7.0 (m, 2 H), 7.64 (m, 8 H), 8.01 (s, 1 H), 8.54 (t, J = 6 Hz, 1 H), 8.77 (s, 2 H), 9.06 (s, 2 H); MS (ES+) :485.57 |
| **32g** | 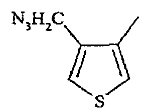 | 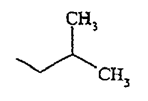 | **31g** | I-2 | MS (ES+) 596..2 |
| **32h** | 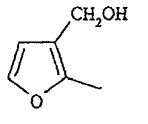 | 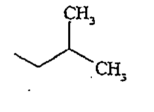 | **31h** | I-2 | ¹H NMR (DMSO-d₆): δ 14.2 (bs, 1 H), 9.1 (bs, 4 H), 8.6 (m, 1 H), 8.15 (s, 1 H), 7.9-7.6 (m, 8 H), 7.2 (m, 2 H), 6.7 (s, 1 H), 5.3 (br s, 1 H), 4.6 (m, 2 H), 3.1 (m, 2 H), 1.9 (m, 1 H), 0.9 (d, J = 6.7 Hz, 6 H); MS (ES+) 555.1 |
| **32i** | 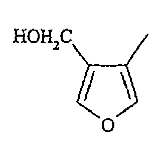 | 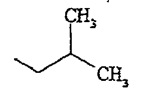 | **31i** | I-2 | ¹H NMR (DMSO-d₆): δ 13.84 (bs, 1 H), 9.01 (bs, 2 H), 8.80 (bs, 2 H), 8.46 (t, J = 6 and 5 Hz, 1 H), 8.03 (s, 1 H), 7.95 (s, 1 H), 7.77 (s, 1 H), 7.67 (m, 2 H), 7.61 (m, 5 H), 7.02 (d, J = 7.7 Hz, 1 H), 6.94 (m, 1 H), 5.13 (t, J = 5 Hz, 1 H), 4.47 (m, 2 H), 2.97 (t, J = 6.8 Hz, 2 H), 1.78 (m, 1 H), 0.80 (d, J = 6.8 Hz, 6 H); MS (ES-) 553.3, (ES+) 555.3 |
| **40** | 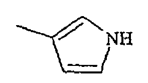 | 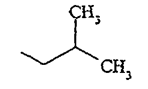 | **39** | I-2 | MS (ES+) 524.3 |
| **44** | 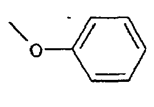 | 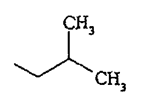 | **43** | I-2 | ¹H NMR (DMSO-d₆): δ 13.82 (s, 1 H), 9.20 (bs, 1 H), 9.10 (bs, 1 H), 8.51 (t, J = 6 Hz, 1 H), 7.97 (s, 1 H), 7.73-7.45 (m, 5 H), 7.43-7.39 (m, 2 H), 7.20 (t, J = 8 Hz, 1 H), 7.10 (m, 6 H), 6.96 (d, J = 8 Hz, 1 H), 3.0 (t, J = 6 Hz, 2 H), 1.80 (m, 1 H), 0.68 (d, J = 6.8 Hz, 6 H); MS (ES⁺) 551.30 |
| **46** | 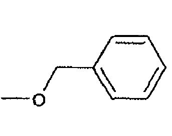 | 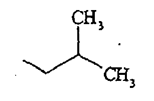 | **45** | I-2 | ¹H NMR (DMSO-d₆): δ 9.21 (2 bs, 2 H each, 4 H), 8.61 (m, 1 H), 8.1 (s, 1H), 7.8-7.4 (m, 10 H), 7.3 (s, 1 H), 7.2 (d, J = 7 Hz, 1 H), 7.1 (m, 2 H), 5.2 (s, 2 H), 3.1 (m, 2 H), 1.8 (m, 1 H), 0.91 (d, J = 6.8 Hz, 6 H); MS (ES⁺) 565.27 |
| **51** | -OCH₃ | 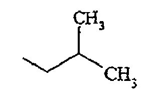 | **50** | I-2 | ¹H NMR (CF₃CO₂D): δ 8.43 (s, 1 H), 8.01 (d, J = 7.5 Hz, 1 H), 7.67 (q, J = 24 and 8.4 Hz, 4 H), 7.56 (d, J = 7.7 Hz, 1 H), 7.38 (s, 1 H), 7.23 (s, 2 H), 3.98 (s, 3 H), 3.43 (d, J = 7 Hz, 2 H), 2.01 (m, 1 H), 1.01 (d, J = 6.8 Hz, 6 H); MS (ES-) 487., (ES+) 489.3 |
| **53** | 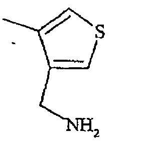 | 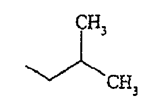 | **52** | I-2 | ¹H NMR (DMSO-d₆): δ 14.00 (bs, 1 H), 8.52 (t, J = 6 and 5 Hz, 1 H), 7.98 (s, 1 H), 7.63 (m, 8 H), 7.07 (d, J = 7.7 Hz, 1 H), 6.96 (d, J = 7.7 Hz, 1 H), 3.83 (s, 2 H), 3.02 (t, J = 6.8 Hz, 2 H), 1.81 (m, 1 H), 0.86 (d, J = 6.8 Hz, 6 H); MS (ES-) 568.1 |
| **70a** | 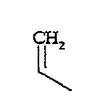 | 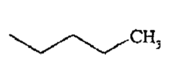 | **68a** | I-2, S | ¹H NMR (DMSO-d₆): δ 13.84 (br s, 1 H), 9.05 (s, 2 H), 8.94 (s, 2 H), 8.48 (t, *J* = 5.7 Hz, 1 H), 7.97 (d, *J* = 1.9 Hz, 1 H), 7.70 (m, 7 H), 7.00 (d, *J* = 7.9 Hz, 1 H), 6.92 (d, *J* = 7.9 Hz, 1 H), 6.84 (dd, *J* = 10.9 and 17.7 Hz, 1 H), 5.93 (d, *J* = 17.7 Hz, 1 H), 5.34 (d, *J* = 10.9 Hz, 1 H), 3.19 (m, 2 H), 1.45 (qui, *J* = 7.0 Hz, 2 H), 1.29 (sex, *J =* 7.0 Hz, 2 H), 0.87 (t, *J* = 7.3 Hz, 3 H); MS (ES⁺): 485.2 |
| **70b** | 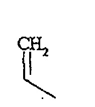 | 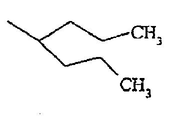 | **68b** | I-2, S | ¹H NMR (DMSO-d₆): δ 12.71 (br s, 1 H), 9.12 (s, 2 H), 8.93 (s, 2 H), 8.20 (m, 2 H), 7.86 (m, 1 H), 7.70 (m, 6 H), 7.20 (m, 2 H), 6.87 (dd, *J* = 10.9 and 17.7 Hz, 1 H), 5.99 (d, *J* = 17.7 Hz, 1 H), 5.40 (d, *J* = 10.9 Hz, 1 H), 3.97 (m, 1 H), 1.50-1.20 (m, 8 H) 0.86 (t, *J* = 7.2 Hz, 6 H); MS (ES⁺): 527.3 |
| **70c** | 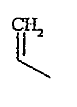 | 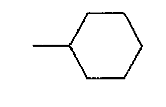 | **68c** | I-2, S | ¹H NMR (DMSO-d₆): δ 12.84 (br s, 1 H), 9.08 (m, 3 H), 8.36 (d, *J* = 7.7 Hz, 1 H), 8.18 (s, 1 H), 7.83 (m, 1 H), 7.67 (m, 6 H), 7.15 (m, 3 H), 6.86 (dd, *J* = 10.9 and 17.7 Hz, 1 H), 5.98 (d, *J* = 17.7 Hz, 1 H), 5.39 (d, *J* = 10.9 Hz, 1 H), 3.74 (m, 1 H), 1.84-1.55 (m, 5 H), 1.38-1.04 (m, 5 H); MS (ES⁺): 511.3 |
| **70d** | 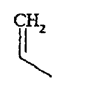 | 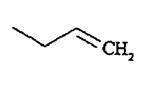 | **68d** | I-2, S | ¹H NMR (DMSO-d₆): δ 9.11 (s, 2 H), 8.89 (s, 2 H), 8.81 (t, *J* = 5.7 Hz, 1 H), 8.21 (s, 1 H), 7.85 (m, 1 H), 7.68 (m, 7 H), 7.17 (m, 3 H), 6.87 (dd, *J* = 10.9 and 17.7 Hz, 1 H), 5.99 (d, *J* = 17.7 Hz, 1 H), 5.88 (m, 1 H), 5.39 (d, *J* = 10.9 Hz, 1 H), 5.12 (m, 2 H), 3.88 (t, *J* = 5.0 Hz, 1 H); MS (ES⁺): 469.2 |
| **70e** | 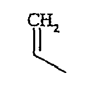 | 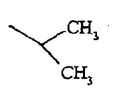 | **68e** | I-2, S | ¹H NMR (DMSO-d₆): δ 9.11 (s, 2 H), 9.01 (s, 2 H), 8.38 (d, *J* = 7.5 Hz, 1 H), 8.18 (s, 1 H), 7.83 (m, 1 H), 7.67 (m, 6 H), 7.16 (m, 3 H), 6.86 (dd, *J* = 10.9 and 17.7 Hz, 1 H), 5.98 (d, *J* = 17.7 Hz, 1 H), 5.39 (d, *J* = 10.9 Hz, 1 H), 4.09 (m, 1 H), 1.15 (d, *J* = 6.6 Hz, 6 H); MS (ES⁺): 471.3 |
| **70f** | 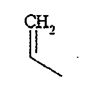 | 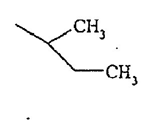 | **68f** | I-2, S | ¹H NMR (DMSO-d₆): δ 9.11 (s, 2 H), 9.05 (s, 2 H), 8.31 (d, *J* = 8.1 Hz, 1 H), 8.20 (s, 1 H), 7.85 (d, *J* = 7.7 Hz, 1 H), 7.69 (m, 6 H), 7.17 (m, 3 H), 6.86 (dd, *J* = 10.9 and 17.7 Hz, 1 H), 5.98 (d, *J* = 17.7 Hz, 1 H), 5.39 (d, *J* = 10.9 Hz, 1 H), 3.91 (m, 1 H), 1.50 (m, 2 H), 1.12 (d, *J* = 6.6 Hz, 3 H). 0.85 (t, *J* = 7.3 Hz, 3 H); MS (ES⁺): 485.3 |
| **70g** | 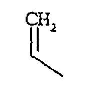 | 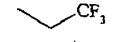 | **68g** | I-2, S | ¹H NMR (DMSO-d₆): δ 12.82 (br s, 1 H), 9.25 (m, 1 H), 9.12 (s, 2 H), 8.91 (s, 2 H), 8.23 (s, 1 H), 7.87 (m, 1 H), 7.68 (m, 7 H), 7.18 (m, 3 H), 6.87 (dd, *J* = 10.9 and 17.7 Hz, 1 H), 5.99 (d, *J* = 17.7 Hz, 1 H), 5.40 (d, *J* = 10.9 Hz, 1 H), 4.07 (m, 2 H); MS (ES⁺): 511.2 |
| **70h** | 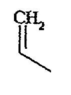 | 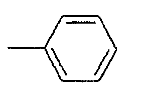 | **68h** | I-2, S | ¹H NMR (DMSO-d₆): δ 10.34 (s, 1 H), 9.05 (m, 4 H) 8.18 (s, 1 H), 7.71 (m, 11 H), 7.34 (t, *J* = 7.8 Hz, 2 H), 7.09 (m, 3 H), 6.86 (dd, *J* = 10.9 and 17.7 Hz, 1 H), 5.98 (d, *J* = 17.7 Hz, 1 H), 5.39 (d, *J* = 10.9 Hz, 1 H); MS (ES⁺): 505.3 |
| **70i** | 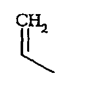 | 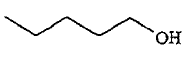 | **68i** | I-2, S | ¹H NMR (DMSO-d₆): δ 12.64 (br s, 1 H), 9.09 (m, 4 H), 8.56 (m, 1 H), 8.09 (s, 1 H), 7.66 (m, 9 H), 7.08 (m, 3 H), 6.86 (dd, *J* = 10.9 and 17.7 Hz, 1 H), 5.96 (d, *J* = 17.7 Hz, 1 H), 5.37 (d, *J* = 10.9 Hz, 1 H), 4.40 (m, 2 H) 3.39 (m, 2 H), 3.22 (m, 2 H), 1.48 (m, 4 H); MS (ES⁺): 501.3 (100%: M⁺¹) |
| **70j** | 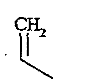 | 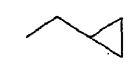 | **68j** | I-2, S | ¹H NMR (DMSO-d₆): δ 9.08 (m, 4 H), 8.69 (t, *J* = 6.0 Hz, 1 H), 8.16 (s, 1 H), 7.69 (m, 5 H), 7.13 (d, *J* = 7.7 Hz, 2 H), 7.09 (m, 3 H), 6.86 (dd, *J* = 10.9 and 17.7 Hz, 1 H), 5.97 (d, *J* = 17.7 Hz, 1 H), 5.38 (d, *J* = 10.9 Hz, 1 H), 3.11 (t, *J* = 6.0 Hz, 2 H), 1.01 (m, 1 H), 0.41 (m, 2 H), 0.21 (m, 2 H); MS (ES⁺): 483.3 |
| **70k** | 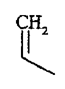 | 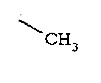 | **68k** | I-2, S | ¹H NMR (DMSO-d₆): δ 9.11 (s, 2 H), 8.97 (s, 2 H), 8.54 (m, 1 H), 8.12 (s, 1 H), 7.68 (m, 7 H), 7.17 (m, 4 H), 6.86 (dd, *J* = 10.9 and 17.7 Hz, 1 H), 5.97 (d, *J* = 17.7 Hz, 1 H), 5.38 (d, *J* = 10.9 Hz, 1 H), 2.75 (d, *J* = 4.3 Hz, 1 H); MS (ES⁺): 443.26 |
| **701** | 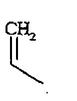 | 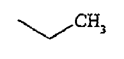 | **68l** | 1-2, S | ¹H NMR (DMSO-d₆): δ 9.07 (s, 2 H), 8.92 (s; 2 H), 8.53 (t, *J* = 5.5 Hz, 1 H), 8.02 (s, 1 H), 7.62 (m, 7 H), 7.01 (m, 2 H), 6.85 (dd, *J* = 10.9 and 17.7 Hz, 1 H), 5.95 (d, *J* = 17.7 Hz, 1 H), 5.36 (d, *J* = 10.9 Hz, 1 H), 3.24 (qui, *J* = 6.7 Hz, 2 H), 1.08 (t, *J* = 7.2 Hz, 3 H); MS (ES⁺): 457.2 |
| **70m** | 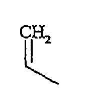 | 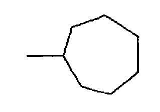 | **68m** | I-2, S | ¹H NMR (DMSO-d₆): δ 12.53 (br s, 1 H), 9.10 (m, 3 H), 8.38 (d, *J* = 7.9 Hz, 1 H), 8.11 (s, 1 H), 7.68 (m, 7 H), 7.12 (m, 3 H), 6.86 (dd, *J* = 10.9 and 17.7 Hz, 1 H), 5.96 (d, *J* = 17.7 Hz, 1 H), 5.37 (d, *J* = 10.9 Hz, 1 H), 3.94 (m, 1 H), 1.88-1.33 (m, 12 H); MS (ES⁺): 525.3 |
| **70n** | 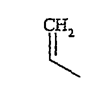 | 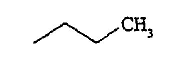 | **68n** | I-2, S | ¹H NMR (DMSO-d₆): δ 9.09 (m, 4 H), 8.59 (t, *J* = 5.2 Hz, 1 H), 8.17 (s, 1 H), 7.70 (m, 7 H), 7.16 (m, 4 H), 6.87 (dd, *J* = 10.9 and 17.7 Hz, 1 H), 5.98 (d, *J* = 17.7 Hz, 1 H), 5.39 (d, *J* = 10.9 Hz, 1 H), 3.20 (q, *J* = 6.7 Hz, 2 H), 1.52 (sex, *J* = 7.2 Hz, 2 H), 0.87 (t, *J* = 7.3 Hz, 3 H); MS (ES⁺): 471.3 |
| **70o** | 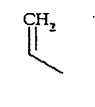 | 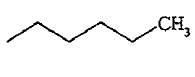 | **68o** | I-2, S | ¹H NMR (DMSO-d₆): δ 12.97 (br s, 1 H), 9.08 (s, 2 H), 8.99 (s, 2 H), 8.53 (t, *J* = 5.1 Hz, 1 H), 8.06 (s, 1 H), 7.64 (m, 7 H), 7.06 (m, 2 H), 6.85 (dd, *J* = 10.9 and 17.7 Hz, 1 H), 5.96 (d, *J* = 17.7 Hz, 1 H), 5.36 (d, *J* = 10.9 Hz, 1 H), 3.20 (q, *J* = 6.5 Hz, 2 H), 1.49 (qui, *J* = 6.6 Hz, 2 H), 1.27 (m, 4 H), 0.86 (t, *J* = 6.6 Hz, 3 H); MS (ES⁺): 499.3 |
| **70p** | 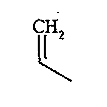 | 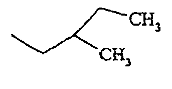 | **68p** | I-2, S | ¹H NMR (DMSO-d₆): δ 9.10 (s, 2 H), 8.91 (s, 2 H), 8.55 (t, J = 5.5 Hz, 1 H), 8.13 (s, 1 H), 7.68 (m, 7 H), 7.12 (m, 2 H), 6.86 (dd, *J* = 10.9 and 17.7 Hz, 1 H), 5.98 (d, *J* = 17.7 Hz, 1 H), 5.38 (d, *J* = 10.9 Hz, 1 H), 3.10 (m, 2 H), 1.62 (m, 1 H), 1.39 (m, 1 H), 1.10 (m, 1 H), 0.86 (m, 6 H); MS (ES⁺): 499.3 |
| **70q** | 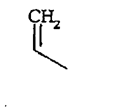 | 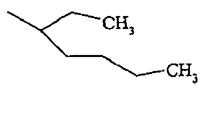 | **68q** | I-2, S | ¹H NMR (DMSO-d₆): δ 9.06 (s, 2 H), 8.82 (s, 2 H), 8.11 (t, *J* = 7.9 Hz, 1 H), 8.00 (s, 1 H), 7.62 (m, 7 H), 6.99 (m, 2 H), 6.85 (dd, *J* = 10.9 and 17.7 Hz, 1 H), 5.95 (d, *J* = 17.7 Hz, 1 H), 5.35 (d, *J* = 10.9 Hz, 1 H), 3.81 (q, *J* = 7.5 Hz, 1 H), 1.45 (m, 4 H), 1.24 (m, 4 H), 0.82 (m, 6 H); MS (ES⁺): 527.3 |
| **70r** | 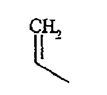 | 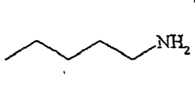 | **68r** | I-2, S | ¹H NMR (DMSO-d₆): δ 13.81 (s, 1 H), 8.44 (m, 4 H), 7.97 (s, 1 H), 7.61 (m, 7 H), 6.90 (m, 3 H), 5.93 (d, *J* = 17.7 Hz, 1 H), 5.34 (d, *J* = 10.9 Hz, 1 H), 3.22 (m, 5 H), 2.73 (m, 2 H), 1.52 (m, 4 H); MS (ES⁺): 500.3 |
| **70s** | 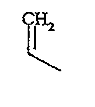 | 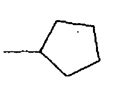 | **68s** | 1-2, S | ¹H NMR (DMSO-d₆): δ 9.09 (s, 2 H), 8.86 (s, 2 H), 8.42 (d, *J* = 7.5 Hz, 1 H), 8.11 (s, 1 H), 7.68 (m, 8 H), 7.10 (m, 2 H), 6.86 (dd, *J* = 10.9 and 17.7 Hz, 1 H), 5.97 (d, *J =* 17.7 Hz, 1 H), 5.38 (d, *J* = 10.9 Hz, 1 H), 4.20 (q, *J* = 7.2 Hz, 1 H), 1.93-1.44 (m, 8 H); MS (ES⁺): 497.2 |
| **70t** | 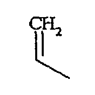 | 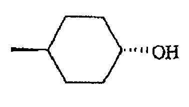 | **68t** | I-2, S | ¹H NMR (DMSO-d₆): δ 13.78 (br s, 1 H), 9.07 (s, 2 H), 8.87 (s, 2 H), 8.25 (d, *J* = 8.1 Hz, 1 H), 8.00 (s, 1 H), 7.62 (m, 7 H), 6.98 (m, 2 H), 6.85 (dd, *J* = 10.9 and 17.7 Hz, 1 H), 5.94 (d, *J* = 17.7 Hz, 1 H), 5.35 (d, *J* = 10.9 Hz, 1 H), 4.55 (d, *J* = 4.1 Hz, 1 H), 3.68 (m, 1 H), 3.39 (m, 1 H), 1.79 (m, 4 H), 1.28 (m, 4 H); MS (ES⁺): 527.2 |
| **70u** | 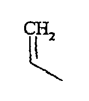 | 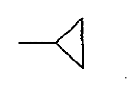 | **68u** | I-2, S | ¹H NMR (DMSO-d₆): δ 13.36 (br s, 1 H), 9.05 (m, 3 H), 8.49 (s, 1 H), 7.98 (s, 1 H), 7.61 (m, 8 H), 6.92 (m, 3 H), 5.94 (d, *J* = 17.7 Hz, 1 H), 5.35 (d, *J* = 10.9 Hz, 1 H), 2.81 (m, 1 H), 0.69-0.48 (m, 4 H); MS (ES⁺): 469.3 |
| **70v** | 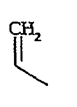 | 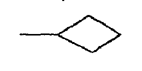 | **68v** | I-2, S | ¹H NMR (DMSO-d₆): δ 9.05 (m, 4 H), 8.75 (d, *J* = 7.5 Hz, 1 H), 8.15 (s, 1 H), 7.70 (m, 7 H), 7.14 (d, *J* = 7.9 Hz, 2 H), 6.86 (dd, *J* = 10.9 and 17.7 Hz, 1 H), 5.97 (d, *J* = 17.7 Hz, 1 H), 5.39 (d, *J* = 10.9 Hz, 1 H), 4.40 (q, *J* = 8.2 Hz, 1 H), 2.12 (m, 4 H) 1.65 (m, 2 H); MS (ES⁺): 483.3 |
| **70w** | 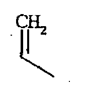 | 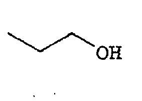 | **68w** | I-2, S | ¹H NMR (DMSO-d₆): δ 13.17 (br s, 1 H), 9.05 (m, 4 H), 8.51 (t, *J* = 5.8 Hz, 1 H), 8.06 (s, 1 H), 7.64 (m, 7 H), 7.03 (m, 2 H), 6.85 (dd, *J* = 10.9 and 17.7 Hz, 1 H), 5.95 (d, *J* = 17.7 Hz, 1 H), 5.36 (d, *J* = 10.9 Hz, 1 H), 4.72 (t, *J* = 5.4 Hz, 1 H) 3.47 (q, *J* = 5.7 Hz, 2 H), 3.28 (m, 2 H); MS (ES⁺): 473.2 |
| **70x** | 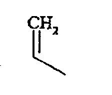 | 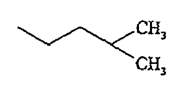 | **68x** | I-2, S | ¹H NMR (DMSO-d₆): δ 9.07 (s, 2 H), 8.90 (s, 2 H), 8.50 (t, *J* = 5.5 Hz, 1 H), 8.04 (s, 1 H), 7.63 (m, 7 H), 7.03 (m, 2 H), 6.85 (dd, *J* = 10.9 and 17.7 Hz, 1 H), 5.96 (d, *J* = 17.7 Hz, 1 H), 5.36 (d, *J* = 10.9 Hz, 1 H), 3.23 (q, *J* = 6.5 Hz, 2 H), 1.59 (m, *J* = 7.0 Hz, 1 H), 1.39 (q, *J* = 6.8 Hz, 2 H), 0.88 (d, *J* = 6.6 Hz, 6 H). |

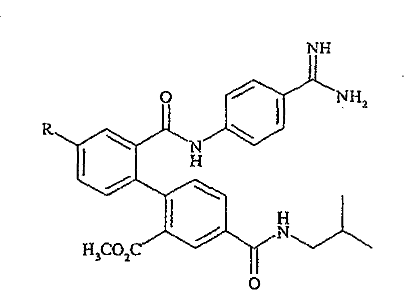

| **Cpd. No.** | **-R** | **Starting From** | **Method Used** | **Analytical Data** |
|---|---|---|---|---|
| **31a** | 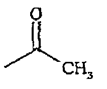 | **30a** | J | ¹HNMR (DMSO-d₆): δ 10.85 (s, 1 H), 9.21(s, 2 H), 8.91 (s, 2 H), 8.71 (t, J = 5.9 Hz, 1 H), 8.21 (d, J = 1.96 Hz, 1 H), 8.23 (d, J = 1.96 Hz, 1 H), 8.19 (d, J = 2.19 Hz, 1 H), 8.17 (d, J = 1.97 Hz, 1 H), 8.09 (d, J = 1.91 Hz, 1 H), 7.77 (s, 4 H), 7.53 (d, J = 7.53 Hz, 1 H), 3.57 (s, 3 H), 3.11 (q, J = 6.89 Hz, 1 H), 2.71 (s, 3 H), 1.86 (m, 1 H), 3.88 (d, 6.87 Hz, 6H); MS (ES+) 515.3 |
| **31b** | 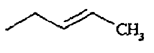 | **30b** | J | MS (ES⁺): 527.2 |
| **31c** | 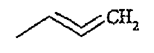 | **30c** | J | Characterized in the next step |
| **31d** | 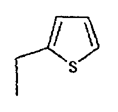 | **30d** | J | ¹HNMR (DMSO-d₆): δ 10.59 (bs, 1 H), 9.16 (s, 2 H), 8.85 (s, 2 H), 8.69 (t, J = 6 and 5 Hz, 1 H), 8.21 (s, 1 H), 8.04 (d, J = 1.5 Hz, 1 H), 7.73 (m, 4 H), 7.58 (s, 1 H), 7.50-7.38 (m, 3 H), 7.32 (m, 1 H), 7.03 (d, J = 7.5 Hz, 2 H), 4.31 (s, 2 H), 3.55 (s, 2 H), 3.07 (t, J = 6.8 Hz, 2 H), 1.85 (m, 1 H), 0.87 (d, J = 6.8 Hz, 6 H),; MS (ES-) 567.3, (ES+) 569.3 |
| **31e** | 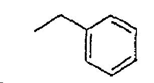 | **30e** | J | MS (ES⁻): 561.4; MS (ES⁺): 563.4 |
| **31f** | 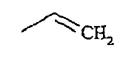 | **30f** | J | ¹H NMR (DMSO-d6): δ 10.73 (s, 1H), 9.24 (s, 2H), 9.00 (s, 2H), 8.71 (t, J = 5.7 Hz, 1H), 8.24 (d, J = 1.9 Hz, 1H), 8.05 (dd, J = 8.0, 1.9 Hz, 1H), 7.77 (m, 5H), 7.71 (dd, J = 79, 1.5 Hz, 1H), 7.42 (d, J = 7.9 Hz, 1H), 7.31 (d, J = 7.9 Hz, 1H), 6.89 (dd, J = 17.6, 11.0 Hz, 1H), 6.04 (d, J = 17.6 Hz, 1H), 5.42 (d, J = 11.0 Hz, 1H), 3.56 (s, 3H), 3.10 (t, J = 6.4 Hz, 2H), 1.85 (m, 1H), 0.89 (d, J = 6.7 Hz, 6H); MS (ES+): 499.3 |
| **31g** | 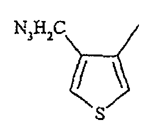 | **30g** | J | ¹HNMR (DMSO-d₆): δ 10.73 (s, 1 H), 9.19 (bs, 2 H), 8.88 (bs, 2 H), 8.71 (t, J = 6 Hz, 1 H), 8.27 (d, J = 2 Hz, 1 H), 8.07 (dd, J = 7.7 and 2 Hz, 1 H), 7.88 (d, 2 Hz, 1 H), 7.8 (d, J = 2 Hz, 1 H), 7.83 (m, 4 H), 7.72 (dd, J = 2 and 7.7 Hz, 1 H), 7.46 (d, J = 7.7, 1 H), 7.41 (d, J = 7.7 Hz, 1 H), 4.56 (s, 2 H), 3.56 (s, 3 H), 3.11 (t, J = 6.8 Hz, 2 H), 1.87 (m, 1 H), 0.92 (d, J = 6.8 Hz, 6 H); MS (ES-) 608.2, (ES+) 610.3 |
| **31h** | 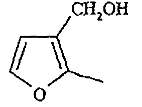 | **30h** | J | Characterized at the next step |
| **31i** | 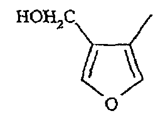 | **30i** | J | ¹HNMR (DMSO-d₆): δ 10.68 (s, 1 H), 9.17 (bs, 2 H), 8.82 (bs, 2 H), 8.68 (t, J = 6 Hz, 1 H), 8.25 (d, J = 2 Hz, 1 H), 8.16 (d, J = 2 Hz, 1 H), 8.05 (dd, J = 8 and 2 Hz, 1 H), 7.87 (m, 1 H), 7.89 (dd, J = 8 and 2 Hz, 1 H), 7.75 (m, 5 H), 7.44 (d, J = 9 Hz, 1 H), 7.36 (d, J = 8 Hz, 1 H), 5.22 (t, J = 5 Hz, 1 H), 4.54 (d, J = 5 Hz, 2 H), 3.57 (s, 3 H), 3.10 (t, J = 6.8 Hz, 2 H), 1.84 (m, 1 H), 0.88 (d, J = 6.8 Hz, 6 H; MS (ES-) 567.4, (ES+) 569.4 |
| **43** | 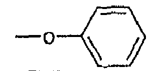 | **42** | J | MS (ES⁻): 563.4 |
| **45** | -Obn | **8** | J | Characterized in the next step |
| **50** | -OCH₃ | **49** | J | MS (ES⁺): 503.1 |
| **52** | 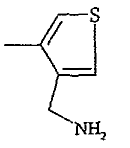 | **31g** | G | Characterized in the next step |

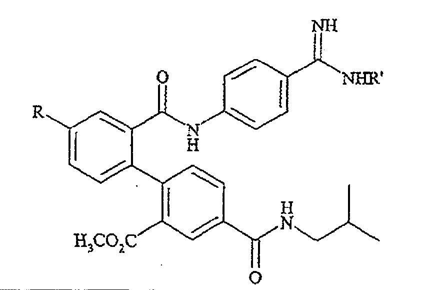

| **Cpd. No.** | **-R** | **-R'** | **Starting From** | **Method Used** | **Analytical Data** |
|---|---|---|---|---|---|
| **34** | -OSO₂CF₃ | -H | **33** | J | MS (ES⁺): 621.2 |
| **35** | -OSO₂CF₃ | 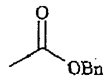 | **34** | P | MS (ES⁺): 755.2; (ES⁻) 753.3 |
| **37** | 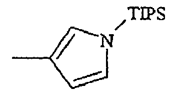 | 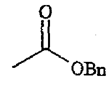 | **35 + 36** | D-2 | MS (ES⁺): 828.5 |
| **38** | 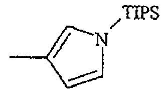 | -H | **37** | G | MS (ES⁺): 694.4; (ES⁻) 692.4 |
| **39** | 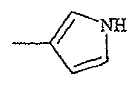 | -H | **38** | Q | Characterized in the next step |

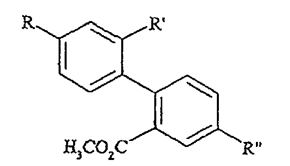

| **Cpd. No.** | **-R** | **-R'** | **-R"** | **Starting From** | **Method Used** | **Analytical Data** |
|---|---|---|---|---|---|---|
| **54** | -OBn | -CHO | -CO₂MEM | **5 + 6** | D-2 | ¹H NMR (DMSO-d₆): δ 9.69 (s, 1 H), 8.49 (d, *J* = 2.0 Hz, 1 H), 8.22 (d, *J* = 6.9 Hz, 1 H), 7.53 (m, 4 H), 7.43 (m, 2 H), 7.37 (m, 2 H), 7.24 (d, *J* = 8.9 Hz, 1 H), 5.57 (s, 2 H), 5.26 (s, 2 H), 3.85 (t, *J* = 4.9 Hz, 2 H), 3.60 (s, 3 H), 3.51 (t, *J* = 4.9 Hz 2 H), 3.32 (s, 3 H); MS (ES⁺): 501.02 (M+Na)⁺ |
| **55** | -OBn | -CO₂H | -CO₂MEM | **54** | E | ¹H NMR (DMSO-d₆): δ 12.65 (s, 1 H), 8.41 (d, *J* = 2.0 Hz 1 H), 8.14 (dd, *J* = 2.0 and 7.9 Hz, 1 H), 7.50 (m, 3 H), 7.38 (m, 4 H), 7.24 (dd, *J* = 3.0 and 8.9 Hz, 1 H), 7.11 (d, *J* = 8.9 Hz, 1 H), 5.54 (s, 2 H), 5.20 (s, 2 H), 3.82 (t, *J* = 4.9 Hz, 2 H), 3.57 (s, 3 H), 3.49 (t, *J* = 4.9 Hz, 2 H), 3.23 (s, 3 H); MS (ES⁻): 493.2 |
| **141** | -OBn | -CHO | 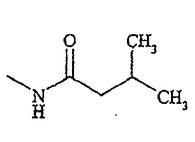 | **140 + 6** | D-2 | ¹H NMR (DMSO-d₆): δ 10.2 (s, 1 H), 9.65 (s, 1 H), 8.25 (d, *J* = 2.0 Hz, 1 H), 7.85 (dd, *J* = 2.0 and 8.9 Hz, 1 H), 7.51 (d, *J* = 7.9 Hz, 2 H), 7.45 (m, 2 H), 7.35 (m, 3 H),7.29 (d, *J* = 7.9 Hz, 1 H) 7.2 (d, *J* = 7.9 Hz, 1 H), 5.24 (s, 2 H), 3.55 (s, 3 H), 2.3 (d, *J* = 6.9 Hz, 2 H) 2.1 (m, *J* = 6.9 Hz, 1 H), 1.0 (d, *J* = 6.9 Hz, 6 H); MS (ES⁺): 446.31 |
| **142** | -OBn | -CO₂H | 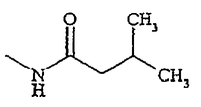 | **141** | E | ¹H NMR (DMSO-d₆): δ 12.38 (s, 1 H), 10.01 (s, 1 H), 8.05 (s, 1 H), 7.68 (d, *J* = 7.9 Hz, 1 H), 7.41 (d, *J* = 7.9 Hz, 2 H), 7.35 (m, 5 H), 7.27 (m, 1 H), 7.11 (d, *J* = 8.9 Hz, 1 H), 7.04 (d, *J* = 8.9 Hz, 1 H), 6.99 (d, *J* = 8.9 Hz, 1 H), 5.11 (s, 2 H), 2.13 (d, *J* = 6.9 Hz, 2 H), 2.02 (m, *J* = 6.9 Hz, 1 H), 0.852 (d, *J* = 6.9 Hz, 6 H); MS (ES⁻): 460.2 |
| **143** | -OBn | -CO₂MEM | 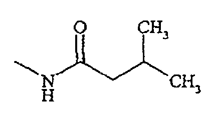 | **142** | F | ¹H NMR (DMSO-d₆): δ 10.12 (s, 1 H), 8.16 (d, *J* = 1.9 Hz, 1 H), 7.80 (dd, *J =* 1.9 and 8.3 Hz, 1 H), 7.42 (m, 6 H), 7.26 (dd, *J* = 2.8 and 8.3 Hz, 1 H), 7.13 (m, 2 H), 5.21 (s, 2 H), 5.17 (s, 2 H), 3.54 (s, 3 H), 3.40 (m, 2 H), 3.32 (m, 2 H), 2.22 (d, *J* = 7.0 Hz, 2 H), 2.10 (m, 4H), 0.95 (d, *J* = 6.4 Hz, 6H); MS (ES⁺): 572.3 (M+Na)⁺ |
| **144** | -OH | -CO₂MEM | 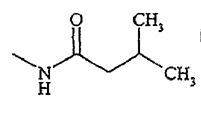 | **143** | G | ¹H NMR (DMSO-d₆): δ 12.7 (br s, 1 H), 9.09 (s, 2 H), 8.91 (s, 2 H), 8.57 (m, 1 H), 8.11 (s, 1 H), 7.92 (d, *J* = 1.9 Hz, 1 H), 7.81 (m, 3 H), 7.67 (m, 5 H), 7.14 (m, 3 H), 6.66 (m, 1 H), 4.40 (t, *J* = 5.3 Hz, 1 H), 3.39 (m, 2 H), 3.22 (m, 2 H), 1.48 (m, 4 H); MS (ES⁻): 592.2. |
| **145** | -OSO₂CF₃ | -CO₂MEM | 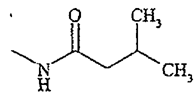 | **144** | B-2 | MS (ES⁺): 592.2 |
| **146a** | 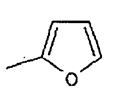 | -CO₂MEM | 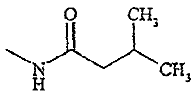 | **145** | D-2 | MS (ES⁺): 532.5 (M+Na)⁺ |
| **146b** | 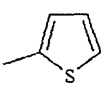 | -CO₂MEM | 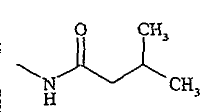 | **145** | D-2 | ¹H NMR (DMSO-d₆): δ 10.1 (s, 1 H), 8.21 (d, *J* = 2.0 Hz, 1 H), 8.10 (d, *J* = 2.0 Hz, 1 H), 7.89 (dd, *J* = 2.0 and 7.9 Hz, 1 H), 7.84 (d, *J* = 3.0 and 8.9 Hz, 1 H), 7.63 (m, 2 H), 7.25 (d, *J* = 7.9 Hz, 1 H), 7.19 (m, 2 H), 5.22 (d, *J* = 14.8 Hz, 2 H), 3.57 (s, 3 H), 3.43 (t, *J* = 4.9 Hz, 2 H), 3.34 (t, *J* = 4.9 Hz, 2 H), 3.20 (s, 3H), 2.23 (d, *J* = 6.9 Hz, 2 H), 2.11 (m, *J* = 6.9 Hz, 1 H), 0.96 (d, *J* = 5.9 Hz, 6 H); MS (ES⁺): 526.48 |
| **146c** | -CH=CH₂ | -CO₂MEM | 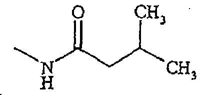 | **145** | D-3 | MS (ES⁺): 470.2 (M+Na)⁺ |
| **147a** | 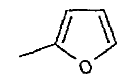 | -CO₂H | 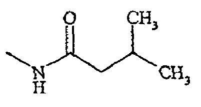 | **146a** | I-1 | MS (ES⁻); 420.29 |
| **147b** | 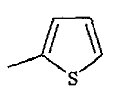 | -CO₂H | 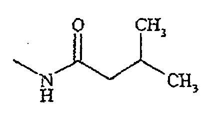 | **146b** | I-1 | ¹H NMR (DMSO-d₆): δ 12.65 (s, 1 H), 10.12 (s, 1 H), 8.18 (d, *J* = 1.9 Hz, 1 H), 8.07 (d, *J* = 3.0 Hz, 1 H), 7.83 (m, 2 H), 7.61 (m, 2 H), 7.19 (m, 3 H), 3.56 (s, 3 H), 2.22 (d, *J* = 6.9 Hz, 2 H), 2.11 (m, *J* = 6.9 Hz, 1 H), 0.96 (d, *J* = 6.9 Hz, 6 H); MS (ES⁺): 438.52 |
| **147c** | -CH=CH₂ | -CO₂H | 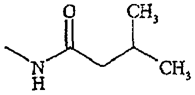 | **146c** | 1-1 | MS (ES⁻): 380.32 |
| **173** | -H | -CHO | 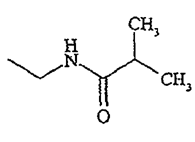 | **172 + 130** | D-2 | ¹H NMR (DMSO-d₆): δ 9.70 (s, 1 H), 8.42 (t, *J* = 6.2 Hz, 1 H), 7.90 (dd, *J* = 1.1 & 6.6 Hz, H), 7.82 (d, *J* = 1.9 Hz, 1 H), 7.72-7.50 (m, 3 H), 7.34 (d, *J* = 7.7 Hz, 1 H), 7.27 (dd, *J* = 1.3 & 6.2 Hz, 1 H), 4.38 (d, *J* = 6.0 Hz, 2 H), 3.53 (s, 3 H), 2.47 (m, 1 H), 1.07 (d, *J* = 7.0 Hz, 6 H); MS (ES⁺): 340.05 |
| **174** | -H | -CO₂H | 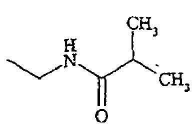 | **173** | E | ¹H NMR (DMSO-d₆): δ 12.35 (br s, 1 H), 8.31 (t, *J* = 7.5 Hz, 1 H), 7.80-7.31 (m, 5 H), 7.06 (m, 2 H), 4.25 (d, *J* = 6.0 Hz, 2 H), 3.41 (s, 3 H), 2.37 (m, 1 H), 0.97 (d, *J* = 7.0 Hz, 6 H); MS (ES⁻), 353.83 |
| **180** | -H | -CHO | 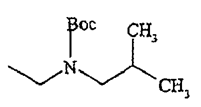 | **179 + 130** | D-2 | ¹H NMR (DMSO-d₆): 5 9.70 (s, 1 H), 7.87 (m, 2 H), 7.69 (m, 1 H), 7.55 (m, 2 H), 7.35 (d, *J* = 7.9 Hz, 1 H), 7.27 (d, *J* = 7.5 Hz, 1 H), 4.51 (s, 2 H), 3.52 (s, 3 H), 3.05 (m, 2 H), 1.92 (m, 1 H), 1.40 (m, 9 H), 0.85 (d, *J* = 6.8 Hz, 6 H); MS (ES⁺): 448.3 (M+Na)⁺ |
| **181** | -H | -CO₂H | 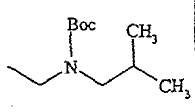 | **180** | E | ¹H NMR (DMSO-d₆): δ 7.81 (m, 2 H), 7.56 (m, 1 H), 7.44 (m, 2 H), 7.16 (m, 2 H), 4.47 (s, 2 H), 3.51 (s, 3 H), 3.02 (m, 2 H), 1.92 (m, *J* = 7.0 Hz, 1 H), 1.41 (m, 9 H), 0.85 (d, *J* = 6 Hz, 6 H), MS (ES⁻): 440.2 |
| **184a** | -OBn | -CHO | 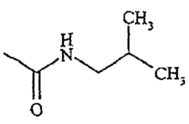 | **3a + 6** | D-2 | ¹H NMR (DMSO-d6): δ9.78 (s, 1H), 8.85 (t, J = 5.7 Hz, 1H), 8.50 (d, J = 2.0 Hz, 1H), 8.20 (dd, J = 8.2, 1.9 Hz, 1H), 7.55 (m, 9H), 5.35 (s, 2H), 3.69 (s, 3H), 3.23 (t, J = 6.5 H₂, 2H), 1.98 (m, 1H), 1.02 (d, J = 6.8 Hz, 6H); MS (ES+): 446.3 |
| **184b** | -OBn | -CHO | 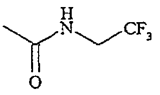 | **3f + 6** | D-2 | MS (ES⁻): 470.2 |
| **184c** | -OBn | -CHO | 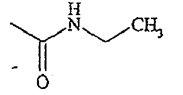 | **3i + 6** | D-2 | MS (ES⁻): 418.3 |
| **184d** | -OBn | -CHO | 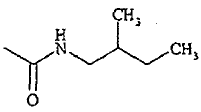 | **3j + 6** | D-2 | MS (ES⁺): 460.3 |
| **185a** | -OH | -CHO | 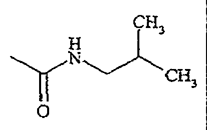 | **184a** | AD | ¹HNMR (DMSO-d₆): δ 10.06 (s, 1 H), 9.63 (s, 1 H), 8.73 (t, J = 6.5 Hz, 1 H), 8.36 (d, J = 2 Hz, 1 H), 8.09 (dd, J = 2 and 8 Hz, 1 H), 7.45 (d, J = 8 Hz, 1 H), 7.28 (s, 1 H), 7.11 (s, 2 H), 3.58 (s, 3 H), 3.13 (d, J = 7 Hz, 2 H), 1.87 (m, 1 H), 0.91 (d, J = 6.8 Hz, 6 H); MS (ES-): 354.2 and (ES⁺) 378.2 (M+Na)⁺) |
| **185b** | -OH | -CHO | 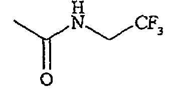 | **184b** | AD | MS (ES⁻): 380.1 |
| **185c** | -OH | -CHO | 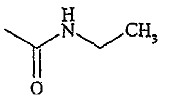 | **184c** | AD | ¹HNMR (DMSO-d₆): δ 10.21 (s, 1 H), 9.78 (s, 1 H), 8.87 (t, J = 5.80 Hz, 1 H), 8.51 (s, 1 H), 8.23 (d, J = 7.92 Hz, 1 H), 7.60 (d, J = 7.9 Hz, 1 H), 7.43 (s, 1 H), 7.25 (s, 2 H), 3.74 (s, 3 H), 3.46 (q, J = 5.65, 2 H), 1.32(t, J = 7.8 Hz, 3 H) |
| **185d** | -OH | -CHO | 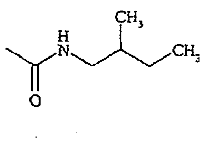 | **184d** | AD | ¹HNMR (DMSO-d₆): δ 10.06 (s, 1 H), 9.62 (s, 1 H), 8.69 (t, J = 5.90 Hz, 1 H), 8.36 (s, 1 H), 8.08 (d, J = 7.92 Hz, 1 H), 7.45 (d, J = 8.1 Hz, 1 H), 7.28 (s, 1 H), 7.10 (s, 2 H), 3.58 (s, 3 H), 3.22 (m, 1 H), 3.11 (m, 1 H), 1.66 (m, 1 H), 1.44 (m, 1 H), 1.18 (m, 1 H), 0.89(t, J = 6.4 Hz, 6 H). |
| **186a** | -OSO₂CF₃ | -CHO | 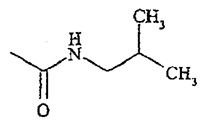 | **185a** | B-2 | MS (ES⁺): 488.24 |
| **186b** | -OSO₂CF₃ | -CHO | 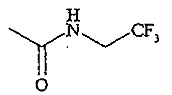 | **185b** | B-2 | ¹HNMR (DMSO-d₆): δ 9.74 (s, 1 H), 9.44 (t, J = 5.90 Hz, 1 H), 8.51 (s, 1 H), 8.11 (d, J = 7.91 Hz, 1 H), 7.54 (m, 4 H), 4.18 (m, 2 H), 3.59 (s, 3 H). |
| **186c** | -OSO₂CF₃ | -CHO | 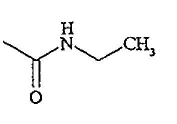 | **185c** | B-2 | ¹HNMR (DMSO-d6): δ 9.45 (s, 1 H), 8.59 (t, J = 5.90 Hz, 1 H), 8.28 (s, 1 H), 7.94 (d, J = 8.10 Hz, 1 H), 7.79 (d, J = 2.8 Hz, 1 H), 7.67 (d, J = 7.9 Hz, 1 H), 7.32 (d, J = 7.9 Hz, 2 H), 3.40 (s, 3 H), 3.12 (q, J = 7.1 Hz, 2 H), 0.97 (t, J = 7.16 Hz, 3 H). |
| **186d** | -OSO₂CF₃ | -CHO | 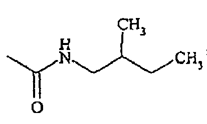 | **185d** | B-2 | ¹HNMR (DMSO-d₆): δ 9.71 (s, 1 H), 8.78 (t, J = 5.90 Hz, 1 H), 8.49 (s, 1 H), 8.18 (d, J = 7.92 Hz, 1 H), 8.00 (s, 1 H), 7.88 (d, J = 8.51 Hz, 1 H), 7.52 (q, J = 8.1 Hz, 2 H), 3.67 (s, 3 H), 3.22 (m, 1 H), 3.16 (m, 1 H), 1.68 (m, 1 H), 1.44 (m, 1 H), 1.18 (m, 1 H), 0.89(t, J = 6.4 Hz, 6 H). |
| **187a** | -CH=CH₂ | -CHO | 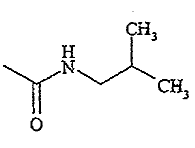 | **186a** | D-3 | ¹HNMR (DMSO-d₆): δ 9.74 (s, 1 H), 8.76 (t, J = 6.5 Hz, 1 H), 8-42 (d, J = 2 Hz, 1 H), 8.11 (dd, J = 2 and 8 Hz, 1 H), 8.00 (d, J =1.7 Hz, 1 H), 7.84 (dd, J = 8 and 2 Hz, 1 H), 7.47 (d, J = 8 Hz, 1 H), 7.27 (d, J = 8 Hz, 1 H), 6.90 (dd, J = 11 and 17.7 Hz, 1 H), 6.01 (d, J = 17.7 Hz, 1 H), 5.42 (d, J = 11 Hz, 1 H), 3.59 (s, 3 H), 3.14 (d, J = 7 Hz, 2 H), 1.88 (m, 1 H), 0.92 (d, J = 6.8 Hz, 6 H); MS (ES-): 364.2 and (ES⁺) 388.2 (M+Na)⁺ |
| **187b** | -CH=CH₂ | -CHO | 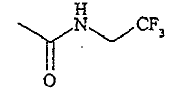 | **186b** | D-3 | MS (ES⁻): 390.1 |
| **187c** | -CH=CH₂ | -CHO | 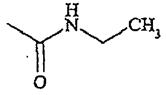 | **186c** | D-3 | MS (ES⁻): 336.2 |
| **187d** | -CH=CH₂ | -CHO | 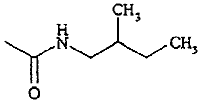 | **186d** | D-3 | MS (ES⁻): 378.2 |

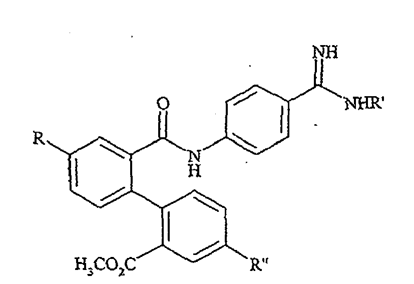

| **Cpd. No.** | **-R** | **-R'** | **-R"** | **Starting From** | **Method Used** | **Analytical Data** |
|---|---|---|---|---|---|---|
| **56** | -OBn | -H | -CO₂MEM | 55 | J | ¹H NMR (DMSO-d₆): δ 10.67 (s, 1 H), 9.2 (s, 2 H), 8.87 (s, 2 H), 8.33 (d, *J* = 2.0 Hz, 1 H), 8.17 (dd, *J* = 2.0 and 7.9 Hz, 1 H), 7.77 (s, 4 H), 7.49 (m, 4 H), 7.39 (m, 2 H), 7.30 (s, 2 H), 5.54 (s, 2 H), 5.27 (s, 2 H), 3.83 (t, *J* = 4.9 Hz, 2 H), 3.57 (s, 3 H), 3.49 (t, *J =* 4.9 Hz, 2 H), 3.23 (s, 3H); MS (ES⁺): 612.4 |
| **57** | -OBn | -Boc | -CO₂MEM | 56 | R | MS (ES⁺): 712.4 |
| **58** | -OH | -Boc | -CO₂MEM | 57 | G | ¹H NMR (DMSO-d₆): δ 10.4 (s, 1 H), 10.0 (s, 1 H), 8.9 (s, 1H), 8.28 (d, *J* = 2.0 Hz, 1 H), 8.12 (dd, *J* = 2.1 and 7.7 Hz, 1 H), 7.89 (d, *J* = 8.4 Hz, 2 H), 7.61 (d, *J* = 8.4 Hz, 2 H), 7.45 (d, *J* = 7.7 Hz, 1 H), 7.13 (d, *J* = 8.4 Hz, 1 H), 7.06 (s, 1 H), 6.98 (dd, *J* = 2.8 and 8.4 Hz, 1 H), 5.52 (s, 2 H), 3.81 (t, *J* = 4.9 Hz, 2 H), 3.56 (s, 3 H), 3.46 (t, *J* = 4.9 Hz, 2 H), 3.20 (s, 3 H), 1.43 (s, 9 H); MS (ES⁻): 620.5 |
| **59** | -OSO₂CF₃ | -Boc | -CO₂MEM | **58** | B-2 | ¹H NMR (DMSO-d₆): δ 10.55 (s, 1 H), 8.38 (d, *J* = 2.0 Hz, 1 H), 8.18 (dd, *J* = 2.0 and 7.9 Hz, 1 H), 7.86 (m, 4 H), 7.75 (dd, *J* = 2.0 and 8.9 Hz, 1 H), 7.54 (m, 5 H), 5.51 (s, 2 H), 3.77 (t, *J* = 4.9 Hz, 2 H), 3.55 (s, 3 H), 3.46 (t, *J* = 4.9 Hz, 2 H), 3.18 (s, 3 H) 1.41 (s, 9 H); MS (ES⁺): 754.3 |
| **60** | 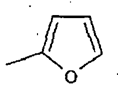 | -Boc | -CO₂MEM | **59** | D-2 | ¹H NMR (DMSO-d₆): δ 10.61 (s, 1 H), 8.94 (s, 1 H), 8.37 (s, 1 H), 8.19 (dd, *J* = 2.0 and 7.9 Hz, 1 H), 8.02 (s, 1 H), 7.89 (m, 5 H), 7.65 (d, *J* = 8.9 Hz, 2 H), 7.54 (d, *J* = 7.9 Hz, 1 H), 7.39 (d, *J* = 7.9 Hz, 1 H), 7.17 (d, J= 3.9 Hz, 1 H), 6.68 (m, 1 H), 5.54 (s, 2 H), 3.82 (t, *J* = 4.9 Hz, 2 H), 3.58 (s, 3 H), 3.49 (t, *J* = 4.9 Hz, 2 H), 3.22 (s, 3 H), 1.45 (s, 9 H); MS (ES⁺): 672.5 |
| **61** | 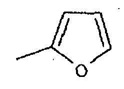 | -Boc | -CO₂H | **60** | I-1 | ¹H NMR (DMSO-d₆): δ 10.50 (s, 1 H), 8.96 (s, 1 H), 8.32 (s, 1 H), 8.07 (d, *J* = 7.9 Hz, 1 H), 7.98 (s, 1 H), 7.87 (m, 5 H), 7.63 (d, *J* = 8.9 Hz, 2 H), 7.38 (m, 2 H), 7.15 (d, *J* = 3.0 Hz, 1 H), 6.67 (m, 1 H), 3.57 (s, 3 H), 1.45 (s, 9 H); MS (ES¹): 582.4 |
| **66** | -CH=CH₂ | -Boc | -CO₂MEM | **59** | D-3 | ¹H NMR (DMSO-d₆): δ 10.56 (s, 1 H), 9.02 (br s, 1 H), 8.35 (d, *J* = 1.7 Hz, 1 H), 8.18 (dd, *J* = 1.9 and 6.0 Hz, 1 H), 7.88 (d, *J* = 9.0 Hz, 2 H), 7.80 (d, *J* =1.3 Hz, 1 H), 7.71 (dd, *J* = 1.7 and 6.2 Hz, 1 H), 7.63 (d, *J* = 8.9 Hz, 2 H), 7.50 (d, *J* = 8.3 Hz, 1 H), 7.32 (d, J= 8.1 Hz, 1 H), 6.89 (dd, *J* = 10.7 and 17.7 Hz, 1 H), 6.04 (d, *J* = 17.4 Hz, 1 H), 5.54 (s, 2 H), 5.43 (d, *J* = 11.7 Hz, 1 H), 3.82 (t, *J* = 4.5 Hz, 2 H), 3.57 (s, 3 H), 3.48 (t, *J* = 4.5 Hz, 2 H), 3.22 (s, 3 H), 1.44 (s, 9 H); MS (ES⁺): 632.1 |
| **67** | -CH=CH₂ | -Boc | -CO₂H | **66** | I-1 | ¹H NMR (DMSO-d₆): δ 10.49 (s, 1 H), 8.99 (br s, 1 H), 8.31 (s, 1 H), 8.07 (d, *J* = 8.3 Hz, 1 H), 7.87 (d, *J* = 9.0 Hz, 2 H), 7.77 (m, 2 H), 7.66 (m, 3 H), 7.38 (d, *J* = 7.7 Hz, 1 H), 7.29 (d, *J* = 7.7 Hz, 1 H), 6.88 (dd, *J* = 10.7 and 17.7 Hz, 1 H), 6.03 (d, *J* = 17.4 Hz, 1 H), 5.41 (d, *J* = 10.9 Hz, 1 H), 3.56 (s, 3 H), 1.43 (s, 9 H); MS (ES⁻): 542.1 |

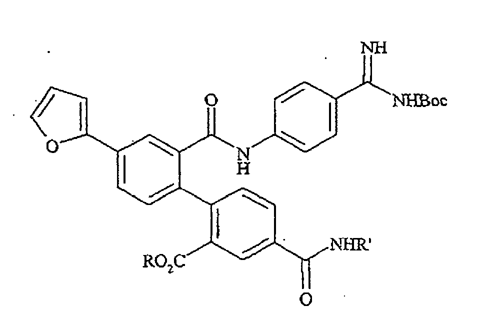

| **Cpd. No.** | **-R** | **-R'** | **Starting From** | **Method Used** | **Analytical Data** |
|---|---|---|---|---|---|
| **62a** | -CH₃ | 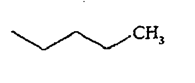 | **61** | A-4 | ¹H NMR (DMSO-d₆): δ 10.57 (s, I H), 8.92 (s, 1 H), 8.64 (t, *J* = 5.4 Hz, 1 H), 8.24 (d, *J* = 2.0 Hz, 1 H), 8.02 (dd, *J* = 2.0 and 7.9 Hz, 1 H), 7.98 (s, 1 H), 7.88 (m, 3 H) 7.84 (s, 1 H), 7.64 (d, *J* = 8.9 Hz, 2 H), 7.42 (d, *J* = 7.9 Hz, 1 H), 7.36 (d, *J* = 7.9 Hz, I H), 7.14 (d, *J* = 3.0 Hz, 1 H), 6.67 (m, 1 H), 3.55 (s, 3 H), 3.26 (m, 2 H), 1.50 (m, *J* = 7.4 Hz, 2 H), 1.43 (s, 9 H), 1.32 (m, *J* = 7.4 Hz, 2 H), 0.89 (t, 3 H); MS (ES⁻): 639.5 |
| **62b** | -CH₃ | 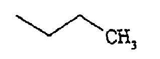 | **61** | A-4 | MS (ES⁺): 625.5 |
| **62c** | -CH₃ | 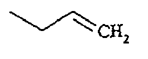 | **61** | A-4 | MS (ES⁺): 623.4 |
| **62d** | -CH₃ | 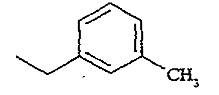 | **61** | A-4 | MS (ES⁺): 687.4 |
| **62e** | -CH₃ | 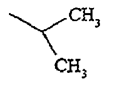 | **61** | A-4 | MS (ES⁺): 625.4 |
| **62f** | -CH₃ | 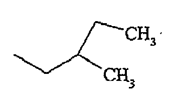 | **61** | A-4 | MS (ES⁺): 653.5 |
| **62g** | -CH₃ | 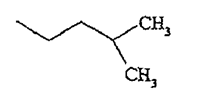 | **61** | A-4 | MS (ES⁺): 653.5 |
| **62h** | -CH₃ | 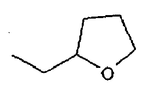 | **61** | A-4 | MS (ES⁺): 667.3 |
| **62i** | -CH₃ | 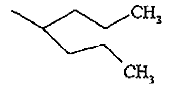 | **61** | A-4 | MS (ES⁺): 681.5 |
| **62j** | -CH₃ -CH₃ | 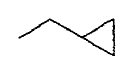 | **61** | A-4 | MS (ES⁺): 637.3 |
| **62k** | -CH₃ | 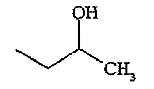 | **61** | A-4 | MS (ES⁺): 640.3 |
| **62i** | -CH₃ | 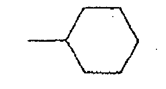 | **61** | A4 | MS (ES⁺): 665.4 |
| **62m** | -CH₃ | 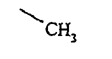 | **61** | A-4 | MS (ES⁺): 597.3 |
| **62n** | -CH₃ | 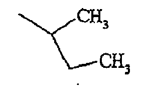 | **61** | A-4 | MS (ES⁺): 639.4 |
| **62o** | -CH₃ | 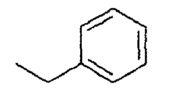 | **61** | A-4 | MS (ES⁺): 695.4 (M+Na)⁺ |
| **62p** | -CH₃ | 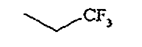 | **61** | A-4 | MS (ES⁻): 665.4 |
| **62q** | -CH₃ | 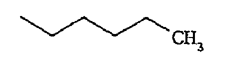 | **61** | A-4 | MS (ES⁺): 653.4 |
| **62r** | -CH₃ | 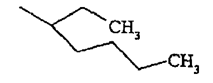 | **61** | A-4 | MS (ES⁺): 567.3 |
| **62s** | -CH₃ | 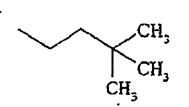 | **61** | A-4 | MS (ES⁺): 667.5 |
| **62t** | -CH₃ | 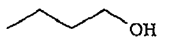 | **61** | A-4 | MS (ES⁺): 641.3 |
| **62u** | -CH₃ | 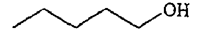 | **61** | A-4 | MS (ES⁺): 655.3 |
| **62v** | -CH₃ | 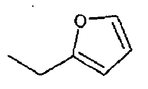 | **61** | A-4 | MS (ES⁺): 663.1 |
| **62w** | -CH₃ | 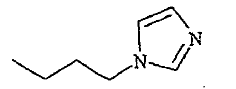 | **61** | A-4 | MS (ES⁻): 577.2 |
| **62x** | -CH₃ | 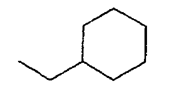 | **61** | A-4 | MS (ES⁺): 679.2 |
| **62y** | -CH₃ | 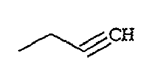 | **61** | A-4 | MS (ES⁺): 621.1 |
| **62z** | -CH₃ | 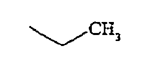 | **61** | A-4 | MS (ES⁺): 611.1 |
| **62aa** | -CH₃ | 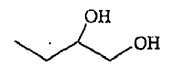 | **61** | A-4 | MS (ES⁺): 657.1 |
| **62ab** | -CH₃ | 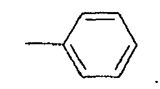 | **61** | A-4 | MS (ES⁺): 659.1 |
| **62ac** | -CH₃ | 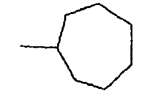 | **61** | A-4 | MS (ES⁺): 679.3 |
| **62ad** | -CH₃ | 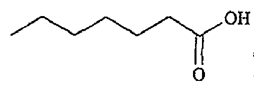 | **61** | A-4 | MS (ES⁻): 695.3 |
| **62ae** | -CH₃ | 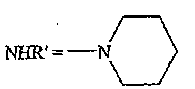 | **61** | A-4 | MS (ES⁺): 651.3 |
| **62af** | -CH₃ | 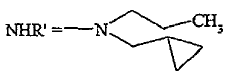 | **61** | A-4 | MS (ES⁺): 679.4 |

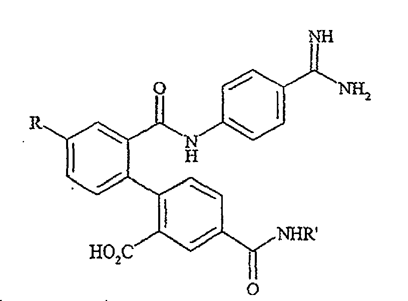

| **Cpd. No.** | **-R** | **-R'** | **Starting From** | **Method Used** | **Analytical Data** |
|---|---|---|---|---|---|
| **64a** | 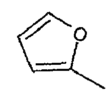 | 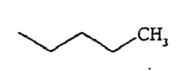 | **62a** | I-2, S | ¹H NMR (DMSO-d₆): δ 12.80 (s, 1 H), 9.09 (s, 2 H), 8.91 (s, 2 H), 8.57 (m, 1 H), 8.15 (s, 1 H), 7.91 (s, 1 H), 7.80 (m, 3 H), 7.67 (m, 4 H), 7.20 (m, 2 H), 7.07 (s, I H), 6.63 (s, 1 H) 3.21 (m, *J* = 5.9 Hz, 2 H), 1.46 (m, *J* = 7.4 Hz, 2 H), 1.28 (m, *J* = 7.4 Hz, 2 H) 0.86 (t, *J* = 7.4 Hz, 3 H); MS (ES⁺): 525.3 |
| **64b** | 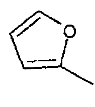 | 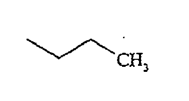 | **62b** | I-2, S | ¹H NMR (DMSO-d₆): δ 12.76 (s, 1 H), 9.10 (s, 2 H), 8.82 (s, 2 H), 8.59 (m, 1 H), 8.20 (s, 1 H), 7.95 (s, 1 H), 7.83 (m, 3 H), 7.70 (s, 4 H), 7.25 (m, 2 H), 7.10 (s, 1 H), 6.65 (s, 1 H), 3.20 (q, *J* = 6.0 Hz, 2 H), 1.51 (m, *J* = 7.4 Hz, 2 H), 0.87 (t, *J* = 7.4 Hz, 3 H); MS (ES⁺): 511.2 |
| **64c** | 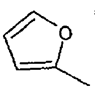 | 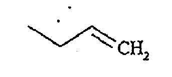 | **62c** | I-2, S | ¹H NMR (DMSO-d₆): δ 12.84 (s, 1 H), 9.11 (s, 2 H), 8.84 (m, 2 H), 8.26 (m, 1 H), 7.94 (m, 2 H), 7.83 (m, 3 H), 7.71 (s, 4 H), 7.28 (m, 2 H), 7.12 (s, 1 H), 6.65 (s, I H), 5.87 (m, 1 H), 5.15 (d, *J* = 17.2 Hz, 1 H), 5.07 (d, *J* = 10.3 Hz, 1 H) 3.88 (t, *J* = 5.2 Hz, 2 H); MS (BS⁺): 509.2 |
| **64d** | 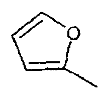 | 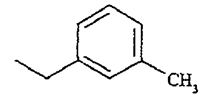 | **62d** | 1-2, S | ¹H NMR (DMSO-d₆): δ 12.78 (s, 1 H), 9.11 (m, 2 H), 8.85 (s, 2 H), 8.22 (s, 1 H), 7.93 (s, 1 H), 7.83 (m, 3 H), 7.68 (s, 4 H), 7.19 (m, 3 H), 7.10 (m, 5 H), 6.65 (s, 1 H), 4.41 (s, 2 H), 2.27 (s, 3 H); MS (ES⁺): 573.3 |
| **64e** | 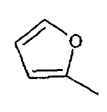 | 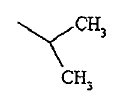 | **62e** | I-2, S | ¹H NMR (DMSO-d₆): δ 12.82 (s, 1 H), 9.11 (s, 2 H), 8.86 (s, 2 H), 8.39 (d, *J* = 7,7 Hz, 1 H), 8.24 (s, 1 H), 7.95 (s, 1 H), 7.90 (m, 1 H), 7.84 (m, 2 H), 7.71 (s, 4 H), 7.28 (m, 2 H), 7.11 (m, 1 H), 6.65 (s, 1 H), 4.08 (m, *J* = 6.9 Hz, 1 H), 1.14 (d, *J* = 6.9 Hz, 6 H); MS (ES⁺): 511.3 |
| **64f** | 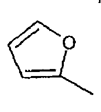 | 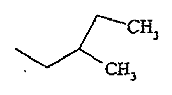 | **62f** | I-2, S | ¹H NMR (DMSO-d₆): δ 13.28 (br s, 1 H), 9.05 (m, 2 H), 8.84 (s, 2 H), 8.46 (m, 1 H), 7.99 (s, 1 H), 7.88 (s, 1 H), 7.77 (m, 2 H), 7.63 (m, 5 H), 7.07 (m, 2 H), 6.96 (m, 1 H), 6.63 (s, 1 H), 3.16-2.96 (m, 2 H), 1.65-1.03 (m, 3 H), 0.85 (m, 6 H); MS (ES⁺): 539.3 |
| **64g** | 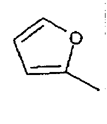 | 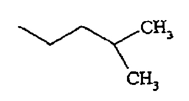 | **62g** | I-2, S | ¹H NMR (DMSO-d₆): δ 13.37 (s, I H), 9.06 (s, 2 H), 8.84 (s, 2 H), 8.47 (m, 1 H), 8.00 (s, 1 H), 7.88 (s, 1 H), 7.78 (m, 2 H), 7.70 (m, 5 H), 7.08 (m, 2 H), 6.97 (s, 1 H), 6.63 (s, 1 H), 3.22 (m, 2 H), 1.58 (m, *J* = 6.0 Hz, 1 H), 1.38 (m, *J* = 6.9 Hz, 2 H), 0.87 (d, *J* = 6.9 Hz, 6 H); MS (ES⁺): 539.3 |
| **64h** | 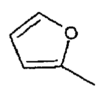 | 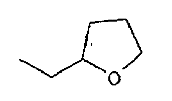 | **62h** | I-2, S | ¹H NMR (DMSO-d₆): δ 12.71 (br s, 1 H), 9.13 (s, 1 H), 8.75 (m, 3 H), 8.31 (m, 1 H), 7.97 (m, 2 H), 7.86 (m, 2 H), 7.73 (m, 4 H), 7.64 (m, 2 H), 7.33 (m, 2 H), 7.13 (m, 1 H), 6.67 (m, 1 H), 3.98 (m, 1 H), 3.77 (q, *J* = 6.9 Hz, 1 H), 3.62 (q, *J* = 6.9 Hz, 1 H), 3.29 (m, 2 H), 1.86 (m, 3 H), 1.59 (m, 1 H); MS (ES⁺): 553.3 |
| **64i** | 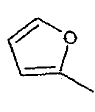 | 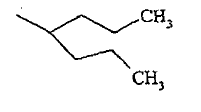 | **62i** | I-2, S | ¹H NMR (DMSO-d₆): δ 12.81 (br s, 1 H), 9.13 (s, 2 H), 8.85 (s, 2 H), 8.26 (m, 2 H), 7.96 (m, 2 H), 7.86 (m, 2 H), 7.74 (m, 5 H), 7.32 (m, 1 H), 7.13 (m, 1 H), 6.67 (m, 1 H), 3.99 (m, 1 H), 1.5.-0.85 (m, 14 H); MS (ES⁺): 567.3 |
| **64j** | 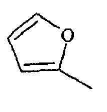 | 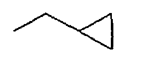 | **62j** | I-2, S | ¹H NMR (DMSO-d₆): δ 13.74 (br s, 1 H), 9.07 (s, 2 H), 8.92 (s, 2 H), 8.62 (t, *J* = 5.6 Hz, 1 H), 8.03 (s, 1 H), 7.89 (d, *J* = 1.7 Hz, 1 H), 7.79 (m, 2 H), 7.64 (m, 4 H), 7.10 (m, 3 H), 6.99 (d, *J* = 8.5 Hz, 1 H), 6.64 (m, 1 H), 3.08 (t, *J* = 6.0 Hz, 2 H), 1.00 (m, I H), 0.40 (m, 2 H), 0.20 (m, 2 H); MS (ES⁺): 523.4 |
| **64k** | 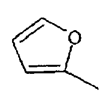 | 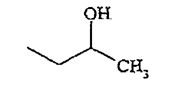 | **62k** | I-2, S | ¹H NMR (DMSO-d₆): δ 9.12 (s, 2 H), 8.88 (s, 2 H), 8.52 (m, 1 H), 8.12 (m, 1 H), 7.92 (m, 2 H), 7.81 (m, 3 H), 7.67 (m, 4 H), 7.14 (m, 3 H), 6.66 (m, 1 H), 4.75 (d, *J* = 4.5 Hz, 1 H), 3.77 (m, 1 H), 3.17 (m, 1 H), 1.04 (d, *J* = 6.0 Hz, 3 H); MS (ES⁺): 527.2 |
| **64l** | 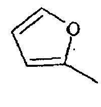 | 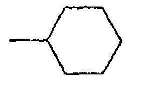 | **62l** | I-2, S | ¹H NMR (DMSO-d₆): δ 13.91 (br s, 1 H), 9.07 (s, 2 H), 8.90 (s, 2 H), 8.29 (d, *J* = 8.1 Hz, 1 H), 8.00 (s, 1 H), 7.89 (m, 1 H), 7.78 (m, 2 H), 7.64 (m, 5 H), 7.08 (m, 2 H), 6.96 (d, *J* = 7.7 Hz I H), 6.64 (m, 1 H), 3.71 (m, 1 H), 1.82-1.03 (m, 10 H)p; MS (ES⁺): 551.33 |
| **64m** | 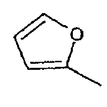 | 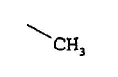 | **62m** | I-2, S | ¹H NMR (DMSO-d₆): δ 13.87 (br s, 1 H), 9.07 (s, 2 H), 8.90 (s, 2 H), 8.48 (m, 1 H), 7.99 (s, 1 H), 7.89 (m, 1 H), 7.79 (m, 2 H), 7.62 (m, 5 H), 7.10 (m, 2 H), 6.97 (d, *J* = 7.9 Hz 1 H), 6.64 (m, 1 H), 2.73 (d, *J* = 4.5 Hz, 3 H); MS (ES⁺): 483.2 |
| **64n** | 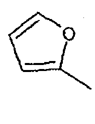 | 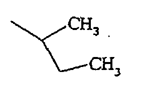 | **62n** | **I-2,** S | ¹H NMR (DMSO-d₆): δ 9.08 (s, 2 H), 8.85 (s, 2 H), 8.26 (d, *J* = 8.7 Hz, I H), 8.07 (s, 1 H), 7.91 (s, 1 H), 7.80 (m, 2 H), 7.67 (m, 5 H), 7.09 (m, 3 H), 6.65 (m, 1 H), 3.89 (m, *J* = 7.0 Hz, I H), 1.49 (m, *J* = 6.9 Hz, 2 H), 1.10 (d, *J* = 6.6 Hz, 3 H), 0.85 (t, *J* = 7.2 Hz, 3 H); MS (ES⁺): 525.2 |
| **64o** | 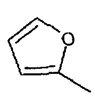 | 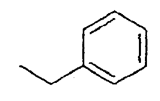 | **62o** | I-2, S | ¹H NMR (DMSO-d₆): δ 9.19 (m, 2 H), 9.10 (s, 2 H), 8.82 (s, 2 H), 8.19 (m, 1 H), 7.94 (s, 1 H), 7.83 (m, 2 H), 7.68 (m, 4 H), 7.33-7.10 (m, 8 H), 6.66 (m, 1 H), 4.45 (d, *J* = 5.7 Hz, 2 Hz); MS (ES⁺): 559.2 |
| **64p** | 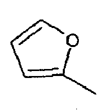 | 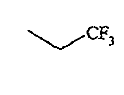 | **62p** | I-2, S | ¹H NMR (DMSO-d₆): δ 9.22 (m, 2 H), 9.09 (s; 2 H), 8.81 (s, 2 H), 8.17 (m, 1 H), 7.95 (s, 1 H), 7.82 (m, 2 H), 7.68 (m, 4 H), 7.16 (m, 4 H), 6.66 (m, 1 H), 4.06 (m, 2 H); MS (ES⁺): 551.22 |
| **64q** | 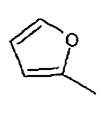 | 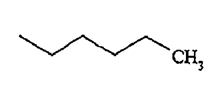 | **62q** | I-2, S | ¹H NMR (DMSO-d₆): δ 9.10 (s, 2 H), 8.86 (s, 2 H), 8.56 (m, 1 H), 8.13 (m, 1 H), 7.93 (s, 1 H), 7.82 (m, 2 H), 7.67 (m, 5 H), 7.15 (m, 3 H), 6.66 (m, 1 H), 3.19 (m, 2 H), 1.50 (m, 2 H), 1.28 (m, 4 H), 0.87 (t, *J* = 7.0 Hz, 3 H); MS (ES⁺): 539.3 |
| **64r** | 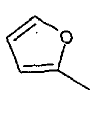 | 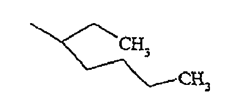 | **62r** | I-2, S | ¹H NMR (DMSO-d₆): δ 9.09 (s, 2 H), 8.90 (m, 2 H), 8.15 (m, 2 H), 7.93 (s, 1 H), 7.81 (m, 3 H), 7.68 (m, 4 H), 7.13 (m, 3 H), 6.66 (m, 1 H), 3.83 (m, 1 H), 1.47 (m, 4 H), 1.25 (m, 4 H), 0.83 (m, 6 H); MS (ES⁺): 567.3 |
| **64s** | 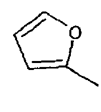 | 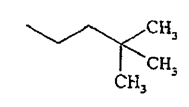 | **62s** | I-2, S | ¹H NMR (DMSO-d₆): δ 9.08 (s, 2 H), 8.86 (s, 2 H), 8.48 (m, 1 H), 8.03 (m, 1 H), 7.90 (s, 1 H), 7.79 (m, 2 H), 7.65 (m, 5 H), 7.12 (m, 2 H), 7.02 (m, 1 H), 6.65 (m, 1 H), 3.22 (m, 2 H), 1.42 (t, *J* = 8.2 Hz, 2 H), 0.91 (s, 9 H); MS (ES⁺): 553.4 |
| **64t** | 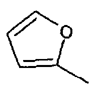 | 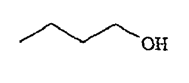 | **62t** | I-2, S | ¹H NMR (DMSO-d₆): δ 13.61 (br s, 1 H), 9.07 (s, 2 H), 9.00 (s, 2 H), 8.52 (t, *J* = 5.5 Hz, 1 H), 8.02 (s, 1 H), 7.90 (d, *J* = 1.9 Hz, 1 H), 7.79 (m, 2 H), 7.64 (m, 5 H), 7.10 (m, 2 H), 7.00 (d, *J* = 7.7 Hz, 1 H), 6.64 (m, 1 H), 4.47 (t, *J* = 5.3 Hz, 1 H), 3.43 (m, 2 H), 3.27 (m, 2 H), 1.64 (qui, *J* = 6.8 Hz, 2 H); MS (ES⁺): 527.23 |
| **64u** | 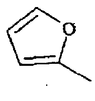 | 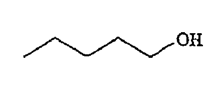 | **62u** | I-2, S | ¹H NMR (DMSO-d₆): δ 12.7 (br s, 1 H), 9.09 (s, 2 H), 8.91 (s, 2 H), 8.57 (m, 1 H), 8.11 (s, 1 H), 7.92 (d, *J* = 1.9 Hz, 1 H), 7.81 (m, 3 H), 7.67 (m, 5 H), 7.14 (m, 2 H), 6.66 (m, 1 H), 4.40 (t, *J* = 5.3 Hz, 1 H), 3.39 (m, 2 H), 3.22 (m, 2 H), 1.48 (m, 4 H); MS (ES⁺): 541.34 |
| **64v** | 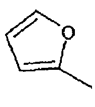 | 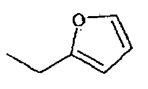 | **62v** | I-2, S | ¹H NMR (DMSO-d₆): δ 9.16-8.89 (m, 4 H), 8.16 (m, 1 H), 7.93 (s, 1 H), 7.81 (m, 3 H), 7.67 (m, 4 H), 7.56 (s, 1 H), 7.15 (m, 5 H), 6.65 (m, 1 H), 6.38 (m, 1 H), 6.26 (m, 1 H), 4.42 (d, *J* = 4.9 Hz, 2 H); MS (ES⁺): 549.27 |
| **64w** | 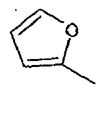 | 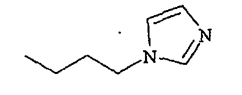 | **62w** | I-2, S | ¹H NMR (DMSO-d₆): δ 11.59 (br s, 1 H), 9.14 (s, 2 H), 8.98 (s, 2 H), 8.70 (t, *J* = 5.7 Hz, 1 H), 8.24 (s, 1 H), 7.99 (m, 2 H), 7.87 (m, 3 H), 7.71 (m, 3 H), 7.36 (s, 1 H), 7.27 (m, 2 H), 7.10 (m, 2 H), 6.67 (m, 1 H), 4.07 (t, *J* = 6.9 Hz, 2 H), 3.24 (q, *J* = 6.5 Hz, 2 H), 1.98 (qui, *J* = 6.7 Hz, 2 H); MS (ES⁺): 577.17 |
| **64x** | 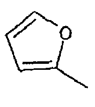 | 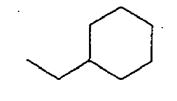 | **62x** | I-2, S | ¹H NMR (DMSO-d₆): δ 13.72 (br s, 1 H), 9.13 (s, 2 H), 9.06 (s, 2 H), 8.50 (t, *J* = 5.7 Hz, 1 H), 8.00 (d, *J* = 1.3 Hz, 1 H), 7.89 (d, *J* = 1.9 Hz, 1 H), 7.78 (m, 2 H), 7.62 (m, 4 H), 7.08 (m, 2 H), 6.96 (d, *J* = 7.9 Hz, 1 H), 6.64 (m, 1 H), 3.04 (t, *J* = 6.5 Hz, 2 H), 1.72-1.43 (m, 6 H), 1.25-1.08 (m, 3 H), 0.88 (m, 2 H); MS (ES⁺): 565.25 |
| **64y** | 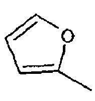 | 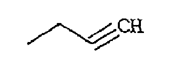 | **62y** | I-2, S | ¹H NMR (DMSO-d₆): δ 9.16-8.87 (m, 4 H), 8.09 (s, 1 H), 7.91 (s, 1 H), 7.80 (m, 2 H), 7.65 (m, 5 H), 7.12 (m, 5 H), 6.65 (m, 1 H), 4.01 (m, 2 H), 3.10 (m, 1 H); MS (ES⁺): 507.2 |
| **64z** | 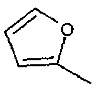 | 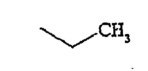 | **62z** | I-2, S | ¹H NMR (DMSO-d₆): δ 9.10 (s, 2 H), 8.97 (s, 2 H), 8.59 (t, *J* = 5.7 Hz, 1 H), 8.13 (s, 1 H), 7.93 (s, 1 H), 7.80 (m, 3 H), 7.68 (m, 4 H), 7.16 (m, 4 H), 6.65 (m, 1 H), 3.26 (qui, *J* = 6.0 Hz, 2 H), 1.10 (t, *J* = 7.2 Hz, 3 H); MS (ES⁺): 497.2 |
| **64aa** | 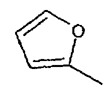 | 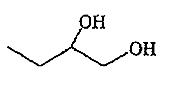 | **62aa** | I-2, S | ¹H NMR (DMSO-d₆): δ 14.1 (br s, 1 H), 9.08 (s, 2 H), 8.79 (s, 2 H), 8.45 (m, 1 H), 8.01 (s, 1 H), 7.90 (s, 1 H), 7.79 (m, 3 H), 7.63 (m, 5 H), 7.09 (m, 2 H), 6.98 (m, 1 H), 6.65 (m, 1 H), 4.80 (d, *J* = 4.7 Hz, 1 H), 4.56 (t, *J* = 6.8 Hz, 1 H), 3.60 (m, 1 H), 3.32-2.90 (m, 3 H); MS (ES⁺): 543.2 |
| **64ab** | 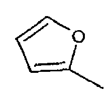 | 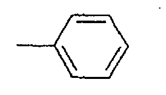 | **62ab** | I-2, S | ¹H NNM (DMSO-d₆): δ 10.34 (s, 1 H), 9.07 (s, 2 H), 8.85 (s, 2 H), 8.18 (s, 1 H), 7.93 (s, 1 H), 7.80 (m, 6 H), 7.66 (m, 4 H), 7.34 (m, 2 H), 7.11 (m, 4 H), 6.65 (m, 1 H); MS (ES⁺): 545.2 |
| **64ac** | 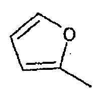 | 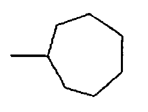 | **62ac** | I-2, S | ¹H NMR (DMSO-d₆): δ 9.07 (m, 4 H), 8.38 (d, *J* = 8.5 Hz, 1 H), 8.10 (s, 1 H), 7.92 (s, 1 H), 7.84-7.62 (m, 7 H), 7.11 (m, 3 H), 6.66 (m, 1 H), 3.94 (m, 1 H), 1.88-1.35 (m, 12 H); MS (ES⁺): 565.3 |
| **64ad** | 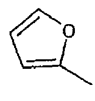 | 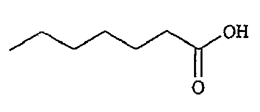 | **62ad** | I-2, S | ¹H NMR (DMSO-d₆): δ 13.71 (m, 2 H), 9.36-8.57 (m, 4 H), 8.50 (m, 1 H), 7.98 (s, 1 H), 7.89 (s, 1 H), 7.78 (2 H), 7.61 (m, 5 H), 7.08 (m, 2 H), 6.95 (d, *J* = 7.9 Hz, 1 H), 6.63 (m, 1 H), 3.19 (m, 2 H), 2.16 (t, *J* = 7.2 Hz, 2 H), 1.48 (m, 4 H), 1.28 (m, 2 H); MS (ES⁻): 581.2 |
| **64ae** | 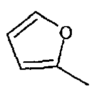 | 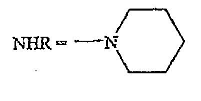 | **62ae** | I-2, S | ¹H NMR (DMSO-d₆): δ 9.12 (s, 2 H), 8.89 (s, 2 H), 7.91 (m, 1 H), 7.81 (m, 2 H), 7.70 (d, *J* = 8.7 Hz, 2 H), 7.62 (d, *J* = 8.9 Hz, 2 H), 7.48 (m, 1 H), 7.22 (m, 2 H), 7.11 (d, *J* = 3.4 Hz, 1 H), 7.05 (d, *J* = 7.2 Hz, 1 H), 6.65 (m, 1 H), 3.53 (m, 2 H), 3.08 (m, 2 H), 1.62-1.21 (m, 6 H); MS (ES⁺): 537.20 |
| **64af** | 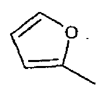 | 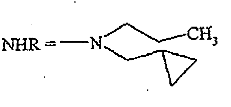 | **62af** | I-2, S | ¹H NMR (DMSO-d₆): δ 12.81 (br s, 1 H), 9.13 (s, 2 H), 8.82 (s, 2 H), 7.95 (s, 1 H), 7.85 (m, 2 H), 7.71 (m, 5 H), 7.43 (m, 1 H), 7.29 (m, 2 H), 7.13 (m, 1 H), 6.67 (m, 1 H), 3.49-2.97 (m, 4 H), 1.67-1.37 (m, 2 H), 1.08 (m, 1 H), 0.90 (m, 3 H), 0.61-0.26 (m, 4 H); MS (ES⁺): 565.3 |

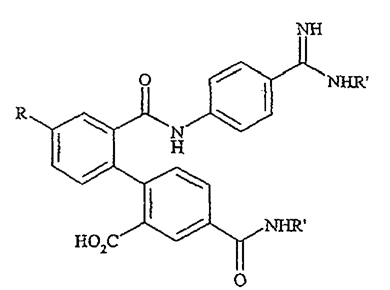

| **Cpd. No.** | **-R** | | **Starting From** | **Method Used** | **Analytical Data** |
|---|---|---|---|---|---|
| **65** | 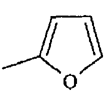 | 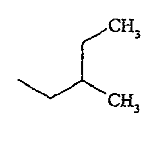 | **61** | A-4, I-2, S | ¹H NMR (DMSO-d₆, D₂O): δ 13.87 (br s, 1 H), 9.56 (m, 2 H) 9.21 (s, 1 H), 8.74 (s, 1 H), 8.47 (m, 1 H), 7.97 (m, 1 H), 7.88 (s, 1 H), 7.78 (m, 3 H), 7.58 (m, 7 H), 7.09 (m, 3 H), 6.96 (m, 1 H), 6.65 (m, 1 H), 3.14 (m, 4 H), 1.77-0.80 (m, 18 H); MS (ES⁺): 609.4 |
| **71a** | -CH=CH₂ | 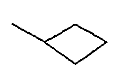 | **67** | A-4, I-2, S | ¹H NMR (DMSO-d₆): δ 13.80 (br s, 1 H), 9.91 (s, 1 H), 9.41 (s, 1 H), 8.63 (m, 2 H), 8.07 (s, 1 H), 7.98 (s, 1 H), 7.60 (m, 8 H), 6.90 (m, 3 H), 5.94 (d, *J* = 17.7 Hz, 1 H), 4.37 (m, 1 H), 4.16 (m, 1 H), 2.41-1.58 (m, 12 H); MS (ES⁺): 537.4 |
| **71b** | -CH=CH₂ | 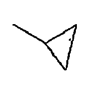 | **67** | A-4, I-2, S | ¹H NMR (DMSO-d₆): δ 9.76 (s, 1 H), 9.41 (s, 1 H), 8.95 (s, 1 H), 8.53 (m, 1 H), 8.07 (s, 1 H), 7.65 (m, 8 H), 7.08 (m, 2 H), 6.85 (dd, *J* = 10.9 and 17.7 Hz, 1 H), 6.92 (m, 3 H), 5.97 (d, *J* = 17.7 Hz, 1 H), 5.37 (d, *J* = 10.9 Hz, 1 H), 2.84 (m, 1 H), 2.70 (m, 1 H), 0.98-0.51 (m, 8H); MS (ES⁺): 509.4 |
| **71c** | -CH=CH₂ | 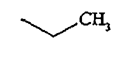 | **67** | A-4, I-2, S | ¹H NMR (DMSO-d₆): δ 12.51 (br s, 1 H), 9.59 (s, 1 H), 9.22 (s, 1 H), 8.79 (s, 1 H), 8.58 (t, *J* = 5.5 Hz, 1 H), 8.17 (s, 1 H), 7.67 (m, 8 H), 7.12 (m, 2 H), 6.86 (dd, *J* = 10.9 and 17.7 Hz, 1 H), 5.98 (d, *J* = 17.7 Hz, 1 H), 5.38 (d, *J* = 10.9 Hz, 1 H), 3.27 (m, 4 H), 1.20 (t, *J* = 7.2 Hz, 1 H), 1.09 (t, *J* = 7.2 Hz, 1 H); MS (ES⁺): 485.3 |

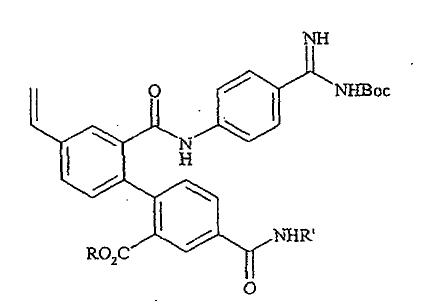

| **Cpd. No.** | **-R** | **-R'** | **Starting From** | **Method Used** | **Analytical Data** |
|---|---|---|---|---|---|
| **68a** | -CH₃ | 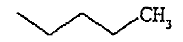 | **67** | A-4 | MS (ES⁺): 599.4 |
| **68b** | -CH₃ | 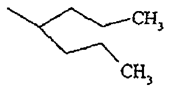 | **67** | A-4 | MS (ES⁺): 641.4 |
| **68c** | -CH₃ | 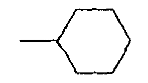 | **67** | A-4 | MS (ES⁺): 625.3 |
| **68d** | -CH₃ | 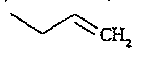 | **67** | A-4 | MS (ES⁺): 583.3 |
| **68e** | -CH₃ | 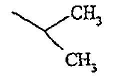 | **67** | A-4 | MS (ES⁺): 585.3 |
| **68f** | -CH₃ | 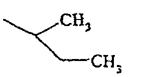 | **67** | A-4 | MS (ES⁺): 599.4 |
| **68g** | -CH₃ | 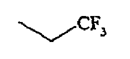 | **67** | A-4 | MS (ES⁺): 625.2 |
| **68h** | -CH₃ | 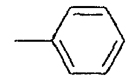 | **67** | A-4 | MS (ES⁺): 619.2 |
| **68i** | -CH₃ | 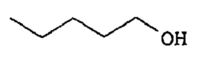 | **67** | A-4 | MS (ES⁺): 615.3 |
| **68j** | -CH₃ | 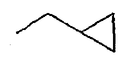 | **67** | A-4 | MS (ES⁺): 597.3 |
| **68k** | -CH₃ | 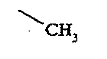 | **67** | A-4 | MS (ES⁺): 557.3 |
| **681** | -CH₃ | 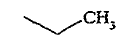 | **67** | A-4 | MS (ES⁺): 571.4 |
| **68m** | -CH₃ | 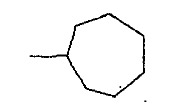 | **67** | A-4 | MS (ES⁺): 639.4 |
| **68n** | -CH₃ | 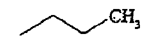 | **67** | A-4 | Characterized in the next step |
| **68o** | -CH₃ | 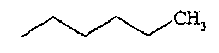 | **67** | A-4 | MS (ES⁺): 613.5 |
| **68p** | -CH₃ | 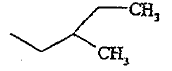 | **67** | A-4 | MS (ES⁺): 613.5 |
| **68q** | -CH₃ | 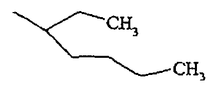 | **67** | A-4 | MS (ES⁺): 641.5 |
| **68r** | -CH₃ | 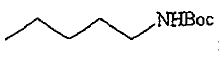 | **67** | A-4 | MS (ES⁺): 714.5 |
| **68s** | -CH₃ | 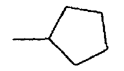 | **67** | A-4 | MS (ES⁺): 611.4 |
| **68t** | -CH₃ | 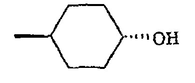 | **67** | A-4 | MS (ES⁺): 641.4 |
| **68u** | -CH₃ | 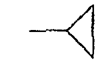 | **67** | A-4 | MS (ES⁺): 583.3 |
| **68v** | -CH₃ | 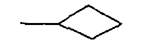 | **67** | A-4 | MS (ES⁺): 597.4 |
| **68w** | -CH₃ | 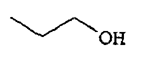 | **67** | A-4 | MS (ES⁺): 587.4 |
| **68x** | -CH₃ | 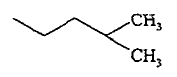 | **67** | A-4 | MS (ES⁺): 613.5 |

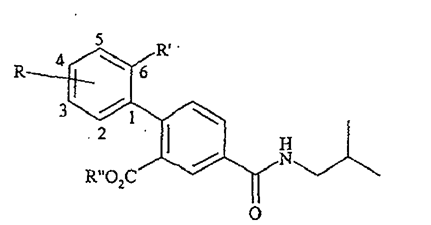

| **Cpd. No.** | **-R (Position with Respect to Phenyl Ring)** | **-R'** | **-R"** | **Starting From** | **Method Used** | **Analytical Data** |
|---|---|---|---|---|---|---|
| **74** | -OCH₃ (3) | -CHO | -CH₃ | **73 + 3a** | D-2 | MS (ES⁻): 368.2 |
| **75a** | -OH (3) | -CHO | -CH₃ | **74** | V-2,W | MS (ES⁻): 354.1 |
| **75b** | -OH (3) | -CHO | -Bn | **74** | V-1, H | MS (ES⁻): 430.2 |
| **76a** | -OSO₂CF₃ (3) | -CHO | -CH₃ | **75a** | B-2 | MS (ES⁺): 488.1 |
| **76b** | -OSO₂CF₃ (3) | -CHO | -Bn | **75b** | B-2 | MS (ES⁻): 562.3 ; MS (ES⁺): 586.3 (M+Na)⁺ |
| **77a** | -CH=CH₂ (3) | -CHO | -CH₃ | **76a** | D-3 | MS (ES⁺): 366.38 |
| **77b** | -OCH₂CO₂C₂H₅ (3) | -CHO | -Bn | **75b** | X | Characterized in the next step |
| **77c** | -OCH₂CONH₂ (3) | -CHO | -Bn | **75b** | X | MS (ES⁻): 487.3; MS (ES⁺): 511.35 (M+Na)⁺ |
| **77d** | 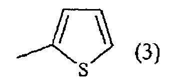 | -CHO | -Bn | **76b** | D-2 | Characterized in the next step |
| **77e** | 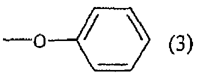 | -CHO | -Bn | **75b** | D-8 | MS (ES⁺): 530.3 (M+Na)⁺); MS (ES⁻): 506.3 |
| **77f** | 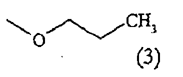 | -CHO | -Bn | **75b** | X | MS (ES⁺): 496.3 (M+Na)⁺ |
| **77g** | 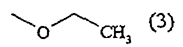 | -CHO | -Bn | **75b** | X | MS (ES+): 482.4 (M+Na)⁺ |
| **77h** | 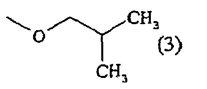 | -CHO | -Bn | **75b** | X | MS (ES⁺): 510.4 (M+Na)⁺ |
| **77i** | 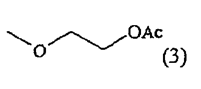 | -CHO | -Bn | **75b** | X | ¹HNMR (CDCl₃): δ 9.59 (s, 1 H), 8.39 (d, J = 2 Hz, 1 H), 8.03 (m, 2 H), 7.84 (d, J = 8.9 Hz, 1 H), 7.35 (d, J = 8 Hz, 1 H), 7.28 (m, 2 H), 7.12 (m, 2 H), 6:93 (dd, J = 2.5 and 8.8 Hz, 1 H), 6.64 (d, J = 2.5 Hz, 1 H), 6.31 (t, J = 6 and 5 Hz, 1 H), 5.06 (m, 2 H), 4.42 (t, J = 4.5 Hz, 2 H), 4.13 (m, 2 H), 3.34 (t, J =6.8 Hz, 2 H), 2.11 (s, 3 H), 1.94 (m, 1 H), 1.01 (d, J = 6.8 Hz, 6 H) |
| **78a** | -CH=CH₂ (3) | -CO₂H | -CH₃ | **77a** | E | MS (ES⁻): 380.1 |
| **78b** | -OSO₅CF₃ (3) | -CO₂H | -Bn | **76b** | E | Characterized in the next step |
| **78c** | -OCH₂CO₂C₂H₅ (3) | -CO₂H | -Bn | **77b** | E | Characterized in the next step |
| **78d** | -OCH₂CONH₂ (3) | -CO₂H | -Bn | **77c** | E | MS (ES⁺): 527.35 (M+Na)⁺ |
| **78e** | 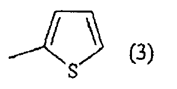 | -CO₂H | -Bn | **77d** | E | MS (ES⁺): 536.4 (M+Na)⁺ |
| **78f** | 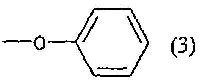 | -CO₂H | -Bn | **77e** | E | MS (ES⁻): 522.3 |
| **78g** | -OCH₃ (3) | -CO₂H | -CH₃ | **74** | E | MS (ES⁻): 384.1 |
| **78h** | 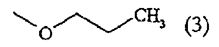 | -CO₂H | -Bn | **77f** | E | MS (ES⁻): 488.3 |
| **78i** | 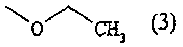 | -CO₂H | -Bn | **77g** | E | MS (ES⁻): 474.4 |
| **78j** | 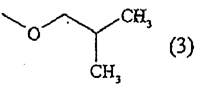 | -CO₂H | -Bn | **77h** | E | MS (ES⁻): 502.4 |
| **78k** | 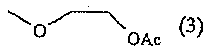 | -CO₂H | -Bn | **77i** | E | Characterized in the next step |
| **90** | -OBn (5) | -CHO | -CH₃ | **89 + 3a** | D-2 | ¹HNMR (CDCl₃): δ 10.47 (s, 1 H), 8.36 (d, J = 2 Hz, 1 H), 7.96 (dd, J = 2.2 and 7.7 Hz, 1 H), 7.68 (m, 2 H), 7.46 (m, 5 H), 7.23 (d, J = 8 Hz, 1 H), 7.12 (d, J = 8.7 Hz, 1 H), 6.73 (d, J = 7.2 Hz, 1 H), 5.23 (q, J = 11 and 15 Hz, 2 H), 3.67 (s, 3 H), 3.31 (t, J = 6.8 Hz, 2 H), 1.94 (m, 1 H), 1.01 (d, J = 6.8 Hz, 6 H), MS (ES+) 468.2 (M+Na)⁺ (ES-) 444.2 |

| | | **-R'** | **-R"** | **Starting From** | **Method Used** | **Analytical Data** |
|---|---|---|---|---|---|---|
| **91** | -OBn (5) | -CO₂H | -CH₃ | **90** | E | ¹HNMR (CDCl₃): δ 8.22 (s, 1 H), 7.83 (d, J = 7.2 Hz, 1 H), 7.34 (m, 8 H), 7.02 (d, J = 8.1 Hz, I H), 6.75 (d, J = 7.4 Hz, I H), 5.16 (s, 2 H), 3.66 (s, 3 H), 3.21 (t, J = 6.8 Hz, 2 H), 1.85 (m, 1 H), 0.94 (d, J = 6.8 Hz, 6 H), MS (ES+) 484.1 (M+Na)⁺ |
| **92** | -OBn (5) | -CO₂MEM | -CH₃ | **91** | F | MS (ES⁺): 572.2 (M+Na)⁺ |
| **93** | -OH (5) | -CO₂MEM | -CH₃ | **92** | G | MS (ES⁺): 482. (M+Na)⁺ |
| **94** | -OSO₂CF₃ (5) | -CO₂MEM | -CH₃ | **93** | B-2 | MS (ES⁺): 614.3 (M+Na)⁺ |
| **95a** | 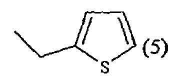 | -CO₂MEM | -CH₃ | **94** | D-3 | MS (ES+) 562.3 (M+Na)⁺ |
| **96a** | 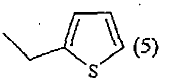 | -CO₂H | -CH₃ | **95a** | 1-1 | MS (ES+) 452.1 (M+Na)⁺ |
| **101** | -OCH₃ (2) | -CHO | -CH₃ | **100 + 3a** | D-2 | MS (ES+) 370.1 |
| **102** | -OCH₃ (2) | -CO₂H | -CH₃ | **101** | E | MS (ES⁻) 384.2; MS (ES⁺) 386.2 |
| **108** | -OBn (2) | -CHO | -CH₃ | **107 + 3a** | D-2 | MS (ES⁺): 446.2 |
| **109** | -OBn (2) | -CO₂H | -CH₃ | **108** | E | MS (ES⁻): 460.1 |
| **131** | -H | -CHO | -CH₃ | **130 + 3a** | D-2 | ¹HNMR (CDCl₃-dₜ): δ 9.79 (s, 1 H), 8.39 (d, J = 1.88 Hz, 1 H), 8.02 (t, J = 6.0 Hz, 2 H), 7.59 (m, 2 H), 7.38 (d, J =.7.9 Hz, I H), 7.22 (d, J = 8.1 Hz, 1 H), 6.30 (b, 1 H), 3.72 (s, 3 H), 3.36 (t, J = 6.6 Hz, 2 H), 1.96 (m, 1 H), 1.02 (d, J = 6.8 Hz, 6 MS 340.1 |
| **132** | -H | -CO₂H | -CH₃ | **131** | E | H), (ES+): ¹HNMR (DMSO-d₆): δ 12.28 (b, 1 H), 8.52 (d, J = 6.03 Hz, 1 H), 8.12 (s, 1 H), 7.86 (d, J = 8.1 Hz, 1 H), 7.74 (d, J = 7.74 Hz, 1 H), 7.41 (t, J = 8.67 Hz, 1 H), 7.31 (t, J = 7.9 Hz, 1 H), 7.12 (d, J = 8.1 Hz, 1 H), 6.97 (d, J = 7.5 Hz, 1 H), 3.39 (s, 3 H), 2.92 (t, J = 6.0 Hz, 2 H), 1.66 (m, 1 H), 0.78 (d, J = 7.4 Hz, 6 H), MS (ES-): 354.1 |
| **193a** | -H | 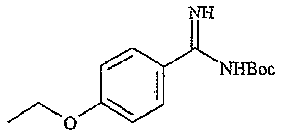 | -CH₃ | **192a + 6a** | D-7 | MS (ES⁺): 560.5 |
| **193b** | -H | 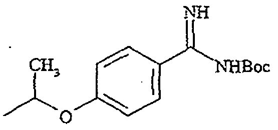 | -CH₃ | **192b + 6a** | D-7 | MS (ES⁺): 574.5) |
| **194a** | -H | 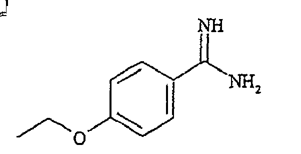 | -CH₃ | **193a** | S-2 | MS (ES⁺): 460.3 |
| **194b** | -H | 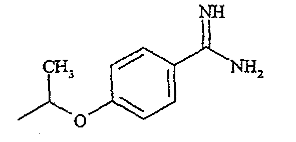 | -CH₃ | **193b** | S-2 | MS (ES⁺): 474.3 |
| **195a** | -H | -H 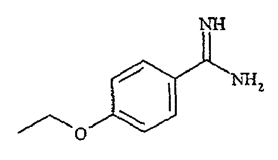 | -H | **194a** | I-2 | ¹HNMR (DMSO-d₆): δ 8.79 (bs, 4 H), 8.63 (t, J = 6.5 Hz, I H), 8.35 (s, 1 H), 7.85 (d, J = 6 Hz, 1 H), 7.62 (d, J = 8.2 Hz, 2 H), 7.26 (m, 5 H), 7.06 (m, 1 H), 5.0 (m, 2 H), 3.09 (t, J = 6.2 Hz, 2 H), 1.86 (m, 1 H), 0.89 (d, J = 6.6 Hz, 6 H); MS (ES-): 444.3 and (ES⁺) 446.3 |
| **195b** | -H | 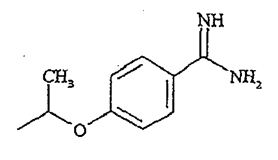 | -H | **194b** | 1-2 | ¹HNMR (DMSO-d₆/DCl): δ 8.24 (d, J = 1.6 Hz, 1 H), 7.91 (dd, J = 7.7 and 1.6 Hz, 1 H), 7.56 (d, J = 8.7 Hz, 1 H), 7.48 (d, J = 8.7 Hz, 1 H), 7.32 (t, J = 8 Hz, 1 H), 7.16 (m, 3 H), 6.91 (t, J = 7.5 Hz, 1 H), 6.76 (d, J = 8.5 Hz, 1 H), 6.66 (d, J = 8.5 Hz, 1 H), 4.99 (m, 1 H), 2.92 (d, J = 6.9 Hz, 2 H), 1.68 (m, 1 H), 1.33 (d, J = 6 Hz, 1.2 H), 1.27 (d, J = 6 Hz, 1.8 H), 0.71 (d, J = 6.5 Hz, 6 H); MS (ES-): 458.2 and (ES⁺) 460.3 |
| **200** | -H | 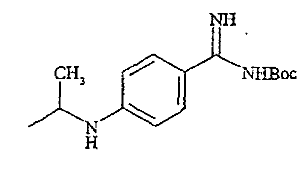 | -CH₃ | **199 + 6a** | D-7 | MS (ES⁺): 573.5 |
| **201** | -H | 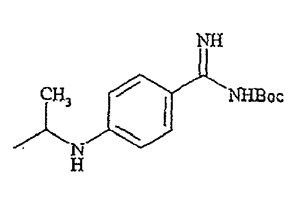 | -H | **200** | 1-2 | ¹HNMR (DMSO-d₆/DCl): δ 8.49 (t, J = 5.6 Hz, 1 H), 8.18 (d, J = 6.9 Hz, 1 H), 7.84 (t, J = 7.8 Hz, 1 H), 7.23 (m, 4 H), 7.01 (m, 2 H), 6.82 (d, J = 7 Hz, 1 H), 6.22 (d, J = 8.5 Hz, 1 H), 6.15 (d, J = 8.5 Hz, I H), 3.95 (m, 1 H), 2.85 (t, J = 5.8 Hz, 1 H), 1.62 (m, 1 H), 1.23 (s, 9 H), 1.1 (d, J = 6.7 Hz, 1.2 H), 1.05 (d, J = 6.7 Hz, 1.8 H), 0.67 (d, J = 6.6 Hz, 6 H); MS (ES+): 559.4 |
| **202** | -H | 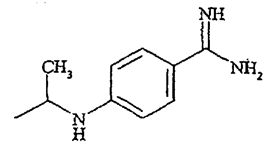 | -H | **201** | S | MS (ES⁺): 459.3 |
| **203** | -OBn(4) | 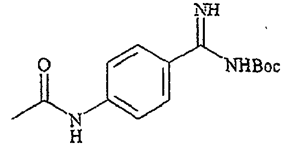 | -CH₃ | **45** | R | MS (ES⁺): 679.4 |
| **204** | -OBn (4) | 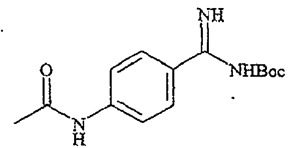 | -H | **203** | 1-2 | MS (ES⁻): 663.4 |
| **209a** | -H | 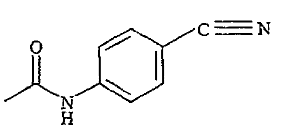 | -CH₃ | **132** | A-7 | MS (ES⁻): 454.3 |
| **209b** | -CH=CH₂ (4) | 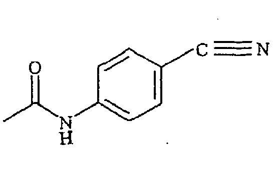 | -CH₃ | **30f** | A-7 J | ¹HNMR (DMSO-d₆): δ 10.72 (s, 1 H), 8.65 (d, J = 6.03 Hz, 1 H), 8.24 (s, I H), 8.03 (d, J = 8.1 Hz, 1 H), 7.75 (m, 6 H), 7.40 (d, J = 7.90 Hz, 1 H), 7.34 (d, J = 8.1 Hz, 1 H), 6.88 (q, J = 11.2 Hz, 1 H), 6.04 (d, J = 7.5 Hz, 1 H), 5.41 (d, = 11.1 Hz, 1 H), 3.55 (s, 3 H), 3.10 (t, J = 6.6 Hz, 2 H), 1.86 (m, 1 H), 0.88 (d, J = 6.6 Hz, 6 H); MS (ES⁻): 480.3 |
| **210b** | -CH=CH₂ (4) | 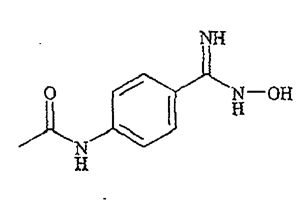 | -CH₃ | **209b** | Y | ¹HNMR (DMSO-d₆): δ 10.12 (s, 1 H), 9.37 (b, 1 H), 8.48 (t, J=6.1 Hz, 1 H), 8.05 (d, J=1.9 Hz, 1 H), 7.85 (d, J=7.9 Hz, 1 H), 7.56 (d, J=7.8 Hz, 1 H), 7.49 (d, J=7.9 Hz, 1 H), 7.36 (s, 4 H), 7.21 (d, J=7.9 Hz, 1 H), 7.10 (d, J=2.8 Hz, I H), 6.69 (m, 1 H), 5.84 (d, J=15.5 Hz, 1 H), 5.60 (b, 1 H), 5.22 (d, J=11.4 Hz, 1 H), 3.38 (s, 3 H), 2.91 (t, J = 6 Hz, 2 H), 1.66 (m, 1 H), 0.71 (d, J = 6.8 Hz, 6 H); MS (ES+) 515.40 |
| **211b** | -CH=CH₂ (4) | 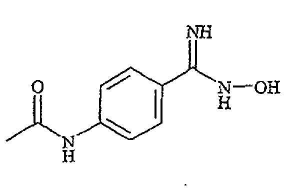 | -H | **210b** | 1-2 | ¹HNMR (DMSO-d₆): δ 12.62 (bs, 1H), 10.24 (s, 1 H), 8.48 (t, J=5.65 Hz, I H), 8.15 (s, I H), 7.81 (d, J=10.9 Hz, 1 H), 7.61 (s, 1 H), 7.50 (d, J=7.9 Hz, 1 H), 7.49 (s, 6 H), 7.16 (d, J=8.1 Hz, 1 H), 7.08 (d, J=8.1 Hz, 1 H), 6.72 (m, 1 H), 5.85 (d, J=13.7 Hz, 1 H), 5.24 (d, J=11.5 Hz, 1 H), 2.93 (t, J = 6 Hz, 2 H), 1.68 (m, 1 H), 0.72 (d, J = 6.8 Hz, 6 H); MS (ES+) 501.40, (ES-) 499.2 |
| **212** | -CH=CH₂ (4) | 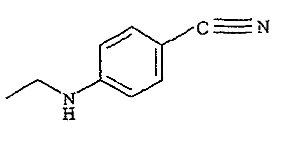 | -CH₃ | **187a** | AE-5 | ¹HNMR (DMSO): δ 8.70 (t, J = 5.6 Hz, 1 H), 8.36 (d, *J* = 1.7 Hz, 1 H), 8.07 (dd, J = 8.1, 1.9 Hz, 1 H), 7.42 (m, 4H), 7.09 (d, *J* = 5.5 Hz, I H), 7.04 (d, *J* = 7.7 Hz, I H), 6.74 (dd, *J* = 17.5, 10.9 Hz, 1 H), 6.49 (d, *J* = 8.8 Hz, 2 H), 5.79 (d, *J* = 17.7 Hz, 1 H), 5.27 (d, *J* = 10.9 Hz, 1 H), 4.0 (t, *J* = 6.0 Hz, 2 H), 3.62 (s, 3 H), 3.11 (t, *J* = 6.2,2 H), 1.86 (m, 1 H), 0.90 (d, *J* = 6.6 Hz, 6 H) |
| **213** | -CH=CH₂ (4) | 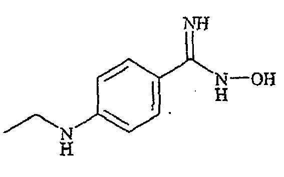 | -CH₃ | **212** | Y | ¹HNMR (DMSO): δ 9.23 (s, 1 H), 8.71 (t, *J* = 6.2 Hz, 1 H), 8.36 (d, *J* = 1.9 Hz, 1 H), 8.09 (dd, J = 7.9, 1.7 Hz, 1 H), 7.49 (d, J = 7.9 Hz, 2 H), 7.40 (d, *J* = 8.3 Hz, 1 H), 7.32 (d, *J* = 8.8 Hz, 2 H), 7.04 (d, *J* = 7.9 Hz, 1 H), 6.73 (dd, *J* = 17.7, 11.1 Hz, 1 H), 6.40 (d, *J* = 8.5 Hz, 2 H), 6.33 (t, *J* = 7.0 Hz, 1 H), 5.78 (d, *J* = 17.7 Hz, 1 H), 5.58 (b, 1 H), 5.26 (d, *J* = 11. Hz, 1 H), 3.96 (m. 2 H), 3.64 (s, 3 H), 3.11 (t, *J* = 6.4 Hz, 2 H), 1.86 (m,, 1 H), 0.90 (d, *J* = 6.8 Hz, 6 H); MS (ES⁺): 501.3 |
| **214** | -CH=CH₂ (4) | 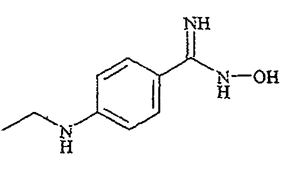 | -H | **213** | I-2 | ¹HNMR (DMSO): δ 8.76 (t, *J*= 5.8 Hz, I H), 8.37 (s, 1 H), 8.04 (d, *J* = 8.7 Hz, 1 H), 7.39 (m, 5 H), 7.06 (d, *J* = 8.3 Hz, 1 H), 6.72 (dd, *J* = 17.9, 11.3 Hz, 1 H), 6.43 (d, *J* = 8.5 Hz, 3 H), 5.76 (d, *J* = 17.9 Hz, 1 H), 5.24 (d, *J* = 11.1 Hz, 1 H), 3.98 (m. 2 H), 3.11 (t, *J* = 6.6 Hz, 2 H), 1.86 (h, *J* = 6.8 Hz, 1 H), 0.90 (d, *J* = 6.8, 6 H); MS (ES⁺): 487.2 |
| **238** | -CH=CH₂ (4) | 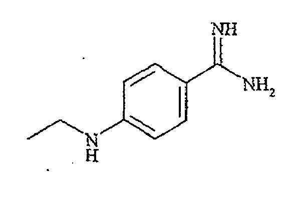 | -H | **237 + 187a** | AE-2 | ¹HNMR (DMSO-d₆): δ 8.68-8.60 (m, 1 H), 8.50 (d, J = 2.4 Hz, 1 H), 7.90-7.80 (m, 1 H), 7.76-7.70 (m, 1 H), 7.56-7.50 (m, 1 H), 7.48-7.42 (d, J = 7.7 Hz, 1 H), 7.30-7.22 (d, J = 7.9 Hz, 1 H), 7.10-7.02 (d, J = 7.7 Hz, 1 H), 6.90-6.75 (dd, J = 17, 11 Hz, 1 H), 6.5 (bs, 1 H), 5.92-5.80 (d, J = 17 Hz, 1 H), 5.40-5.30 (d, 11 Hz, 1 H), 4.50-4.20 (m, 2 H), 3.20-3.10 (t, J =6.6 Hz, 2 H), 2.10-1.88 (m, 1 H), 1.2-0.94 (d, J = 6.6 Hz, 6 H); MS (ES⁺ ) 471.3 |
| **256** | -H | 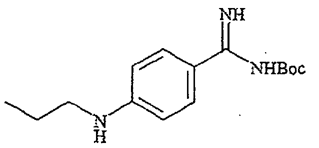 | -CH₃ | **255 + 6a** | D-6 | MS (ES⁺): 573.3 |
| **257** | -H | 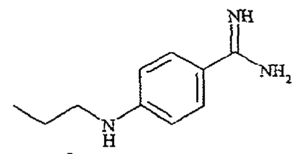 | -H | **256** | I-2, S | MS (ES⁺): 459.1 |

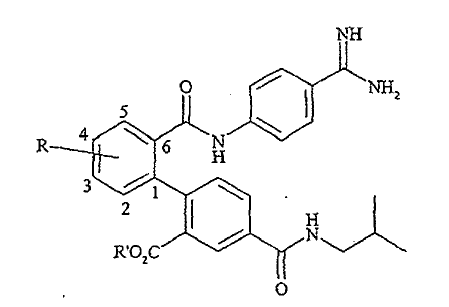

| **Cpd. No.** | **-R** | **-R'** | **Starting From** | **Method Used** | **Analytical Data** |
|---|---|---|---|---|---|
| **79a** | -CH=CH₂ (3) | -CH₃ | **78a** | J | MS (ES⁺): 499.2 |
| **79b** | -OSO₂CF₃ (3) | -CH₂C₆H₅ | **78b** | J | Characterized in the next step |
| **79c** | -OCH₂CO₂C₂H₅ (3) | -CH₂C₆H₅ | **78c** | J | Characterized in the next step |
| **79d** | -OCH₂CONH₂ (3) | -CH₂C₆H₅ | **78d** | J | MS (ES⁺): 622.4; (ES⁻) 620.4 |
| **79e** | 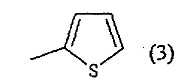 | -CH₂C₆H₅ | **78e** | J | Characterized in the next step |
| **79f** | 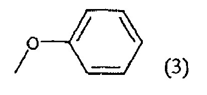 | -CH₂C₆H₅ | **78f** | J | Characterized in the next step |
| **79g** | -OCH₃ (3) | -CH₃ | **78g** | J | ¹HNMR (DMSO-d₆): δ 10.6 (bs, 1 H), 9.29-9.32 (bs, 1 H), 9.06 (bs, 1 H), 8.82-8.75 (t, J = 5.84 Hz, 1 H), 8.32 (d, J = 1.88 Hz, 1 H), 8.13 (d, J = 1.7 Hz, 1 H), 7.83 (s, 4 H), 7.78 (d, J = 8.67 Hz, 1 H), 7.50 (d, J = 7.9 Hz, 1 H), 7.20-7.15 (dd, J = 8.67, 2.3 Hz, 1 H), 6.92 (d, J = 2.4 Hz, 1 H), 3.94 (s, 3 H), 3.64 (s, 3 H), 3.21-3.14 (t, J = 6 Hz, 2 H), 2.0-1.86 (m, 1 H), 1.0-0.94 (d, J = 6.5 Hz, 6 H); MS (ES⁺) 503.3 |
| **79h** | 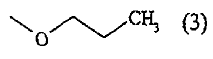 | -Bn | **78h** | J | MS (ES⁺): 607.3 |
| **79i** | 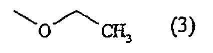 | -Bn | **78i** | J | MS (ES⁺): 593.4 |
| **79j** | 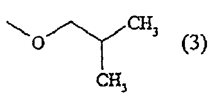 | -Bn | **78j** | J | MS (ES⁺): 621.4 |
| **79k** | -O-CH₂-CH₂-OAc (3) | -Bn | **78k** | J | MS (ES⁺): 651.4 |
| **80a** | -CH=CH₂ (3) | -H | **79a** | I-2 | ¹HNMR (DMSO-d₆): δ 9.1 (s, 2 H), 8.87 (s, 2 H), 8.53 (t, J = 6 Hz, 1 H), 8.02 (s, 1 H), 7.64 (m, 7 H), 7.1 (s, 1 H), 6.98 (d, 7.4 Hz, 1 H), 6.80 (dd, J = 11 Hz, J = 17.6 Hz, 1 H), 5.90 (d, J = 17.6 Hz, 1 H), 5.35 (d, J = 12 Hz, 1 H), 3.03 (t, 6 Hz, 2 H), 1.83 (m, 1 H), 0.86 (d, J = 6.7 Hz, 6 H); MS (ES⁺ ) 485.2 |
| **80b** | -OH (3) | -H | **79b** | 1-2 | ¹HNMR (DMSO-d₆): δ 10.37 (s, 1 H), 9.20 (m, 3 H), 8.72 (t, J = 6 Hz, 1 H), 8.2 (s, 1 H), 8.85 (m, 6 H), 7.65 (d, J = 8 Hz, 1 H), 7.12 (d, 8 Hz, 1 H), 7.02 (dd, J = 2.5 Hz, J = 8 Hz, 1 H), 6.60 (d, J = 2.5 Hz, 1 H), 3.25 (t, J = 6.5 Hz, 2 H), 2.0 (m, 1 H), 1.07 (d, J = 6.8 Hz, 6 H); MS (ES⁺ ) 475.2 |
| **80c** | -OCH₂CO₂H (3) | -H | **79c** | 1-2 | ¹HNMR (DMSO-d₆): δ 12.7 (2H, bs, 1 H), 9.01, 8.87 (2 bs, 4 H), 8.36 (m, 1H), 7.83 (s, 1H), 7.44 (m, 6 H), 6.75 (m, 2H), 6.31 (d, J=2.2 Hz, 1H), 4.42 (s, 2H), 2.84 (m, 2H), 1.63 (m, 1H), 0.67 (d, J=6.5 Hz, 6H); MS(ES+): 533.4 |
| **80d** | -OCH₂CONH₂ (3) | -H | **79d** | G | ¹H NMR (DMSO-d₆): δ 9.13 (bs, 5H), 8.59 (t, J=6.28 Hz, 1H), 8.14 (d, J = 1.7 Hz, 1H), 7.63 (m, 9H), 7.42 (s, 1H), 7.09 (d, J = 7.5 Hz, 1H), 7.03 (dd, J = 2.5, 12.7 Hz, 1H), 6.70 (d, J =2.5 Hz, 1H), 4.48 (s, 2H), 3.05 (t, J= 6.6 Hz, 2H), 1.83 (m, 1H), 0.87 (d, J=6.8 Hz, 6H); MS(ES+): 532.4 |
| **80e** | 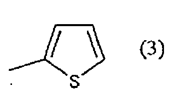 | -H | **79e** | 1-2 | ¹H NMR (DMSO-d₆): δ 12.6 (1H, bs, COOH), 8.98, 8.67 (2 bs, 4H), 8.46 (m, 1H), 8.08 (m,1H), 7.76 (m, 1H), 7.53 (m, 6 H), 7.39 (m, 2H), 7.06 (m; 1H), 7.04 (m, 1H), 2.89 (m, 2H), 1.66 (m, 1H), 0.69 (d, J=6.5 Hz, 6H); MS(ES+): 541.4 |
| **80f** | 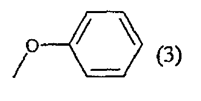 | -H | **79f** | 1-2 | ¹HNMR (DMSO-d₆): δ 9.14 (d, J = 10 Hz, 4 H), 8.60 (t, J = 6 Hz, 1 H), 8.22 (bs, 1 H), 7.87-7.62 (m, 7 H), 7.47 (t, J = 8 Hz, 2 H), 7.26 (t, 7 Hz, 1 H), 7.22 (m, 4 H), 6.70 (bs, 1 H), 3.09 (t, J = 6 Hz, 2 H), 1.83 (m, 1 H), 0.91 (d, J = 6.8 Hz, 6 H); MS (ES⁺ ) 551.4 |
| **80g** | -OCH₃ (3) | -H | **79g** | 1-2 | ¹HNMR (DMSO-d₆): δ 9.13 (bs, 2 H), 8.78 (bs, 2H), 8.65 (t, J = 6 Hz, 1 H), 8.25 (bs, 1 H), 7.78 (m, 1 H), 7.76 (m, 5 H), 7.25 (s, 1 H), 7.17 (m, 1 H), 6.73 (bs, 1 H), 3.83 (s, 3 H), 3.10 (t, J = 6 Hz, 2 H), 1.80 (m, 1 H), 0.88 (d, J = 6.8 Hz, 6 H); MS (ES⁺ ) 489.3 |
| **80h** | 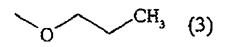 | -H | **79h** | I-2 | MS (ES⁺): 517.7 |
| **80i** | 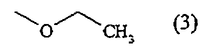 | -H | **79i** | I-2 | MS (ES⁺): 503.4 ; MS (ES⁻): 501.4 |
| **80j** | 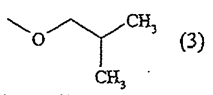 | -H | **79j** | I-2 | MS (ES⁺): 531.4 ; MS (ES⁻): 529.4 |
| **80k** | -O-CH₂-CH₂-OH (3) | -H | **79k** | I-2 | ¹HNMR (DMSO-d₆): δ 13.52 (bs, 1 H), 9.16 (bs, 2 H), 9.03 (bs, 2 H), 8.50 (t, J = 6 Hz, 1 H), 7.96 (d, J = 1.7 Hz, 1 H), 7.56 (m, 6 H), 7.00 (dd, J = 2.5 and 8.5 Hz, 1 H), 6.90 (d, J = 8 Hz, 1 H), 6.48 (d, J = 2.5 Hz, 1 H), 4.91 (t, J = 5.5 Hz, 1 H), 4.00 (t, J = 4.5 Hz, 2 H), 3.69 (q, J = 5.5 and 10 Hz, 2 H), 3.05 (t, J = 6.8 Hz, 2 H), 1.80 (m, 1 H), 0.84 (d, J = 6.8 Hz, 6 H); MS (ES⁺): 519.3, (ES-) 517.3 |
| **86a** | -CH(OH)CH₂OH (3) | -H | **82** | S, I-2 | ¹HNMR (DMSO-d₆): δ 9.15 (bs, 3 H), 8.65 (t, J = 6 Hz, 1 H), 8.12 (s, 2 H), 7.82-7.56 (m, 7 H), 7.55-6.96 (m, 4 H), 5.5 (bs, 1 H), 4.90 (bs, 1 H), 4.65 (bs, 1 H), 3.10 (t, J = 6 Hz, 2 H), 1.90 (m, 1 H), 0.92 (d, J = 6.8 Hz, 6 H); MS (ES⁺) 519.3 |
| **86b** | -CH₂OH (3) | -H | **84** | S, I-2 | ¹HNMR (DMSO-d₆): δ 8.82 (bs, 2 H), 8.68 (bs, 2 H), 8.40 (t, J = 6 Hz, 1 H), 7.88 (bs, 1 H), 7.53 (m, 5 H), 7.45 (d, 8 Hz, I H), 7.25 (d, J = 8 Hz, 1 H), 6.81 (m, 2 H), 5.22 (d, J = 5.5 Hz, 1 H), 4.41 (d, J = 5.5 Hz, 2 H), 2.88 (t,J = 6 Hz, 2 H), 1.65 (m, 1 H), 0.71 (d, J = 6.8 Hz, 6 H); MS (ES⁺) 489.2 |
| **86c** | -CO₂H (3) | -H | **85** | S, I-2 | ¹HNMR (DMSO-d₆-D₂O): δ 13.7 (bs, 1 H), 8.32 (t, J = 6 Hz, 1 H), 7.63-7.17 (m, 7 H), 6.72 (d, 7.0 Hz, 1 H), 2.81 (t, J = 6 Hz, 2 H), 1.53 (m, 1 H), 0.64 (d, J = 6.8 Hz, 6 H); MS (ES⁺) 503.2 |
| **97a** | 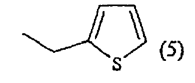 | -CH₃ | **96a** | J | MS (ES⁺): 569.2 |
| **97b** | -OBn (5) | -CH₃ | **91** | J | ¹HNMR (DMSO-d₆): δ 10.62 (s, 1 H), 9.15 (bs, 2 H), 8.82 (bs, 2 H), 8.67 (t, J = 6 Hz, 1 H), 8.25 (d, J = 2 Hz, 1 H), 7.99 (dd, J = 8.1 and 2 Hz, 1 H), 7.69 (q, 8.8 and 16.2 Hz, 4 H), 7.44 (m, 3 H), 7.28 (m, 3 H), 6.89 (d, J = 7.7 Hz, 1 H), 5.5 (s, 2 H), 3.6 (s, 3 H), 3.08 (t, J = 5.8 and 6.8 Hz, 2 H), 1.83 (m, 1 H), 0.87 (d, J = 6.8 Hz, 6 H); MS (ES-) 577.2, (ES+) 579.3 |
| **98a** | 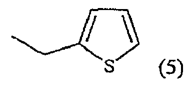 | -H | **97a** | I-2 | ¹HNMR (DMSO-d₆): δ 13.45 (bs, 1 H), 9.06 (s, 2 H), 8.99 (s, 2 H), 8.51 (t, J = 6 and 5 Hz, 1 H), 7.99 (s, 1 H), 7.62 (m, 5 H), 7.47 (s, 1 H), 7.36 (m, 2 H), 6.99 (m, 4 H), 4.26 (s, 2 H), 3.02 (t, J = 6.8 Hz, 2 H), 1.80 (m, 1 H), 0.86 (d, J = 6.8 Hz, 6 H); MS (ES-) 553.2, (ES+) 555.2 |
| **98b** | -OBn (5) | -H | **97b** | I-2 | ¹HNMR (DMSO-d₆): δ 13.52 (bs, 1 H), 9.09 (bs, 2 H), 9.04 (bs, 2 H), 8.48 (t, J = 6 Hz, 1 H), 7.94 (s, 1 H), 7.61 (m, 4 H), 7.49 (s, 1 H), 7.46 (s, 1 H), 7.34 (m, 5 H), 7.15 (d, J = 8.2 Hz, 1 H), 7.00 (d, J = 8.2, 1 H), 6.02 (d, J = 7.4 Hz, 1 H), 5.21 (s, 2 H), 3.01 (t, J = 6.8 Hz, 2 H), 1.80 (m, 1 H), 0.85 (d, J = 6.8 Hz, 6 H); MS (ES-) 563.2, (ES+) 565.2 |
| **98c** | -OH (5) | -H | **98b** | G | ¹HNMR (DMSO-d₆): δ 9.85 (s, 1 H), 9.07 (s, 2 H), 8.98 (s, 2 H), 8.50 (t, J = 6 and 5 Hz, 1 H), 7.99 (d, J = 1.7 Hz, 1 H), 7.63 (m, 5 H), 7.20 (t, J = 8 Hz, 2 H), 6.90 (d, J = 7.9 Hz, 1 H), 6.49 (d, J = 7.2 Hz, 1 H), 3.21 (t, J = 6.8 Hz, 2 H), 1.80 (m, 1 H), 0.85 (d, J = 6.8 Hz, 6 H); MS (ES+) 475.2; (ES-) 473.2 |
| **103** | -OCH₃ (2) | -CH₃ | **102** | J | MS (ES+) 503.1 |
| **104** | -OCH₃ (2) | -H | **103** | I-2 | ¹HNMR (DMSO-d₆): δ 9.08 (bs, 2 H), 8.80 (bs, 2 H), 8.52 (t, J = 6 Hz, 1 H), 8.02 (s, 1 H), 7.64 (m, 5 H), 7.16 (m, 2 H), 7.03 (m, 2 H), 3.84 (s, 3 H), 3.03 (t, J = 6.8 Hz, 2 H), 1.81 (m, 1 H), 0.86 (d, J = 6.8 Hz, 6 H); MS (ES-) 487.3, (ES+) 489.3 |
| **110** | -OBn (2) | -CH₃ | **109** | J | MS (ES⁺): 579.3 |
| **111** | -OH (2) | -CH₃ | **110** | G | MS (ES⁺): 489.3 |
| **126** | 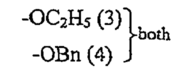 | -CH₃ | **118b** | J | Characterized in the next step |
| **127** | 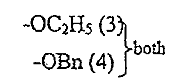 | -H | **126** | I-2 | ¹H NMR (DMSO-d6): δ 9.06-9.09 (m, 3H), 8.56-8.50 (m, 1H), 8.05 (s, 1H), 7.71-7.58 (m, 6H), 7.55-7.28 (m, 6H), 7.10-7.01 (m, 1H), 6.63 (s, 1H), 5.19 (s, 2H), 4.05-3.97 (m, 2H), 3.05-3.01 (m, 2H), 1.86-1.77 (m, 1H), 1.29 (t, *J*=6.7 Hz, 3H), 0.87 (d, *J*=6.8 Hz, 6H) |
| **129** | 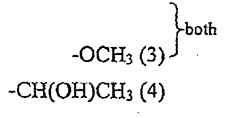 | -H | **128** | I-2, S | ¹H NMR (DMSO-*d₆*): 13.64 (br s, 1 H), 8.99 (br s, 2 H), 8.49 (t, J= 5.1 Hz, 1 H), 7.99 (s, 1 H), 7.73-7.56 (m, 5 H), 7.32-6.83 (m, 5 H), 6.50 (s, 1 H), 5.17 (d, *J* = 4.3 Hz, 1 H), 5.01 (m, 1 H), 3.75 (s, 3 H), 3.03 (t, *J* = 6.0 Hz, 1 H), 1.81 (m, 1 H), 1.32 (d, *J* = 6.2 Hz, 3 H), 0.86 (d, *J =* 6.6 Hz, 6 H); MS (ES⁺): 533.4 (100% M⁺¹) |

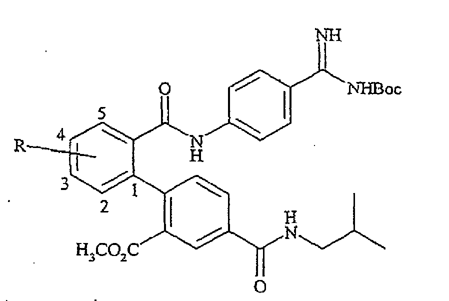

| **Cpd. No.** | **-R (With Respect to Phenyl Ring)** | **Starting From** | **Method Used** | **Analytical Data** |
|---|---|---|---|---|
| **81** | -CH=CH₂ (3) | **79a** | R | MS (ES⁻): 597.2 |
| **82** | -CH(OH)CH₂OH (3) | **81** | L | MS (ES⁻¹): 631.3 |
| **83** | -CH=O (3) | **82** | M | MS (ES⁺): 601.3 |
| **84** | -CH₂OH (3) | **83** | K | MS (ES⁻¹): 601.4 |
| **85** | -CO₂H (3) | **83** | E | MS (ES⁻¹): 615.3 |
| **128** | 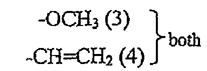 | **124a** | R | MS (ES⁺): 629.4 |

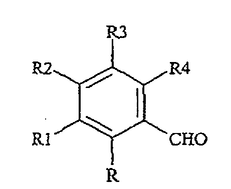

| **Cpd. No.** | **-R** | **-R1** | **-R2** | **-R3** | **-R4** | **Starting From** | **Method Used** | **Analytical Data** |
|---|---|---|---|---|---|---|---|---|
| **88** | -Br | -H | -H | -H | -OBn | **87** | X | ¹HNMR (CDCl₃): δ 10.48 (s, 1 H), 7.42 - 7.25 (m, 7 H), 7.00 (dd, J = 2 and 7.4 Hz, 1 H), 5.19 (s, 2 H); IR (KBr) 1701, 1585, 1452, 1262, 1009 cm⁻¹; MS (ES+) 313.0, 315.0 (M+Na)⁺ |
| **89** | -B(OH)₂ | -H | -H | -H | -OBn | **88** | T, U-1 | ¹HNMR (CDCl₃): δ 10.61 (s, 1 H), 7.65 (d, J = 7.2 Hz, 1 H), 7.60 (t, J = 7.9 and 7.2 Hz, 1 H), 7.41 (m, 5 H), 7.19 (d, J = 7.9 Hz, 1 H), 6.81 bs, 2 H), 5.20 (s, 2 H) |
| **100** | -B(OH)₂ | -OCH₃ | -H | -H | -H | **99** | T, U-3 | ¹HNMR (DMSO-d₆): δ 10.2 (s, 1 H), 8.34 (s, 2 H), 7.92 (d, J = 9.4 Hz, 1 H), 7.13 (m, 2 H), 3.92 (s, 3 H); MS (ES⁻) 179.0 |
| **107** | -B(OH)₂ | -OBn | -H | -H | -H | **106** | T, U-1 | ¹HNMR (DMSO-d6): δ 10.1 (s, 1 H), 7.3-7.6 (m, 8 H), 5.3 (m; 2 H) |
| **114a** | -Br | -H | -OCH₃ | -OH | -H | **113** | Z | MS (ES⁻): 229.0 and 231.0) |
| **114b** | -Br | -H | -OC₂H₅ | -OH | -H | **113** | Z-1 | MS (ES⁻): 242.9 and 244.9 |
| **114c** | -Br | -H | -OCH(CH₃)₂ | -OH | -H | **113** | Z-1 | MS (ES⁻): 257.0 and 259.0 |
| **115a** | -Br | -H | -OCH₃ | -OBn | -H | **114a** | X | MS (ES⁺): 321.0 and 323.0 |
| **115b** | -Br | -H | -OC₂H₅ | -OBn | -H | **114b** | X | MS (ES⁺): 335.0 and 337.0 |
| **115c** | -Br | -H | -OCH(CH₃)₂ | -OBn | -H | **114c** | X | MS (ES⁺): 349.0 and 351.0 |
| **115d** | -Br | -H | 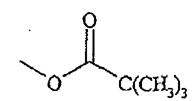 | -OBn | -H | **115a** | X, V-4, AH | Characterized in the next step |
| **116a** | -B(OH)₂ | -H | -OCH₃ | -OBn | -H | **115a** | T, U-1 | Characterized in the next step |
| **116b** | -B(OH)₂ | -H | -OC₂H₅ | -OBn | -H | **115b** | T, U-1 | Characterized in the next step |
| **116c** | -B(OH)₂ | -H | -OCH(CH₃)₂ | -OBn | -H | **115c** | T, U-1 | Characterized in the next step |

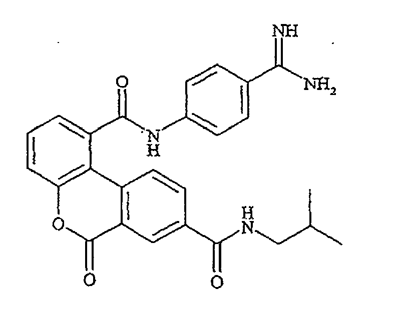

| **Cpd. No.** | **Starting From** | **Method Used** | **Analytical Data** |
|---|---|---|---|
| **112** | **111** | I-2 | ¹HNMR (DMSO-d₆): δ 11.28 (s, 1 H), 9.31 (s, 2 H), 9.0 (s, 2 H), 8.88 (d, J = 11.30 Hz, 1 H), 8.82 (d, J = 1.88 Hz, 1 H), 8.25 (d, J = 1.88 Hz, I H), 8.18 (d , J = 1.88 Hz, 1 H), 8.04 (d, J = 8.47 Hz, 1H), 7.92 (m, J = 24.48 Hz, 2 H), 7.75 (m, J = 15.82, 1 H), 7.75 (m, J = 8.28 Hz, 1 H), 7.55 (m, J = 8.66 Hz, 1 H), 3.10 (m, J = 12.6 Hz, 1 H), 2.5 (m, J = 3.5 Hz, 1 H), 1.8 (m, J = 19.9 Hz, 2 H), 0.88 (m, J = 6.6 Hz, 6 H). |

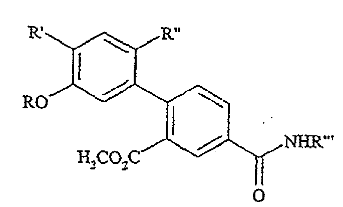

| **Cpd. No.** | **-R** | **-R'** | **-R"** | **-R"'** | **Starting From** | **Method Used** | **Analytical Data** |
|---|---|---|---|---|---|---|---|
| **117a** | -CH₃ | -OBn | -CHO | 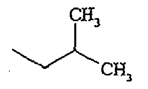 | **3a + 116a** | D-2 | MS (ES⁻): 474.2 |
| **117b** | -C₂H₅ | -OBn | -CHO | 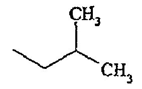 | **3a+ 116b** | D-2 | MS (ES⁻): 488.2 |
| **117c** | -CH(CH₃)₂ | -OBn | -CHO | 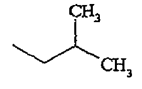 | **3a+ 116c** | D-2 | MS (ES⁻): 502.3 |
| **117d** | -CH₃ | -OBn | -CHO | 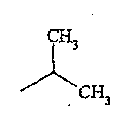 | **3b+ 116a** | D-2 | ¹H NMR (CDCl₃): δ 9.56 (s, 1 H), 8.34 (d, J = 1.7 Hz, 1 H), 8.5 (s, 1 H), 8.01 (dd, J = 7.9 and 1.9 Hz, 1 H), 7.40 (m, 7 H), 6.9 (s, 1 H), 5.24 (m, 2 H), 4.2 (m, 1 H), 3.80 (s, 3 H), 3.52 (s, 3 H), 1.02 (d, J = 7 Hz, 6 H); MS (ES+) : 484.3 (M+Na)⁺ |
| **117e** | -CH₃ | -OBn | -CHO | 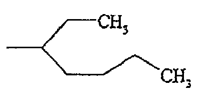 | **3c + 116a** | D-2 | ¹HNMR (DMSO-d₆): δ 8.43 (d, J = 1.65 Hz, 1 H), 8.31 (d, J = 8.66 Hz, 1), 8.12 (dd, J = 1.69 Hz, 1H), 7.98 (s, 1H), 7.41 (d, J = 8 and 10 Hz, 1H), 7.19 (d, J = 8.1 Hz, 1H), 5.20 (dd, J = 6.2 Hz, 1H), 3.98 (dd, J = 7.75 Hz, 3H), 3.94 (s, 3H), 3.42 (m, 3H), 3.32 (m, 3H), 3.19 (s, 3H), 2.5 (m, 3H), 2.0 (s, 4H), 1.5 (m, 2H), 1.28 (m, 3H), 0.88 (d, J = 6.59 Hz, 3H); MS (ES+): 664.3 |
| **117f** | -CH₃ | -OBn | -CHO | 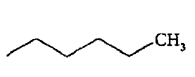 | **3d + 116a** | D-2 | ¹HNMR (CDCl₃): δ 9.50 (s, 1 H), 8.40 (d, J = 2.1 Hz, 1 H), 8.04 (dd, *J* = 8.1, 2.1 Hz, 1 H), 7.57 (s, 1 H), 7.48 (m, 5 H), 7.38 (m, 5 H), 6.67 (s, 1 H), 6.50 (broad, 1 H),) 5.27 (d, *J* = 11.9 Hz, 1 H), 5.22 (dd, *J* = 11.7, 1 H), 4.63,(m,3H) 4.17 (m, 4 H), 3.92 (s, 3 H), 3.66 (s, 3 H); MS (ES⁻): 488.3 |
| **117g** | -CH₃ | -OBn | -CHO | 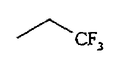 | **3f + 116a** | D-2 | ¹H NMR (CDCl₃): δ 9.50 (s, 1 H), 8.40 (d, *J* = 2.1 Hz, 1 H), 8.04 (dd, *J* = 8.1, 2.1 Hz, I H), 7.57 (s, 1 H), 7.48 (m, 2 H), 7.38 (m, 3 H), 6.67 (s, 1 H), 6.50 (broad, 1 H), 5.27 (d, *J* = 11.9 Hz, 1 H), 5.22 (dd, *J* = 11.7, 2 H), 4.17 (m, 2 H), 3.92 (s, 3 H), 3.66 (s, 3 H); MS (ES⁻): 500 |
| **117h** | -CH₃ | -OBn | -CHO | 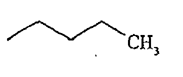 | **3e + 116a** | D-2 | ¹H NNM (CDCl₃): δ 9.56 (s, 1 H), 8.34 (d, *J* = 1.7 Hz, 1 H), 8.01 (dd, *J* = 7.9, 1.9 Hz, H), 7.57 (s, 1 H), 7.50 (dd, *J* = 7.2, 1.5, 2 H), 7.40 (m, 4 H), 6.67 (s, 1 H), 6.21 (broad, 1 H), 5.24 (d, *J* = 2.8 Hz, 2 H), 3.92 (s, 3 H), 3.65 (s, 3 H), 3.52 (m, 2 H), 1.65 (m, 2 H), 1.46 (m, 2 H), 0.99 (t, J = 7.3 Hz, 3 H). |
| **117i** | -CH₃ | -OBn | -CHO | 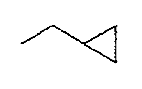 | **3g + 116a** | D-2 | ¹H NMR (CDCl₃): δ 9.57 (s, 1 H), 8.37 (d, *J* = 1.9 Hz, 1 H), 8.03 (dd, *J* = 7.9, 1.9 Hz, 1 H), 7.58 (s, 1 H), 7.50 (d, *J* = 7.2 Hz, 2 H), 7.38 (m, 3 H), 6.68 (s, I H), 6.33 (broad, 1H), 5.26 (d, *J* = 11.5 Hz, 1 H), 5.21 (d, *J* = 11.9 Hz, 1 H), 3.92 (s, 3 H), 3.65 (s, 3 H), 3.37 (dd, *J* = 7.2, 5.3 Hz, 2 H), 1.09 (m, 1 H), 0.60 (m, 2 H), 0.32 (m, 2 H); MS (ES⁺): 474.2 |
| **117j** | -CH₃ | -OBn | -CHO | 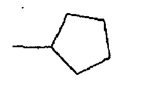 | **3h + 116a** | D-2 | ¹H NMR (CDCl₃): δ 9.55 (s, 1 H), 8.32 (d, *J* = 1.9 Hz, 1 H), 8.00 (dd, *J* =1.9 and 7.9 Hz, 1 H), 7.59-7.30 (m, 7 H), 6.67 (s, 1 H), 5.23 (m, 2 H), 4.45 (q, *J* = 7.0 Hz, 1 H), 3.91 (s, 3 H), 3.64 (s, 3 H), 2.21-1.46 (m, 8 H); MS (ES⁺): 510.3 (M + Na)⁺ |
| **117k** | 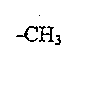 | -OBn | -CHO | 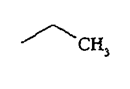 | **3i + 116a** | D-2 | ¹H NMR (CDCl₃): δ 9.56 (s, 1 H), 8.35 (d, *J* = 1.9 Hz, 1 H), 8.02 (dd, *J* = 1.9 and 7.9 Hz, 1 H), 7.58-7.33 (m, 7 H), 6.68 (s, 1 H), 5.24 (m, 2 H), 3.92 (s, 3 H), 3.65 (s, 3 H), 3.56 (m, 2 H), 1.30 (t, *J* = 7.2 Hz, 3 H); MS (ES⁺): 470.3 (M+ Na)⁺ |
| **117l** | 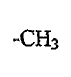 | -OBn | -CHO | 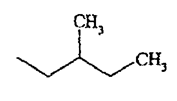 | **3j + 116a** | D-2 | ¹H NNM (CDCl₃): δ 9.56 (s, I H), 8.35 (d, *J* = 1.9 Hz, I H), 8.02 (dd, *J* = 1.9 and 7.9 Hz, 1 H), 7.58-7.33 (m, 7 H), 6.68 (s, 1 H), 5.24 (m, 2 H), 3.92 (s, 3 H), 3.65 (s, 3 H), 3.40 (m, 2 H), 1.80-0.94 (m, 9 H); MS (ES⁺): 512.2 (M+ Na)⁺ |
| **117m** | 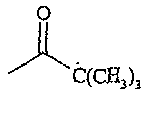 | -OBn | -CHO | 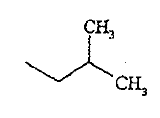 | **6a + 115d** | D-6 | ¹HNMR (DMSO-d₆): δ 9.73 (s, 1 H), 8.86 (t, J = 5.7 Hz, 1 H), 8.52 (d, J = 1.5 Hz, 1 H), 8.22 (dd, J = 8 and 2 Hz, 1 H), 7.79 (s, 1 H), 7.60 (d, J = 8 Hz, 1 H), 7.5 (m, 5 H), 7.22 (s, I H), 5.35 (g, J = 11 and 17 Hz, 1 H), 3.70 (s, 3 H), 3.23 (t, J = 6.5 Hz, 2 H), 1.98 (m, 1 H), 1.3 (s, 9 H), 1.01 (d, J = 6.8 Hz, 6 H); MS (ES⁺): 546.4 |
| **118a** | -CH₃ | -OBn | -CO₂H | 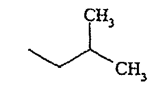 | **117a** | E | MS (ES⁻): 490.2 |
| **118b** | -C₂H₅ | -OBn | -CO₂H | 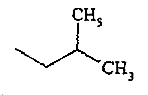 | **117b** | E | MS (ES⁻): 504.2 |
| **118c** | -CH(CH₃)₂ | -OBn | -CO₂H | 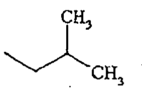 | **117c** | E | MS (ES⁻): 518.2 |
| **118d** | -CH₃ | -OBn | -CO₂H | 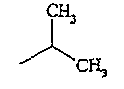 | **117d** | E | Characterized in the next step |
| **118e** | -CH₃ | -OBn | -CO₂H | 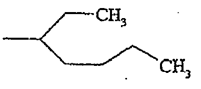 | **117e** | E | MS (ES⁺): 534.3 |
| **118f** | -CH₃ | -OBn | -CO₂H | 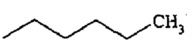 | **117f** | E | MS (ES⁺): 506.3 |
| **118g** | -CH₃ | -OBn | -CO₂H | 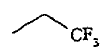 | **117g** | E | Characterized in the next step |
| **118h** | -CH₃ | -OBn | -CO₂H | 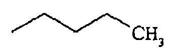 | **117h** | E | MS (ES⁻¹): 490.2 |
| **118i** | -CH₃ | -OBn | -CO₂H | 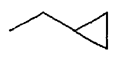 | **117i** | E | MS (ES⁻¹): 488.3 |
| **118j** | -CH₃ | -OBn | -CO₂H | 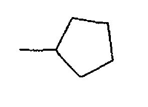 | **117j** | E | ¹H NMR (DMSO-*d₆*): δ 12.19 (br s, 1 H), 8.50 (d, *J* = 7.4 Hz, 1 H), 8.31 (d, *J* = 1.9 Hz, 1 H), 8.02 (dd, *J* = 1.7 and 7.9 Hz, 1 H), 7.58-7.29 (m, 7 H), 6.71 (s, 1 H), 5.17 (s, 2 H), 4.27 (q, *J* = 6.4 Hz, 1 H), 3.80 (s, 3 H), 3.57 (s, 3 1.97-1.51 8 |
| **118k** | -CH₃ | -OBn | -CO₂H | 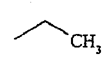 | **117k** | E | H), (m, H) MS (ES⁻): 462.3 |
| **118l** | -CH₃ | -OBn | -CO₂H | 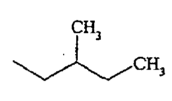 | **117l** | E | ¹H NMR (CDCl₃): δ 8.30 (d, *J* = 1.9 Hz, 1.H), 7.95 (dd, *J* = 1.7 and 7.9 Hz, 1 H), 7.66 (s, 1 H), 7.52-7.27 (m, 6 H), 6.62 (s, 1 H), 6.49 (m, 1 H), 5.21 (s, 2 H), 3.88 (s, 3 H), 3.61 (s, 3 H), 3.38 (m, 2 H), 1.79-0.94 (m, 9 H); MS (ES⁻): 504.4 |
| **118m** | 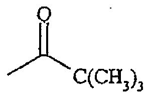 | -OBn | -CO₂H | 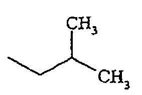 | **117m** | E | Characterized in the next step |
| **119a** | -CH₃ | -OBn | -CO₂MEM | 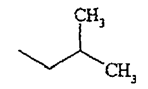 | **118a** | F | MS (ES⁻): 578.3 |
| **119b** | -C₂H₅ | -OBn | -CO₂MEM | 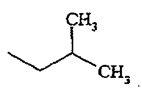 | **118b** | F | MS (ES⁻): 592.3 |
| **119c** | -CH(CH₃)₂ | -OBn | -CO₂MEM | 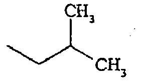 | **118c** | F | MS (ES⁻): 606.3 |
| **119d** | -CH₃ | -OBn | -CO₂MEM | 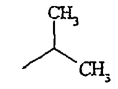 | **118d** | F | MS (ES⁻): 564.2 |
| **119e** | -CH₃ | -OBn | -CO₂MEM | 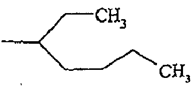 | **118e** | F | MS (ES⁻): 620.1 |
| **119f** | -CH₃ | -OBn | -CO₂MEM | 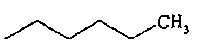 | **118f** | F | MS (ES⁻): 592.3 |
| **119g** | -CH₃ | -OBn | -CO₂MEM | 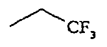 | **118g** | F | Characterized in the next step |
| **119h** | -CH₃ | -OBn | -CO₂MEM | 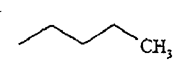 | **118h** | F | ¹H NMR (CDCl₃): δ 8.32 (d, *J* = 1.9 Hz, 1 H), 7.96 (dd, *J* = 7.9, 1.9 Hz, 1H), 7.68 (s, 1 H), 7.50 (m, 2 H), 7.35 (m, 4 H), 6.62 (s, 1 H), 6.33 (t, *J* = 5.4 Hz, 1 H), 5.24 (m, 4 H), 3.88 (s, 3 H), 3.63 (s, 3 H), 3.46 (m, 6 H), 3.34 (s, 3 H), 1.63 (m, 2 H), 1.44 (m, 2 H), 0.98 (t, *J* = 7.3 Hz, 3 H) |
| **119i** | -CH₃ | -OBn | -CO₂MEM | 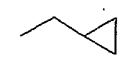 | **118i** | F | ¹H NMR (CDCl₃): δ 8.34 (d, *J* = 1.9 Hz, 1 H), 8.00 (dd, *J* = 7.9, 2.1 Hz, 1 H), 7.68 (s, 1 H), 7.50 (m, 2 H), 7.36 (m, 4 H), 6.63 (s, 1 H), 6.42 (broad, 1 H), 5.24 (m, 4 H), 3.89 (s, 3 H), 3.64 (s, 3 H), 3.45 (s, 3 H), 3.35 (m, 5 H), 1.07 (m, 1 H), 0.58 (m, 2 H), 0.30 (m, 2 H) |
| **119j** | -CH₃ | -OBn | -CO₂MEM | 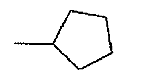 | **118j** | F | ¹H NMR (DMSO-*d₆*): δ 8.55 (d, *J* = 7.4 Hz, 1 H), 8.39 (d, *J* = 1.9 Hz, 1 H), 8.10 (dd, *J* = 1.7 and 7.9 Hz, 1 H), 7.63-7.35 (m, 7 H), 6.81 (s, 1 H), 5.25-5.12 (m, 4 H), 4.31 (q, *J* = 6.4 Hz, 1 H), 3.86 (s, 3 H), 3.62 (s, 3 H), 3.3 (s, 3 H), 3.23 (s, 3 H) 1.99-1.53 (m, 8 H); MS (ES⁺): 614.3 (M+ Na)⁺ |
| **119k** | -CH₃ | -OBn | -CO₂MEM | 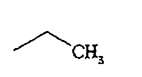 | **118k** | F | ¹H NMR (DMSO-*d₆*): δ 8.70 (t, *J* = 5.5 Hz, 1 H), 8.35 (d, *J* = 1.9 Hz, 1 H), 8.05 (dd, *J* = 1.7 and 7.9 Hz, 1 H), 7.59-7.30 (m, 7 H), 6.77 (s, 1 H), 5.21-5.08 (m, 4 H), 3.82 (s, 3 H), 3.58 (s, 3 H), 3.40-3.29 (m, 6 H), 3.18 (s, 3 H), 1.14 (t, *J* = 7.2 Hz, 3 H); MS (ES⁺): 574.3 (M+ Na)⁺ |
| **119l** | -CH₃ | -OBn | -CO₂MEM | 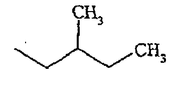 | **118l** | F | ¹H NMR (DMSO-*d₆*): δ 8.68 (t, *J* = 5.8 Hz, 1 H), 8.35 (d, *J* = 1.9 Hz, 1 H), 8.05 (dd, *J* = 1.7 and 7.9 Hz, 1 H), 7.63-7.33 (m, 7 H), 6.77 (s, 1 H), 5.22-5.08 (m, 4 H), 3.82 (s, 3 H), 3.58 (s, 3 H), 3.39-3.22 (m, 6 H), 3.18 (s, 3 H), 1.56 (qui, *J* = 7.0 Hz, 2 H), 1.27 (m, 1 H), 0.94-0.75 (m, 6 H); MS (ES⁺): 616.3 (M+ Na)⁺ |
| **119m** | 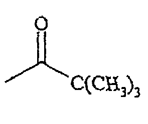 | -OBn | -CO₂MEM | 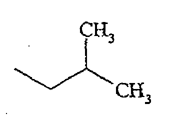 | **118m** | F | ¹HNMR (DMSO-d₆): δ 8.72 (t, J = 5.6 Hz, 1 H), 8.38 (d, J = 1.8 Hz, 1 H), 8.70 (dd, J = 1.8 and 8.1 Hz, 1 H), 7.71 (s, 1 H), 7.40 (m, 6 H), 7.02 (s, 1 H), 5.20 (m, 4 H), 3.59 (s, 3 H), 3.37 (m, 2 H), 3.31 (m, 2 H), 3.17 (s, 3 H), 3.12 (t, J = 6.5 Hz, 2 H), 1.87 (m, 1 H), 1.21 (s, 9 H), 0.91 (d, J = 6.8 Hz, 6 H); MS (ES+): 650.4 and 672.3 (M+ Na)⁺ |
| **120a** | -CH₃ | -OH | -CO₂MEM | 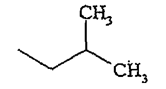 | **119a** | G | MS (ES⁻): 488.1 |
| **120b** | -C₂H₅ | -OH | -CO₂MEM | 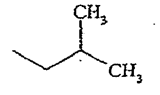 | **119b** | G | MS (ES⁻): 502.2 |
| **120c** | -CH(CH₃)₂ | -OH | -CO₂MEM | 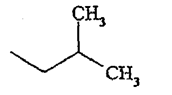 | **119c** | G | MS (ES⁻): 516.3 |
| **120d** | -CH₃ | -OH | -CO₂MEM | 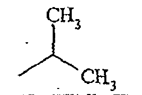 | **119d** | G | MS (ES⁻): 474.3 |
| **120e** | -CH₃ | -OH | -CO₂MEM | 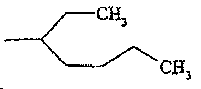 | **119e** | G | MS (ES⁻): 530.4 |
| **120f** | -CH₃ | -OH | -CO₂MEM | 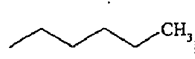 | **119f** | G | MS (ES⁻): 502.3 |
| **120g** | -CH₃ | -OH | -CO₂MEM | 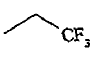 | **119g** | G | Characterized in the next step |
| **120h** | -CH₃ | -OH | -CO₂MEM | 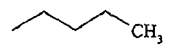 | **119h** | G | Characterized in the next step |
| **120i** | -CH₃ | -OH | -CO₂MEM | 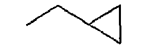 | **119i** | G | MS (ES⁻): 486.3 |
| **120j** | -CH₃ | -OH | -CO₂MEM | 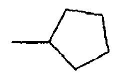 | **119j** | G | MS (ES⁺): 524.3 (M+ Na)⁺ |
| **120k** | -CH₃ | -OH | -CO₂MEM | 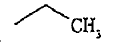 | **119k** | G | MS (ES⁺): 484.2 (M+ Na)⁺ |
| **120l** | -CH₃ | -OH | -CO₂MEM | 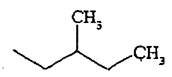 | **119l** | G | MS (ES⁻): 502.3 |
| **120m** | 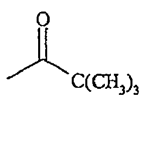 | -OH | -CO₂MEM | 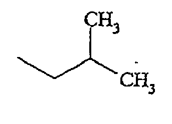 | **119m** | G | ¹HNMR (DMSO-d₆): δ 10.83 (bs, 1 H), 8.77 (t, J = 5.6 Hz, 1 H), 8.42 (d, J = 1.8 Hz, 1 H), 8.12 (dd, J = 1.8 and 8.1 Hz,1 H), 7.68 (s, 1 H), 7.41 (d, J = 8.1 Hz, 1 H), 6.73 (s, 1 H), 5.21 (q, J = 21 and 6 Hz, 2 H), 3.65 (s, 3 H), 3.48 (m, 2 H), 3.37 (m, 2 H), 3.24 (s, 3 H), 3.18 (t, J = 6.5 Hz, 2 H), 1.94 (m, 1 H), 1.39 (s, 9 H), 0.97 (d, J = 6.8 Hz, 6 H); MS (ES+): 560.5 and 582.4 (M+ Na)⁺, (ES⁻) 558.4 |
| **121a** | -CH₃ | -OSO₂CF₃ | -CO₂MEM | 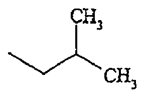 | **120a** | B-2 | MS (ES⁺): 644.1 (M+ Na)⁺ |
| **121b** | -C₂H₅ | -OSO₂CF₃ | -CO₂MEM | 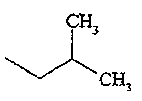 | **120b** | B-2 | MS (ES⁺): 658.2 (M+ Na)⁺ |
| **121c** | -CH(CH₃)₂ | -OSO₂CF₃ | -CO₂MEM | 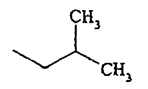 | **120c** | B-2 | MS (ES⁺): 672.2 (M+ Na)⁺ |
| **121d** | -CH₃ | -OSO₂CF₃ | -CO₂MEM | 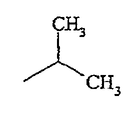 | **120d** | B-2 | ¹HNMR (DMSO-d₆): δ 8.43 (d, J = 1.9 Hz, 1 H), 8.31 (s, 1 H), 8.12 (d, J = 1.69 Hz, 1 H), 7.98 (s, 1 H), 7.41 (d, J = 8.1 Hz, 1 H), 7.19 (s, 1 H), 5.20 (m, 2 H), 3.98 (m, 1 H), 3.94 (s, 3 H), 3.42 (s, 3 H), 3.19 (s, 3 H), 2.50 (m, 2 H), 1.08 (d, J = 6.59, 6 H); MS (ES+) 608.3 |
| **121e** | -CH₃ | -OSO₂CF₃ | -CO₂MEM | 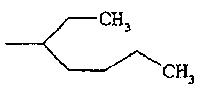 | **120e** | B-2 | ¹HNMR (DMSO-d₆): δ 8.49 (s, 1 H), 8.34 (d, J = 1.8 Hz, 1 H), 8.2 (d, J = 1.8 Hz, 1 H), 7.97 (s, 1 H), 7.4 (d, J = 7.8 Hz, 1 H), 7.2 (s, I H), 5.2 (q, J = 6 and 10 Hz, 2 H), 4.0 (m, 3 H), 3.6 (s, 3 H), 3.4 (m, 4 H), 3.2 (s, 3 H), 1.5 (m, 4 H), 1.3 (m, 4 H), 0.85 (m, 6 H); MS (ES+): 664.3 |
| **121f** | -CH₃ | -OSO₂CF₃ | -CO₂MEM | 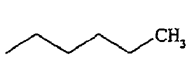 | **120f** | B-2 | ¹HNMR (DMSO-d₆): δ 8.83 (d, J = 5.46, 1 H), 8.55 (d, J = 1.88 Hz, 1 H), 8.23 (dd, J = 1.88 Hz, 1 H), 8.19 (s, 1 H), 7.73 (d, J = 7.93 Hz, 1 H), 7.29 (s, 1 H), 5.29 (dd, J = 6.217 Hz, 2 H), 4.06 (s, 3 H), 3.71 (s, 2 H), 3.54 (m, 5 H), 2.62 (t, J = 3.57 Hz, 3 H), 1.66 (t, J = 6.59 Hz, 2 H), 1.42 (m, 6 H), 0.99 (t, J = 6.79 Hz, 3 H); MS (ES+) 636.6 |
| **121g** | -CH₃ | -OSO₂CF₃ | -CO₂MEM | 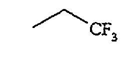 | **120g** | B-2 | ¹H NMR (CDCl₃): δ 8.43 (d, *J* = 1.9 Hz, 1 H), 8.03 (dd, *J* = 7.9 Hz, 2.1 Hz, 1 H), 8.00 (s, 1 H), 7.35 (d, *J* = 7.9 Hz, 1 H), 6.79 (m, 2 H), 5.29 (d, *J* = 6.2 Hz, 1 H), 5.26 (d, *J* = 6.2 Hz, 1 H), 4.16 (m, 2 H), 3.94 (s, 3 H), 3.67 (s, 3 H), 3.48 (m, 4 H), 3.36 (s, 3 H); MS (ES⁻): 646.3 |
| **121h** | -CH₃ | -OSO₂CF₃ | -CO₂MEM | 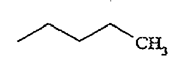 | **120h** | B-2 | ¹H NMR (CDCl₃): δ 8.41 (s, 1 H), 7.96 (d, J = 8.3 Hz, 2 H), 7.8 (m, 1 H), 6.80 ( s, 1 H), 6.34 (m, 1 H), 5.32 (m, 2 H), 3.90 (s, 3 H), 3.66 (s, 3 H), 3.55 (m, 6 H), 3.4 ( s, 3 H), 1.7 (m, 2 H), 1.45 (m, 2 H), 0.98 (t, J = 7.3 Hz, 3 H); MS (ES⁻): 620 |
| **121i** | -CH₃ | -OSO₂CF₃ | -CO₂MEM | 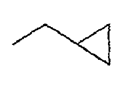 | **120i** | B-2 | ¹H NMR (CDCl₃): δ 8.41 (d, *J* = 2.1 Hz, 1 H), 8.03 (dd, *J* = 7.9, 1.9 Hz, 1 H), 8.00 (s, 1 H), 7.32 (d, *J* = 7.9 Hz, 1 H), 6.43 (t, *J* = 4.9 Hz, 1 H), 5.30 (q, *J* = 6.0 Hz, 2 H), 3.94 (s, 3 H), 3.67 (s, 3 H), 3.55 (m, 2 H), 3.48 (m, 2 H), 3.35 (m, 5 H), 1.09 (m, 1 H), 0.59 (m, 2 H), 0.31 (m, 2 H); MS (ES⁻): 618.4 |
| **121j** | -CH₃ | -OSO₂CF₃ | -CO₂MEM | 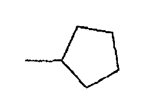 | **120j** | B-2 | ¹H NMR (CDCl₃): δ 8.35 (d, *J* = 1.9 Hz, 1 H), 8.00 (m, 2 H), 7.31 (d, *J* = 7.9 Hz, 1 H), 6.77 (s, 1 H), 6.27 (m, 1 H), 5.28 (m, 2 H), 4.44 (q, *J* = 7.0 Hz, 1 H), 3.94 (s, 3 H), 3.66 (s, 3 H), 3.57-3.45 (m, 4 H), 3.35 (s, 3 H), 2.19-1.45 (m, 8 H); MS (ES⁺): 656.3 (M+ Na)⁺ |
| **121k** | -CH₃ | -OSO₂CF₃ | -CO₂MEM | 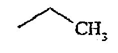 | **120k** | B-2 | ¹H NMR (CDCl₃): δ 8.38 (s, 1 H), 8.00 (m, 2 H), 7.31 (d, *J* = 7.9 Hz, 1 H), 6.78 (s, 1 H), 6.37 (m, 1 H), 5.27 (m, 2 H), 3.94 (s, 3 H), 3.66 (s, 3 H), 3.59-3.43 (m, 6 H), 3.35 (s, 3 H), 1.28 (t, *J* = 7.2 Hz, 3 H); MS (ES⁺): 616.3 (M+ Na)⁺ |
| **1211** | -CH₃ | -OSO₂CF₃ | -CO₂MEM | 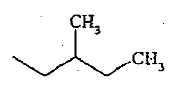 | **1201** | B-2 | ¹H NMR (CDCl₃): δ 8.38 (s, 1 H), 8.00 (m, 2 H), 7.31 (d, *J* = 7.9 Hz, 1 H), 6.78 (s, 1 H), 6.37 (m, 1 H), 5.27 (m, 2 H), 3.94 (s, 3 H), 3.66 (s, 3 H), 3.57-3.25 (m, 9 H), 1.78-0.92 (m, 9 H); MS (ES⁺): 658.4 (M+ Na)⁺ |
| **121m** | 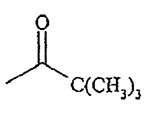 | -OSO₂CF₃ | -CO₂MEM | 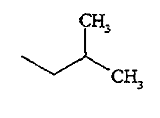 | **121m** | B-2 | ¹HNMR (DMSO-d₆): δ 8.75 (t, J = 5.6Hz, 1 H), 8.45 (d, J = 1.8 Hz, 1 H), 8.11 (dd, J = 1.8 and 8.1 Hz, 1 H), 8.04 (s, 1 H), 7.57 (s, 1 H), 7.42 (d, J = 8.1 Hz, 1 H), 5.23 (q, J = 21 and 6 Hz, 2 H), 3.60 (s, 3 H), 3.41 (m, 2 H), 3.32 (m, 2 H), 3.17 (s, 3 H), 3.13 (t, J = 6.5 Hz, 2 H), 1.87 (m, 1 H), 1.37 (s, 9 H), 0.91 (d, J = 6.8 Hz, 6 H); MS (ES-): 690.4 |
| **122a** | -CH₃ | -CH=CH₂ | -CO₂MEM | 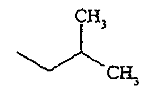 | **121a** | D-3 | Characterized in the next step |
| **122b** | -C₂H₅ | -CH=CH₂ | -CO₂MEM | 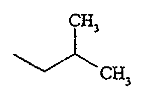 | **121b** | D-3 | MS (ES⁺): 536.3 (M+ Na)⁺ |
| **122c** | -CH(CH₃)₂ | -CH=CH₂ | -CO₂MEM | 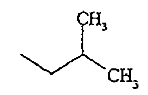 | **121c** | D-3 | MS (ES⁺): 550.3 (M+ Na)⁺ |
| **122d** | -CH₃ | -CH=CH₂ | -CO₂MEM | 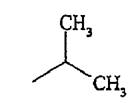 | **121d** | D-3 | MS (ES⁺): 486.2 |
| **122e** | -CH₃ | -CH=CH₂ | -CO₂MEM | 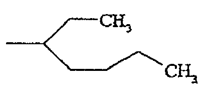 | **121e** | D-3 | MS (ES⁺): 564.5 (M+ Na)⁺ |
| **122f** | -CH₃ | -CH=CH₂ | -CO₂MEM | 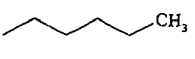 | **121f** | D-3 | MS (ES⁺): 514.4 (M+ Na)⁺ |
| **122g** | -CH₃ | -CH=CH₂ | -CO₂MEM | 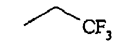 | **121g** | D-3 | Characterized in the next step |
| **122h** | -CH₃ | -CH=CH₂ | -CO₂MEM | 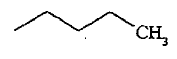 | **121h** | D-3 | Characterized in the next step |
| **122i** | -CH₃ | -CH=CH₂ | -CO₂MEM | 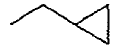 | **121i** | D-3 | Characterized in the next step |
| **122j** | -CH₃ | -CH=CH₂ | -CO₂MEM | 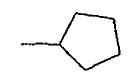 | **121j** | D-3 | MS (ES⁻): 422.3 [(M-MeM)-1] |
| **122k** | -CH₃ | -CH=CH₂ | -CO₂MEM | 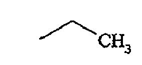 | **121K** | D-3 | MS (ES⁺): 494.2 (M+ Na)⁺ |
| **122l** | -CH₃ | -CH=CH₂ | -CO₂MEM | 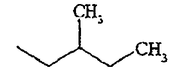 | **121l** | D-3 | MS (ES⁺): 536.42 (M+ Na)⁺ |
| **122m** | 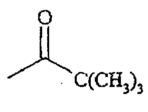 | -CH=CH₂ | -CO₂MEM | 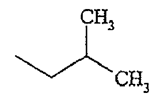 | **121m** | D-3 | ¹HNMR (DMSO-d₆): δ 8.73 (t, J = 5.6 Hz, 1 H), 8.43 (d, J = 1.8 Hz, 1 H), 8.11 (dd, J = 1.8 and 8.1 Hz, 1 H), 7.61 (s, I H), 7.57 (s, 1 H), 7.42 (d, J = 8.1 Hz, 1 H), 6.72 (dd, J = 11 and 17.5 Hz, 1 H), 6.03 (d, J = 17.5 Hz, 1 H), 5.52 (d, J = 11 Hz, 1 H), 5.19 (q, J = 18 and 6 Hz, 2 H), 3.60 (s, 3 H), 3.41 (m, 2 H), 3.32 (m, 2 H), 3.18 (s, 3 H), 3.13 (t, J = 6.5 Hz, 2 H), 1.89 (m, 1 H), 1.38 (s, 9 H), 0.91 (d, J = 6.8 Hz, 6 H); MS (ES-): 480.4 [(M-MEM)-1] |
| **123a** | -CH₃ | -CH=CH₂ | CO₂H | 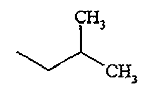 | **122a** | I-1 | MS (ES⁻): 410.2 |
| **123b** | -C₂H₅ | -CH=CH₂ | CO₂H | 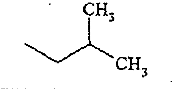 | **122b** | I-1 | MS (ES⁻): 424.2 |
| **123c** | -CH(CH₃)₂ | -CH=CH₂ | CO₂H | 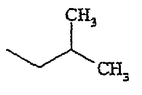 | **122c** | I-1 | MS (ES⁻): 438.2 |
| **123d** | -CH₃ | -CH=CH₂ | CO₂H | 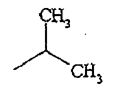 | **122d** | I-1 | MS (ES⁻): 396.2 |
| **123e** | -CH₃ | -CH=CH₂ | CO₂H | 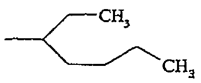 | **122e** | I-1 | MS (ES⁺): 454.3 |
| **123f** | -CH₃ | -CH=CH₂ | CO₂H | 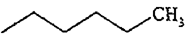 | **122f** | I-1 | MS (ES⁺): 426.3 |
| **123g** | -CH₃ | -CH=CH₂ | CO₂H | 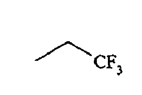 | **122g** | I-1 | ¹H NMR (DMSO): δ 12.37 (s, I H), 9.35 (t, *J* = 6.0 Hz, 1 H), 8.42 (d, *J* = 1.7 Hz, 1 H), 8.10 (dd, *J* = 8.1 Hz, 1.9 Hz, 1 H), 8.06 (s, 1 H), 7.40 (d, *J* = 7.9 Hz, 1 H), 6.98 (dd, *J* = 17.9, 11.5 Hz, 1 H), 6.77 (s, 1 H), 5.89 (dd, *J* =17.7, 1.3 Hz, 1 H), 5.37 (dd, *J* = 11.1, 1.3 Hz, 1 H), 4.14 (m, 2 H), 3.84 (s, 3 H), 3.61 (s, 3 H); MS (ES⁻): 436.3 |
| **123h** | -CH₃ | -CH=CH₂ | CO₂H | 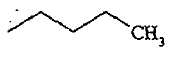 | **122h** | I-1 | ¹H MMR (DMSO): δ 8.66 (t, *J* = 5.5 Hz, 1 H), 8.35 (d, *J* = 1.7 Hz, 1 H), 8.05 (s, 1 H), 8.03 (dd, *J* = 8.1, 1.9 Hz, 1 H), 7.34 (d, *J* = 7.9 Hz, 1 H), 6.98 (dd, *J* = 17.9, 11.3 Hz, 1 H), 6.75 (s, 1 H), 5.88 (dd, *J* = 17.7, 1.3, 1H), 5.36 (dd, *J*= 11.3, 1.3 Hz, 1 H), 3.84 (s, 3 H), 3.60 (s, 3 H), 3.30 (q, *J* = 5.6 Hz, 2 H), 1.52 (m, 2 H), 1.33 (m, 2 H), 0.96 (t, *J* = 7.3 Hz, 3 H); MS (ES⁻): 410.4 |
| **123i** | -CH₃ | -CH=CH₂ | CO₂H | 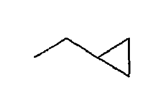 | **122i** | I-1 | ¹H NMR (DMSO): δ 12.34 (s, 1 H), 8.80 (t, *J* = 6.1 Hz, 1 H), 8.37 (d, *J* = 1.9 Hz, 1 H), 8.06 (dd, *J* = 9.8, 7.9 Hz, I H), 8.05 (s, 1 H), 7.36 (d, *J* = 7.9 Hz, 1 H), 6.98 (dd, *J* = 17.9, 11.3 Hz, I H), 6.76 (s, 1 H), 5.89 (dd, J = 17.9, 1.5 Hz, 1 H), 5.36 (dd, *J* = 10.9, 1.5 Hz, 1 H), 3.84 (s, 3 H), 3.60 (s, 3 H), 3.18 (t, 6.2, 2 H), 1.06 (m, 1 H), 0.45 (m, 2 H), 0.25 (m, 2 H); MS (ES⁻): 408.4 |
| **123j** | -CH₃ | -CH=CH₂ | CO₂H | 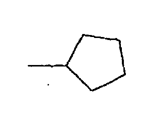 | **122j** | I-1 | ¹H NMR (DMSO-*d₆*): δ 12.31 (br s, 1 H), 8.52 (d, *J* = 7.3 Hz, 1 H), 8.34 (d, *J* = 1.7 Hz, 1 H), 8.05 (m, 2 H), 7.34 (d, *J* = 7.9 Hz, 1 H), 6.97 (dd, *J* = 11.5 and 17.9 Hz, 1 H), 6.74 (s, I H), 5.89 (d, *J* = 17.9 Hz, 1 H), 5.37 (d, J= 11.5 Hz, 1 H), 4.27 (q, *J* = 7.3 Hz, 1 H), 3.84 (s, 3 H), 3.60 (s, 3 H), 1.98-1.50 (m, 8 H); MS (ES⁻): 422.3 |
| **123k** | -CH₃ | -CH=CH₂ | CO₂H | 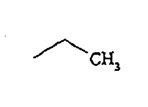 | **122k** | I-1 | ¹H NMR (DMSO-*d₆*): δ 12.27 (br s, 1 H), 8.58 (m, 1 H), 8.23 (s, 1 H), 7.92 (m, 2 H), 7.47 (m, 1 H), 7.22 (m, 1 H), 6.84 (m, I H), 6.63 (s, 1 H), 5.76 (d, *J* = 17.9 Hz, 1 H), 5.24 (d, *J* = 11.5 Hz, 1 H), 3.71 (s, 3 H), 3.47 (s, 3 H), 1.02 (m, 3 H); MS (ES⁻): 382.2 |
| **1231** | -CH₃ | -CH=CH₂ | CO₂H | 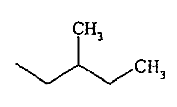 | **122l** | I-1 | ¹H NMR (DMSO-*d*₆): δ 12.30 (br s, 1 H), 8.52 (d, *J* = 6.0 Hz, 1 H), 8.33 (d, *J* = 1.7 Hz, 1 H), 8.02 (m, 2 H), 7.31 (d, *J* = 7.9 Hz, 1 H), 6.95 (dd, *J* = 11.5 and 17.9 Hz, 1 H), 6.73 (s, 1 H), 5.86 (d, *J =* 17.9 Hz, 1 H), 5.33 (d, *J* = 11.5 Hz, 1 H), 3.81 (s, 3 H), 3.57 (s, 3 H), 3.14 (m, 2 H), 1.65 (m, 1 H), 1.39 (m, 1 H), 1.11 (m, 1 H), 0.87 (m, 6 H) |
| **123m** | 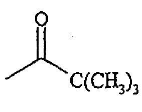 | -CH=CH₂ | -CO₂H | 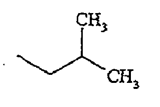 | **122m** | I-1 | ¹HNMR (DMSO-d₆): δ 12.81 (bs, 1 H), 8.72 (t, J = 5.6 Hz, 1 H), 8.38 (d, J = 1.8 Hz, 1 H), 8.08 (dd, J = 1.8 and 8.1 Hz, 1 H), 7.61 (s, 1 H), 7.57 (s, 1 H), 7.39 (d, J = 8 Hz, 1 H), 6.72 (dd, J = 11 and 17.5 Hz, 1 H), 5.99 (d, J = 17.5 Hz, 1 H), 5.49 (d, J = 11 Hz, 1 H), 3.57 (s, 3 H), 3.13 (t, J = 6.5 Hz, 2 H), 1.87 (m, 1 H), 1.37 (s, 9 H), 0.91 (d, J = 6.8 Hz, 6 H); MS (ES-): 480.3 |

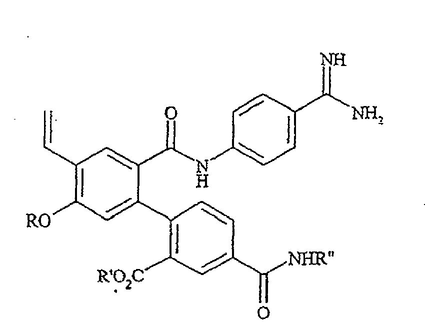

| **Cpd. No.** | **-R** | **-R'** | **R"** | **Starting From** | **Method Used** | **Analytical Data** |
|---|---|---|---|---|---|---|
| **124a** | -CH₃ | -CH₃ | 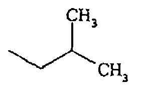 | **123a** | J | MS (ES⁺): 529.3 |
| **124b** | -C₂H₅ | -CH₃ | 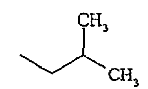 | **123b** | J | MS (ES⁺): 543.3 |
| **124c** | -CH(CH₃)₂ | -CH₃ | 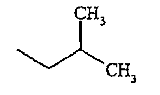 | **123c** | J | MS (ES⁺): 557.3 . |
| **124d** | -CH₃ | -CH₃ | 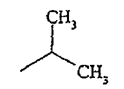 | **123d** | J | Characterized in the next step |
| **124e** | -CH₃ | -CH₃ | 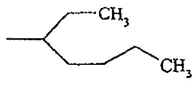 | **123e** | J | MS (ES⁺): 571.6 |
| **124f** | -CH₃ | -CH₃ | 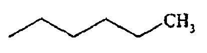 | **123f** | J | MS (ES⁺): 543.6 |
| **124g** | -CH₃ | -CH₃ | 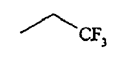 | **123g** | J | ¹H NMR (DMSO): δ 10.62 (s, 1 H), 9.35 (t, *J* = 6.6 Hz, 1 H), 9.20 (s, 2 H), 8.90 (s, 2 H), 8.30 (d, *J* = 1.9 Hz, 1 H), 8.11 (dd, *J =* 8.1, 1.9 Hz, 1 H), 7.86 (s, 1 H), 7.76 (s, 4 H), 7.50 (d, *J* = 8.1 Hz, 1 H), 7.04 (dd, *J* = 17.9, 11.5 Hz, 1 H), 6.94 (s, 1 H), 6.01 (dd, *J* = 17.7, 1.3, 1 H), 5.42 (dd, *J* = 11.3, 13 Hz, 1 H), 4.11 (m, 2 H), 3.89 (s, 3 H), 3.57 (s, 3 H) |
| **124h** | -CH₃ | -CH₃ | 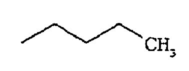 | **123h** | J | ¹H NMR (DMSO): δ 9.03 (broad, 3 H), 8.49 (broad, 1 H), 8.04 (s, 1 H), 7.65 (m, 6 H), 6.99 (m, 2 H), 6.61 (s, 1 H), 5.90 (d, *J*= 17.5 Hz, 1 H), 5.35 (d, *J* = 11.5 Hz, 1 H), 3.78 (s, 3 H), 3.20 (m, 2 H), 1.46 (m, 2 H), 1.28 (m, 2 H), 0.87 (t, *J* = 7.3 Hz, 3 H) |
| **124i** | -CH₃ | -CH₃ | 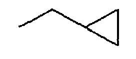 | **123i** | J | MS (ES⁺): 527.4 |
| **124j** | -CH₃ | -CH₃ | 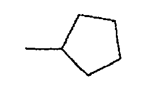 | **123j** | J | MS (ES⁺): 541.4 |
| **124k** | -CH₃ | -CH₃ | 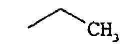 | **123K** | J | MS (ES⁺): 501.3 |
| **124l** | -CH₃ | -CH₃ | 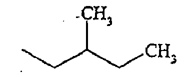 | **123l** | J | MS (ES⁺): 543.3 |
| **124m** | 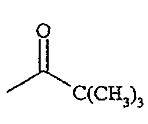 | -CH₃ | 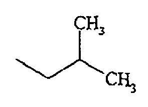 | **123m** | J | ¹HNMR (DMSO-d₆): δ 10.67 (s, 1H), 9.19 (bs, 2 H), 8.88 (bs, 2 H), 8.71 (t, J = 5.6 Hz, 1 H), 8.25 (d, J = 1.8 Hz, 1 H), 8.07 (dd, J = 1.8 and 8.1 Hz, 1 H), 7.73 (m, 4 H), 7.65 (s, 1 H), 7.50 (d, J = 8 Hz, 1 H), 7.45 (s, 1 H), 6.73 (dd, J = 11 and 17.5 Hz, 1 H), 6.03 (d, J = 17.5 Hz, 1 H), 5.49 (d, J = 11 Hz, 1 H), 3.56 (s, 3 H), 3.09 (t, J = 6.5 Hz, 2 H), 1.85 (m, 1 H), 1.37 (s, 9 H), 0.89 (d, J = 6.8 Hz, 6 H); MS (ES-): 597.3 and (ES⁺) 599.5 |
| **125a** | -CH₃ | -H | 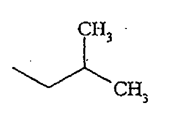 | **124a** | 1-2 | ¹HNMR (DMSO): δ 13.40 (bs, 1H), 9.26 and 9.03 (2s, 4H), 8.53-8.49 (t, J = 6 Hz, 1H), 8.02 (d, *J*=1.28 Hz, 1H), 7.71-7.53 (m, 6H), 7.0-6.9 (m, 2H), 6.5 (s, 1H), 5.89 (d, *J*=17.6 Hz, 1H), 5.33 (d, *J*=12.4 Hz, 1H), 3.77 (s, 3H), 3.04-2.99 (m, 2H), 1.85-1.75 (m, 1H), 0.86-0.84 (d, *J*=76.8 Hz, 6H); MS (ES⁺): 515.3 |
| **125b** | -C₂H₅ | -H | 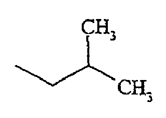 | **124b** | 1-2 | ¹HNMR (DMSO): δ 9.17 and 8.92 (s, 3H), 8.67-8.63 (m, 1H), 8.28 (s, 1H), 7.95-7.93 (m, 1H), 7.83 (s, 1H), 7.73 (s, 5H), 7.29 (d, *J*=8.1 Hz, 1H), 7.02 (dd, *J*=17.7 Hz, 11.3 Hz, 1H), 6.82 (s, 1H), 6.00 (d, 17.7 Hz, 1H), 5.38 (d, 11.3 Hz, 1H), 4.14-4.06 (m, 2H), 3.11-3.04 (q, *J*=6.8 Hz, 2H), 1.89-1.80 (m, 1H), 1.35 (t, *J*=6.8 Hz, 3H), 0.88 (d, *J*=6.8 Hz, 6H); MS (ES⁺): 529.2 |
| **125c** | -CH(CH₃)₂ | -H | 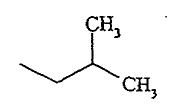 | **124c** | 1-2 | ¹HNMR (DMSO): δ 13.74 (s, 1H), 8.99 (s, 3H), 8.59-8.41 (m, 1H), 7.95 (s, 1H), 7.69 (s, 1H), 7.65-7.53 (m, 6H), 7.06-6.91 (m, 2H), 6.53 (s, 1H), 5.89 (d, *J*=17.7 Hz, 1H), 5.32 (d, *J*=11.5 Hz, 1H), 4.62-4.54 (m, 1H), 3.03-2.99 (m, 2H), 1.87-1.71 (m, 1H), 1.25 (d, *J*=6.1 Hz, 6H), 0.85 (d, *J*=6.8 Hz, 6H); MS (ES⁻): 541.2 |
| **125d** | -CH₃ | -H | 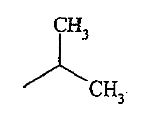 | **124d** | I-2 | ¹HNMR (DMSO-d₆): δ 8.9 (d, J = 33.74, 4 H), 8.08 (d, J = 7.91, 1 H), 7.81 (s, 1 H), 7.51 (s, 1 H), 7.41(s, 4 H), 6.78 (s, 1 H), 6.3 (s, 2 H), 5.70 (d, J = 7.78 Hz, 1 H), 5.15 (d, J = 11.8 Hz, 2 H),) 3.82 (m, J = 20.34 Hz, 2 H), 3.56 (bs, 3 H) 0.92 (d, 6H); MS (ES+) 501.3 |
| **125e** | -CH₃ | -H | 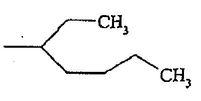 | **124e** | I-2 | ¹HNMR (DMSO-d₆): δ 9.05 (s, 2 H), 8.85 (s, 2 H), 7.96 (d, J = 9.04 Hz, 1 H), 7.88 (s, 1 H), 6,86 (m, J = 17.8 Hz, 3 H), 7.62 (m, 1 H), 7.24 (d, J = 7.8 Hz, 1 H), 6.95 (d, J = 7.8 Hz, 1 H), 7.45 (m, J = 28.63 Hz, 5 H), 7.55 (s, 1 H), 5.75 (d, J = 17.5 Hz, 1 H); 5.61 (d, J= 11.11, 1 H) 3.61(s, 3H) 1.30 (bs, 3 H) 1.05 (s, 4 H) 0.66 (m, 6 H); MS (ES+) 555.3(100% M⁺¹) |
| **125f** | -CH₃ | -H | 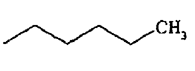 | **124f** | I-2 | ¹H NMR (DMSO-d₆): δ 12.7 (bs, 1H), 9.01 (bs, 2H), 8.87 (bs, 2H), 8.36 (t, J = 6 Hz, 1H), 7.83 (s; 1H), 7.44 (m, 6H), 6.75 (m, 2H), 6.31 (d, J = 2.2 Hz, 1H), 5.7 (d, J = 17 Hz, 1H), 5.1 (d, J = 11 Hz, 1H), 3.5 (s, 3H), 2.84 (m, 2H), 1.3 (m, 2H), 1.1 (m, 4H), 0.7 (m, 3H); MS (MS+): 529.4 |
| **125g** | -CH₃ | -H | 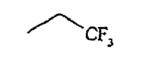 | **124g** | I-2 | ¹H NMR (DMSO): δ 9.22 (broad, 1 H), 9.09 (s, 2 H), 8.9 (s, 2 H), 8.18 (s, 1 H), 7.80 (m, 2 H), 7.66 (m, 4 H), 7.16 (s, 1 H), 7.00 (dd, J= 17.7, 11.1 Hz, 1 H), 6.70 (s, 1 H), 5.94 (d, *J* = 17.7 Hz, 1 H), 5.37 (d, *J* = 10.9 Hz, 1 H), 4.07 (m, 2 H), 3.81 (s, 3 H); MS (ES⁻) 539.3 |
| **125h** | -CH₃ | -H | 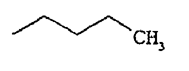 | **124h** | 1-2 | ¹H NMR (DMSO): δ 9.03 (bs, 4 H), 8.49 (bs, 1 H), 8.04 (s, 1 H), 7.65 (m, 6 H), 6.99 (m, 2 H), 6.61 (s, 1 H), 5.90 (d, *J* =17.5 Hz, 1 H), 5.35 (d, J = 11.5 Hz, 1 H), 3.78 (s, 3 H), 3.20 (m, 2 H), 1.46 (m, 2 H), 1.28 (m, 2 H), 0.87 (t, *J* = 7.3 Hz, 3 H); MS (ES⁺) 515.4 |
| **125i** | -CH₃ | -H | 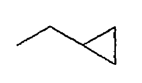 | **124i** | 1-2 | ¹H NMR (DMSO): δ 8.86 (s, 2 H), 8.78 (s, 2 H), 8.44 (broad, 1 H), 7.89 (s, 1 H), 7.53 (m, 2 H), 7.43 (m, 4 H), 6.86 (s, 1 H), 6.78 (dd, *J* = 17.5, 11.3 Hz, 1 H), 6.44 (s, 1 H), 5.71 (d, *J* = 17.5 Hz, 1 H), 5.14 (d, *J* = 11.1 Hz, 1 H), 3.59 (s, 3 H), 2.89 (m, 2H), 0.79 (m, 1 H), 0.20 (m, 2 H), 0.01 (m, 2 H); MS (ES⁻) 513.4 |
| **125j** | -CH₃ | -H | 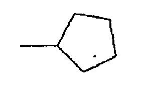 | **124j** | I-2 | ¹H NMR (DMSO): δ 13.14 (br s, 1 H), 8.84 (m, 3 H), 8.12 (d, *J* = 7.3 Hz, 1 H), 7.79 (s, 1 H), 7.40 (m, 8 H), 6.74 (m, 2 H), 6.33 (s, 1 H), 5.66 (d, *J* = 19.2 Hz, 1 H), 5.10 (d, *J* = 11.7 Hz, 1 H), 3.94 (m, 1 H), 3.54 (s, 3 H), 1.66-0.93 (m, 8 H); MS (ES⁺) 527.4 |
| **125k** | -CH₃ | -H | 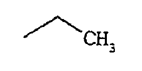 | **124k** | I-2 | ¹N MR (DMSO): δ 9.25 (m, 4 H), 8.73 (t, *J* = 5.7 Hz, 1 H), 8.28 (s, 1 H), 7.86 (m, 7 H), 6.84 (s, 1 H), 6.10 (d, *J* = 17.7 Hz, 1 H), 5.55 (d, *J* = 11.3 Hz, 1 H), 3.99 (s, 3 H), 3.43 (qui, *J* = 6.2 Hz, 2 H), 1.28 (t, *J* = 7.2 Hz, 3 H); MS (ES⁺): 487.2 |
| **125l** | -CH₃ | -H | 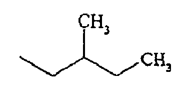 | **124l** | 1-2 | ¹H NMR (DMSO): δ 8.91 (m, 4 H), 8.38 (t, *J*= 5.5 Hz, 1 H), 7.96 (s, 1 H), 7.53 (m, 5 H), 6.86 (m, 2 H), 6.52 (s, 1 H), 5.77 (d, *J* = 17.7 Hz, 1 H), 5.21 (d, *J*= 11.5 Hz, 1 H), 3.65 (s, 3 H), 2.94 (m, I H), 1.57-0.56 (m, 11 H); MS (ES⁺): 529.3 |
| **125m** | -H | -H | 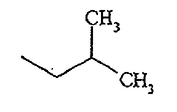 | **124m** | I-2 | ¹HNMR (DMSO-d₆): δ 10.07 (bs, 1H), 9.05 (bs, 2 H), 8.98 (bs, 2 H), 8.49 (t, J = 5.6 Hz, 1 H), 7.96 (s, 1 H), 7.62 (m, 5 H), 7.06 (s, 1 H), 7.03 (s, 1 H), 6.94 (dd, J = 11 and 18 Hz, 1 H), 5.78 (d, J = 18 Hz, 1 H), 5.26 (d, J = 11 Hz, 1 H), 3.02 (t, J = 5.7 Hz, 2 H), 1.81 (m, 1 H), 0.85 (d, J = 6.8 Hz, 6 H); MS (ES-): 499.2 and (ES⁺) 501.3 |

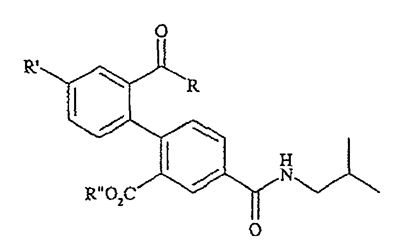

| **Cpd. No.** | **-R** | **-R'** | | **Starting From** | **Method Used** | **Analytical Data** |
|---|---|---|---|---|---|---|
| **133a** | 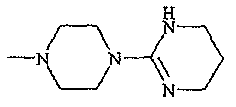 | -H | -CH₃ -CH₃ | **132** | A-5 | MS (ES⁺): 506.4 |
| **133b** | 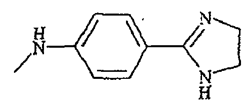 | -H | -CH₃ | **132** | J | MS (ES⁺): 499.3 |
| **133c** | 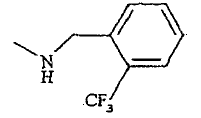 | -H | -CH₃ | **132** | A-5 | Characterized in the next step |
| **133d** | 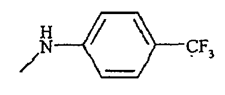 | -H | -CH₃ | **132** | A-5 | Characterized in the next step |
| **133e** | 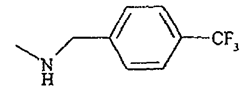 | -H | -CH₃ | **132** | A-5 | Characterized in the next step |
| **133f** | 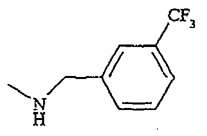 | -H | -CH₃ | **132** | A-5 | Characterized in the next step |
| **133g** | 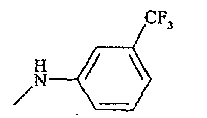 | -H | -CH₃ | **132** | A-5 | Characterized in the next step |
| **133h** | 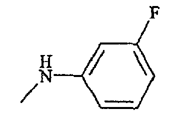 | -H | -CH₃ | **132** | A-5 | Characterized in the next step |
| **133i** | 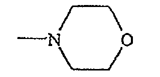 | -H | -CH₃ | **132** | A-5 | Characterized in the next step |
| **133j** | 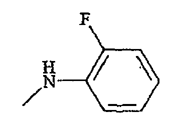 | -H | -CH₃ | **132** | A-5 | Characterized in the next step |
| **133k** | 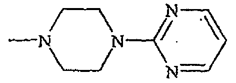 | -H | -CH₃ | **132** | J | MS (ES⁺): 502.3 |
| **133l** | 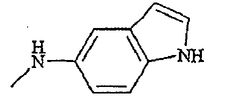 | -H | -CH₃ | **132** | J | MS (ES⁺): 470.2 |
| **133m** | 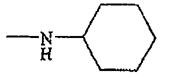 | -H | -CH₃ | **132** | J | MS (ES⁺): 437.3 |
| **133n** | 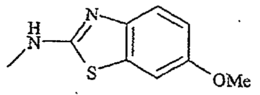 | -H | -CH₃ | **132** | J | MS (ES⁺): 518.2 |
| **133o** | 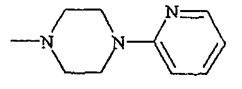 | -H | -CH₃ | **132** | J | MS (ES⁺): 501.3 |
| **133p** | 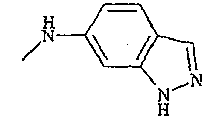 | -H | -CH₃ | **132** | J | MS (ES⁻): 469.1 |
| **133q** | 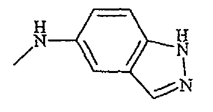 | -H | -CH₃ | **132** | J | MS (ES⁻): 469.1; MS (ES⁺): 471.2 |
| **133r** | 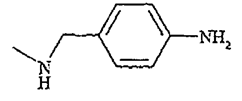 | -H | -CH₃ | **132** | A-5 | Characterized in the next step |
| **133s** | 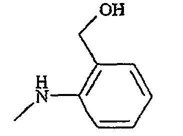 | -H | -CH₃ | **132** | A-5 | MS (ES⁺): 483.2 (M+Na) |
| **133u** | 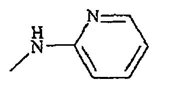 | -H | -CH₃ | **132** | A-5 | MS (ES⁺): 432.2 |
| **133v** | 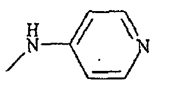 | -H | -CH₃ | **132** | A-5 | MS (ES⁺): 432.2 |
| **133w** | 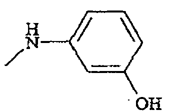 | -H | -CH₃ | **132** | A-5 | MS (ES⁺): 447.2 |
| **133x** | 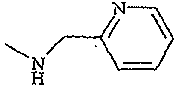 | -H | -CH₃ | **132** | A-5 | Characterized in the next step |
| **133y** | 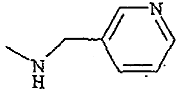 | -H | -CH₃ | **132** | A-5 | MS (ES⁺): 446.3 |
| **133z** | 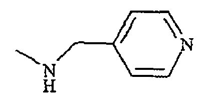 | -H | -CH₃ | **132** | A-5 | MS (ES⁺): 446.2 |
| **133aa** | 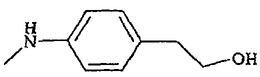 | -H | -CH₃ | **132** | A-4 | MS (ES⁺): 475.3 |
| **133ab** | 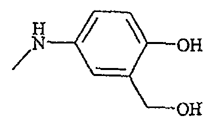 | -H | -CH₃ | **132** | J | MS (ES⁺): 499.3 (M+Na) |
| **133ac** | 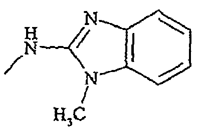 | -H | -CH₃ | **132** | A-4 | MS (ES⁻): 483.2; MS (ES⁺): 485.2 |
| **133ad** | 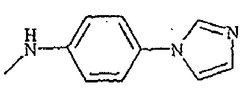 | -H | -CH₃ | **132** | A-4 | MS (ES⁺): 497.2; MS (ES⁻): 495.2 |
| **133ae** | 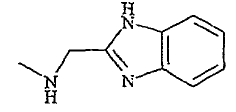 | -H | -CH₃ | **132** | A-4 | MS (ES⁻): 483.2; MS (ES⁺): 485.2 |
| **133af** | 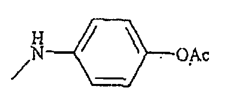 | -H | -CH₃ | **132** | J | MS (ES⁺): 511.3 (M+Na)⁺; MS (ES⁻): 487.3 |
| **133ag** | 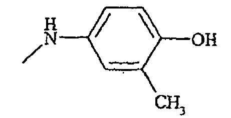 | -H | -CH₃ | **132** | J | MS (ES⁻): 451.3 |
| **133ai** | 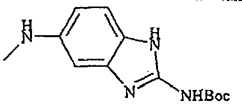 | -H | -CH₃ | **132** | J | MS (ES⁻): 584.4 |
| **134a** | 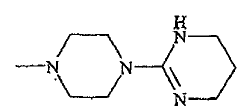 | -H | -H | **133a** | 1-2 | ¹HNMR (DMSO-d₆): δ 13.13 (bs, 1 H), 8.76 (t, J = 6 and 5 Hz, 1 H), 8.32 (m, 2 H), 8.02 (dd, J = 1.9 and 8.1 Hz, 1 H), 7.42 (m, 4 H), 7.25 (m, 1 H), 3.62-3.19 (m, 12 H), 3.11 (t, J = 6.8 Hz, 2 H), 1.87 (m, 1 H), 1.76 (m, 2 H), 0.90 (d, J = 6.8 Hz, 6 H); MS (ES-) 490.3; (ES+) 492.3 |
| **134b** | 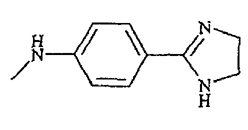 | -H | -H | **133b** | 1-2 | ¹HNMR (DMSO-d₆): 8 13.82 (bs, 1 H), 10.57 (bs, 2 H), 8.50 (t, J = 6 and 5 Hz, 1 H), 7.99 (d, J = 1.5 Hz, 1 H), 7.83 (s, 1 H), 7.8 (s, 1 H), 7.59 (m, 4 H), 7.46 (m, 2 H), 7.03 (m, 1 H), 6.92 (d, J = 7.9 Hz, 1 H), 3.89 (s, 4 H), 3.02 (t, J = 6.8 Hz, 2 H), 1.81 (m, 1 H), 0.8 (d, J = 6.8 Hz, 6 H);MS (ES⁻): 483.3; MS (ES⁺): 485.4 |
| **134c** | 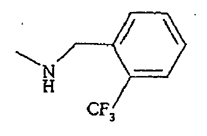 | -H | -H | **133c** | I-2 | ¹HNMR (DMSO-d₆): δ 8.71 (t, J=5.5 Hz, 1 H), 8.40 (t, J=5.3 Hz, 1H), 8.30 (s, 1 H), 8.00(d, J= 7.8 Hz, 1 H), 7.63 (d, J=4.3 Hz, 2 H), 7.40 (d, J=7.4 Hz, 4 H), 7.27(d, J=8.1 Hz, 1 H), 7.18 (s, 1 H), 6.91 (d, J=7.1 Hz, 1 H), 4.42 (b, 2 H), 3.13 (t, J = 6.5 Hz, 2 H), 1.93 (m, 1 H), 0.91 (d, J = 6.8 Hz, 6 H); MS (ES-) 497.3 |
| **134d** | 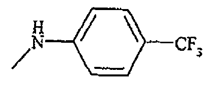 | -H | -H | **133d** | 1-2 | ¹HNMR (DMSO-d₆)_{:} δ 10.45 (s, 1 H), 8.63 (s, 1 H), 8.27 (s, 1 H), 7.93 (d, J=8.1Hz, I H), 7.67 (t, J=6.8 Hz, 2 H), 7.55 (m, 2 H), 7.27 (m 3 H), 7.12 (m, 2 H), 3.06 (t, J = 6 Hz, 2 H), 1.82 (m, 1 H), 0.86 (d, J = 6.8 Hz, 6 H); MS(ES-) 483.3 |
| **134e** | 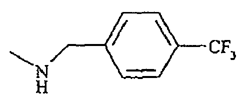 | -H | -H | **133e** | I-2 | ¹HNMR (DMSO-d₆): δ 12.92 (bs, 1 H), 8.71 (t, J=5.8Hz, 1 H), 8.49(t, J=6.2 Hz, 1 H), 8.32 (s, 1 H), 8.01 (d, J=7.8 Hz, 1 H), 7.52 (m, 5 H), 7.27 (d, J=7.9 Hz, 1 H), 7.18 (m, 1 H), 7.08 (d, J=8.2 Hz, 2 H), 4.32 (d, J=4.2 Hz, 2 H), 3.12 (t, J = 6.5 Hz, 2 H), 1.88 (m, 1 H), 0.91 (d, J = 6.8 Hz, 6 H); MS(ES-) 498.2 |
| **134f** | 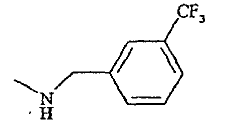 | -H | -H | **133f** | I-2 | ¹HNMR (DMSO-d₆): δ 8.66 (t, J=5.7 Hz, 1 H), 8.27 (s, 1 H), 7.92 (d, J=8.1 Hz, 1 H), 7.45 (m, 7 H), 7.18 (m, 3 H), 4.32 (d, J=5.9 Hz, 2 H), 3.12 (t, J = 6 Hz, 2 H), 1.89 (m, 1 H), 0.91 (d, J = 6.8 Hz, 6 H); MS(ES-) 497.2 |
| **134g** | 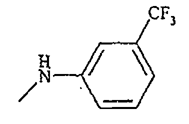 | -H | -H | **133g** | I-2 | ¹HNMR (DMSO-d₆): δ 13.1 (s, 1 H), 9.58 (s, 1 H), 8.65 (s, 1 H), 8.29 (s, 1 H), 7.98 (d, J=5.9Hz, 1 H), 7.75 (d, J=5.2 Hz, 2 H), 7.30 (d, J=8 Hz, 2 H), 7.12 (d, J=12.0 Hz, 1 H), 7.12 (m, 4 H), 3.06 (t, J = 6 Hz, 2 H), 1.85 (m, I H), 0.86 (d, J = 6.8 Hz, 6 H); MS (ES-) 483.2 |
| **134h** | 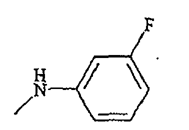 | -H | -H | **133h** | I-2 | ¹HNMR (DMSO-d₆): δ 10.31 (s, 1 H), 8.65 (t, J=6.2 Hz, 1 H), 8.31 (s, 1 H), 7.98 (d, J= 7.9 Hz, 1 H), 7.66 (m, 1 H), 7.53 (m, 3 H), 7.27 (m, 4 H), 6.85 (m, 1 H), 3.09 (t, J = 6.5 Hz, 2 H), 1.86 (m, 1 H), 0.89 (d, J = 6.8 Hz, 6 H); MS (ES-) 433.1(M⁻¹) |
| **134i** | 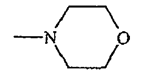 | -H | -H | **133i** | I-2 | ¹HNMR (DMSO-d₆): δ 8.71 (t, J=5.7 Hz, 1 H), 8.31 (s, 1 H), 8.01 (d, J= 7.9 Hz, 1 H), 7.46 (m, 2 H), 7.39 (m, 2 H), 7.24 (s, 1 H), 3.38 (b, 8 H), 3.11 (t, J = 6.5 Hz, 2 H), 1.86 (m, 1 H), 0.91(d, J = 6.8 Hz, 6 H); MS(ES-) 409.3 |
| **134j** | 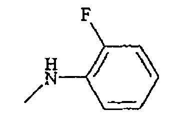 | -H | -H | **133j** | I-2 | ¹HNMR (DMSO-d₆): δ 9.61 (s, 1 H), 8.67 (t, J=5.5 Hz, 1 H), 8.32 (s, I H), 7.98 (d, J= 7.9 Hz, 1 H), 7.71 (m, 2 H), 7.54 (m, 2 H), 7.29 (d, J=7.9 Hz, 1 H), 7.04 (m, 4 H), 3.10 (t, J = 6.5 Hz, 2 H), 1.86 (m, 1 H), 0.89 (d, J = 6.8 Hz, 6 H); MS (ES-) 433.3 |
| **134k** | 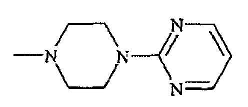 | -H | -H | **133k** | I-2 | ¹HNMR (DMSO-d₆): δ 8.59 (t, J = 6 and 5 Hz, 1 H), 8.3 (d, J = 5 Hz, 2 H), 8.18 (s, 1 H), 7.86 (d, J = 8 Hz, 1 H), 7.36 (m, 5 H), 6.6 (t, J = 4.7 Hz, 1 H), 4.0 (m, 1 H), 3.75 (m, 2 H), 3.37 (m, 5 H), 3.07 (t, J = 6.8 Hz, 2 H), 1.81 (m, 1 H), 0.85 (d, J = 6.8 Hz, 6 H) |
| **1341** | 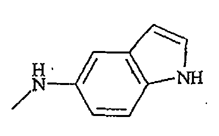 | -H | -H | **133l** | I-2 | ¹HNMR (DMSO-d₆): δ 10.92 (bs, 1 H), 8.55 (t, J = 6 and 5 Hz, 1 H), 8.14 (s, 1 H), 7.76 (d, J = 7 Hz, 1 H), 7.68 (m, 1 H), 7.62 (m, 1 H), 7.45 (m, 2 H), 7.24 (t, J = 2.6 Hz, 1 H), 7.19 (s, 1 H), 7.15 (s, 1 H), 7.10 (m, 2 H), 6.95 (dd, J = 1.5 and 8.7 Hz, 1 H), 6.28 (s, 1 H), 3.04 (t, J = 6.8 Hz, 2 H), 1.82 (m, 1 H), 0.86 (d, J = 6.8 Hz, 6 H); MS (ES-) 454.3; (ES+) 456.3 |
| **134m** | 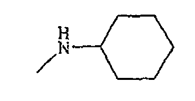 | -H | -H | **133m** | I-2 | ¹HNMR (DMSO-d₆): δ 13.30 (bs, 1 H), 8.62 (t, J = 6 and 5 Hz, 1 H), 8.18 (s, 1 H), 7.87 (d, J = 7.9 Hz, 1 H), 7.42 (m, 3 H), 7.09 (m, 2 H), 3.03 (m, 1 H), 3.1 (t, J = 6.8 Hz, 2 H), 1.86 (m, 1 H), 1.4 (m, 4 H), 1.09 (m, 1 H), 0.89 (d, J = 6.8 Hz, 6 H); MS (ES-) 421.2; (ES+) 423.2 |
| **134n** | 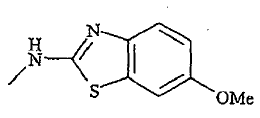 | -H | -H | **133n** | I-2 | ¹HNMR (DMSO-d₆): δ 15.89 (bs, 1 H), 8.56 (t, J = 6 and 5 Hz, 1 H), 8.06 (s, 1 H), 7.67 (m, 2 H), 7.54 (d, J = 8.8 Hz, 1 H), 7.48 (m, 4 H), 7.05 (m, 1 H), 6.96 (m, 2 H), 3.77 (s, 3 H), 3.03 (t, J = 6.8 Hz, 2 H), 1.81 (m, 1 H), 0.84 (d, J = 6.8 Hz, 6 H); MS (ES-) 502.3; (ES+) 504.3 |
| **134o** | 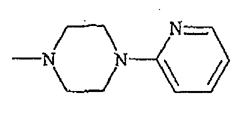 | -H | -H | **133o** | I-2 | ¹HNMR (DMSO-d₆): δ 13.07 (bs, 1 H), 8.63 (t, J = 6 and 5 Hz, 1 H), 8.26 (s, 1 H), 8.05 (d, J = 4 Hz, 1 H), 7.94 (d, J = 8 Hz, 1 H), 7.43 (m, 5 H), 7.28 (m, 1 H), 6.72 (d, J = 8.8 Hz, 1 H), 6.62 (dd, J = 5.5 and 6.5 Hz, 1 H), 3.34 (m, 8 H), 3.07 (t, J = 6.8 Hz, 2 H), 1.82 (m, 1 H), 0.85 (d, J = 6.8 Hz, 6 H); MS (ES-) 486.3; (ES+) 488.3 |
| **134p** | 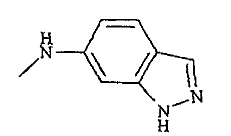 | -H | -H | **133p** | I-2 | ¹HNMR (DMSO-d₆): δ 12.94 (bs, 1 H), 10.20 (bs, I H), 8.63 (t, J = 6 and 5 Hz, 1 H), 8.28 (d, J = 1.5 Hz, 1 H), 7.96 (m, 2 H), 7.92 (d, J = 8.3 Hz, 1 H), 7.68 (m, 1 H), 7.52 (m, 2 H), 7.4 (m, 1 H), 7.3 (m, 2 H), 7.24 (m, 1 H), 3.08 (t, J = 6.8 Hz, 2 H), 1.84 (m, 1 H), 0.88 (d, J = 6.8 Hz, 6 H); MS (ES-) 455.2; (ES+) 479.2 (M+Na) |
| **134q** | 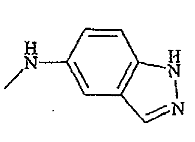 | -H | -H | **133q** | I-2 | ¹HNMR (DMSO-d₆): δ 12.84 (bs, 1 H), 10.45 (bs, 1 H), 8.62 (t, J = 6 and 5 Hz, 1 H), 8.27 (d, J = ,1.5 Hz, 1 H), 8.01 (s, 1 H), 7.93 (s, 2 H), 7.9 (d, J = 1.5 Hz, 1 H), 7.69 (m, 1 H), 7.57 (d, J = 8.7 Hz, 1 H), 7.52 (m, 2 H), 7.29 (d, J = 8 Hz; 1 H), 7.23 (m, 1 H), 7.02 (dd, J = 1.5 and 8.7 Hz, 1 H), 3.07 (t, J = 6.8 Hz, 2 H), 1.83 (m, 1 H), 0.87 (d, J = 6.8 Hz, 6 H), MS (ES-) 455.2; (ES+) 479.3 (M+Na) |
| **134r** | 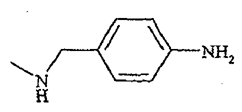 | -H | -H | **133r** | 1-2 | ¹HNMR (DMSO-d₆): δ 8.64 (t, J=5.5 Hz, 1 H), 8.16 (s, 1 H), 7.87 (d, J=7.1 Hz, 1H), 7.50 (m, I H), 7.40 (d, J=4.1 Hz, 2 H), 7.19 (b, 3 H), 7.07 (m, 2 H), 6.51 (m, 2 H), 6.35 (d, J=7.8 Hz, 2 H), 3.97 (d, J=5.6 Hz, 2 H), 3.13 (t, J = 6.5 Hz, 2 H), 1.90 (m, 1 H), 0.91 (d, J = 6.8 Hz, 6 H) |
| **134s** | 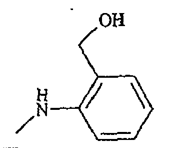 | -H | -H | **133s** | 1-2 | ¹HNMR (DMSO-d₆): δ 9.53 (bs, 1 H), 8.67 (t, J=4.7 Hz, 1 H), 8.32 (s, 1 H), 7.99 d, J=8.1 Hz, 1 H), 7.70 (d, J=7.6 Hz, 1 H), 7.52 (m, 2 H), 7.46 (d, J=11.5 Hz, 1 H), 7.32 (m, 3 H), 7.18 (m, 3 H), 4.33 (s, 2 H), 3.10 (t, J = 6.5 Hz, 2 H), 1.86 (m, 1 H), 0.89 (d, J = 6.8 Hz, 6 H); MS (ES-) 445.2 |
| **134t** | -OH | -H | -H | **132** | I-2 | ¹HNMR (DMSO-d₆): δ 12.57 (b, 1 H), 8.69 (t, J=5.6 Hz, 1 H), 8.36 (s, 1 H), 7.99 (d, J= 7.9 Hz, 1 H), 7.92 (d, J=7.7 Hz, 1 H), 7.57(t, J=7.5 Hz, 1H), 7.46 (t, J=7.7 Hz, 1H), 7.23 (d, J=5.2 Hz, 1H), 7.17 (d, J=7.5 Hz, 1H), 3.12 (t, J = 6.5 Hz, 2 H), 1.88 (m, 1 H), 0.91 (d, J = 6.8 Hz, 6 H); MS (ES-) 340.2 |
| **134u** | 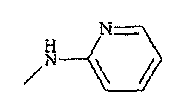 | -H | -H | **133u** | 1-2 | ¹HNMR (DMSO-d₆): δ 8.56 (t, J=5.0 Hz, 1 H), 8.16 (d, J=7.0 Hz, 2 H), 7.94 (d, J=8.4 Hz, 1 H), 7.75 (d, J=7.4 Hz, 1 H), 7.63 (m, 2 H), 7.46 (m, 2 H), 7.21 (b, 1 H), 7.07 (s, 2 H), 6.99 (t, J=5.1 Hz, I H), 3.05 (t, J = 6.5 Hz, 2 H), 1.83 (m, I H), 0.86(d, J = 6.8 Hz, 6 H); MS (ES-) 416.3 |
| **134v** | 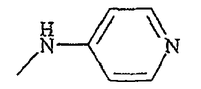 | -H | -H | **133v** | 1-2 | ¹HNMR (DMSO-d₆): δ 8.60 (t, J=5.6 Hz, 1 H), 8.32 (d, J=5.3 Hz, 2 H), 8.11(s, 1 H), 7.78 (d, J=7.7 Hz, 1 H), 7.65 (d, J=5.5 Hz, 1 H), 7.55 (m, 2 H), 7.43 (d, J=4.5 Hz, 2 H), 7.14 (m, 3 H), 3.06 (t, J = 6.5 Hz, 2 H), 1.83 (m, 1 H), 0.86 (d, J = 6.8 Hz, 6 H); MS (ES-) 416.2 |
| **134w** | 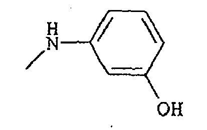 | -H | -H | **133w** | I-2 | ¹HNMR (DMSO-d₆)_{:} δ 10.10 (bs, 1 H), 9.31 (s, 1 H), 8.65 (t, J=5.7 Hz, 1 H), 8.27 (s, 1 H), 7.93 (d, J=8.1 Hz, 1 H), 7.62 (d, J=5.3 Hz, 1 H), 7.48 (m, 2 H), 7.28(s, 1 H), 7.20 (d, J=12.0 Hz, I H), 7.09 (s, 1 H), 6.98 (d, J=7.0 Hz, 1 H), 6.81 (d, J=7.3 Hz, I H), 6.37 (t, J=7.6 Hz, I H), 3.09 (t, J = 6.5 Hz, 2 H), 1.85 (m, 1 H), 0.90(d, J = 6.8 Hz, 6 H); MS (ES-) 431.1 |
| **134x** | 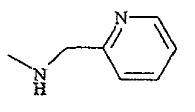 | -H | -H | **133x** | I-2 | ¹HNMR (DMSO-d₆): δ 10.28 (bs, 1 H), 8.63 (t, J=5.3 Hz, 1 H), 8.34 (d, J=4.7 Hz, 1 H), 8.06 (s, 1 H), 7.82 (d, J=6.6 Hz, 1 H), 7.53 (m, 1 H), 7.42 (m, 2 H), 7.34 (t, J=8.6 Hz, 1 H), 7.18 (s, 1 H), 7.07 (d, J=2.7Hz, 2 H), 6.10 (b; 1 H), 4.43 (b; I H), 4.12 (b, 1 H), 3.12 (t, J = 6.5 Hz, 2 H), 1.89 (m, 1 H), 0.90(d, J = 6.8 Hz, 6 H); MS (ES+) 432.3, (ES-) 430.2 |
| **134y** | 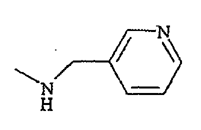 | -H | -H | **133y** | I-2 | ¹HNMR (DMSO-d₆): δ 9.79 (bs, 1 H), 8.62 (t, J=6.0 Hz, 1 H), 8.31 (d, J=4.5 Hz, 1 H), 8.20 (s, 1 H), 8.08 (s, 1 H), 7.78 (d, J=2.1 Hz, 1 H), 7.51 (m, 1 H), 7.42 (m, 2 H), 7.06 (m, 3 H), 6.88 (m, 1 H), 4.02 (b, 2 H), 3.13 (t, J = 6.5 Hz, 2 H), 1.90 (m, 1 H), 0.93 (d, J = 6.8 Hz, 6 H); MS (ES+) 432.3, (ES-) 430.3 |
| **134z** | 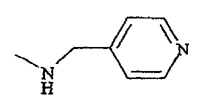 | -H | -H | **133z** | I-2 | ¹HNMR (DMSO-d₆): δ 10.71 (bs, 1 H), 8.64 (t, J=5.9 Hz, 1 H), 8.21 (d, J=5.2 Hz, 2 H), 8.05 (s, 1 H), 7.81 (d, J=7.7 Hz, 1 H), 7.51 (m, 1 H), 7.42 (m, 2 H), 7.18 (s, 1 H), 7.04 (t, J=1.4Hz, 2 H), 6.51 (b, 2H), 4.41 (b, 1 H), 4.01 (b, 1 H), 3.13 (t, J = 6.5 Hz, 2 H), 1.91 (m, 1 H), 0.91 (d, J = 6.8 Hz, 6 H); MS (ES+) 432.2, (ES-) 430.2 |
| **134aa** | 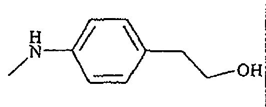 | -H | -H | **133aa** | I-2 | ¹HNMR (DMSO-d₆): δ 10.02 (bs, 1 H), 8.65 (t, J = 5.7 Hz, 1 H), 8.26(s, 1 H), 7.94(d, J= 7.7 Hz, 1 H), 7.66(d, J=5.8 Hz, 1 H), 7.51(m, 2 H), 7.36 (d, J=8.4 Hz, 2 H), 7.29 (d, J=7.9 Hz, 1 H), 7.22 (d, J=5.5 Hz, 1 H), 7.07(d, J=8.3 Hz, 2 H), 4.57 (t, J=9.0 Hz, 1 H), 3.51 (m, 2 H), 3.09 (t, J = 6.5 Hz, 2 H), 2.62 (t, J=6.6 Hz, 2 H), 1.85 (m, 1 H), 0.90(d, J = 6.8 Hz, 6 H), MS(ES-) 459.2 |
| **134ab** | 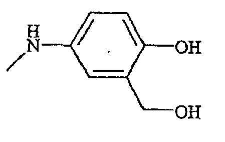 | -H | -H | **133ab** | I-2 | ¹HNMR (DMSO-d₆): δ 9.05 (s, 1 H), 8.70 (t, J=5.7 Hz, 1 H), 8.56 (s, 1 H), 8.36 (s, 1 H), 8.12 (m, 2 H), 7.79 (m, 1 H), 7.60 (m, 1 H), 7.44 (s, 2 H), 7.09 (m, 2 H), 6.56 (d, J=8.9 Hz, 1 H), 4.89 (t, J=4.4 Hz, 1 H), 4.38 (d, J=5.6 Hz, 2 H), 3.11 (t, J = 6.5 Hz, 2 H), 1.84 (m, 1 H), 0.90 (d, J = 6.8 Hz, 6 H), MS(ES-) 461.1 |
| **134ac** | 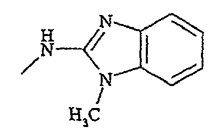 | -H | -H | **133ac** | I-2 | ¹HNMR (DMSO-d₆): δ 8.60 (t, J = 6 and 5 Hz, 1 H), 8.13 (s, 2 H), 7.85 (d, J = 2 Hz, 1 H), 7.46 (m, 4 H), 7.36 (d, J = 7.7 Hz, 1 H), 7.16 (m, 4 H), 7.10 (m, 1 H), 3.17 (s, 3 H), 3.08 (t, J = 6.8 Hz, 2 H), 1.85 (m, 1 H), 0.89 (d, J = 6.8 Hz, 6 H), MS (ES-) 469.2; (ES+) 471.3 |
| **134ad** | 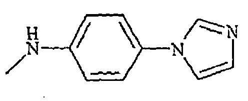 | -H | -H | **133ad** | I-2 | ¹HNMR (DMSO-d₆): δ 8.55 (t, J = 6 and 5 Hz, 1 H), 8.10 (s, 2 H), 7.73 (d, J = 7.2 Hz, 1 H), 7.54 (m, 4 H), 7.46 (m, 5 H), 7.08 (m, 3 H), 3.04 (t, J = 6.8 Hz, 2 H), 1.82 (m, 1 H), 0.86 (d, J = 6.8 Hz, 6 H), MS (ES-) 481.1; (ES+) 483.3 |
| **134ae** | 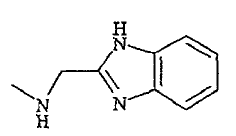 | -H | -H | **133ae** | 1-2 | ¹HNMR (DMSO-d₆): δ 9.66 (bs, 1H), 8.54 (t, J = 6 and 5 Hz, 1 H), 8.12 (s, 2 H), 7.77 (dd, J = 8 and 2 Hz, 1 H), 7.6 (dd, J = 7 and 2 Hz, 1 H), 7.45 (m, 5 H), 7.10 (m, 4 H), 4.36 (bs, 2 H), 3.09 (t, J = 6.8 Hz, 2 H), 1.86 (m, 1 H), 0.89 (d, J = 6.8 Hz, 6 H), MS (ES-) 469.2; (ES+) 471.3 |
| **134af** | 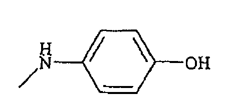 | -H | -H | **133af** | 1-2 | ¹HNMR (DMSO-d₆): δ 9.76 (s, 1 H), 9.17 (s, 1 H), 8.63 (t, J=5.0 Hz, 1 H), 8.29 (s, 1 H), 7.90 (d, J=1.6 Hz, 1 H), 7.60 (s, 1 H), 7.51 (d, J=8 Hz 1 H), 7.30 (d, J=3.6 Hz, 2 H), 7.28 (d, J=8.2Hz, 1 H), 7.22 (t, 3 H), 6.60 (d, J=8.9 Hz, 1 H), 3.06 (t, J = 6 Hz, 2 H), 1.85 (m, 1 H), 0.86 (d, J = 6.8 Hz, 6 H); MS (ES-) 431.2 |
| **134ag** | 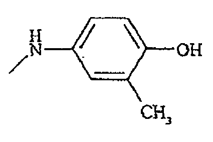 | -H | -H | **133ag** | I-2 | ¹HNMR (DMSO-d₆): δ 9.64 (s, 1 H), 9.06 (s, 1 H), 8.66 (t, J=5.6 Hz, 1 H), 8.29 (s, 1 H), 7.95 (d, J=7.9 Hz, 1 H), 7.63 (m, 1 H), 7.50 (m, 2 H), 7.29 (d, J=3.1 Hz, 1 H), 7.20 (d, J=8.9 Hz, 1 H), 7.11 (m, 1 H), 7.03 (m, 1 H), 6.60 (d, J=8.9 Hz, 1 H), 3.08 (t, J = 6 Hz, 2 H), 2.05 (s, 3 H), 1.85 (m, 1 H), 0.86 (d, J = 6.8 Hz, 6 H); MS (ES-) 445.2, MS (ES+) 469.3 (M+Na) |
| **134ai** | 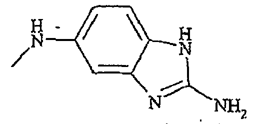 | -H | -H | **133ai** | I-2, S | MS (ES⁺): 472.2; MS (ES⁻): 470.2 |
| **135a** | 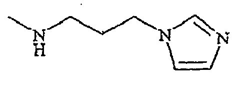 | -CH=CH₂ | -CH₃ | **30f** | A-4 | MS (ES⁺): 489.3 |
| **135b** | 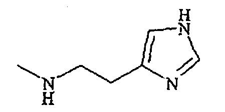 | -CH=CH₂ | -CH₃ | **30f** | A-4 | MS (ES⁺): 475.3; MS (ES⁺): 473.3 |
| **135c** | 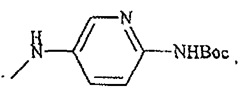 | -CH=CH₂ | -CH₃ | **30f** | J | MS (ES⁺): 573.5; MS (ES⁻): 571.3 |
| **135d** | 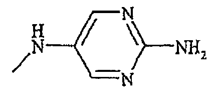 | -CH=CH₂ | -CH₃ | **30f** | A-4 | MS (ES⁻): 472.2 |
| **135e** | 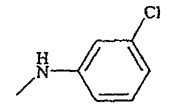 | -CH=CH₂ | -CH₃ | **30f** | J | MS (ES⁻): 489.1 |
| **135f** | 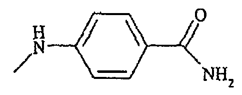 | -CH=CH₂ | -CH₃ | **30f** | J | MS (ES⁻): 498.1 |
| **135g** | 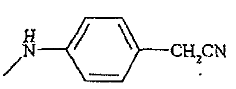 | -CH=CH₂ | -CH₃ | **30f** | J | MS (ES⁻): 494.3 |
| **135h** | 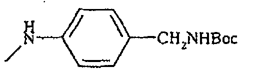 | -CH=CH₂ | -CH₃ | **30f** | J | MS (ES⁻): 584.2 |
| **136a** | 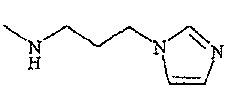 | -CH=CH₂ | -H | **135a** | I-2 | ¹HNMR (DMSO-d₆): δ 8.66 (t, J = .55 Hz, 1 H), 8.35 (t, J = 4 and 6.4 Hz,1 H), 8.28 (d, J = 2 Hz, 1 H), 7.95 (dd, J = 7.9 and 2 Hz, 1 H), 7.69 (s, 1 H), 7.59 (m, 2 H), 7.25 (d, J = 8.1 Hz, 2 H), 7.15 (m, 2 H), 6.93 (s, 1 H), 6.88 (dd, J = 17.7 and 11.5 Hz, 1 H), 5.95 (d, J = 17.7 Hz, 1 H), 5.37 (d, J = 11.5 Hz, 1 H), 3.76 (t, J = 6.8 Hz, 2 H), 3.10 (t, J = 6.4 Hz, 2 H), 2.96 (m, 2 H), 1.86 (m, 1 H), 1.67 (m, 2 H), 0.89 (d, J = 6.8 Hz, 6 H); MS (ES-) 473.3; (ES+) 475.3 |
| **136b** | 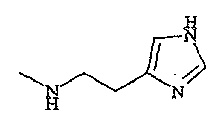 | -CH=CH₂ | -H | **135b** | I-2 | ¹HNMR (DMSO-d₆): δ 8.64 (t, 1 H), 8.51 (s, I H), 821(s, 1 H), 7.88 (d, J=7.8 Hz, 1 H), 7.74 (s, 1 H), 7.56 (s, 2 H), 7.15 (m, 2 H), 6.80 (t, 2 H), 5.90 (d, J=17 Hz, 1 H), 5.36 (d, J=11.0Hz, 1 H), 3.18 (m, 2 H), 3.06 (t, J = 6 Hz, 2 H), 2.43 (m, 2 H), 1.85 (m, 1 H), 0.86 (d, J = 6.8 Hz, 6 H); MS (ES+) 461.2, MS (ES-) 459.2 |
| **136c** | 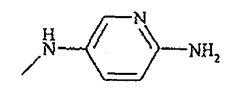 | -CH=CH₂ | -H | **135c** | I-2, S | ¹HNMR (DMSO-d₆/D₂O): δ 8.71 (t, 1 H), 8.27 (d, J= 3 Hz, 1 H), 8.21 (d, J=3 Hz, 1 H), 7.96 (q, 1 H), 7.79 (q, 1 H), 7.72 (s, 1 H), 7.63 (d, J=8 Hz 1 H), 7.30 (d, J=6 Hz, 1 H), 7.24 (d, J=7 Hz, 1 H), 6.87 (q, 2 H), 6.00 (d, J=8 Hz, 1 H), 5.41 (d, J=8 Hz, 1 H), 3.06 (t, J = 6 Hz, 2 H), 1.85 (m, 1 H), 0.86 (d, J = 6.8 Hz, 6 H); MS (ES+) 459.2 |
| **136d** | 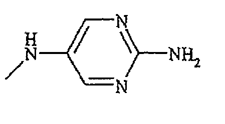 | -CH=CH₂ | -H | **135d** | I-2 | ¹HNMR (DMSO-d₆): δ 12.86 (bs, 1 H), 9.17 (s, 1 H), 8.65 (t, J = 6 Hz, 1 H), 8.29 (d, J = 2 Hz, 1 H), 8.26 (s, 2 H), 7.97 (dd, J = 8 and 2 Hz, 1 H), 7.76 (s, 1 H), 7.63 (d, 8 Hz, 1 H), 7.31 (d, J = 8 Hz, 1 H), 7.24 (d, J = 8 Hz, 1 H), 6.86 (dd, J = 10.7 and 17.5 Hz, 1 H), 6.49 (s, 1 H), 5.99 (d, J = 17.5, 1 H), 5.40 (d, J = 10.7 Hz, 1 H), 3.10 (t, J = 6.8 Hz, 2 H), 1.86 (m, I H), 0.89 (d, J = 6.8 Hz, 6 H); MS (ES-) 458.2, (ES+) 460.3 |
| **136e** | 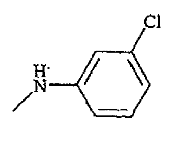 | -CH=CH₂ | -H | **135e** | I-2 | ¹HNMR (DMSO-d₆): δ 12.72 (s, broad, 1 H), 8.65(t, J=5.7 Hz, 1 H), 8.29 (s, 1 H), 7.93 (d, J=7.9 Hz, 1 H), 7.74 (m, 2 H), 7.65 (d, J=6 Hz 1 H), 7.42 (d, J=7.9 Hz, 1 H), 7.24 (m, 3 H), 7.11 (m, 1 H), 6.84 (q, J=11.1, 17.8 Hz, 1 H), 5.97 (d, J=18 Hz, 1 H), 5.58 (d, 1 H), 5.41 (d, I H), 3.08 (t, J = 6 Hz, 2 H), 1.85 (m, 1 H), 0.86 (d, J = 6.8 Hz, 6 H); MS (ES-) 475.1 |
| **136f** | 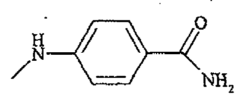 | -CH=CH₂ | -H | **135f** | I-2 | ¹HNMR (DMSO-d₆): δ 8.67 (t, J=6.06 Hz, 1 H), 8.28 (s, 1 H), 7.90 (d, J=7.7Hz, 1 H), 7.67 (m, 4 H), 7.32 (m, 5 H), 7.09 (d, J=7.9 Hz 1 H), 6.89 (q, J=10.9 & 18.0 Hz, 1 H), 5.99 (d, J=17.5Hz, 1 H), 5.42 (d, J=11 Hz, 1 H), 3.08 (t, J=6.3 Hz, 2 H), 1.88 (m, 1 H), 0.87 (d, J = 6.8 Hz, 6 H); MS (ES-) 484.2 |
| **136g** | 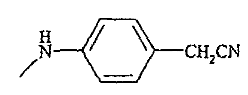 | -CH=CH₂ | -H | **135g** | I-2 | ¹HNMR (DMSO-d₆): δ 10.38 (s, 1 H), 8.66 (t, J=6.06 Hz, 1 H), 8.29 (s, 1 H), 7.95 (d, J=6.1 Hz, 1 H), 7.75 (s, 1 H), 7.63 (d, 2 H), 7.43 (d, 2 H), 7.26 (m, 3 H), 7.00 (d, J=7.7 Hz, 1 H), 6.85 (q, J=10.9 & 18.0 Hz, I H), 5.98 (d, J=17.5Hz, 1 H), 5.40 (d, J=11 Hz, 1 H), 3.98 (s, 2 H), 3.08 (t, J=6.3 Hz, 2 H), 1.86 (m, 1 H), 0.88 (d, J = 6.8 Hz, 6 H); MS (ES-) 480.2 |
| **136h** | 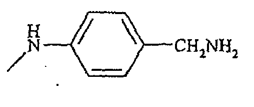 | -CH=CH₂ | -H | **135h** | S, I-2 | ¹HNMR (DMSO-d₆): δ 8.55 (t, J=6.06 Hz, 1 H), 8.02 (s, 1 H), 7.60(m, 4H), 7.21 (t, J=7.1, 2 H), 6.99(m, 2 H), 6.83 (d, J=6.8 Hz, 1H), 6.81 (q, J=10.9 & 18.0 Hz, 1H), 5.92 (d, J=17.5Hz, 1 H), 5.35 (d, J=11 Hz, I H), 3.89 (s, 2H), 3.03 (t, J=6.3 Hz, 2 H), 1.36 (m, 1 H), 0.86 (d, J = 6.8 Hz, 6 H) |

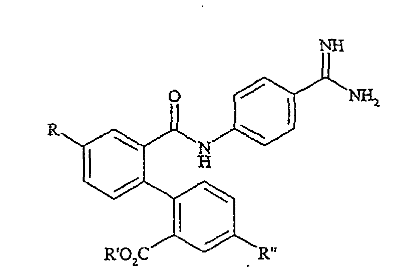

| **Cpd. No.** | **-R** | **-R'** | **-R"** | **Starting From** | **Method Used** | **Analytical Data** |
|---|---|---|---|---|---|---|
| **148a** | 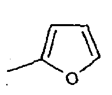 | -CH₃ | 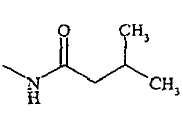 | **147a** | J | ¹H NMR (DMSO-d₆): δ 10.65 (s, 1 H), 10.15 (s, 1 H), 9.19 (s, 2 H), 8.88 (s, 2 H), 8.10 (d, *J* = 2.1 Hz, 1 H), 7.92 (s, 1 H), 7.93-7.75 (m, 6 H), 7.31 (dd, *J* = 8.4 and 23.9 Hz, 1 H), 7.12 (d, *J* = 3.5 Hz, 1 H), 6.67 (m, 1 H), 3.53 (s, 3 H), 2.20 (d, *J* = 7.0 Hz, 2 H), 2.07 (m, 1 H), 0.94 (d, *J* = 6.3 Hz, 6 H). |
| **148b** | 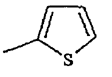 | -CH₃ | 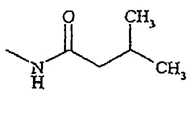 | **147b** | J | ¹H NMR (DMSO-d₆): δ 10.65 (s, 1 H), 10.09 (s, 1 H), 9.17 (s, 1 H), 8.83 (s, 1 H), 8.10 (d, *J*= 2.0 Hz, 1 H), 7.85 (d, *J* = 2.0 Hz, 2 H), 7.81 (d, *J*= 2.0 and 7.9 Hz, 2 H), 7.76 (m, 5 H), 7.66 (d, J= 3.9 Hz, 1 H), 7.62 (d, *J* = 4.9 Hz, 1 H), 7.31 (d, *J =* 7.9 Hz, 1 H), 7.26 (d, *J* = 7.9 Hz, 1 H), 7.19 (t, *J* = 3.9 Hz, 1 H), 3.53 (s, 1 H), 2.19 (d, *J* = 6.9 Hz, 2 H), 2.06 (m, *J* = 6.9 Hz, 1 H), 0.92 (d, *J* = 6.9 Hz, 6 H); MS (ES⁺): 555.67 |
| **148c** | -CH=CH₂ | -CH₃ | 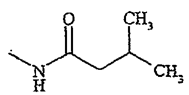 | **147c** | J | Characterized in the next step |
| **149a** | 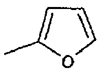 | -H | 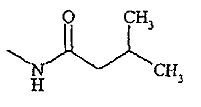 | **148a** | I-2 | MS (ES⁺): 525.3 |
| **149b** | 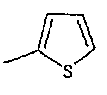 | -H | 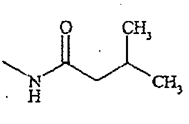 | **148b** | 1-2 | ¹H NMR (DMSO-d₆): δ 13.95 (s, 1 H), 9.79 (s, 1 H), 8.87 (s, 4 H), 7.76 (s, 1 H), 7.65 (m, 8 H), 7.46 (dd, *J* = 2.1 and 8.4 Hz, 1 H), 7.16 (t, *J* = 4.2 Hz, 1 H), 7.04 (d, *J* = 7.7 Hz, 1 H), 6.76 (d, *J* = 8.4 Hz, 1 H), 2.13 (d, *J* = 7.0 Hz, 2 H), 2.03 (m, *J* = 6.3 and 7.0 Hz, 1 H), 0.90 (d, *J* = 6.3 Hz, 6 H); MS (ES⁺): 541.62 |
| **149c** | -CH=CH₂ | -H | 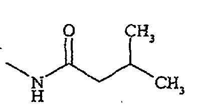 | **148c** | 1-2 | MS (ES⁺): 485.6 |
| **175** | -H | -CH₃ | 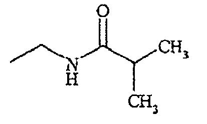 | **174** | J | ¹H NMR (DMSO-d₆): δ 8.81 (m, 4 H), 8.37 (t, *J* = 6.0 Hz, 1 H), 7.74-7.23 (m, 11 H), 4.31 (d, *J* = 6.2 Hz, 2 H), 3.51 (s, 3 H), 2.44 (m, 1 H), 1.04 (d, *J* = 7.0 Hz, 6 H); MS (ES⁺): 473.3 |
| **176** | -H | -H | 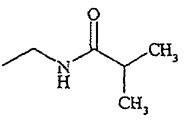 | **175** | 1-2 | ¹H ¹NMR (DMSO-d₆): δ 13.79 (br s, 1 H), 9.03 (m, 3 H), 8.25 (m, 1 H), 7.78-7.35 (m, 7 H), 6.99 (m, 2 H), 6.79 (m, 1 H), 4.20 (br s, 2 H), 3.51 (s, 3 H), 2.39 (m, 1 H), 1.00 (d, *J* = 6.8 Hz, 6 H); MS (ES⁺): 459.3 |
| **182** | -H | -CH₃ | 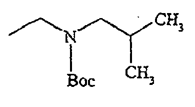 | **178** | J | ¹H NMR (DMSO-d₆): δ 8.96 (m, 2 H), 7.79-7.38 (m, 9 H), 7.29 (dd, *J* = 7.5 and 1.7 Hz, 2 H), 4.42 (s, 2 H), 3.50 (s, 3 H), 2.97 (s, 2 H), 1.87 (m, 1 H), 1.36 (m, 9 H), 0.81 (d, *J* = 6.8 Hz, 6 H); MS (ES⁺): 559.5 |
| **183** | -H | -H | 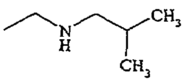 | **182** | I-2, S | ¹H NMR (DMSO-d₆): δ 9.11 (m, 4 H), 7.86 (s, 1 H), 7.66 (m, 5 H), 7.49 (m, 2 H), 7.38 (m, 1 H), 7.08 (m, 2 H), 4.12 (s, 2 H), 2.59 (m, 2 H), 1.87 (m, I H), 0.81 (d, *J* = 6.6 Hz, 6 H); MS (ES⁺): 445.32 |

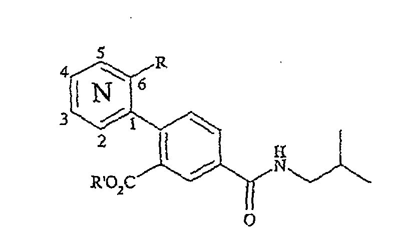

| **Cpd. No.** | **N (in Ring With Respect to Phenyl)** | **-R** | **-R'** | **Starting From** | **Method Used** | **Analytical Data** |
|---|---|---|---|---|---|---|
| **151** | 3 | -CHO | -CH₃ | **150 + 3a** | D-9 | MS (ES⁻): 339.3 |
| **152** | 3 | -CO₂H | -CH₃ | **151** | E | ¹H NMR (CDCl₃): δ 8.69 (t, *J* = 5.8 Hz, 1 H), 8.50 (d, *J* = 4.9 Hz, I H), 8.33 (d, *J* = 1.7 Hz, 1 H), 8.24 (s, 1 H), 8.01 (dd, *J* = 7.9, 1.9 Hz, 1 H), 7.53 (d, *J* = 5.1 Hz, 1 H), 7.34 (d, *J* = 8.1 Hz, 1 H), 3.56 (s, 3 H), 3.12 (m, 2 H), 1.87 (m, 1 H), 0.91 (d, *J* = 6.6 Hz, 6 H) |
| **153** | 3 | 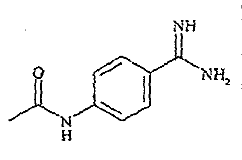 | -CH₃ | **152** | J | ¹H NMR (CD₃OD): δ 8.75 (d, J = 4.7 Hz, 2 H), 8.55 (s, 1 H), 8.42 (d, *J* = 1.9 Hz, 1 H), 8.07 (dd, J = 8.1, 1.9, 1 H), 7.74 (s, 3 H), 7.70 (d, *J* = 5.1 Hz, 1 H), 7.51 (d, *J* = 8.1 Hz, 1 H), 3.69 (s, 3 H), 3.21 (m, 2 H), 1.94 (m, 1 H), 0.98 (d, *J* = 6.6 Hz, 6 H); MS (ES⁺): 474 |
| **154** | 3 | 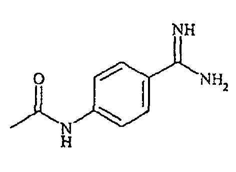 | -H | **153** | 1-2 | ¹H NMR (DMSO): δ 11.18 (s, 1 H), 9.31 (s, 2 H), 9.10 (s, 2 H), 8.92 (d, *J* = 5.1 Hz, 1 H), 8.78 (m, 2 H), 8.43 (d, *J* = 1.5 Hz, 1 H), 8.07 (dd, *J* = 7.9, 1.3 Hz, 1 H), 7.97 (d, *J* = 5.3 Hz, 1 H), 7.82 (d, *J* = 8.7 Hz, 2 H), 7.72 (d, *J* = 8.8 Hz, 2 H), 7.50 (d, *J* = 7.9 Hz, 1 H), 3.10 (t, *J* = 6.0 Hz, 2 H), 1.86 (m, 1 H), 0.89 (d, *J* =6.6 Hz, 6 H); MS (ES⁺) 460 |
| **156** | 4 | -CHO | -CH₃ | **155+ 3a** | D-9 | MS (ES⁺): 341.4 |
| **157** | 4 | -CO₂H | -CH₃ | **156** | E | ¹H NMR (CDCl₃): δ 8.80 (s, 1 H), 8.46 (d, *J* = 5.1 Hz, 1 H), 8.29 (s, 1 H), 7.85 (d, *J* = 7.9 Hz, 1 H), 7.13 (d, *J* = 7.9 Hz, 1 H), 7.00 (d, *J* = 5.1 Hz, 1 H), 6.83 (bs, 2 H), 3.45 (s, 3 H), 3.15 (m, 2 H), 1.84 (m, 1 H), 0.90 (d, *J* = 6.6 Hz, 6 H); MS (ES⁻ ): 355.2 |
| **158** | 4 | 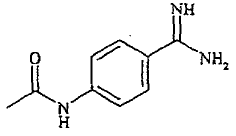 | -CH₃ | **157** | J | ¹H NMR (CD₃OD): δ 8.85 (s, 1 H), 8.75 (d, *J* = 5.3 Hz, 1 H), 8.41 (d, J = 1.9 Hz, 1 H), 8.07 (dd, J = 8.1, 2.1, 1 H), 7.74 (s, 4 H), 7.48 (d, *J* = 8.1 Hz, 1 H), 7.45 (d, *J* = 5.1 Hz, 1 H), 3.69 (s, 3 H), 3.21 (m, 2 H), 1.94 (m, 1 H), 0.97 (d, *J* = 6.8 Hz, 6 H); MS (ES⁻): 472.4 |
| **159** | 4 | 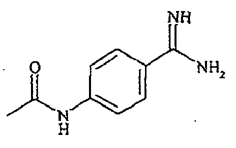 | -H | **158** | 1-2 | ¹H NMR (DMSO): δ 10.97 (s, 1 H), 9.24 (s, 2 H), 8.96 (s, 3 H), 8.79 (m, 2 H), 8.40 (d, *J* = 1.8 Hz, 1 H), 8.06 (d, *J* = 7.7 Hz, 1 H), 7.77 (s, 4 H), 7.52 (m, 1 H), 7.38 (d, *J* = 7.5 Hz, 1 H), 3.10 (m, 2 H), 1.85 (m, 1 H), 0.89 (d, *J* = 5.3, 6 H); MS (ES⁺) 460.2 |

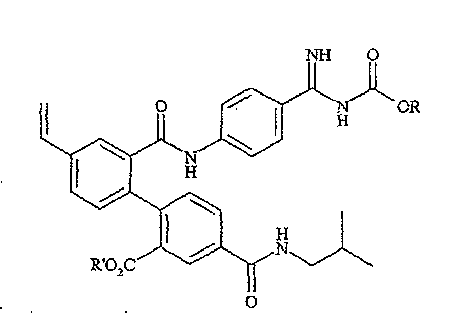

| **Cpd. No.** | **-R** | **-R'** | **Starting From** | **Method Used** | **Analytical Data** |
|---|---|---|---|---|---|
| **161a** | -CH₃ | -CH₃ | **31f** | AB-2 | ¹H NMR (DMSO-d6): δ 10.55 (s, 1H), 9.00 (bs, 2H), 8.68 (t, J = 5.8 Hz, 1H), 8.24 (d, J = 1.9 Hz, 1H), 8.04 (d, J = 8.1 Hz, 1H), 7.91 (d, J = 8.8 Hz, 2H), 7.77 (d, J = 1.3 Hz, 1H), 7.67 (m, 3H), 7.40 (d, J = 7.9 Hz, 1H), 7.29 (d, J = 7.9 Hz, 1H), 6.90 (dd, J = 17.7, 11.0 Hz, 1H), 6.03 (d, J = 17.7 Hz, 1H), 5.42 (d, J = 11.0 Hz, 1H), 3.61 (s, 3H), 3.56 (s, 3H), 3.10 (t, J = 6.4 Hz, 2H), 1.85 (m, 1H), 0.90 (d, J = 6.5 Hz, 6H); MS (ES+): 557.3 |
| **161b** | -C₂H₅ | -CH₃ | **31f** | AB-2 | ¹H NMR (DMSO-d6): δ 10.54 (s, 1H), 9.20 (bs, 4H), 8.67 (t, J=6 Hz, 1H), 8.24 (1H), 8.02 (1H), 7.91 (2H), 7.77 (1H), 7.66 (m, 3H), 7.40 (1H), 7.29 (1H), 6.88 (dd, J = 17.3, 10.7 Hz, 1H), 6.03 (d, J = 17.3 Hz, 1H), 5.42 (d, J = 10.7 Hz, 1H), 3.56 (s, 3H), 3.5 (m, 3H), 3.09 (2H), 1.85 (m, 1H), 0.89 (6H); MS (ES+): 571.3 |
| **161c** | -CH₂C₆H₅ | -CH₃ | **31f** | AB-2 | ¹H NMR (DMSO-d6): δ 10.54 (s, 1H), 9.20 (bs, 2H), 8.68 (t, J = 5.8 Hz, 1H), 8.24 (d, J = 1.9 Hz, 1H), 8.03 (d, J = 8.1 Hz, 1H), 7.92 (d, J = 8.8 Hz, 2H), 7.77 (s, 1H), 7.68 (m, 4H), 7.36(m, 6H), 6.89 (dd, J = 17.7, 11.2 Hz, 1H), 5.05 (s, 2H), 6.03 (d, J = 17.7 Hz, 1H), 5.42 (d, J = 11.2 Hz, 1H), 3.56 (s, 3H), 3.09 (t, J = 6.6 Hz, 2H), 1.84 (m, 1H), 0.89 (d, J = 6.6 Hz, 6H); MS (ES+): 633.3 |
| **161d** | -C(CH₃)₃ | -CH₃ | **31f** | AB-2 | MS (ES⁺): 599.3 and 499.3 |
| **161e** | -CH₂-CCl₃ | -CH₃ | **31f** | AB-2 | ¹H NMR (DMSO-d6): δ 10.59 (s, 1H), 9.24(s, 2H), 8.68 (t, J = 5.6 Hz, 1H), 8.24 (d, J = 1.8 Hz, 1H), 8.03 (dd, J = 8.9, 1.9 Hz, 1H), 7.96 (d, J = 8.9 Hz, 2H), 7.79 (d, J = 1.5 Hz, 1H), 7.69 (m, 3H), 7.41 (d, J = 8.1 Hz, 1H), 7.29 (d, J = 8.0 Hz, 1H), 6.89 (dd, J = 17.7, 11.1 Hz, 1H), 6.03 (d, J = 17.7 Hz, 1H), 5.42 (d, J = 11.1 Hz, 1H), 4.88 (s, 2H), 3.56 (s, 3H), 3.10 (t, J = 6.6 Hz, 2H), 1.85 (m, 1H), 0.89 (d, J = 6.6 Hz, 6H); MS (ES+): 674.97 |
| **161f** | 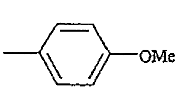 | -CH₃ | **31f** | AB-2 | ¹H NMR (DMSO-d6): δ 10.58 (s, 1H), 9.15 (s, 2H), 8.69 (t, J = 5.4 Hz, 1H), 8.25 (d, J = 1.8 Hz, 1H), 8.04 (dd, J = 8.1, 1.9 Hz, 1H), 7.95 (d, J = 8.9 Hz, 2H), 7.78 (s, 1H), 7.68 (m, 3H), 7.40 (d, J = 8.0 Hz, 1H), 7.29 (d, J = 8.0 Hz, 1H), 7.07 (d, J = 8.8 Hz, 2H), 6.93 (d, J = 8.8 Hz, 2H), 6.89 (dd, J = 17.7, 11.1 Hz, 1H), 6.03 (d, J = 17.7 Hz, 1H), 5.42 (d, J = 11.1 Hz, 1H), 3.75 (s, 3H), 3.57 (s, 3H), 3.10 (t, J = 6.6 Hz, 2H), 1.85 (m, 1H), 0.89 (d, J = 6.6 Hz, 6H); MS (ES+): 649.3 |
| **161g** | 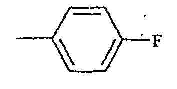 | -CH₃ | **31f** | AB-2 | ¹H NMR (DMSO-d6): δ 10.59 (s, 1H), 9.19 (s, 2H), 8.68 (t, J = 5.7 Hz, 1H), 8.25 (d, J = 1.8 Hz, 1H), 8.03 (dd, J = 8.1, 1.9 Hz, 1H), 7.95 (d, J = 8.9 Hz, 2H), 7.78 (d, J = 1.7 Hz, 1H), 7.70 (m, 3H), 7.41 (d, J = 8.1 Hz, 1H), 7.29 (d, J = 7.9 Hz, 1H), 7.20 (m, 4H), 6.90 (dd, J = 17.9, 11.1 Hz, 1H), 6.03 (d, J = 17.9 Hz, 1H), 5.42 (d, J = 11.1 Hz, 1H), 3.57 (s, 3H), 3.10 (t, J = 6.8Hz, 2H), 1.85 (m, 1H), 0.89 (d, J = 6.6 Hz, 6H); MS (ES+): 637.5 |
| **161h** | 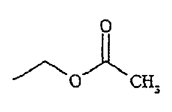 | -CH₃ | **31f** | AB-1 | ¹H NMR (DMSO-d6): δ 10.5 (s, 1H), 9.00 (bs, 2H), 8.68 (t, J = 5.9 Hz, 1H), 8.24 (d, J = 1.9 Hz, 1H), 8.03 (d, J = 8.1 Hz, 1H), 7.94 (d, J = 8.9 Hz, 2H), 7.78 (d, J = 1.5 Hz, 1H), 7.68 (m, 3H), 7.40 (d, J = 8.1 Hz, 1H), 7.29 (d, J = 8.1 Hz, 1H), 6.89 (dd, J = 17.5, 11.0 Hz, 1H), 6.03 (d, J = 17.5 Hz, 1H), 5.71 (s, 2H), 5.42 (d, J = 11.0 Hz, 1H), 3.56 (s, 3H), 3.10 (t, J = 6.2 Hz, 2H), 2.07 (s, 3H), 1.85 (m, 1H), 0.89 (d, J = 6.6 Hz, 6H); MS (ES+): 615.3 |
| **161i** | 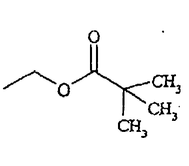 | -CH₃ | **31f** | AB-1 | ¹H NMR (DMSO-d6): δ 10.57 (s, 1H), 9.22 (s, 2H), 8.67 (t, J = 5.9 Hz, 1H), 8.24 (d, J = 1.9 Hz, 1H), 8.03 (dd, J = 8.1, 1.9 Hz, 1H), 7.94 (d, J = 8.9 Hz, 2H), 7.78 (d, J = 1.5 Hz, 1H), 7.69 (m, 3H), 7.41 (d, J = 7.9 Hz, 1H), 7.29 (d, J = 7.9 Hz, 1H), 6.89 (dd, J = 17.7, 11.1 Hz, 1H), 6.03 (d, J = 17.7 Hz, 1H), 5.73 (s, 2H), 5.42 (d, J = 11.1 Hz, 1H), 3.56 (s, 3H), 3.09 (t, J = 6.6 Hz, 2H), 1.85 (m, 1H), 1.14 (s, 9H), 0.89 (d, J = 6.7 Hz, 6H); MS (ES+): 657.52 |
| **161j** | 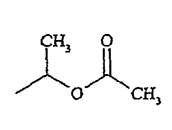 | -CH₃ | **31f** | AB-1 | ¹H NMR (DMSO-d6): δ 10.57 (s, 1H), 9.24 (s, 1 H), 9.17 (s, 1H), 8.68 (t, J = 6.2 Hz, 1H), 8.25 (s, 1H), 8.04 (d, J = 8.2Hz, 1H), 7.94 (d, J = 7.5 Hz, 2H), 7.67 (s, 1H), 7.67 (m, 3H), 7.40 (d, J = 7.9 Hz, 1H), 7.29 (d, J = 7.9 Hz, 1H), 6.90 (dd, J = 17.8, 11.1 Hz, 1H), 6.71 (q, J = 5.5 Hz, 1H), 6.03 (d, J = 17.7 Hz, 1H), 5.42 (d, J = 11.1 Hz, 1H), 3.56 (s, 3H), 3.10 (t, J = 6.6 Hz, 2H), 2.00 (s, 3H), 1.85 (m, 1H), 1.43 (d, J = 5.5 Hz, 3H), 0.89 (d, J = 6.7 Hz, 6H); MS (ES+): 629.4 |
| **162a** | -CH₃ | -H | **161a** | 1-2 | ¹H NMR (DMSO-d6): δ 9.04 (bs, 3H), 8.57 (t, J = 5.4 Hz, 1H), 8.16 (s, 1H), 7.86 (d, J = 8.5 Hz, 2H), 7.79 (d, J = 7.9 Hz, 1H), 7.72 (s, 1H), 7.58 (m, 3H), 7.12 (d, J = 8.0 Hz, 2H), 6.87 (dd, J = 17.7,11.0 Hz, 1H), 5.97 (d, J = 17.7 Hz, 1H), 5.37 (d, J = 11.0 Hz, 1H), 3.59 (s, 3H), 3.05 (t, J = 6.6 Hz, 2H), 1.83 (m, 1H), 0.87 (d, J = 6.6 Hz, 6H); MS (ES+): 543.38 |
| **162b** | -C₂H₅ | -H | **161b** | I-2 | ¹H NMR (DMSO-d6): δ 12.8 (bs, 1H), 10.8 (bs, 1H), 9.20 (bs, 2H), 8.68 (t, J = 5.9 Hz, 1H), 8.24 (d, J = 1.9 Hz, 1H), 7.91 (m, 3H), 7.77 (d, J = 1.5 Hz, 1H), 7.64 (m, 3H), 7.28 (d, J = 8.1 Hz, 1H), 7.22 (d, J = 8.1 Hz, 1H), 6.87 (dd, J = 17.7, 11.4 Hz, 1H), 6.01 (d, J = 17.7 Hz, 1H), 5.42 (d, J = 11.4 Hz, 1H), 4.05 (q, J = 7.2 Hz, 2H), 3.08 (t, J = 6.4 Hz, 2H), 1.84 (m, 1H), 1.21 (t, J = 7.2 Hz, 3H), 0.88 (d, J = 6.6 Hz, 6H); MS (ES⁻): 555.2 |
| **162c** | -CH₂C₆H₅ | -H | **161c** | I-2 | ¹H NMR (DMSO-d6): δ 12.7 (bs, 1H), 10.75 (bs, 1H), 9.15 (b, 2H), 8.63 (t, J = 5.8 Hz, 1H), 8.27 (bs, 1H), 7.90 (d, J = 8.3 Hz, 2H), 7.77 (s, 1H), 7.43-7.15 (m, 8H), 7.40 (d, J = 8.1 Hz, 1H), 7.29 (d, J = 8.1 Hz, 1H), 6.87 (dd, J = 17.4, 11.0 Hz, 1H), 6.03 (d, J = 17.5 Hz, 1H), 5.71 (s, 2H), 5.42 (d, J = 11.0 Hz, 1H), 5.09 (s, 2H), 3.08 (t, J = 6.4 Hz, 2H), 1.85 (m, 1H), 0.88 (d, J = 6.6 Hz, 6H); MS (ES+1): 619.2 |
| **162d** | -C(CH₃)₃ | -H | **161d** | 1-2 | ¹H NMR (DMSO-d6): δ 12.6 (bs, 1H), 11.0 (bs, 1H), 9.04 (b, 2H), 8.62 (t, J = 5.4 Hz, 1H), 8.24 (s, 1H), 7.86 (m, 3H), 7.77 (s, 1H), 7.62 (m, 3H), 7.24 (d, J = 8.2 Hz, 1H), 7.20 (d, J = 8.0 Hz, 1H), 6.87 (dd, J = 17.2, 11.0 Hz, 1H), 6.00 (d, J = 17.7 Hz, 1H), 5.40 (d, J = 11.0 Hz, 1H), 3.07 (t, J = 6.3 Hz, 2H), 1.84 (m, 1H), 1.44 (s, 9H), 0.88 (d, J = 6.6 Hz, 6H); MS (ES+1): 585.4 |

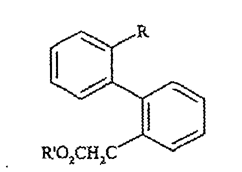

| **Cpd. No.** | **-R** | **-R'** | **Starting From** | **Method Used** | **Analytical Data** |
|---|---|---|---|---|---|
| **164** | -CHO | -CH₃ | **163 + 130** | D-2 | ¹HNMR (DMSO-d₆): δ 9.58 (s, 1 H), 7.91 (dd, J = 1.2, 8.0 Hz, 1 H), 7.71 (dt, J = 1.2 and 7.4 Hz, 1 H), 7.58 (t, J = 7.4 Hz, 1 H), 7.41 (m, 2 H), 7.38 (m, 1 H), 7.32 (d, J = 8 Hz, 1 H), 7.24 (d, J = 7.4 Hz, 1 H), 3.52 (q, J = 16 and 26 Hz, 2 H), 3.35 (s, 3 H); MS (ES+): 255.32 |
| **165** | -CO₂H | -CH₃ | **164** | E | Characterized in the next step |
| **166** | 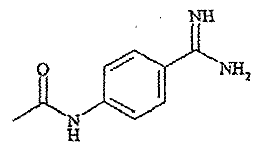 | -CH₃ | **165** | J | ¹HNMR (DMSO-d₆): δ 10.34 (s, 1 H), 9.18 (s, 2 H), 8.92 (s, 2 H), 7.72-7.5 (m, 7 H), 7.34-7.14 (m, 5 H), 3.60 (q, J = 17 & 40 Hz, 2 H), 3.48 (s, 3 H); MS (ES+) 388.67 |
| **167** | 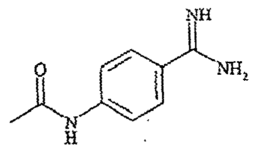 | -H | **166** | I-2 | ¹HNMR (DMSO-d₆): δ 11.74 (bs, 1 H), 9.90 (s, I H), 8.79 (bs, 2 H), 7.64 (m, 1 H), 7.50 (m, 7 H), 7.33 (d, J = 8.6 Hz, 1 H), 7.26 (d, J = 7.4 Hz, 1 H), 7.12 (t, J = 7.4 Hz, 1 H), 7.02 (t, J = 7.4 Hz, 1 H), 6.89 (d, J = 6.8 Hz, 1 H), 3.83 (d, J = 15 Hz, 2 H); MS (ES+) 374.79 |

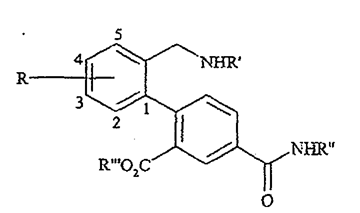

| **Cpd. No.** | **-R** | **-R'** | **-R"** | **-R"'** | **Starting From** | **Method Used** | **Analytical Data** |
|---|---|---|---|---|---|---|---|
| **188a** | -CH=CH₂ (4) | 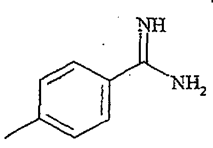 | 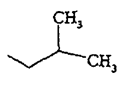 | -H | **187a** | AE-3 | MS (ES⁺): 485.4 (100% M⁺¹) |
| **188b** | -CH=CH₂ (4) | 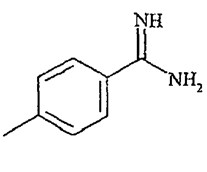 | 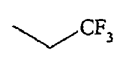 | -H | **187b** | AE-3 | ¹HNMR (DMSO-d₆/D₂O): δ 8.5 (d; J = 2 Hz, 1 H), 8.17 (dd, J = 8 Hz, 2 H), 7.65 (s, 1 H), 7.63 (s, 1 H), 7.54 (d, J = 8 Hz, 1 H), 7.49 (bs, 2 H), 7.14 (d, J = 7.7 Hz, 1 H), 6.78 (dd, J = 11 and 17 Hz, 1 H), 6.62 (d, J = 9 hz, 1 H), 5.83 (d, J = 17 hz, 1 H), 5.33 (d, J = 11 hz, 1 H), 4.17 (d, J = 9 hz, 1 H), 4.12 (s, 2 H); MS (ES+) : 497.3 |
| **188c** | -CH=CH₂ (4) | 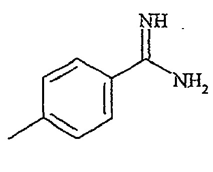 | 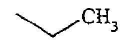 | -H | **187c** | AE-3 | ¹HNMR (DMSO-d₆/D₂O): δ 8.6 (m, 3 H), 8.3 (m, 3 H), 7.9 (d, J = 7.9 Hz, 1 H), 7.45 (d, J = 8.8 Hz, 1 H), 7.3 (m, 3 H), 7.1 (m, 1 H), 7.0 (d, J = 8.1 Hz, 1 H), 6.6 (dd, J = 6 and 28 Hz, 1 H), 6.4 (d, J = 8.8 Hz, 2 H), 5.7 (d, J = 17 Hz, 1 H), 5.15 (d, J = 11 Hz, 1 H), 3.9 (m, 2 H), 3.25 (m, 2 H), 1.1 (t, J = & Hz, 3 H); MS (ES+) : 443.3 |
| **188d** | -CH=CH₂ (4) | 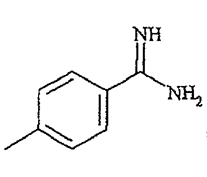 | 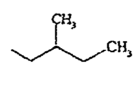 | H | **187d** | AE-3 | ¹HNMR (DMSO-d₆): δ 8.8 (m, 2 H), 8.7 (m, 1 H), 8.4 (m, 2 H), 8.1 (m, 1 H), 7.6 (m, 2 H), 7.5 (m, 3 H), 7.3 (m, 1 H), 7.2 (m, 1 H), 6.8 (m, 1 H), 6.6 (m, 2 H), 5.8 (m, 1 H), 5.3 (m, 1 H), 4.1 (m, 2 H), 3.31 (m, 1 H), 3.2 (m, 1 H), 1.7 (m, 1 H), 1.6 (m, 1 H), 1.3 (m, 1 H), 1.0 (m, 6 H); MS (ES+): 485 |
| **189a** | -OCH₃ (3) | 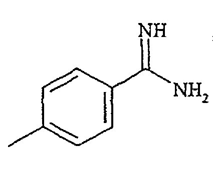 | 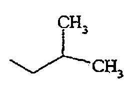 | -H | **74** | AE-4, 1-2 | ¹HNMR (DMSO-d₆): δ 8.60 (t, J = 6 Hz, 1 H), 8.39 (bs, 2 H), 8.28 (bs, 1 H), 7.78 (m, 1 H), 7.56 (m, 1 H), 7.43 (dd, J = 5.8 Hz, 3.8 Hz, 2 H), 7.18 (m, 2 H), 6.80 (m, 3 H), 6.51 (bs, 1 H), 4.10 (m, 1 H), 3.85 (m, 1 H), 3.70 (s, 3 H), 3.17 (t, J = 6 Hz, 2 H), 1.80 (m, 1 H), 0.89 (d, J = 6.8 Hz, 6 H); MS (ES⁺) 475.2 |
| **189b** | -OBn (4) | 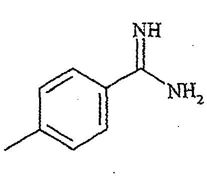 | 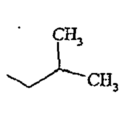 | -H | **184a** | AE-3 | ¹HNMR (DMSO-d₆/D₂O): δ 8.24 (d, J = 1.5 Hz, 1 H), 7.86 (d, J = 7 Hz, 1 H), 7.49 (m, 2 H), 7.36 (m, 4 H), 7.26 (d, J = 8.3 Hz, 1 H), 6.94 (m, 3 H), 6.66 (d, J = 8.7 Hz, 2 Hz, 2 H), 5.03 (s, 2 H), 4.06 (q, J = 16 and 21 Hz, 2 H), 3.02 (d, J = 7 Hz, 2 H), 1.86 (m, 1 H), 0.89 (d, J = 6.8 Hz, 6 H); MS (ES-): 549.2 and (ES⁺) 551.4 |
| **189c** | -OH (4) | 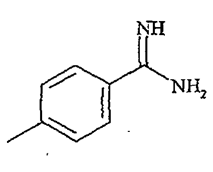 | 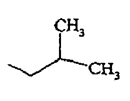 | -H | **189b** | G | ¹HNMR (DMSO-d₆): δ 11.3 (bs, 1 H), 9.07 (s, 1 H), 8.46 (t, J = 6 Hz, 1 H), 8.27 (bs, 2 H), 8.15 (bs, 2 H), 7.66 (d, J = 7.7 Hz, 1 H), 7.36 (d, J = 8.5 Hz, 2 H), 7.03 (d, J = 8.1 Hz, 1 H), 6.77 (m, 2 H), 6.68 (d, J = 8.3 Hz, 2 Hz, 2 H), 6.6 (s, 1 H), 6.47 9d, J = 8.2 Hz, 1 H), 4.05 (d, J = 14 Hz, 1 H), 3.09 (d, J = 14 Hz, 1 H), 3.01 (t, J = 7 Hz, 2 H), 1.79 (m, 1 H), 0.82 (d, J = 6.8 Hz, 6 H); MS (ES-): 459.2 and (ES⁺) 461.4 |
| **189d** | -H | 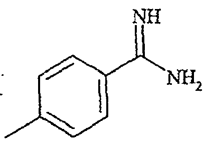 | 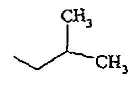 | -H | **131** | AE-3 | MS (ES⁺): 445.4; MS (ES⁻): 443.3 |
| **189e** | -H | 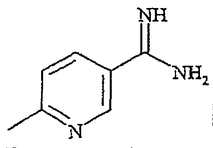 | 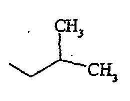 | -H | **131** | AE-3 | MS (ES⁺): 446.46; MS (ES⁻): 444.45 |

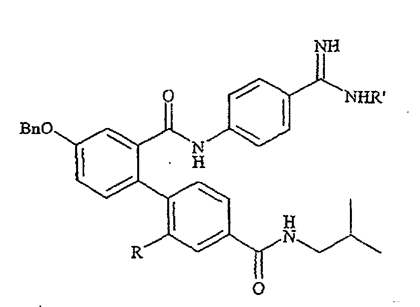

| **Cpd. No.** | **-R** | **-R'** | **Starting From** | **Method Used** | **Analytical Data** |
|---|---|---|---|---|---|
| **205** | 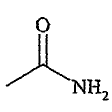 | -Boc | **204** | A-4 | ¹HNMR (DMSO-d₆): δ 11.04 (s, 0.6 H), 10.97 (bs, 0.4 H), 8.66 (t, J = 5.6 Hz, 0.6 H), 8.56 (t, J = 5.6 Hz, 0.4 H), 8.22 (s, 1 H), 8.11 (d, J = 2 Hz, 0.6 H), 8.03 (d, J = 2 Hz, 0.4 H), 7.94 (dd, J = 2 and 8 Hz, 1 H), 7.82 (m, 4 H), 7.40 (m, 8 H), 7.18 (m, 2 H), 7.04 (m, 2 H), 5.21 (s, 0.8 H), 5.11 (s, 1.2 H), 3.11 (t, J = 6.2 Hz, 1.2 H), 3.06 (t, J = 6.2 Hz, 0.8 H), 1.84 (m, 1 H), 1.43 (s, 5.4 H), 1.42 (s, 3.6 H), 0.91 (d, J = 6.8 Hz, 3.6 H), 0.88 (d, J = 6.8 Hz, 2.4 H); MS (ES+): 665.5 |
| **206** | -CH₂OH | -Boc | **204** | A-6 | ¹HNMR (DMSO-d₆): δ 12.15 (bs, 1 H), 11.07 (bs, I H), 10.69 (s, 1 H), 10.38 (bs, I H), 8.68 (t, J = 5.6 Hz, 1 H), 8.12 (d, J = 1.7 Hz, 1 H), 8.00 (dd, 1.8, Hz, 1 H), 7.68 (m, 4 H), 7.46-7.30 (m, 6 H), 7.16 (d, J = 2.8 Hz, 1 H), 7.01 (d, J = 8.5 Hz, 1 H), 6.86 (dd, J = 8.5 and 2.8 Hz, 1 H), 5.07 (s, 2 H), 4.30 (d, J =7.4 Hz, 2 H), 3.15 (t, J = 6.2 Hz, 2 H), 1.86 (m, 1 H), 1.53 (s, 9 H), 0.89 (d, J = 6.8 Hz, 6 H); MS (ES-): 649.4 |
| **207** | -CH₂OH | -H | **206** | S-2 | ¹HNMR (DMSO-d₆/D₂O): δ 10.66 (s, 1 H), 9.19 (bs, 2 H), 8.86 (bs, 2 H), 8.69 (t, J = 5.5 Hz, 1 H), 8.13 (d, J = 2 Hz, 1 H), 8.02 (dd, J = 8 and 2 Hz, 1 H), 7.72 (m, 4 H), 7.38 (m, 6 H), 7.17 (d, J = 2.6 Hz, 1 H), 7.03 (d, J = 8.5 H, 1 H), 6.87 (dd, J = 8.5 and 2.5 Hz, 1 H), 5.39 (t, J = 4.7 Hz, 1 H), 5.08 (s, 2 H), 4.30 (m, 2 H), 3.13 (t, J = 6.5 Hz, 2 H), 1.87 (m, 1 H), 0.91 (d, J = 6.5 Hz, 6 H); MS (ES⁺) 551.4 |
| **208** | 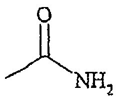 | -H | **205** | S-2 | ¹HNMR (DMSO-d₆): δ 11.26 (s, 0.6 H), 11.20 (bs, 0.4 H), 9.15 (bs, 1.2 H), 9.11 (bs, 0.8 H), 8.84 (bs, 1.2 H), 8.82 (bs, 0.8 H), 8.67 (t, J = 5.6 Hz, 0.6 H), 8.58 (t, J = 5.6 Hz, 0.4 H), 8.3 (s, 1 H), 8.12 (d, J = 2 Hz, 0.6 H), 8.04 (d, J = 2 Hz, 0.4 H), 7.96 (dd, J = 2 and 8 Hz, 1 H), 7.84 (m, 1 H), 7.70 (m, 2 H), 7.57 (m, 3 H), 7.40 (m, 4 H), 7.22 (m, 2 H), 7.02 (m, 2 H), 5.21 (s, 0.8 H), 5.11 (s, 1.2 H), 3.12 (t, J = 6.5 Hz, 1.2 H), 3.06 (t, J = 6.5 Hz, 0.8 H), 1.84 (m, 1 H), 0.90 (d, J = 6.5 Hz, 3.6 H), 0.86 (d, J = 6.5 Hz, 2.4 H); MS (ES+): 564.5 |

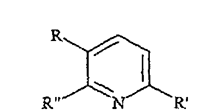

| **Cpd. No.** | **-R** | **-R'** | **-R"** | **Starting From** | **Method Used** | **Analytical Data** |
|---|---|---|---|---|---|---|
| **217** | -OCH₃ | 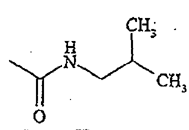 | -Br | **216** | A-3 | ¹H NMR (DMSO-*d₆*): δ 8.48 (t, *J* = 6.2 Hz, 1 H), 8.06 (d, *J* = 8.3 Hz, 1 H), 7.69 (d, *J* = 8.5 Hz, 1 H), 4.01 (s, 3 H), 3.15 (t, *J* = 6.5 Hz, 2 H), 1.91 (m, 1 H), 0.91 (d, *J* = 6.6 Hz, 6 H); MS (ES⁺): 287.1 |
| **218** | -OCH₃ | 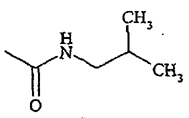 | -CH=CH₂ | **217** | D-12 | ¹H NMR (CDCl₃): δ 8.08 (m, 2 H), 7.20 (m, 2 H), 6.39 (dd, *J* = 2.0 and 17.3 Hz, 1 H), 5.53 (dd, *J* = 2.0 and 10.9 Hz, 1 H), 4.01 (s, 3 H); 3.15 (t, *J* = 6.5 Hz, 2 H), 1.91 (m, 1 H), 0.91 (d, *J* = 6.6 Hz, 6 H) |
| **219** | -OH | 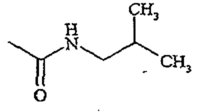 | -CO₂CH₃ | **218** | E-2, V-3, W-2 | ¹H NMR (DMSO-*d₆*): δ 11.05 (s, 1 H), 8.48 (t, *J* = 6.2 Hz, 1 H), 8.06 (d, *J* = 8.7 Hz, 1 H), 7.53 (d, *J* = 8.5 Hz, 1 H), 3.90 (s, 3 H), 3.12 (t, *J* = 6.6 Hz, 2 H), 1.85 (m, 1 H), 0.86 (d, *J* = 6.6 Hz, 6 H); MS |
| **220** | -OSO₂CF₃ | 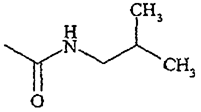 | -CO₂CH₃ | **219** | B-2 | (ES⁺): 253.2 MS (ES⁺): 407.2 (M+Na)⁺ |
| **237** | 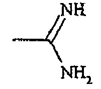 | -NH₂ | -H | **236** | AF-1 | MS (ES⁺): 137.1 |

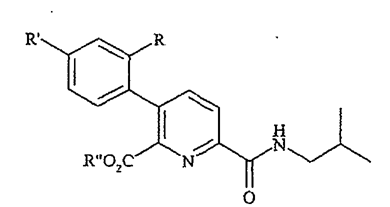

| **Cpd. No.** | **-R** | **-R'** | **-R"** | **Starting From** | **Method Used** | **Analytical Data** |
|---|---|---|---|---|---|---|
| **221** | -CHO | -OBn | -CH₃ | **220 + 6** | D-2 | ¹H NMR (CDCl₃): δ 9.77 (s, I H), 8.40 (d, *J* = 7.9 Hz, 1 H), 8.13 (d, *J* = 6.8 Hz, I H), 7.83 (d, *J* = 7.9 Hz, 1 H), 7.61 (d, *J* = 2.60 Hz, 1 H), 7.20 (m, 5 H), 7.21 (m, 1 H), 7.18 (d, *J* = 8.3 Hz, 1 H), 5.18 (s, 2 H), 3.72 (s, 3 H), 3.35 (q, *J* = 5.8 Hz, 2 H), 1.96 (m, 1 H), 1.01 (d, *J* = 6.8 Hz, 6 H); MS (ES⁺): 447.4 |
| **222** | -CO₂H | -OBn | -CH₃ | **221** | E | MS (ES⁻): 461.3 |
| **223** | -CO₂MEM | -OBn | -CH₃ | **222** | F | MS (ES⁺): 573.33 (M+Na)⁺ |
| **224** | -CO₂MEM | -OH | -CH₃ | **223** | G | MS (ES⁺): 461.36 |
| **225** | -CO₂MEM | -OSO₂CF₃ | -CH₃ | **224** | B-2 | MS (ES⁺): 615.58 (M+Na)⁺ |
| **226** | -CO₂MEM | -CH=CH₂ | -CH₃ | **225** | D-3 or D-12 | MS (ES⁻): 381.35 [(M-MEM)-1] |
| **227** | -CO₂H | -CH=CH₂ | -CH₃ | **226** | I-1 | MS (ES⁻): 381.35 |
| **228** | 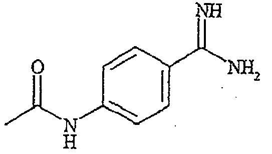 | -CH=CH₂ | -CH₃ | **227** | J | MS (ES⁺): 500.35 |
| **229** | 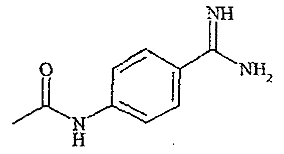 | -CH=CH₂ | -H | **228** | I-2 | MS (ES⁺): 486.32 |
| **245** | -CHO | -OH | -CH₃ | **221** | AD | MS (ES⁺): 357.40 |
| **246** | -CHO | -OSO₂CF₃ | -CH₃ | **245** | B-2 | Characterized in the next step |
| **247** | -CHO | -CH=CH₂ | -CH₃ | **246** | D-3 | MS (ES⁺): 367.42 |
| **248** | 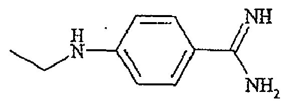 | -CH=CH₂ | -H | **247** | AE-3 | MS (ES⁺): 472.39 |
| **249** | 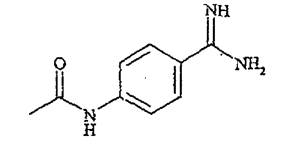 | -OBn | -CH₃ | **222** | J | MS (ES⁺): 580.4 |
| **250** | 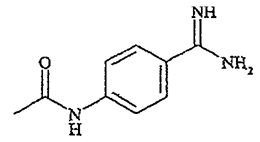 | -OBn | -H | **249** | I-2 | MS (ES⁺): 566.4 MS (ES⁻): 564.3 |
| **251** | 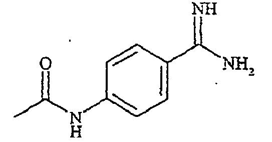 | -OH | -H | **250** | G | MS (ES⁺): 476.3 MS (ES⁻): 474.2 |
| **252** | 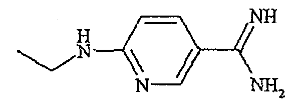 | -CH=CH₂ | -H | **247** | AE-3 | MS (ES⁺): 473.44 MS (ES⁻): 471.43 |

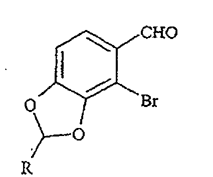

| **Cpd. No.** | **-R** | **Starting From** | **Method Used** | **Analytical Data** |
|---|---|---|---|---|
| **231b** | -CO₂CH₃ | **230** | AG-3 | ¹H NMR (CDCl₃): δ 10.17 (d, J = 0.75 Hz, 1 H), 7.62 (d, J = 8.3 Hz, 1 H), 6.94 (dd, J = 8.3, 0.75 Hz, 1 H), 6.51 (s, 1 H), 3.90 (s, 3 H) |

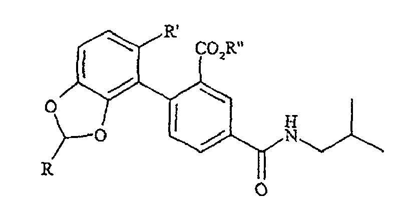

| **Cpd. No.** | **-R** | **-R'** | | **Starting From** | **Method Used** | **Analytical Data** |
|---|---|---|---|---|---|---|
| **232a** | -H | -CHO | **-R"** -CH₃ | **231a + 6a** | D-6 or D-7 | ¹HNMR (CDCl₃): δ 9.64 (s, 1 H), 8.44 (d, J = 2 Hz, 1 H), 8.02 (dd, J = 8 and 2 Hz, 1 H), 7.60 (d, J = 8.3 Hz, 1 H), 7.40 (d, J = 8 Hz, 1 H), 6.96 (d, J = 8 Hz, 1 H), 6.32 (t, J = 6 and 5 Hz, 1 H), 6.01 (s, 2 H), 3.72 (s, 3 H), 3.33 (t, J = 6.5 Hz, 2 H), 1.93 (m, 1 H), 1.00 (d, J = 6.8 Hz, 6 H); MS (ES⁺): 384.3 and 406.3 (M+Na)⁺ |
| **232b** | -CO₂H | -CHO | -CH₃ | **231b + 6a** | D-6 or D-7 | ¹HNMR (DMSO-d₆): δ 9.87 (s, 1 H), 9.49 (s, 1 H), 8.64 (d, J = 2 Hz, 1 H), 8.3 (s, I H), 7.97 (d, J = 8 Hz, 1 H), 7.43 (dd, J = 8 and 2.6 Hz, 1 H), 7.35 (m, 2 H), 6.94 (m, 1 H), 6.05 (s, 0.4 H), 5.98 (s, 0.6 H), 3.55 (s, 1.8 H), 3.52 (s, 1.2 H), 3.02 (t, J = 6.5 Hz, 2 H), 1.78 (m, 1 H), 0.81 (d, J = 6.6 Hz, 6 H); MS (ES⁻): 426.2 |
| **233a** | -H | -CO₂H | -CH₃ | **232a** | E | ¹HNMR (DMSO-d₆): δ 12.29 (bs, 1 H), 8.69 (t, J = 5.5 Hz, 1 H), 8.38 (d, J = 2 Hz, 1 H), 8.03 (dd, J = 8 and 2 Hz, 1 H), 7.58 (d, J = 8.5 Hz, 1 H), 7.36 (d, J = 8 Hz, 1 H), 7.00 (d, J = 8.5 Hz, 1 H), 6.02 (s, 2 H), 3.64 (s, 3 H), 3.12 (t, J = 6.5 Hz, 2 H), 1.87 (m, 1 H), 0.91 (d, J = 6.8 Hz, 6 H); MS (ES⁻): 398.2 |
| **233b** | -CO₂H | -CO₂H | -CH₃ | **232b** | E | ¹HNMR (DMSO-d₆): δ 8.64 (t, J = 5.5 Hz, I H), 8.38 (d, J = 4 Hz, 1 H), 8.00 (dd, J = 8.5 and 4 Hz, 1 H), 7.59 (dd, J = 8.5 and 4 Hz, 1 H), 7.30 (dd, J = 8 and 2.5 Hz, 1 H), 6.52 (s, 0.5 H), 6.48 (s, 0.5 H), 3.60 (s, 1.5 H), 3.58 (s, 1.5 H), 3.08 (t, J = 6.5 Hz, 2 H), 1.84 (m, 1 H), 0.88 (d, J = 6.8 Hz, 6 H) |
| **234a** | -H | 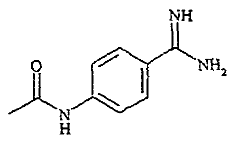 | -CH₃ | **233a** | J | MS (ES⁺): 517.4 |
| **234b** | -CO₂H | 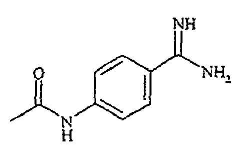 | -CH₃ | **233b** | J | ¹HNMR (DMSO-d₆): δ 12.41 (bs, 1 H), 11.09 (s, 1 H), 10.96 (s, 1 H), 9.22 (bs. 2 H), 8.96 (bs, 2 H), 8.70 (m, 1 H), 8.38 (dd, J = 2 and 13 Hz, 1 H), 8.04 (d, J = 8 Hz, 1 H), 7.82 (m, 4 H), 7.65 (dd, J = 8 and 5 Hz, 1 H), 7.39 (dd, J = 8 and 2.5 Hz, 1 H), 7.11 (dd, J = 8.5 and 1.7 Hz, 1 H), 6.05 (s, 1 H), 3.67 (s, 1.5 H), 3.50 (s, 1.5 H), 3.10 (t, J = 6.5 Hz, 2 H), 1.88 (m, 1 H), 0.90 (d, J = 6.8 Hz, 6 H) |
| **235a** | -H | 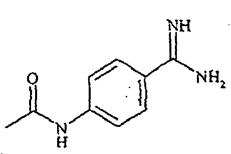 | -H | **234a** | I-2 | ¹HNMR (DMSO-d₆+DCl one drop): δ 8.34 (d, J = 2 Hz, 1 H), 7.97 (dd, J = 8 and 2 Hz, 1 H), 7.75 (m, 4 H), 7.33 (dd, J = 3.8 and 8.1 Hz, 2 H), 7.04 (d, J = 8.1 Hz, 1 H), 6.01 (d, J = 6 Hz, 2 H), 3.07 (t, 3 = 6.5 Hz, 2 H), 1.83 (m, 1 H), 0.86 (d, J = 6.8 Hz, 6 H); MS (ES-) 501.3; (ES+) 503.3 |

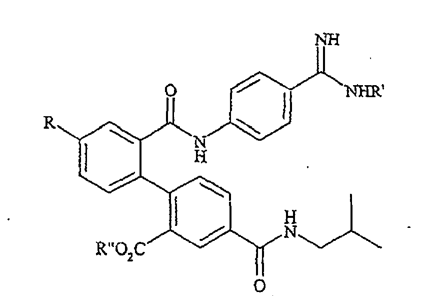

| **Cpd. No.** | **-R** | **-R'** | **-R"** | **Starting From** | **Method Used** | **Analytical Data** |
|---|---|---|---|---|---|---|
| **240** | -CH(OH)-CH₂OH | -Boc | -CH₃ | **161d** | L | ¹H NMR (DMSO-d6): δ 10.47 (s, 1H), 9.07 (s, 2H), 8.72 (t, J = 5.7 Hz, 1H), 8.29 (d, J = 2 Hz, 1 H), 8.08 (dd, J = 8.0, 2 Hz, 1H), 7.95 (s, 1H), 7.92 (s, 1 H), 7.67 (m, 2 H), 7.62 (d, J = 6.5 Hz, 1 H), 7.46 (d, J = 8 Hz, 1H), 7.31 (d, J = 8 Hz, 1H), 5.50 (d, J = 4.5 Hz, 1H), 4.91 (t, J = 5.7 Hz, 1H), 4.74 (m, 1 H), 4.25 (s, 1 H), 3.63 (s, 3H), 3.15 (t, J = 6.4 Hz, 2H), 1.91 (m, 1H), 1.50 (s, 9 H), 0.95 (d, J = 6.7 Hz, 6H) |
| **241** | -CHO | -Boc | -CH₃ | **240** | M | ¹H NMR (DMSO-d6): δ 10.69 (s, 1H), 10.17 (s, 1 H), 9.10 (bs, 2 H), 8.72 (t, J = 5.7 Hz, 1H), 8.30 (d, J = 1.5 Hz, 1H), 8.22 (d, J = 1.5 Hz, 1H), 8.22 (dd, J = 1.5 and 8 Hz, 1 H), 8.07 (dd, J = 1.5 and 8 Hz, 1 H), 7.89 (s, 1H), 7.86 (s, 1 H), 7.65 (s, 1 H), 7.62 (s, 1 H), 7.57 (d, J = 8 Hz, 1H), 7.44 (d, J = 8 Hz, 1H), 3.57 (s, 3H), 3.11 (t, J = 6.4 Hz, 2H), 1.85 (m, 1H), 1.44 (s, 9 H), 0.89 (d, J = 6.7 Hz, 6H) |
| **242** | -CH(OH)-CH=CH₂ | -Boc | -CH₃ | **241** | AG | MS (ES⁺): 629.39 |
| **243** | -CH(OH)-CH=CH₂ | -H | -CH₃ | **242** | S | MS (ES⁺): 529.38 |
| **244** | -CH(OH)-CH=CH₂ | -H | -H | **243** | I-2 | MS (ES⁻): 515.35 |

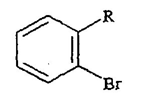

| **Cpd. No.** | **-R** | **Starting From** | **Method Used** | **Analytical Data** |
|---|---|---|---|---|
| **254** | 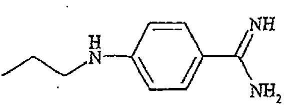 | **253** | AE-3 | MS (ES⁺): 318.2, 320.2 |
| **255** | 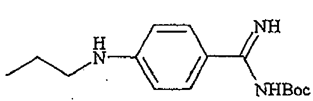 | **254** | R | MS (ES⁺): 418 |

The following non-limiting examples are presented to further illustrate the present invention.
2'-[({4-[Amino(imino)methyl]phenyl}amino)carbonyl]-4-[(isobutylamino)carbonyl]-4'-thien-2-yl-1,1'-biphenyl-2-carboxylic acid
2'-[({4-[Amino(imino)methyl]phenyl}amino)carbonyl]-4-[(isobutylamino)carbonyl]-4'-thien-3-yl-1,1'-biphenyl-2-carboxylic acid
2'-[({4-[Amino(imino)methyl]phenyl}amino)carbonyl]-4-[(isobutylamino)carbonyl]-1,1':4',1"-terphenyl-2-carboxylic acid
2'-[({4-[Amino(imino)methyl]phenyl}amino)carbonyl]-4'-(3-furyl)-4-[(isobutylamino)carbonyl]-1,1'-biphenyl-2-carboxylic acid
2'-[({4-[Amio(imino)methyl]phenyl}amino)carbonyl]-4-[(isobutylamino)carbonyl]-4'-pyridin-4-yl-1,1'-biphenyl-2-carboxylic acid
2'-[({4-[Amino(imino)methyl]phenyl}amino)carbonyl]-4-[(isobutylamino)carbonyl]-4'-(1H-pyrrol-2-yl)-1,1'-biphenyl-2-carboxylic acid
2'-[({4-[Amino(imino)methyl]phenyl}amino)carbonyl]-4'-[2-(hydroxymethyl)thien-3-yl]-4-[(isobutylamino)carbonyl]-1,1'-biphenyl-2-carboxylic acid
2'-[({4-[Amino(imino)methyl]phenyl}amino)carbonyl]-4'-[3-(hydroxymethyl)thien-2-yl]-4-[(isobutylamino)carbonyl]-1,1'-biphenyl-2-carboxylic acid
2'-[({4-[Amino(imino)methyl]phenyl}amino)carbonyl]-4-[(isobutylamino)carbonyl]-4'-vinyl-1,1'-biphenyl-2-carboxylic acid
4'-Allyl-2'-[({4-[amino(imino)methyl]phenyl}amino)carbonyl]-4-[(isobutylamino)carbonyl]-1,1'-biphenyl-2-carboxylate
2'-[({4-[Amino(imino)methyl]phenyl}amino)carbonyl]-4-[(isobutylamino)carbonyl]-4'-(1,3-thiazol-2-yl)-1,1'-biphenyl-2-carboxylic acid
2'-[({4-[Amino(imino)methyl]phenyl}amino)carbonyl]-4'-[3-(hydroxymethyl)-2-furyl]-4-[(isobutylamino)carbonyl]-1,1'-biphenyl-2-carboxylic acid
2'-[({4-[Amino(imino)methyl]phenyl}amino)carbonyl]-4-[(isobutylamino)carbonyl]-4'-prop-1-ynyl-1,1'-biphenyl-2-carboxylic acid
2'-[({4-[Amino(imino)methyl]phenyl}amino)carbonyl]-4'-(3-hydroxy-3-methylbut-1-ynyl)-4-[(isobutylamino)carbonyl]-1,1'-biphenyl-2-carboxylic acid
2'-[({4-[Amino(imino)methyl]phenyl}amino)carbonyl]-4-[(3-methylbutanoyl)amino]-4'-vinyl-1,1'-biphenyl-2-carboxylic acid
2'-[({4-[Amino(imino)methyl]phenyl}amino)carbonyl]-4'-(4-hydroxybut-1-ynyl)-4-[(isobutylamino)carbonyl]-1,1'-biphenyl-2-carboxylic acid
2'-[({4-[Amino(imino)methyl]phenyl}amino)carbonyl]-4-[(isobutylamino)carbonyl]-4'-[(1E)-3-methylbuta-1,3-dienyl]-1,1'-biphenyl-2-carboxylic acid
2'-[({4-[Amino(imino)methyl]phenyl}amino)carbonyl]-4'-(3-hydroxyprop-1-ynyl)-4-[(isobutylamino)carbonyl]-1,1'-biphenyl-2-carhoxylic acid
2'-[({4-[Amino(imino)methyl]phenyl}amino)carbonyl]-4'-(2-furyl)-4-[(propylamino)carbonyl]-1,1'-biphenyl-2-carboxylic acid
2'-[({4-[Amino(imino)methyl]phenyl}amino)carbonyl]-4-[(sec-butylamino)carbonyl]-4'-(2-furyl)-1,1'-biphenyl-2-carboxylic acid
2'-[({4-[Amino(imino)methyl]phenyl}amino)carbonyl]-4'-(2-furyl)-4-{[(2,2,2-trifluoroethyl)amino]carbonyl}-1,1'-biphenyl-2-carboxylic acid
2'-[({4-[Amino(imino)methyl]phenyl}amino)carbonyl]-4'-(2-furyl)-4-{[(4-hydroxybutyl)amino]carbonyl}-1,1'-biphenyl-2-carboxylic acid
2'-[({4-[Amino(imino)methyl]phenyl}amino)carbonyl]-4-[(ethylamino)carbonyl]-4'-(2-furyl)-1,1'-biphenyl-2-carboxylic acid
2'-[({4-[Amino(imino)methyl]phenyl}amino)carbonyl]-4-[(isobutylamino)carbonyl]-5'-methoxy-4'-vinyl-1,1'-biphenyl-2-carboxylic acid
2'-[({4-[Amino(imino)methyl]phenyl}amino)carbonyl]-4-[(isobutylamino)carbonyl]-4'-(thien-2-ylmethyl)-1,1'-biphenyl-2-carboxylic acid
2-{3-[({4-[Amino(imino)methyl]phenyl}amino)carbonyl]pyridin-4-yl}-5-[(isobutylamino)carbonyl]benzoic acid
2'-[({4-[Amino(imino)methyl]phenyl}amino)carbonyl]-4-[(cyclopentylamino)carbonyl]-4'-vinyl-1,1'-biphenyl-2-carboxylic acid
2'-[({4-[Amino(imino)methyl]phenyl}amino)carbonyl]-5'-ethoxy-4-[(isobutylamino)carbonyl]-4'-vinyl-1,1'-biphenyl-2-carboxylic acid
Methyl 2'-[({4-[({[(acetyloxy)methoxy]carbonyl}amino)(imino)methyl]phenyl} amino)carbonyl]-4-[(isobutylamino)carbonyl]-4'-vinyl-1,1'-biphenyl-2-carboxylate
Methyl 2'-[({4-[{[(benzyloxy)carbonyl]amino}(imino)methyl]phenyl}amino) carbonyl]-4-[(isobutylamino)carbonyl]-4'-vinyl-1,1'-biphenyl-2-carboxylate
N'-{4-[Amino(imino)methyl]phenyl}-N8-isobutyl-6-oxo-6H-benzo[c]chromene-1,8-dicarboxamide
2'-[({4-[Amino(imino)methyl]phenyl}amino)methyl]-4-[(isobutylamino)carbonyl]-4'-vinyl-1,1'-biphenyl-2-carboxylic acid
2'-({[4-(4,5-Dihydro-1H-imidazol-2-yl)phenyl]amino}carbonyl)-4-[(isobutylamino)carbonyl]-1,1'-biphenyl-2-carboxylic acid
2'-[({4-[Amino(imino)methyl]phenyl}amino)carbonyl]-4-[(isobutylamino)carbonyl]-5'-thien-2-yl-1,1'-biphenyl-2-carboxylic acid
2'-[({4-[Amino(imino)methyl]phenyl}amino)carbonyl]-5'-(2-amino-2-oxoethoxy)-4-[(isobutylamino)carbonyl]-1,1'-biphenyl-2-carboxylic acid
2'-[({4-[Amino(imino)methyl]phenyl}amino)carbonyl]-4'-ethoxy-4-[(isobutylamino)carbonyl]-1,1'-biphenyl-2-carboxylic acid
2-{5-[({4-[Amino(imino)methyl]phenyl}amino)carbonyl]-1,3-benzodioxol-4-yl}-5-[(isobutylamino)carbonyl]benzoic acid
2'-[1-({4-[Amino(imino)methyl]phenyl}amino)ethyl]-4-[(isobutylamino)carbonyl]-1,1'-biphenyl-2-carboxylic acid
3-[2-({4-[Amino(imino)methyl]phenyl}amino)carbonyl]-4-(benzyloxy)phenyl]-6-[(isobutylamino)carbonyl]pyridine-2-carboxylic acid
3-[2-(4-Carbamimidoyl-phenylcarbamoyl)-4-vinyl-phenyl]-6-isobutylcarbamoyl-pyridine-2-carboxylic acid
2'-[(5-Carbamimidoyl-pyridin-2-ylamino)-methyl]-4-isobutylcarbamoyl-4'-vinyl-biphonyl-2-carboxylic acid
2'-{[4-(N-Hydroxycarbamimidoyl)-phenylamino]-methyl}-4-isobutylcarbamoyl-4'-vinyl-biphenyl-2-carboxylic acid
2'-{[4-(N-Hydroxycarbamimidoyl)-phenylamino]-methyl}-4-isobutylcarbamoyl-4'-vinyl-biphenyl-2-carboxylic acid methyl ester
3-{2-[(4-Carbamimidoyl-phenylamino)-methyl]-4-vinyl-phenyl}-6-isobutylcarbamoyl-pyridine-2-carboxylic acid

### Biological Assay Methods

### In Vitro Assay for Inhibition of TF/FVIIa

To assess the inhibition of the test compounds against the target enzyme, TF/FVIIa, an amidolytic assay based upon the absorbance of p-Nitroanalide (pNA) at OD₄₀₅ was utilized. The IC₅₀ of the test compounds was determined by using KC4A data reduction software (Bio-Tek Instruments) to interpolate percent inhibition from observed Vmax values.

TR/FVIIa assay reactions were performed in a 200 µL mixture containing 4 nM FVIIa, 10 nM lipidated tissue factor, in an assay buffer containing 100 mM Tris, pH 7.2, 150 mM NaCl, 5 mM calcium chloride, 0.1 % bovine serum albumin (BSA), and 10% dimethyl sulfoxide (DMSO). TF and FVIIa were allowed to equilibrate at room temperature for 15 minutes. Test compounds dissolved in DMSO were incubated at varied concentrations with TF/FVIIa for 10 minutes, followed by addition of 500 □M substrate Spectrozyme-FVIIa. Reactions were incubated for 5 minutes at room temperature prior to measuring the change in OD₄₀₅ nm for 10 minutes at 21 second intervals with a Powerwave x (Bio-Tek Instruments) microplate reader.

### In Vitro Assay for Human Thrombin

This colorimetric assay was used to assess the ability of the test compounds to inhibit the human thrombin, enzyme. IC₅₀ of the test compounds was determined by using KC4A data reduction software (Bio-Tek Instruments) to interpolate percent inhibition from observed Vmax values.

Thrombin assay reactions were performed in a 200 µL mixture containing human thrombin at (1 U/mL) in an assay buffer containing 100 mM HEPES, 10 mM calcium chloride, and 10 % DMSO, pH 7.5. Test compounds dissolved in DMSO were added to thrombin enzyme reactions at varied concentrations, followed by the addition of substrate Nα-Benzoyl-Phe-Val-Arg p-Nitroanilide at a final concentration of 1 mM. Reactions were incubated for 5 minutes at room temperature prior to measuring the change in OD₄₀₅ nm for 10 minutes at 21 second intervals with a Powerwave x (Bio-Tek Instruments) microplate reader.

### In Vitro Assay for Human Trypsin

This enzymatic assay was employed to evaluate the ability of the test compounds to inhibit human pancreatic trypsin. IC₅₀ of the test compounds was determined by using KC4A data reduction software (Bio-Tek Instruments) to interpolate percent inhibition from observed Vmax values.

Trypsin assay reactions were performed in a 200 µL mixture containing human pancreatic trypsin at 1 µg/mL in an assay buffer containing 200 mM triethanolamine (TEA), 10 mM calcium chloride, 10 % DMSO, pH 7.8. Test compounds dissolved in DMSO were added to trypsin enzyme reactions at varied concentrations, followed by the addition of substrate Nα-Benzoyl-L-Arginine p-Nitroanilide (L-BAPNA) at a final concentration of (0.25 mg/mL). Reactions were incubated for 5 minutes at room temperature prior to measuring the change in OD₄₀₅ nm for 10 minutes at 21 second intervals with a Powerwave x (Bio-Tek Instruments) microplate reader.

### Biological Data

### IC₅₀ Values of Some Selected Compounds on Different Serine Protease Enzymes

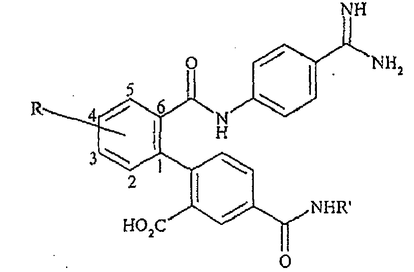

| **R (With Respect to Phenyl Ring** | **R'** | **TF/FVIIa** | **Trypsin** | **Thrombin** |
|---|---|---|---|---|
| 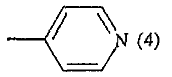 | 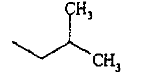 | ++ | + | + |
| 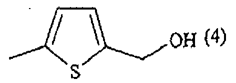 | 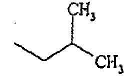 | ++ | + | + |
| 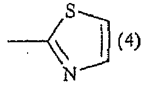 | 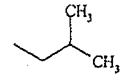 | ++ | + | + |
| 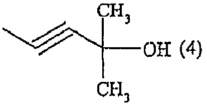 | 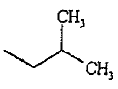 | ++ | - | - |
| 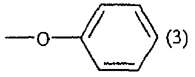 | 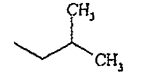 | + | - | - |
| 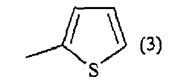 | 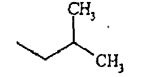 | ++ | - | - |
| 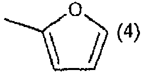 | 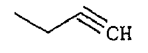 | +++ | + | |
| 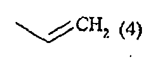 | 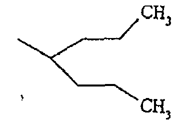 | +++ | ++ | + |
| 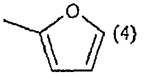 | 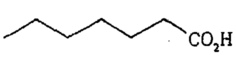 | +++ | ++ | + |
| 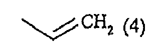 | 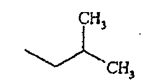 | +++ | ++ | + |

| | | | | |
|---|---|---|---|---|
| IC₅₀ values: + mean> µM; ++ means >100 nM; +++ means <100 nM | | | | |

A comparison of Examples with R group and without R group illustrates the greatly-enhanced activity achieved pursuant to the present invention.

Compounds of the present invention are useful as inhibitors of trypsin-like serine protease enzymes such as thrombin, factor VIIa, TF/FVIIa, and trypsin.

These compounds may be employed to inhibit the coagulation cascade and prevent or limit coagulation.

These compounds may be used to inhibit the formation of emboli or thromboli in blood vessels.

These compounds may be used to treat thrombolymphangitis, thrombosinusitis, thromboendocarditis, thromboangitis, and thromboarteritis.

These compounds may be used to inhibit thrombus formation following angioplasty. These may be used in combination with other antithrombolytic agents such as tissue plasminogen activators and their derivatives, streptokinase and its derivatives, or urokinase and its derivatives to prevent arterial occlusion following thrombolytic therapy.

These compounds may also be used in matastatic diseases, or for any disease where inhibition of coagulation is indicated.

These compounds may be used as diagnostic reagents in vitro for inhibiting clotting of blood in the tubes.

These compounds may be used alone or in combination with other compounds such as heparin, aspirin, or warfarin and any other anticoagulant agents.

These compounds may be used as anti-inflammatory agents.

According to a further aspect of the invention, compounds may be employed in preventing ex vivo coagulation such as that encountered in the extracorporeal perfusion of blood through for example artificial valves, prothesis, stents or catheters. According to this aspect of the invention the extracorporeal device may be coated with the compositions of the invention resulting in a lower risk of clot formation due to extrinsic pathway activation.

### Dosage and Formulation

The compounds of this invention can be administered by any means that produces contact of the active agent's site of action with factor VIIa and other serine proteases in the body of a human, mammal, bird, or other animal. They can be administered by any conventional means, such as oral, topical, transdermal, parenteral, subcutaneous, intraperitoneal, intrapulmonary, and intranasal, available for use in conjunction with pharmaceuticals, either as individual therapeutic agents or in a combination of therapeutic agents. Parenteral infusion includes intramuscular, intravenous, and intraarterial. They can be administered alone, but generally administered with a pharmaceutical carrier elected on the basis of the chosen route of administration and standard pharmaceutical practice.

The dosage administered will, or course, vary depending upon known factors, such as the pharmacodynamic characteristics of the particular agent and its mode and route of administration; the age, health and weight of the recipient; the nature and extent of the symptoms, the kind of concurrent treatment; the frequency of treatment; and the effect desired. A daily dosage of active ingredient can be expected to be about 0.0001 to 1000 milligram (mg) per kilogram (kg) of body weight, with the preferred dose being 0.1 to about 30 mg/kg.

Dosage forms (compositions suitable for administration) contain from about mg to about 500 mg of compound per unit. In these pharmaceutical compositions, the compound of the present invention will ordinarily be present in an amount of about 0.5-95% by weight based on the total weight of the composition.

The daily dose of the compounds of the invention that is to be administered can be a single daily dose or can be divided into several, for example, two, three or four, part administrations. The pharmaceutical compositions or medicaments of the invention can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

Gelatin capsules contain a compound of the present invention and powdered carriers, such as lactose, starch, cellulose derivatives, biocompatible polymers, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated to mask by unpleasant taste and protect the tablet from the atmosphere, or enteric coated for selective disintegration in the gastrointestinal tract.

Liquid dosage forms for oral administration can contain coloring and flavoring to increase patient acceptance. They may also contain buffering agents, surfactants and preservatives. Liquid oral products can be developed to have sustained-release properties. They may also contain cyclodextrin derivatives to enhance the solubility of the active ingredient and to promote its oral uptake.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water-soluble salt of the active ingredient, suitable stabilizing agents, and, if necessary, buffering agents. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propylparaben, and chlorobutanol.

Suitable pharmaceutical carriers are described in Remington's Pharmaceutical Sciences, Mack Publishing Company and in the Handbook of Pharmaceuticals Excipients, American Pharmaceutical Association, both standard reference texts in this field.

Useful pharmaceutical dosage forms for administration of the compounds according to the present invention can be illustrated as follows:

### Hard Shell Capsules

A large number of unit capsules are prepared by filling standard two-piece hard gelatin capsules each with 100 mg of powdered 1500 mg of lactose, 50 mg of cellulose, and 6 mg of magnesium stearate.

### Soft Gelatin Capsules

A mixture of active ingredient in a digestible oil such as soybean oil, cottonseed oil, or olive oil is prepared and injected by means of a positive displacement pump into molten gelatin to form soft gelatin capsules containing 100 mg of the active ingredient. The capsules are washed and dried. The prodrug can be dissolved in a mixture of polyethylene glycol, glycerin and sorbitol to prepare a water miscible medicine mix.

### Tablets

A large number of tablets are prepared by conventional procedures so that the dosage unit was 100 mg of active ingredient, 0.2 mg of colloidal silicon dioxide, 5 mg of magnesium stearate, 275 mg of microcystalline cellulose, 11 mg of starch, and 9.98 mg of lactose. Appropriate aqueous and non-aqueous coatings may be applied to increase palatability improve elegance and stability or delay absorption.

### Immediate Release Tablets/Capsules

These are solid oral dosage forms made by conventional and novel processes. These units are taken orally without water for immediate dissolution and delivery of the medication. The drug is mixed containing ingredient such as sugar, gelatin, pectin, and sweeteners. These liquids are solidified into solid tablets or caplets by freeze drying and solid thermoelastic sugars and polymers or effervescent components to produce porous matrices intended for immediate release, without the need of water.

Moreover, the compounds of the present invention can be administered in the form of nose drops, metered dose nasal or buccal inhalers. The drug is delivered from a nasal solution as a fine mist or from a powder as an aerosol.

A compound of the invention can be used in an assay to identify the presence of factor VIIa and other serine protease or to isolate factor VIIa and other serine protease in a substantially purified form. For example, the compound of the invention can be labeled with, for example, a radioisotope, and the labeled compound is detected using a routine method useful for detecting the particular label. In addition, a compound the invention can be used advantageously as a probe to detect the location or amount of factor VIIa and other serine protease activity in vivo, in vitro or ex vivo.

Various modifications of the invention in addition to those shown and described herein will be apparent to those skilled in the art from the foregoing description. Such modifications are also intended to fall within the scope of the appended claims.

## Claims

1. The compound represented by the structure 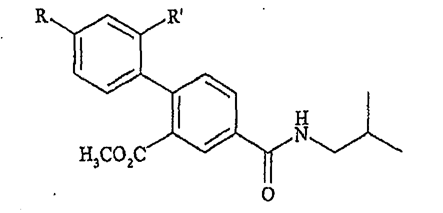 wherein R is selected from the group consisting of
-OBn, -OH, -OSO₂CF₃, 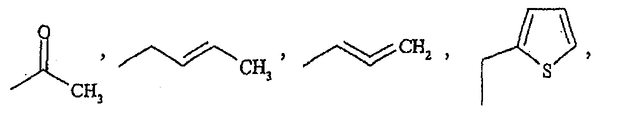 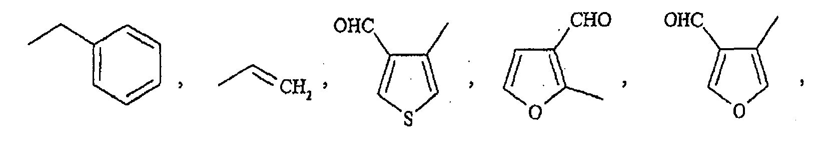 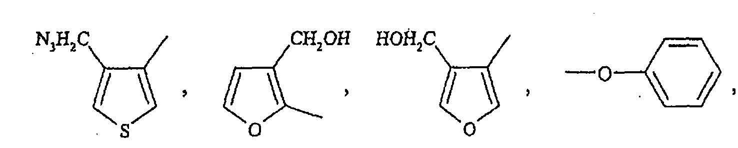 and
-OCH₃;
and R' is selected from the group consisting of -CHO, -CO₂H, and -CO₂MEM; and pharmaceutically acceptable salts thereof;
wherein MEM designates a methoxyethoxymethyl group.

2. The compound represented by the structure 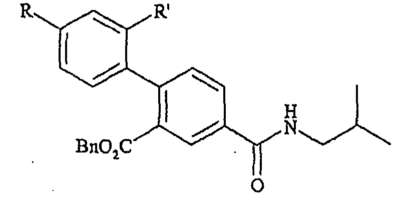 wherein R is selected from the group consisting of
-OBn, -OH, -OSO₂CF₃, 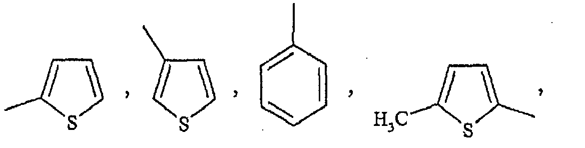 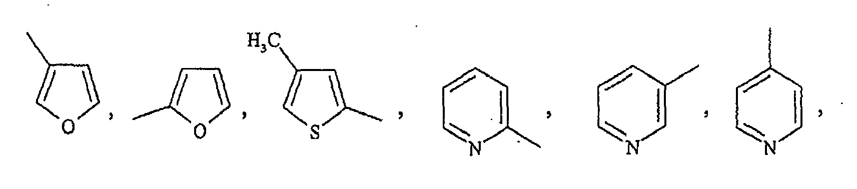 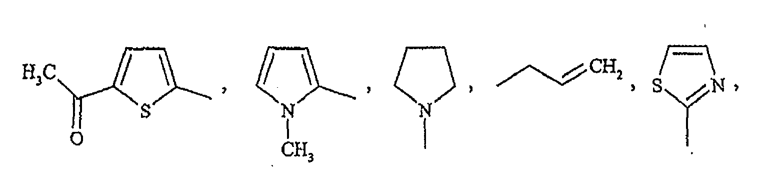 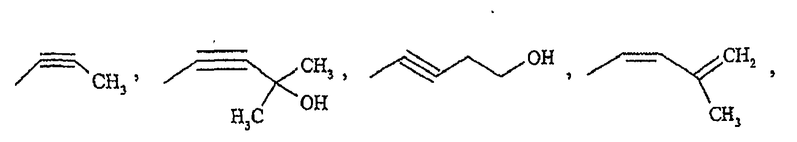 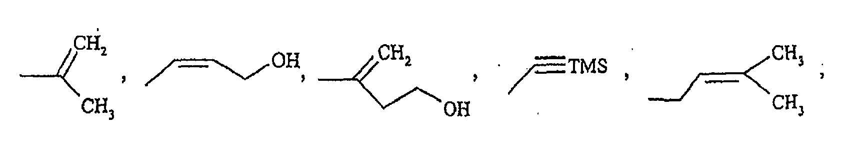 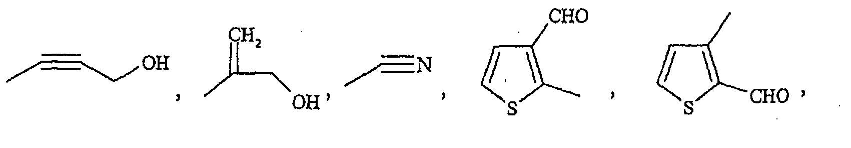 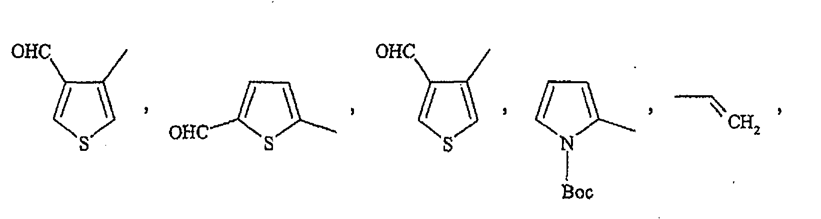 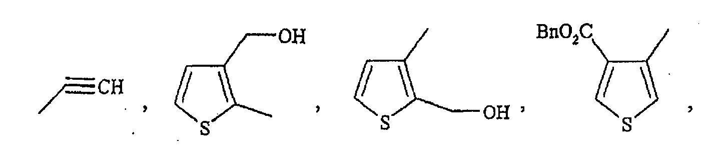 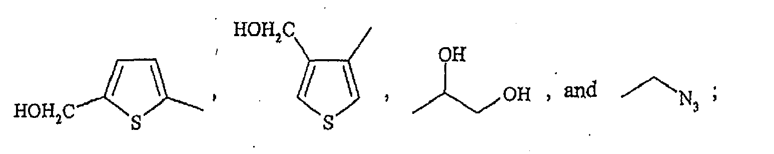 and R' is selected from the group consisting of -CHO, -CO₂H, and -CO₂MEM; and pharmaceutically acceptable salts thereof;
wherein MEM designates a methoxyethoxymethyl group.

3. The compound represented by the structure 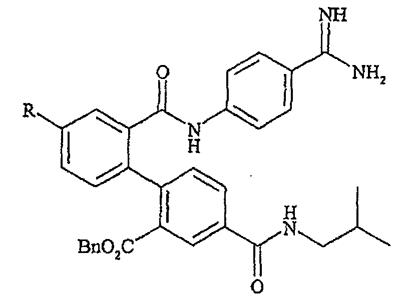 wherein R is selected from the group consisting of 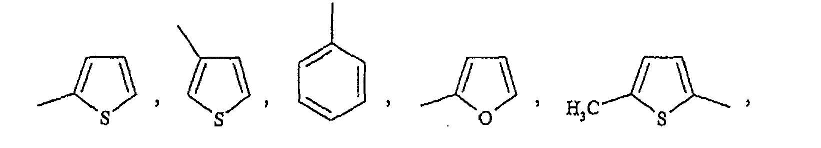 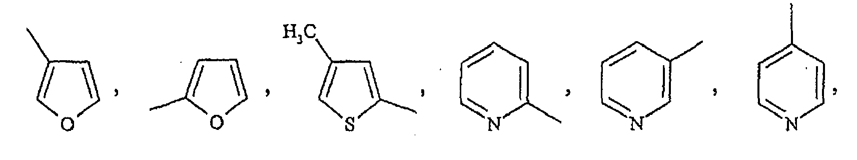 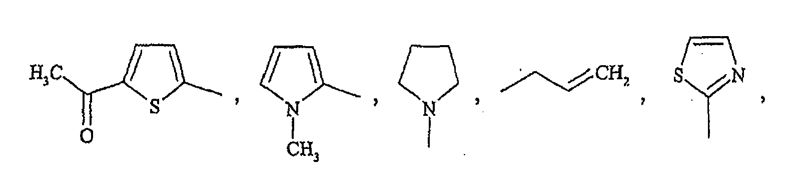 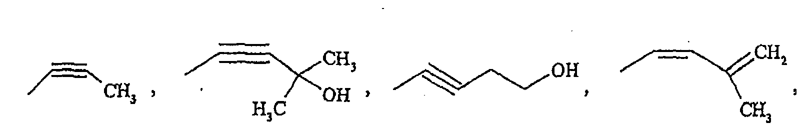 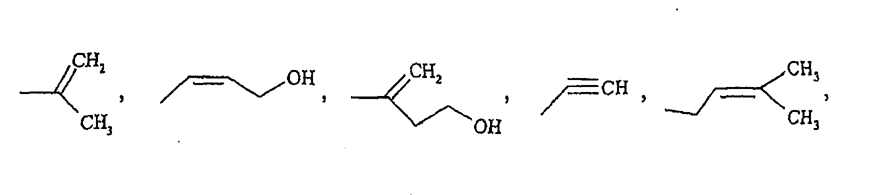 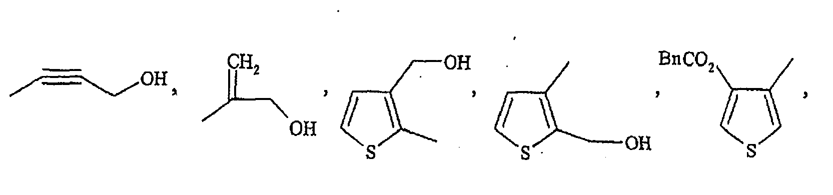 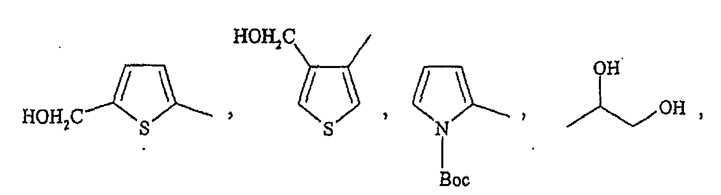 and 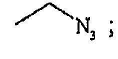 and pharmaceutically acceptable salts thereof.

4. The compound represented by the structure 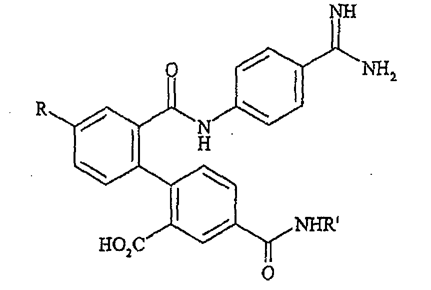 wherein R is selected from the group consisting of 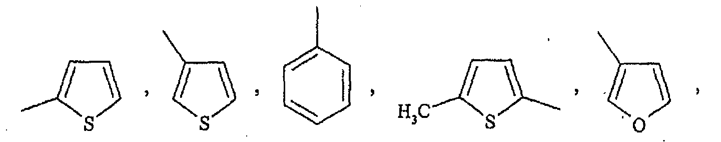 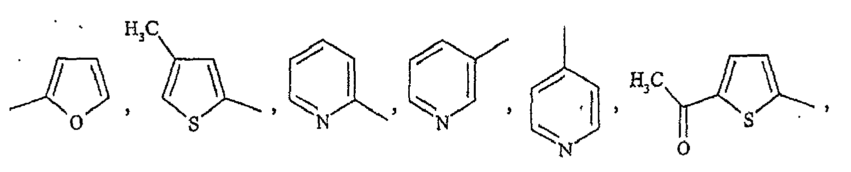 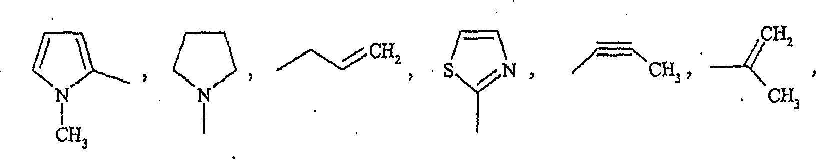 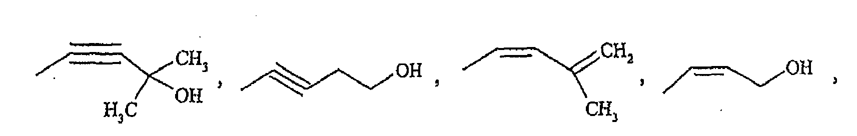 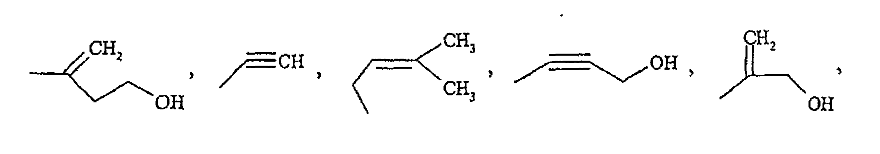 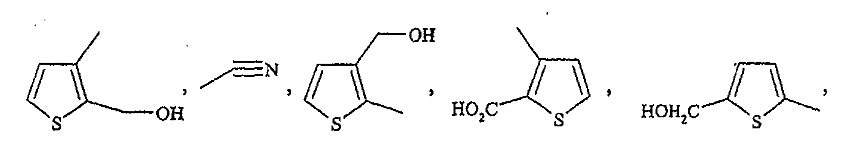 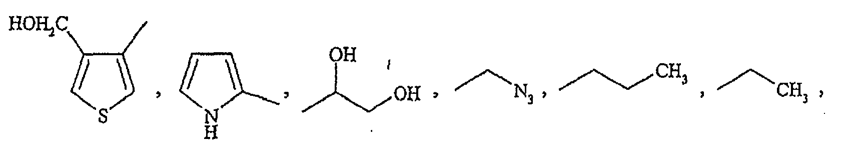 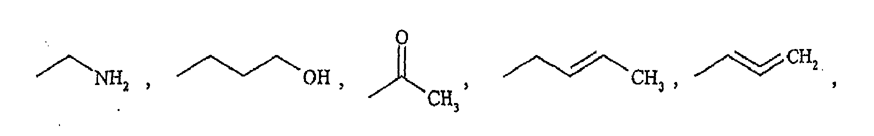 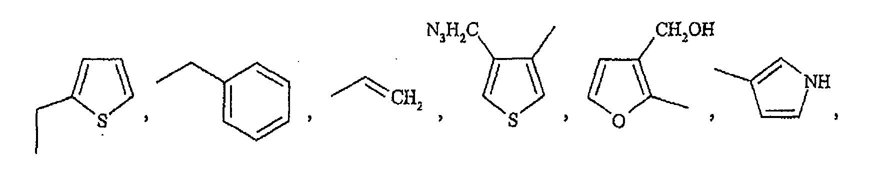 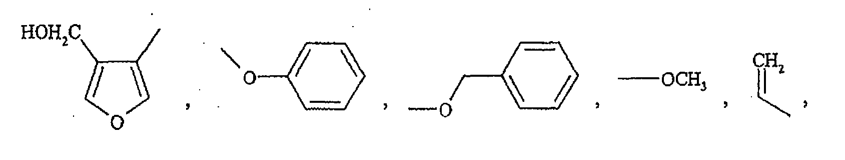 and 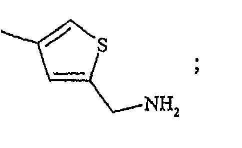 and R' is selected from the group consisting of 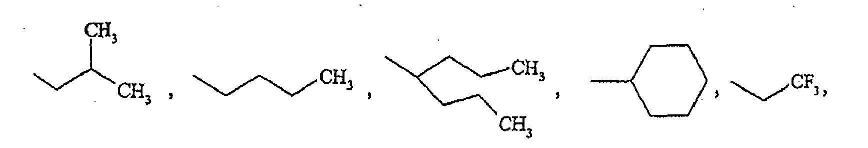 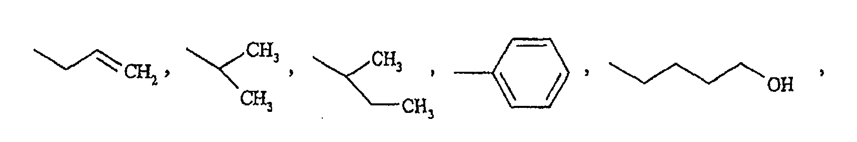 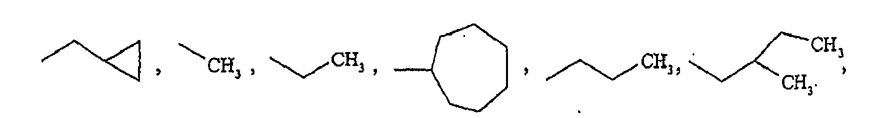 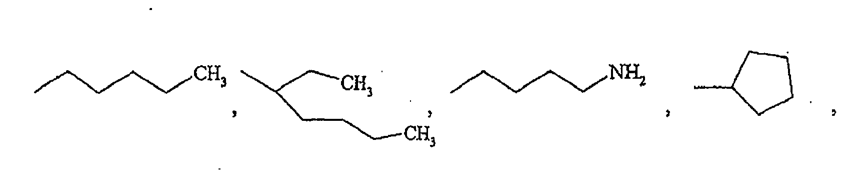 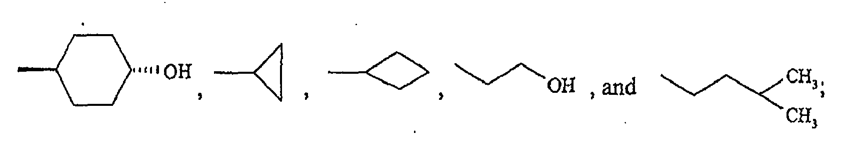 and pharmaceutically acceptable salts thereof.

5. The compound represented by the structure 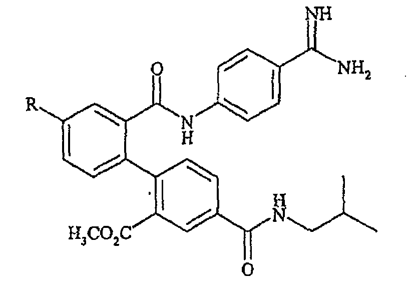 wherein R is selected from the group consisting of 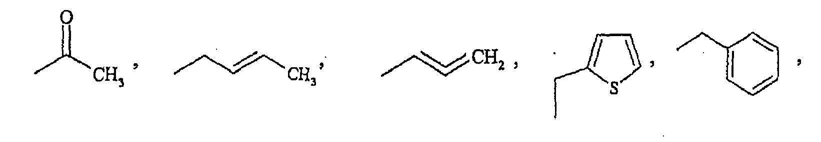 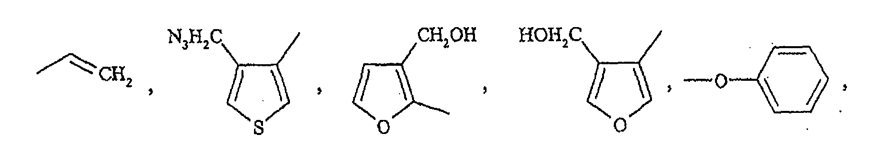 -OBn, -OCH₃, and 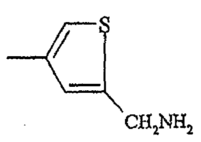 ; and pharmaceutically acceptable salts thereof.

6. The compound represented by the structure 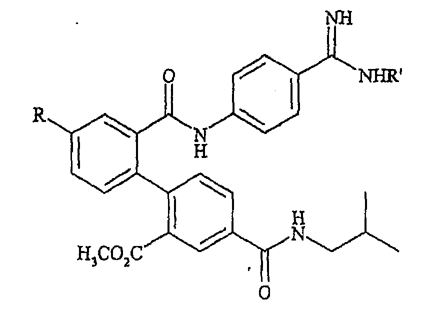 wherein R is selected from the group consisting of
-OSO₂CF₃, 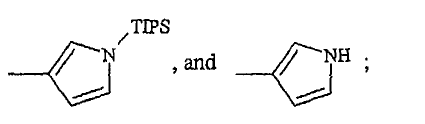 and R' is -H or 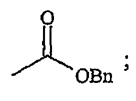 and pharmaceutically acceptable salts thereof.

7. The compound represented by the structure 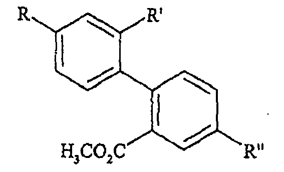 wherein R is selected from the group consisting of
-OBn, -OH, -OSO₂CF₃, 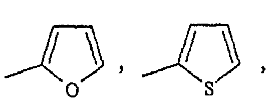 -CH=CH₂, and -H;
R' is selected from the group consisting of -CHO, -CO₂H, and -CO₂MEM;
and R" is selected from the group consisting of
-CO₂MEM, 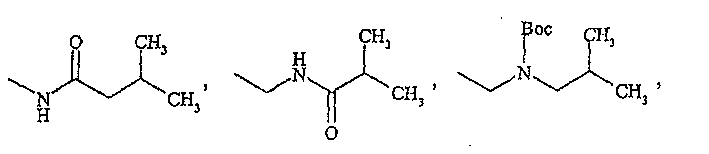 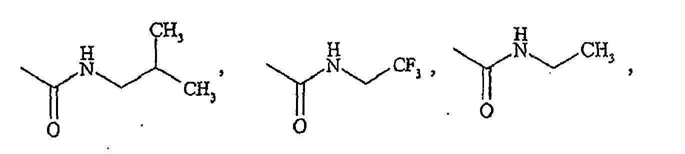 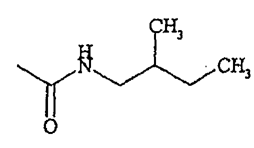 and CO₂H ;
and pharmaceutically acceptable salts thereof.

8. The compound represented by the structure 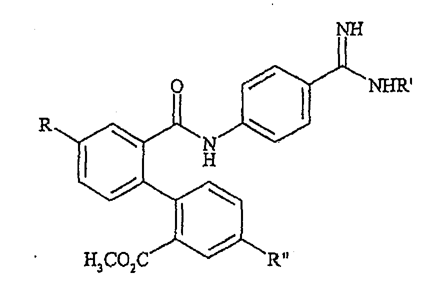 wherein R is selected from the group consisting of
-OBn, -OH, -OSO₂CF₃, 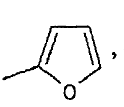 -CH=CH₂;
R' is -H or -Boc; and R" is -CO₂MEM or -CO₂H; and pharmaceutically acceptable salts thereof;
wherein MEM designates a methoxyethoxymethyl group.

9. The compound represented by the structure 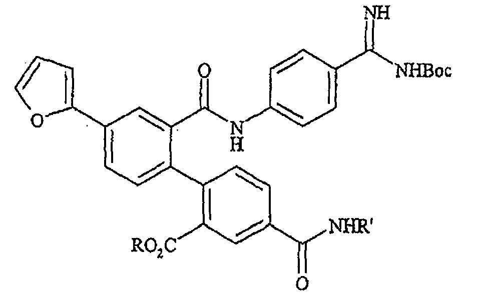 wherein R is -CH₃ and R' is selected from the group consisting of 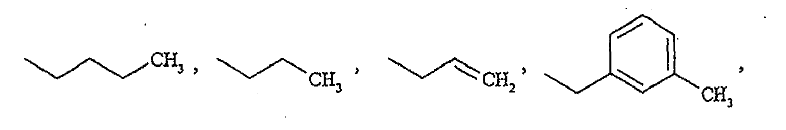 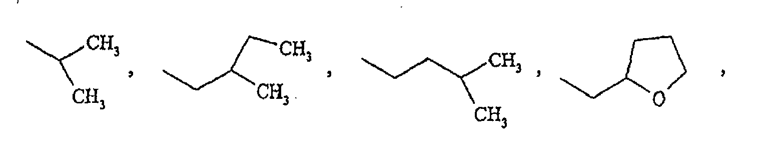 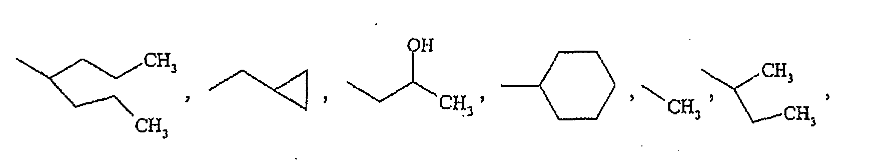 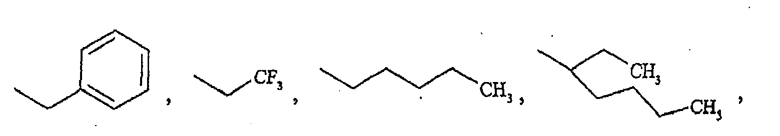 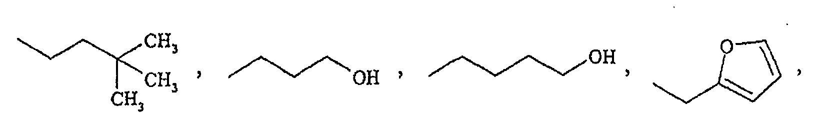 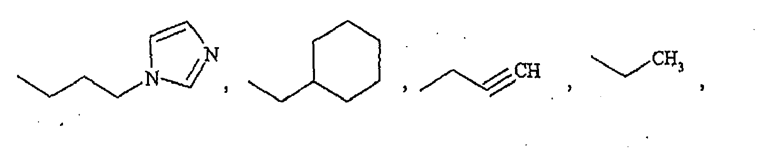 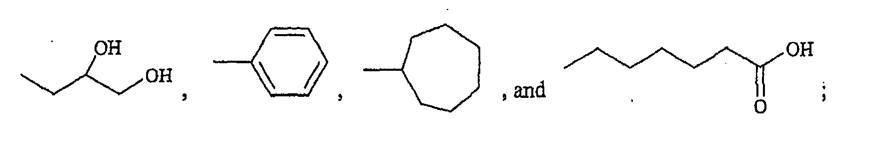 or 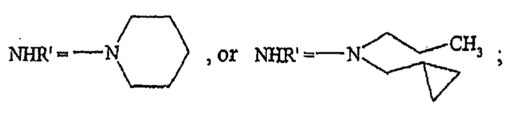 and pharmaceutically acceptable salts thereof.

10. The compound represented by the structure 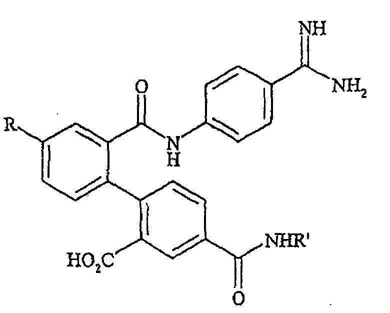 wherein R is 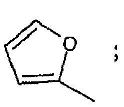 and R' is selected from the group consisting of 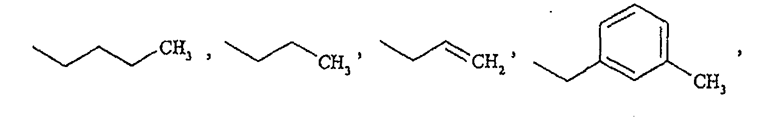 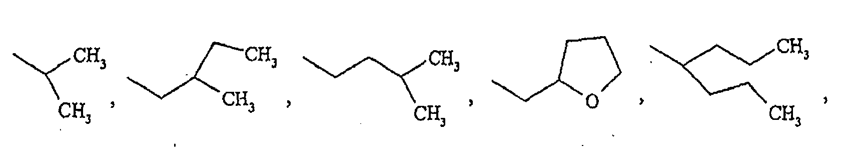 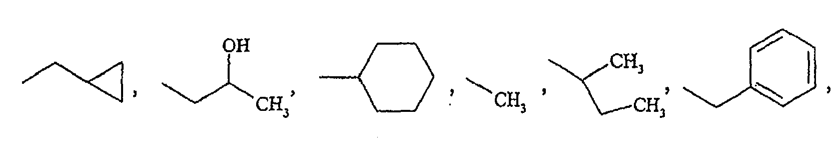 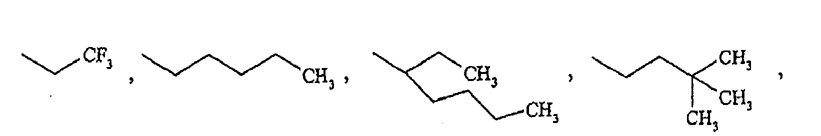 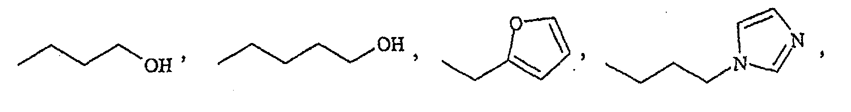 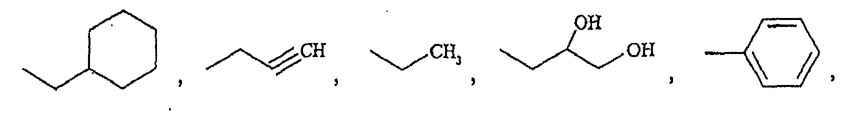 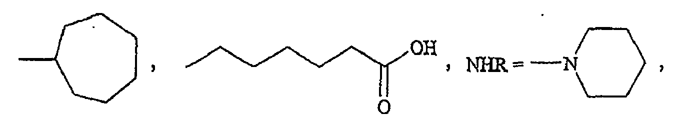 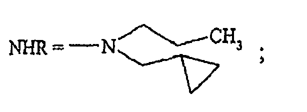 and pharmaceutically acceptable salts thereof.

11. The compound of claim 1 represented by the structure 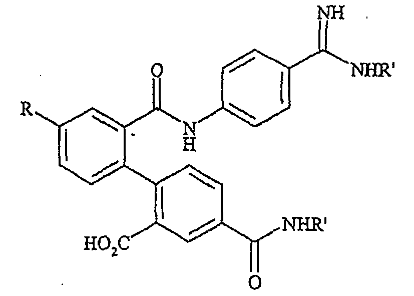 wherein R is 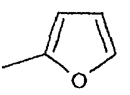 and -CH=CH₂ and R' is selected from the group consisting of 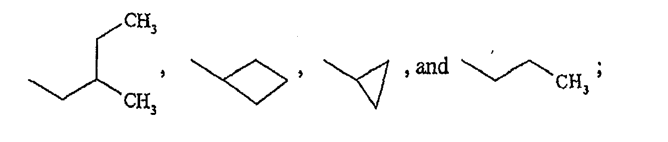 and pharmaceutically acceptable salts thereof.

12. The compound represented by the structure 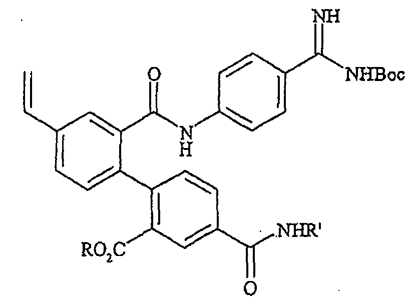 wherein R is -CH₃ and R' is selected from the group consisting of 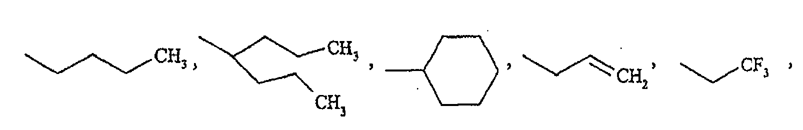 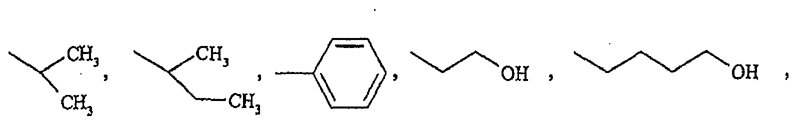 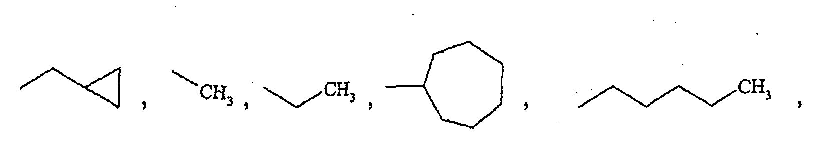 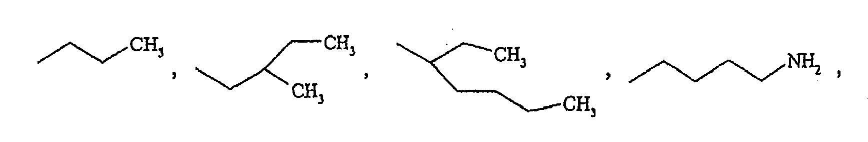 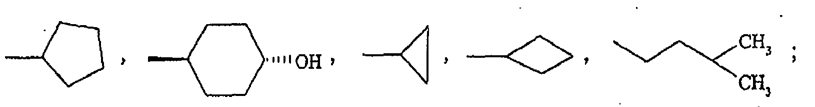 and pharmaceutically acceptable salts thereof.

13. The compound represented by the structure 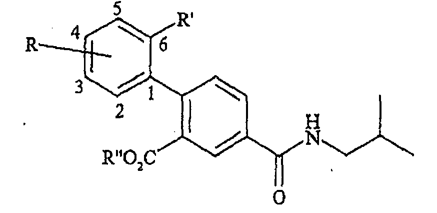 wherein at least one R is selected from the group consisting of
-OCH₃, -OH, -OSO₂CF₃, -CH=CH₂, -OCH₂CO₂C₂H₅,
-OCH₂CONH₂, 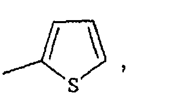 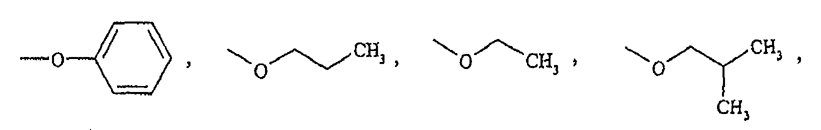 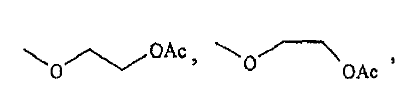 -OBn, -OH, -OSO₂CF₃, 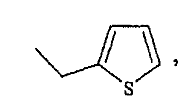 -OCH₃, -OBn, -H, -OBn, and -CH=CH₂;
R' is selected from the group consisting of
-CHO, -CO₂H, -CO₂MEM, 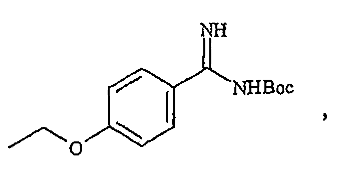 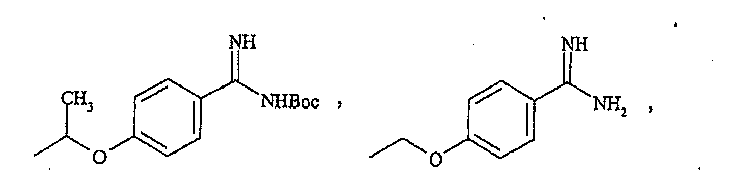 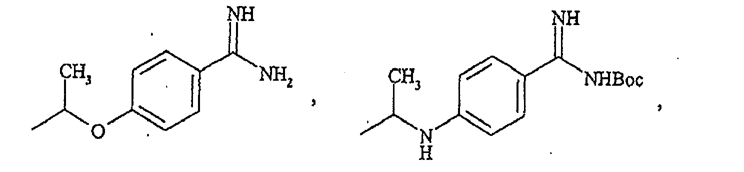 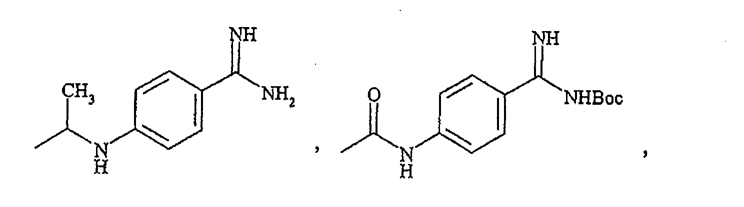 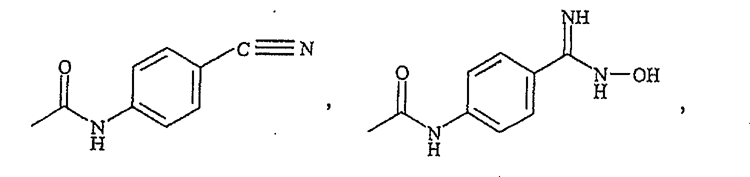 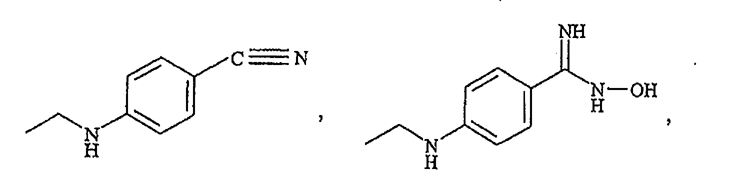 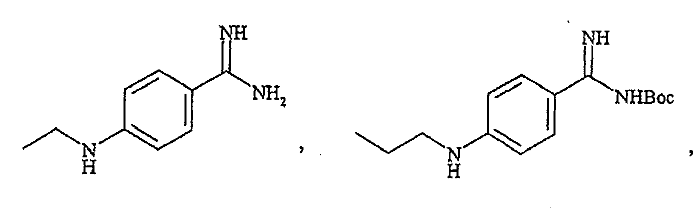 and 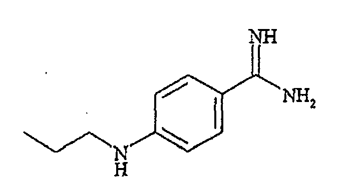 and R" is selected from the group consisting of -H, -CH₃ and -Bn; and pharmaceutically acceptable salts thereof;
wherein MEM designates a methoxyethoxymethyl group.

14. The compound represented by the structure 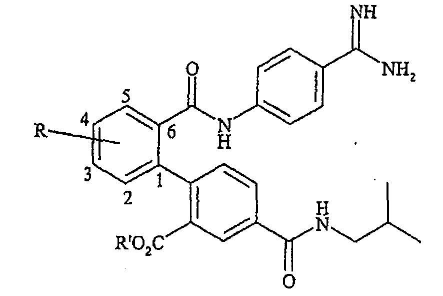 wherein at least one R is selected from the group consisting of -CH=CH₂, -OSO₂CF₃, -OCH₂CO₂C₂H₅, -OCH₂CONH₂, 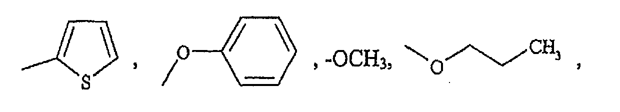 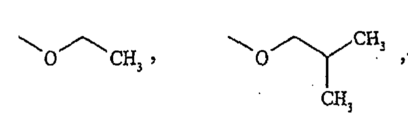 -O-CH₂-CH₂-OAc, -OH,
-OCH₂CO₂H, -O-CH₂-CH₂-OH, -CH(OH)CH₂OH, -CH₂OH, -CO₂H, 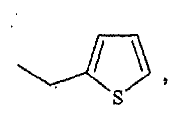 -OBn, -OH, -OCH₃, -OBn, -OH, -OC₂H₅,
-OBn, -OCH₃, and -CH(OH)CH₃; and R' is selected from the group consisting of -CH₃, -CH₂C₆H₅, -Bn, -H; and pharmaceutically acceptable salts thereof;

15. The compound represented by the structure 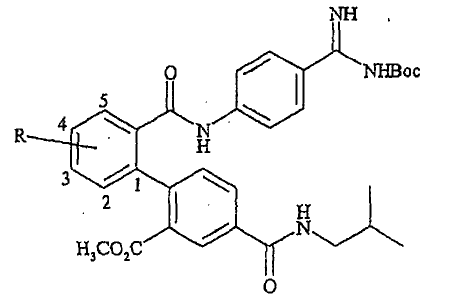 wherein at least one R is selected from the group consisting of -CH=CH₂, -CH(OH)CH₂OH, -CH=O, -CH₂OH, -CO₂H, -OCH₃, -CH=CH₂; and pharmaceutically acceptable salts thereof;

16. The compound represented by the structure 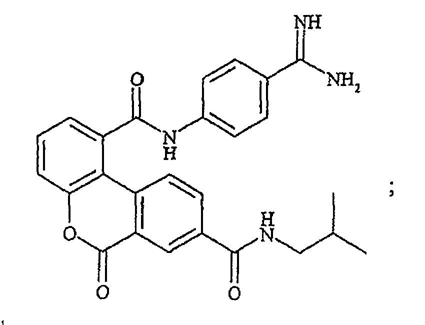 and pharmaceutically acceptable salts thereof.

17. The compound represented by the structure 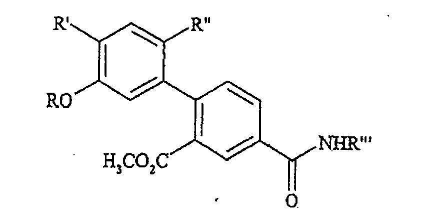 wherein R is selected from the group consisting of
-CH₃, -C₂H₅, -CH(CH₃)₂, 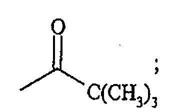
R' is selected from the group consisting of -OBn, -OH, -OSO₂CF₃, and -CH=CH₂;
R" is selected from the group consisting of -CO₂H, -CO₂MEM, or -CHO;
and R"' is selected from the group consisting of 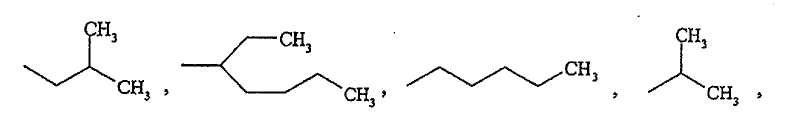 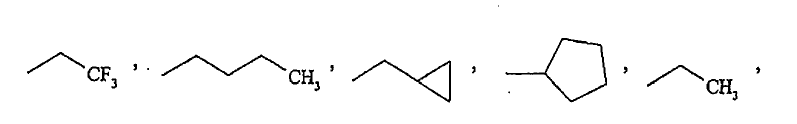 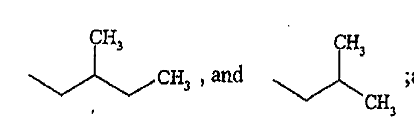
and pharmaceutically acceptable salts thereof; wherein MEM designates a methoxyethoxymethyl group.

18. The compound represented by the structure 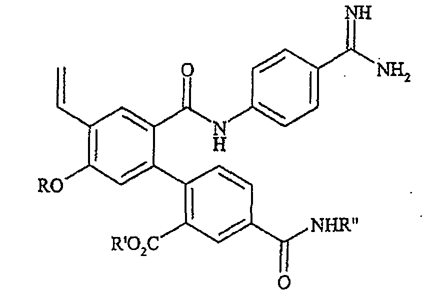 wherein R is selected from the group consisting of
-CH₃, -C₂H₅, -CH(CH₃)₂, 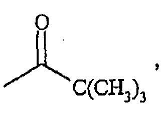 and -H;
R' is -H or alkyl; and R" is selected from the group consisting of 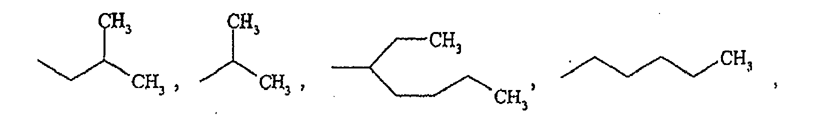 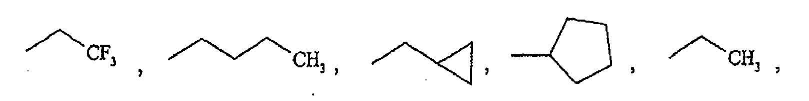 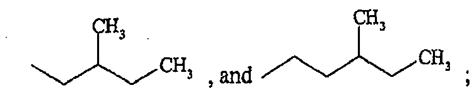
and pharmaceutically acceptable salts
thereof;

19. The compound of claim 18 wherein said alkyl is -CH₃.

20. The compound represented by the structure 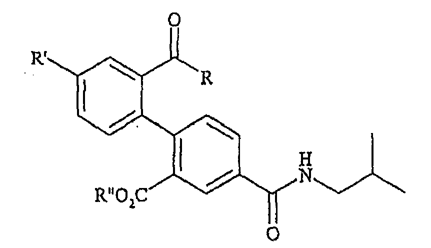 wherein R is selected from the group consisting of 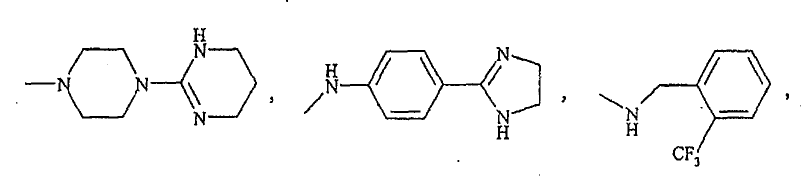 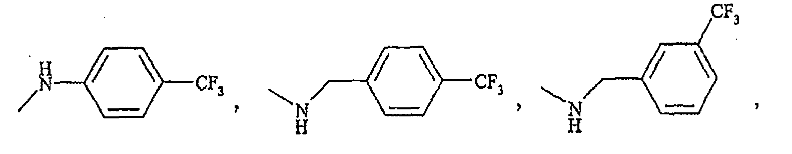 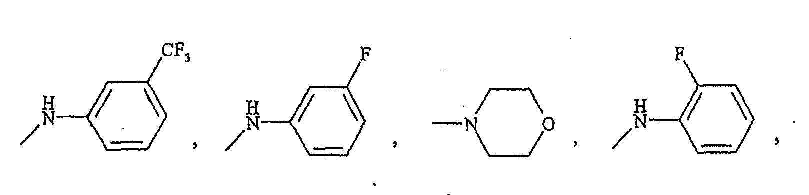 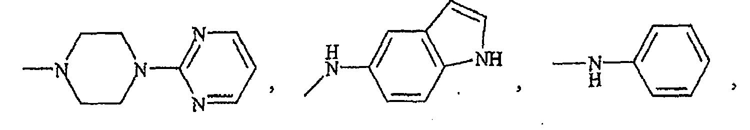 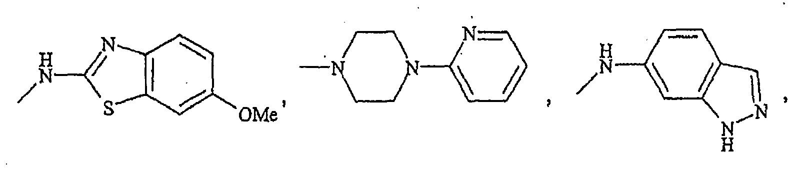 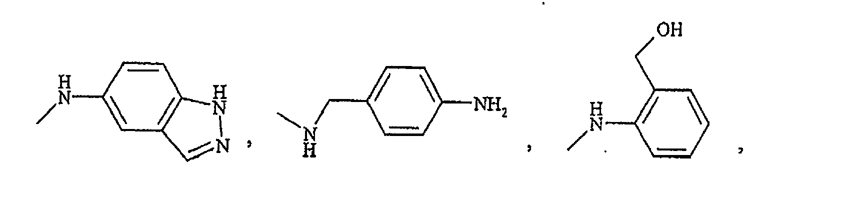 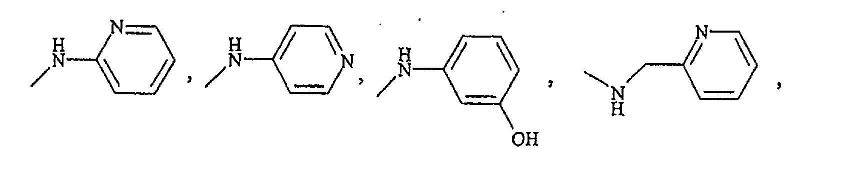 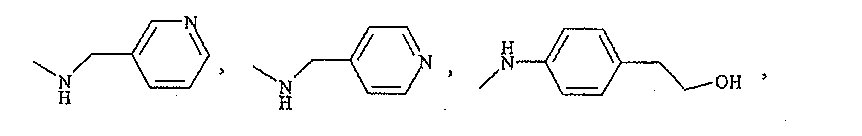 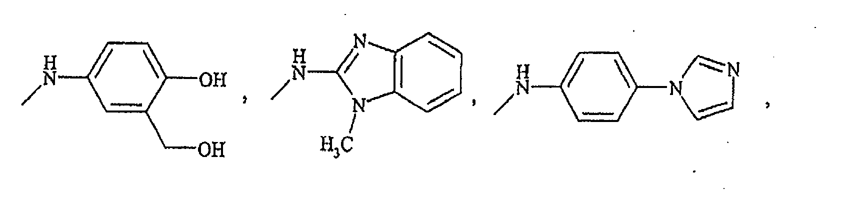 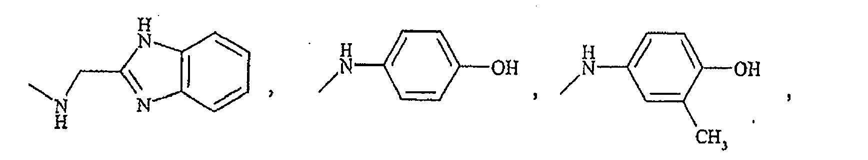 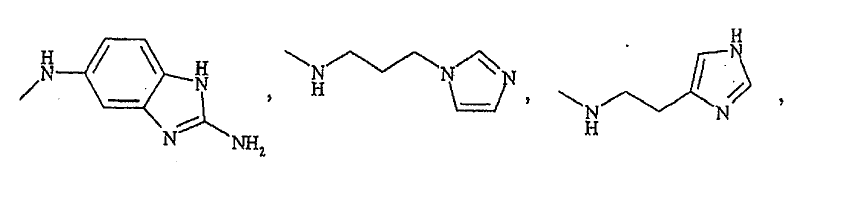 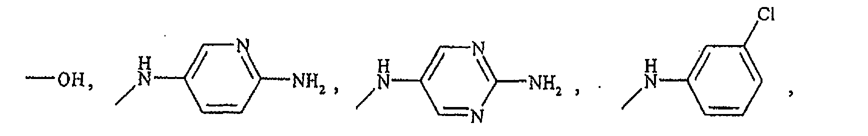 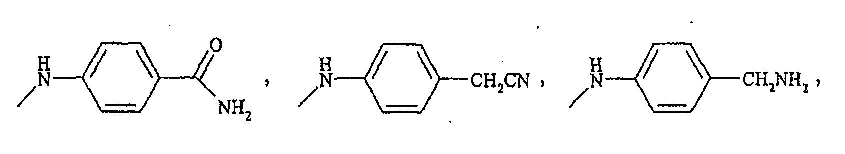 and 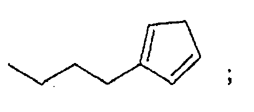
R' is -H, -CH=CH₂; and R" is -H or alkyl; and pharmaceutically acceptable salts thereof;

21. The compound of claim 20 wherein said alkyl is -CH₃,

22. The compound represented by the structure 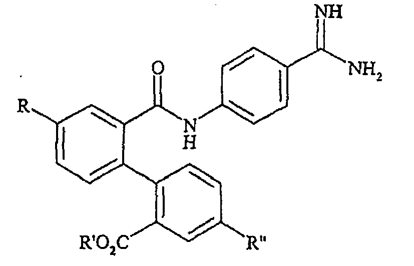 wherein R is selected from the group consisting of 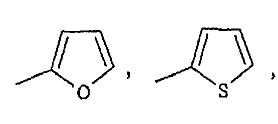 -CH₌CH₂, and -H; R' is -H or alkyl; and R" is selected
from the group consisting of 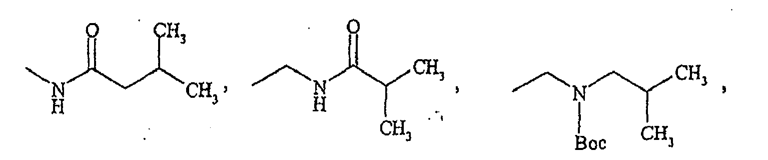 and 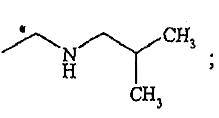 and pharmaceutically acceptable salts thereof;

23. The compound of claim 22 wherein said alkyl is -CH₃.

24. The compound represented by the structure 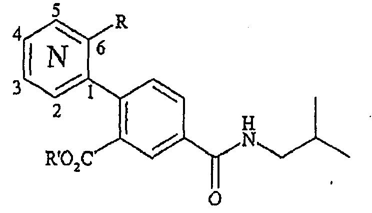 wherein N is located at position 3 or 4 in the phenyl ring; R is selected from the group consisting of -CHO, -CO₂H, and 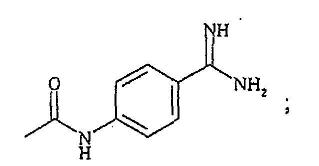 and R' is -H or alkyl; and pharmaceutically acceptable salts thereof;

25. The compound of claim 24 wherein said alkyl is -CH₃.

26. The compound represented by the structure 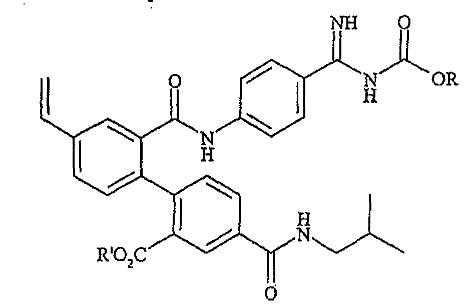 wherein R is selected from the group consisting of 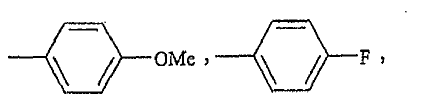 -CH₃, -C₂H₅, -CH₂C₆H₅, -C(CH₃)₃, -CH₂-CCl₃, 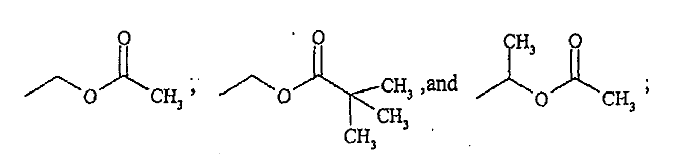 and R' is -H or alkyl; and pharmaceutically acceptable salts thereof;

27. The compound of claim 26 wherein alkyl is CH₃.

28. The compound represented by the structure 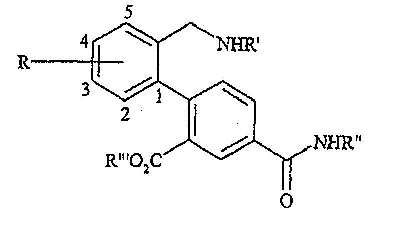 wherein at least one R is selected from the group consisting of -CH=CH₂, -OCH₃, -OBn, -OH, and -H; R' is 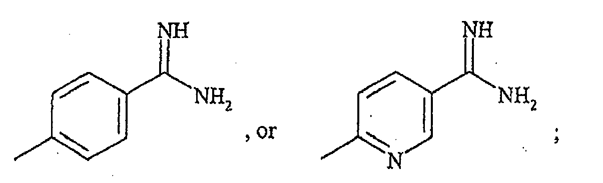
R" is selected from the group consisting of 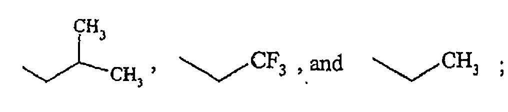
and R"' is -H; and pharmaceutically
acceptable salts thereof;

29. The compound represented by the structure 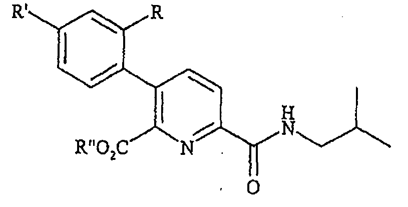 wherein R is selected from the group consisting of -CHO, -CO₂H, -CO₂MEM, 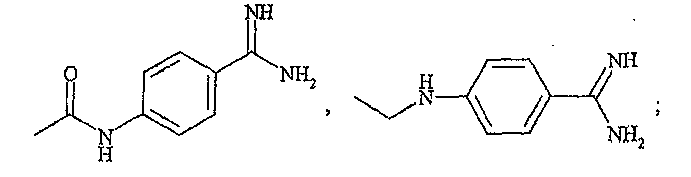
R' is selected from the group consisting of -OBn, -OH, -OSO₂CF₃, and -CH=CH₂;
and R" is -H or alkyl; and pharmaceutically acceptable salts thereof;

30. The compound of claim 29 wherein said alkyl is -CH₃.

31. The compound represented by the structure 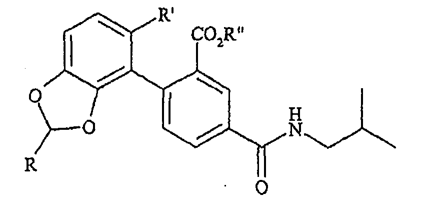 wherein R is -H or -CO₂H;
R' is selected from the group consisting of -CHO, -CO₂H, and 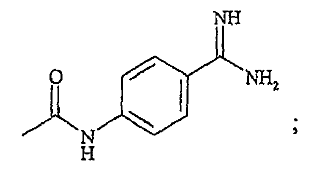
and R" is -H or alkyl; and pharmaceutically acceptable salts thereof.

32. The compound of claim 31 wherein said alkyl is -CH₃.

33. The compound represented by the structure 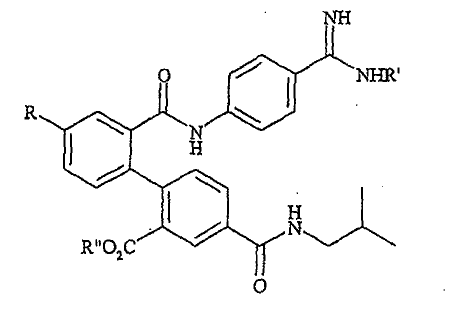 wherein R is selected from the group consisting of -CH(OH)-CH₂OH, -CHO, and -CH(OH)-CH=CH₂;
R' is -Boc or -H; and R" is -H or alkyl; and pharmaceutically acceptable salts thereof;

34. The compound of claim 33 wherein said alkyl is -CH₃.

35. The compound represented by the structure 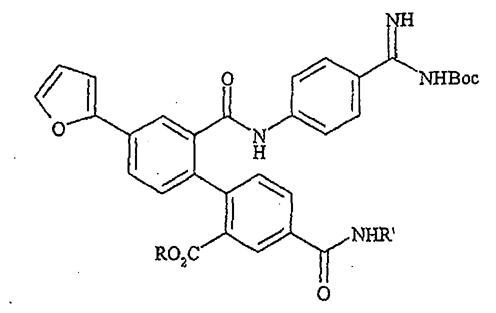 wherein R is alkyl and R' is selected from the group consisting of 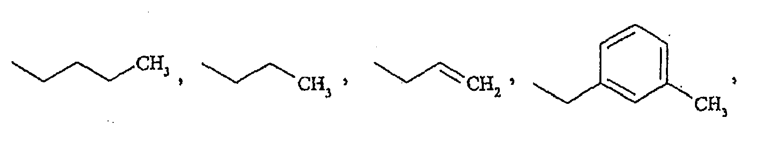 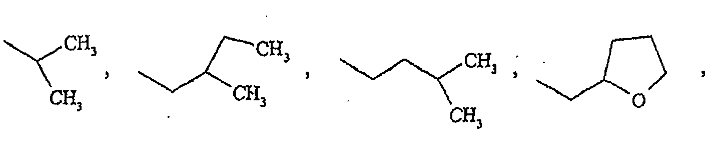 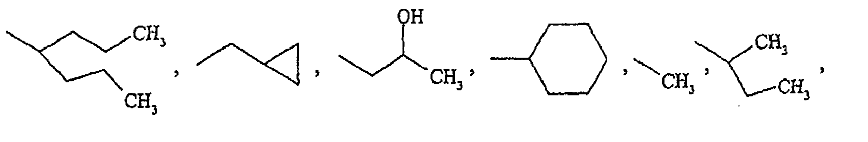 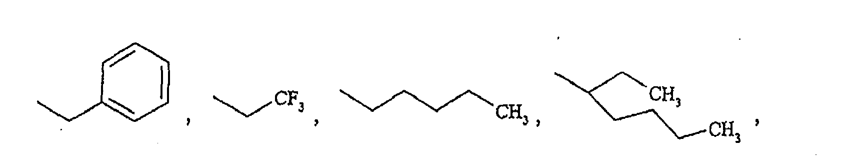 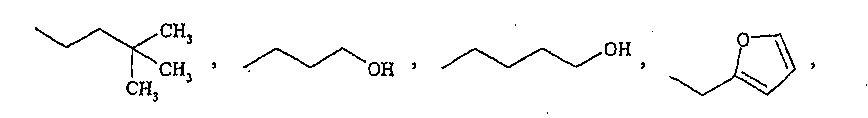 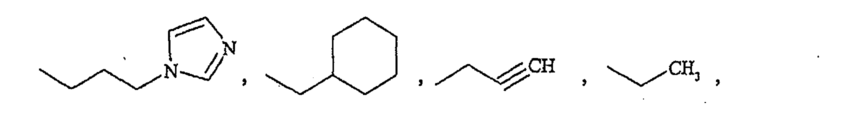 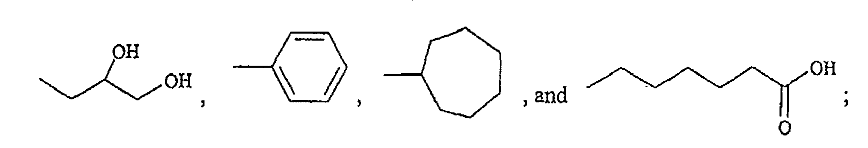 or 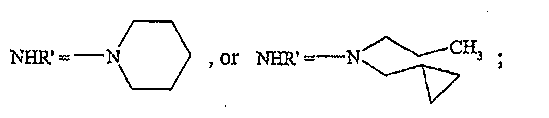 and pharmaceutically acceptable salts thereof.

## Patentansprüche

1. Verbindung, wiedergegeben durch die Struktur 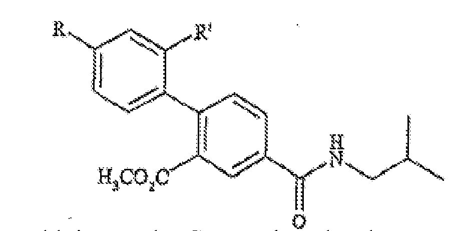 worin R ausgewählt ist aus der Gruppe, bestehend aus
-OBn, -OH, -OSO₂CF₃, 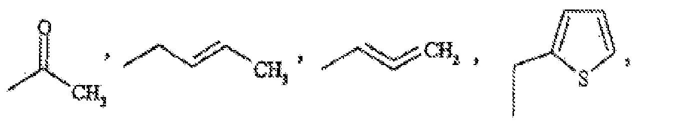 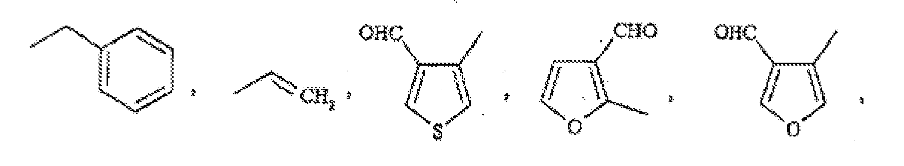 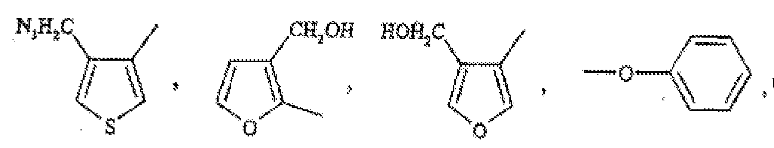 und
-OCH₃;
und R' ausgewählt ist aus der Gruppe, bestehend aus -CHO, -CO₂H, und -CO₂MEM; und pharmazeutisch verträgliche Salze derselben; worin MEM eine Methoxyethoxymethylgruppe bezeichnet.

2. Verbindung, wiedergegeben durch die Struktur 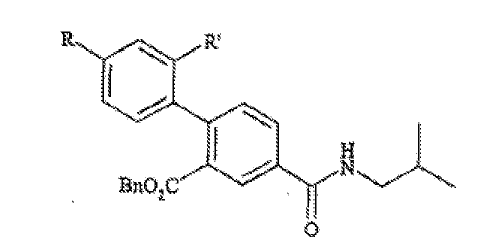 worin R ausgewählt ist aus der Gruppe, bestehend aus 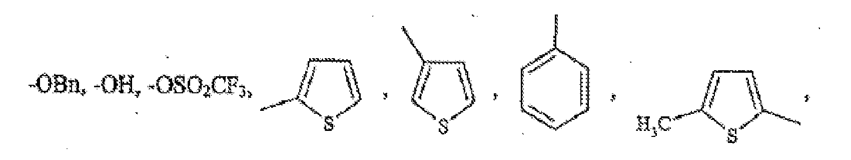 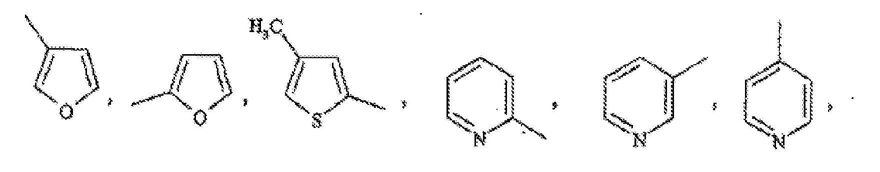 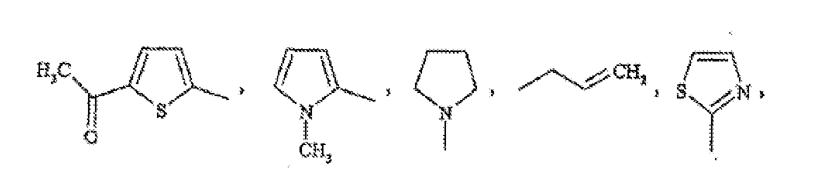 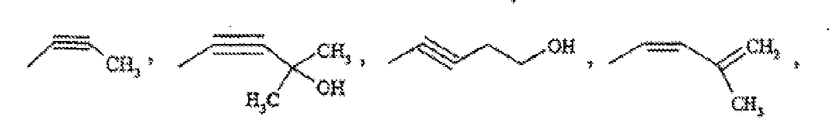 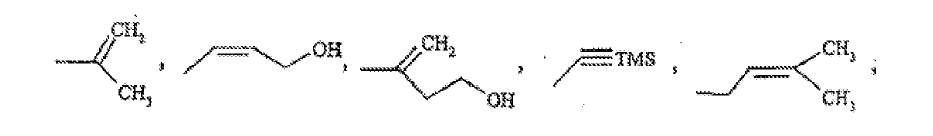 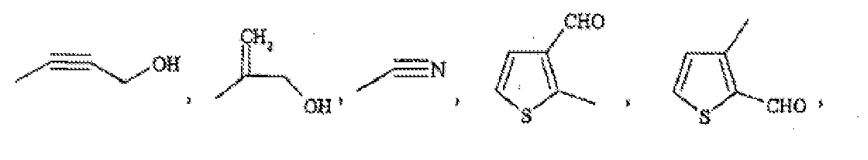 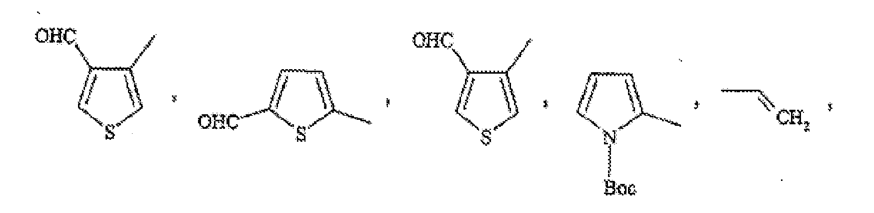 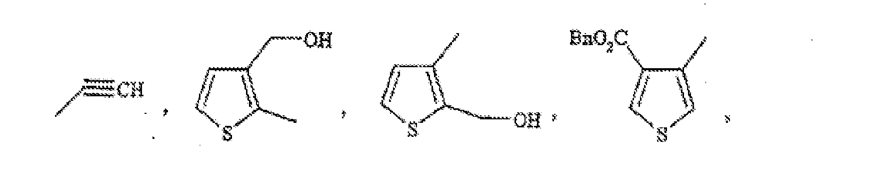 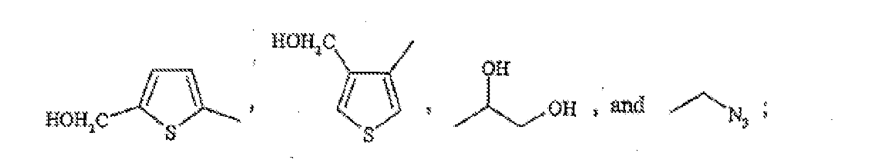 und R' ausgewählt ist aus der Gruppe, bestehend aus -CHO, -CO₂H, und -CO₂MEM; und pharmazeutisch verträgliche Salze derselben; worin MEM eine Methoxyethoxymethylgruppe bezeichnet.

3. Verbindung, wiedergegeben durch die Struktur 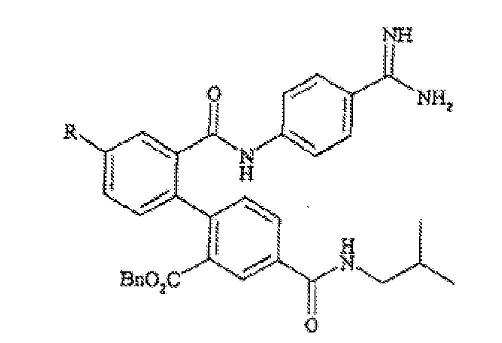 worin R ausgewählt ist aus der Gruppe, bestehend aus 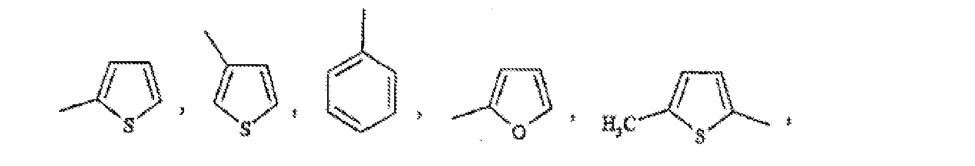 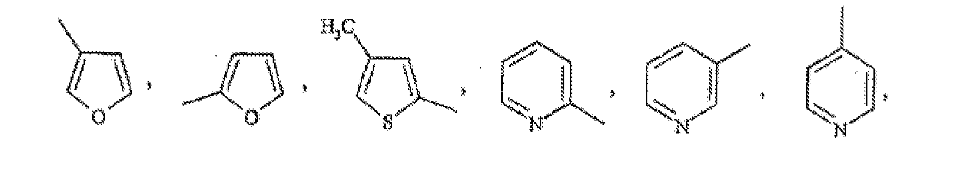 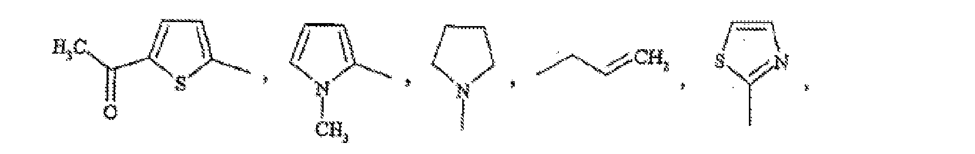 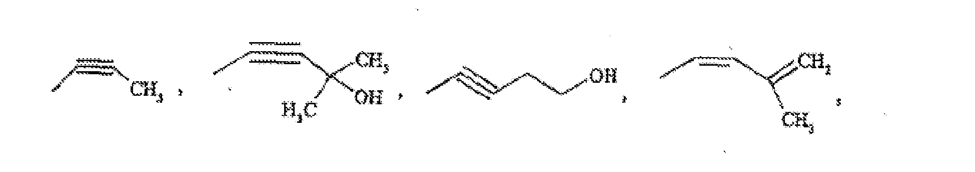 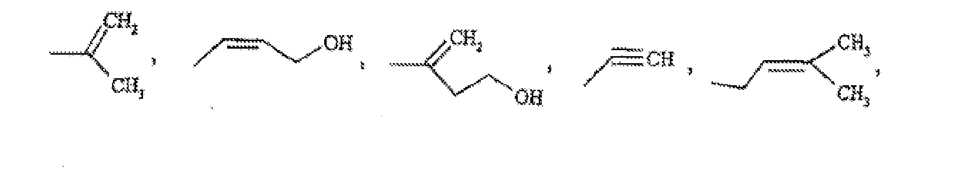 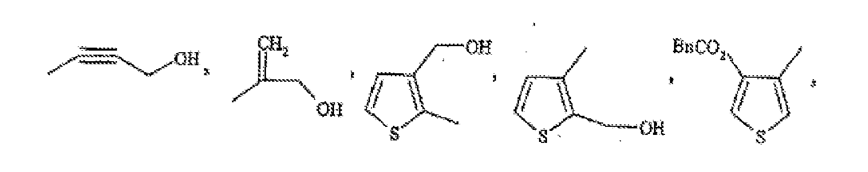 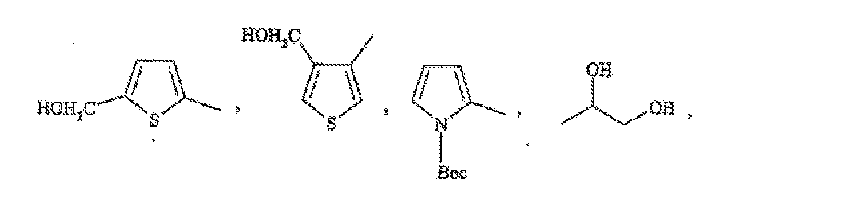 und 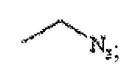 und pharmazeutisch verträgliche Salze derselben.

4. Verbindung, wiedergegeben durch die Struktur 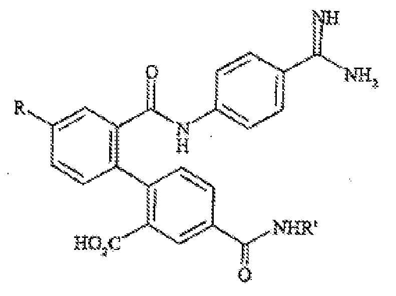 worin R ausgewählt ist aus der Gruppe, bestehend aus 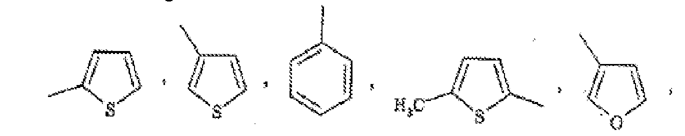 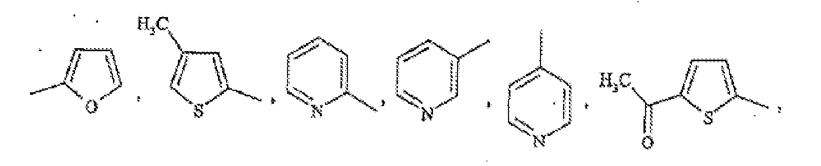 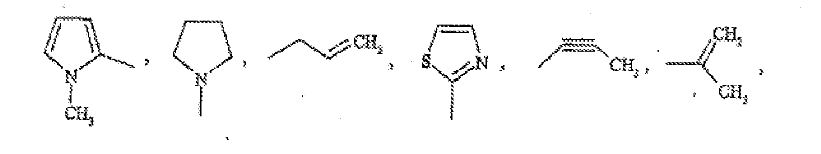 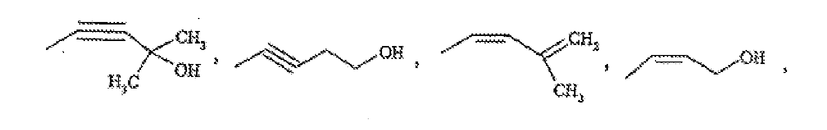 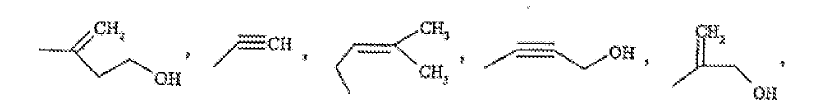 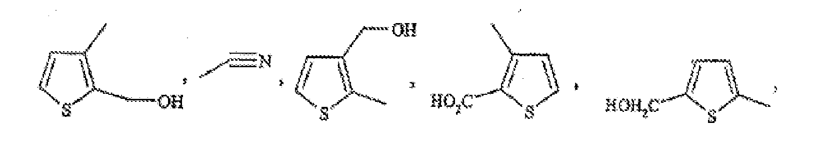 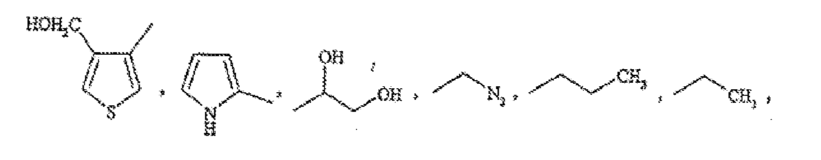 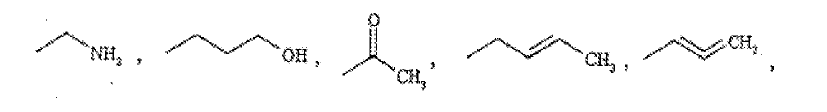 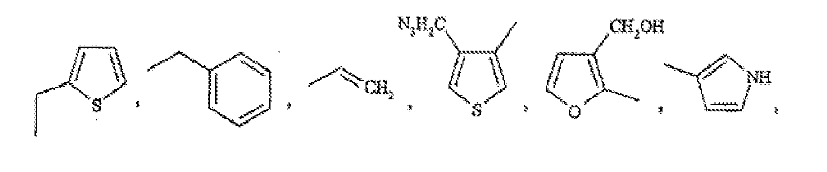 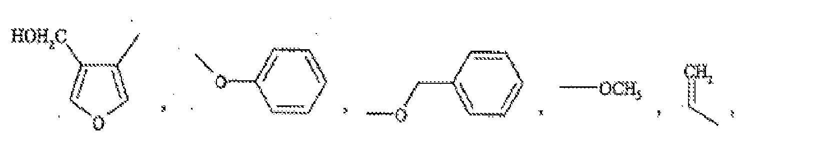 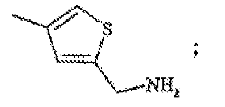 und
und R' ausgewählt ist aus der Gruppe, bestehend aus 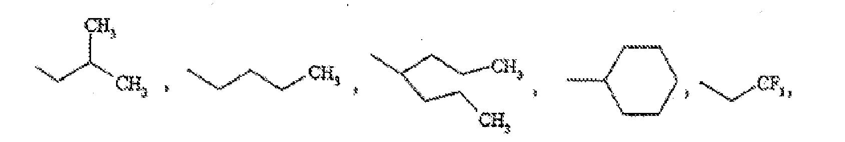 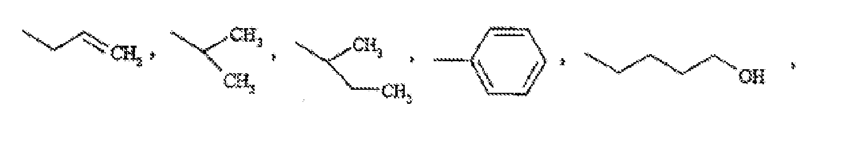 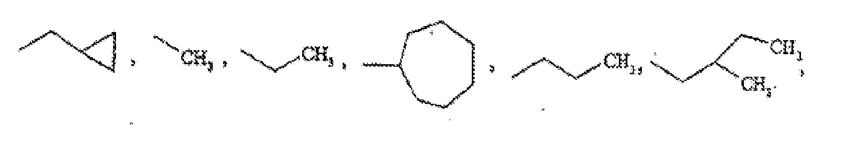 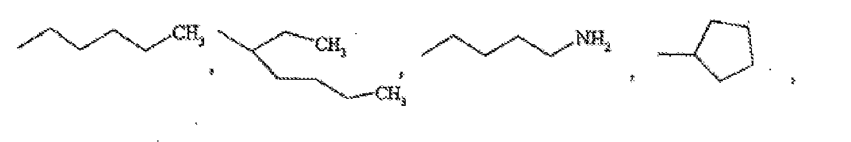 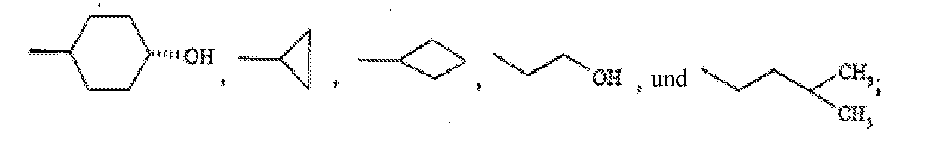 und pharmazeutisch verträgliche Salze derselben.

5. Verbindung, wiedergegeben durch die Struktur 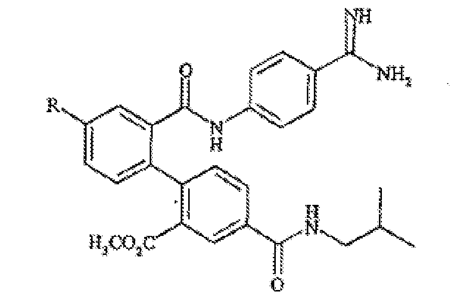 worin R ausgewählt ist aus der Gruppe, bestehend aus 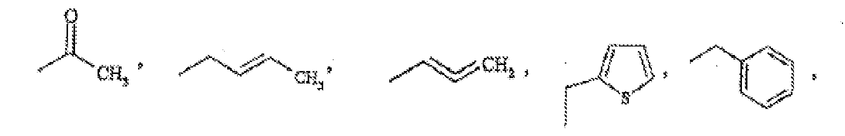 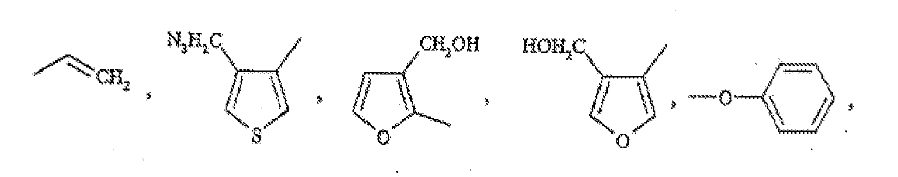 -OBn, -OCH₃, und 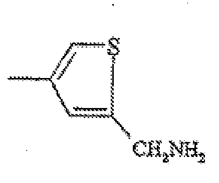 ; und pharmazeutisch verträgliche Salze derselben.

6. Verbindung, wiedergegeben durch die Struktur 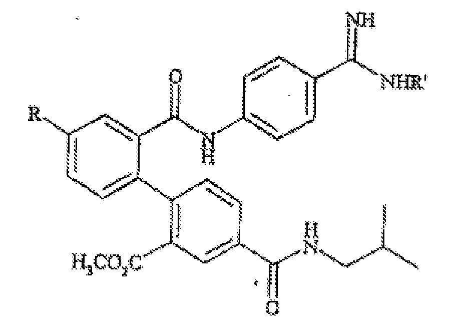 worin R ausgewählt ist aus der Gruppe, bestehend aus
-OSO₂CF₃, 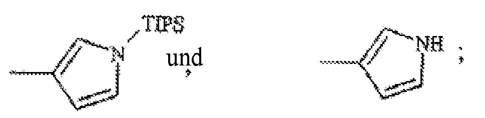 und R' H ist oder 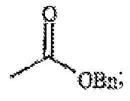 und pharmazeutisch verträgliche Salze derselben.

7. Verbindung, wiedergegeben durch die Struktur 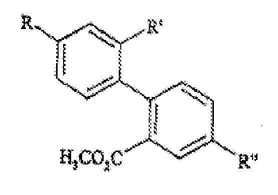 worin R ausgewählt ist aus der Gruppe, bestehend aus
-OBn, -OH, -OSO₂CF₃, 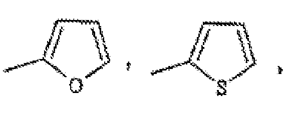 -CH=CH₂, und -H;
und R' ausgewählt ist aus der Gruppe, bestehend aus -CHO, -CO₂H, und -CO₂MEM;
und R" ausgewählt ist aus der Gruppe, bestehend aus
-CO₂MEM, 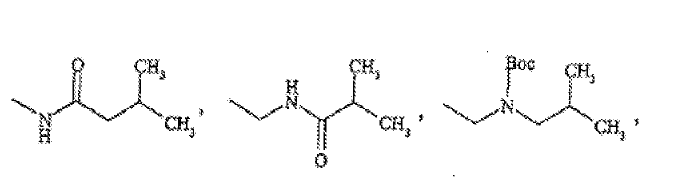 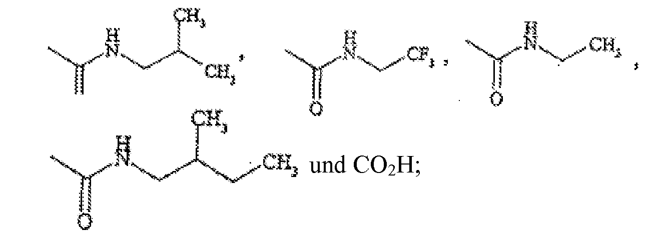 und pharmazeutisch verträgliche Salze derselben.

8. Verbindung, wiedergegeben durch die Struktur 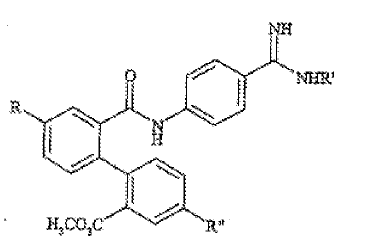 worin R ausgewählt ist aus der Gruppe, bestehend aus
-OBn, -OH, -OSO₂CF₃, 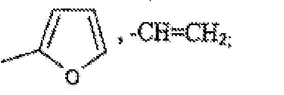
R' -H oder -Boc ist; und R" -CO₂MEM oder -CO₂H ist; und pharmazeutisch verträgliche Salze derselben, worin MEM eine Methoxyethoxymethylgruppe bezeichnet.

9. Verbindung, wiedergegeben durch die Struktur 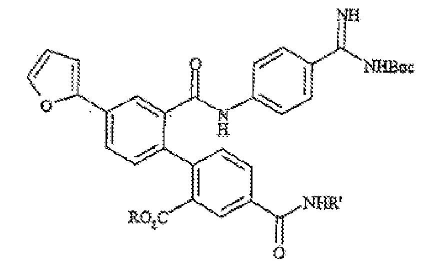 worin R -CH₃ ist und R' ausgewählt ist aus der Gruppe, bestehend aus 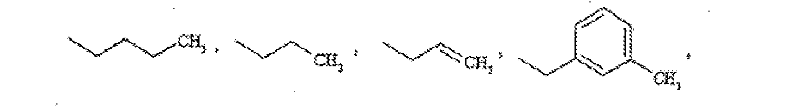 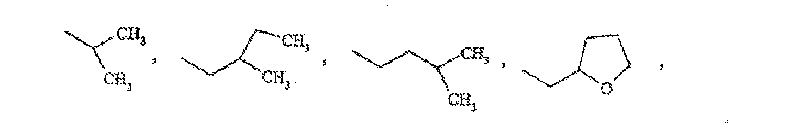 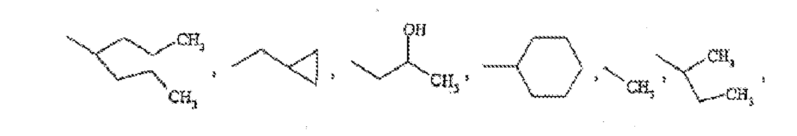 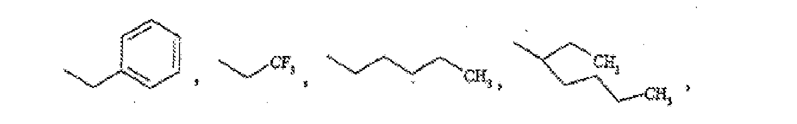 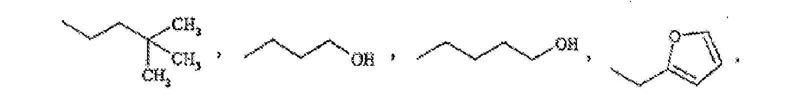 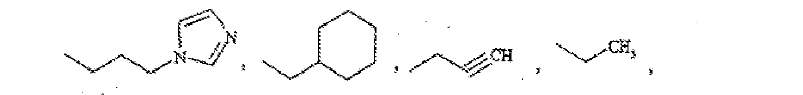 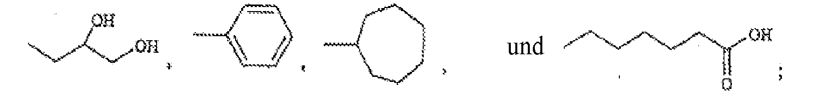 oder 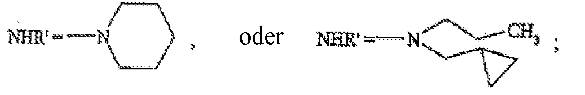 oder pharmazeutisch verträgliche Salze derselben.

10. Verbindung, wiedergegeben durch die Struktur 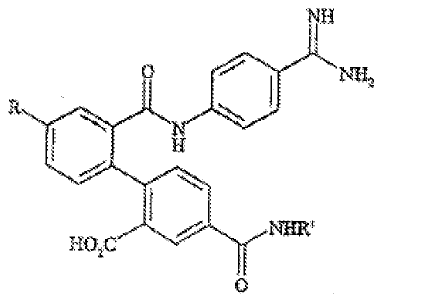 worin R 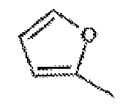 ist ; und R' ausgewählt ist aus der Gruppe, bestehend aus 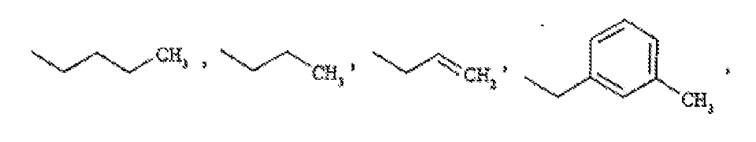 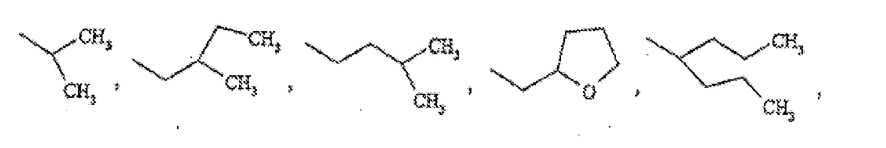 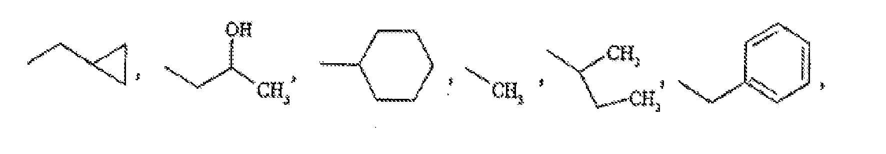 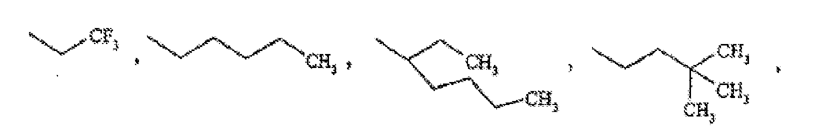 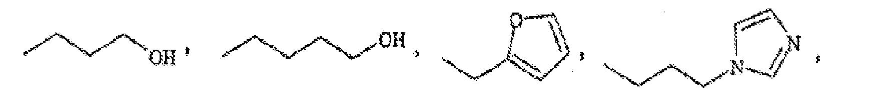 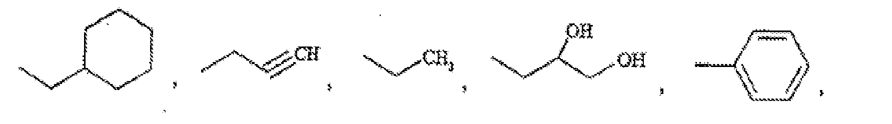 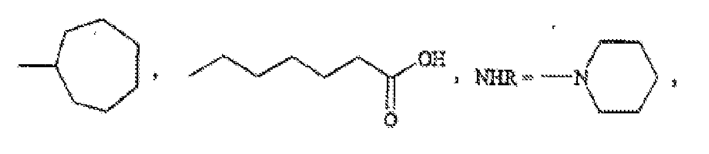 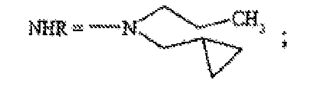 und pharmazeutisch verträgliche Salze derselben.

11. Verbindung, wiedergegeben durch die Struktur 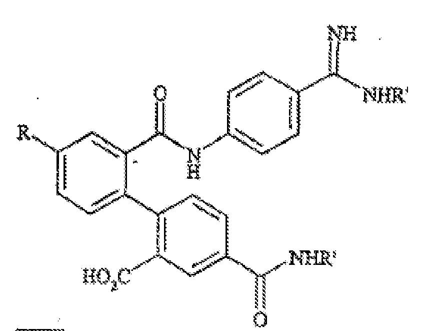 worin R 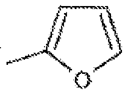 und -CH=CH₂ ist und R' ausgewählt ist aus der Gruppe, bestehend aus 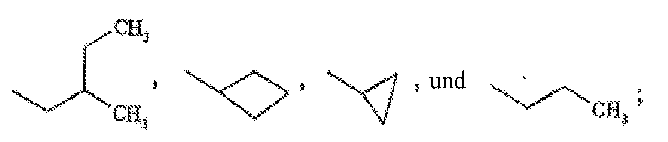 und pharmazeutisch verträgliche Salze derselben.

12. Verbindung, wiedergegeben durch die Struktur 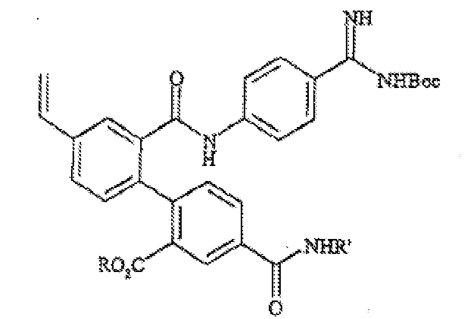 worin R -CH₃ ist und R' ausgewählt ist aus der Gruppe, bestehend aus 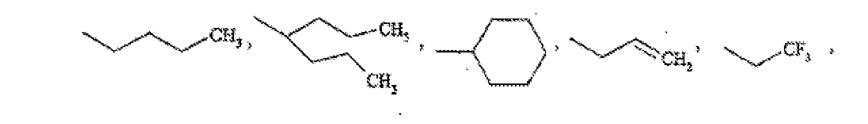 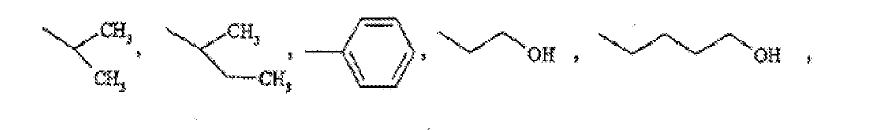 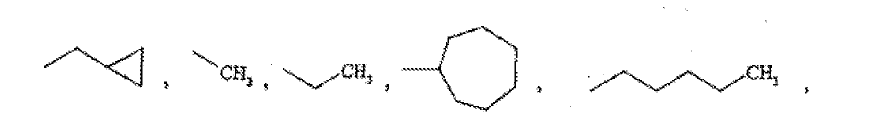 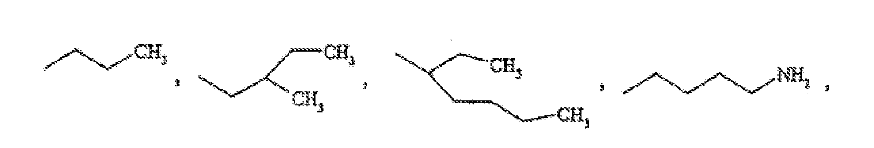 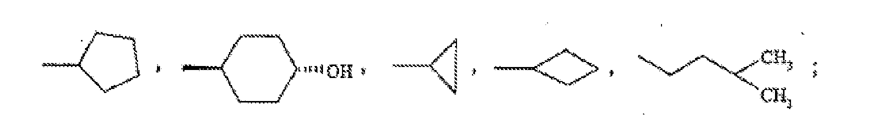 und pharmazeutisch verträgliche Salze derselben.

13. Verbindung, wiedergegeben durch die Struktur 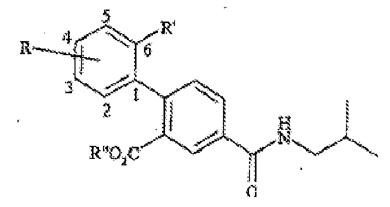 worin wenigstens ein R ausgewählt ist aus der Gruppe, bestehend aus -OCH₃, -OH, -OSO₂CF₃, -CH=CH₂, -OCH₂CO₂C₂H₅,
-OCH₂CONH₂, 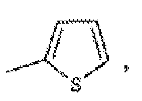 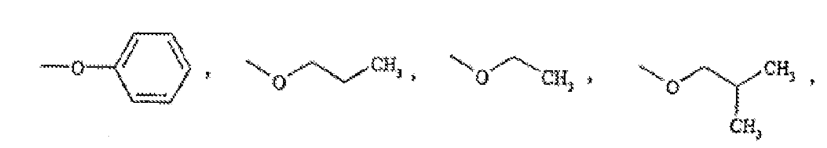 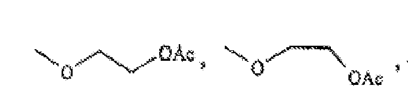 -OBn, -OH, -OSO₂CF₃, 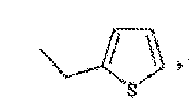 -OCH₃, -OBn, -H, -OBn, and -CH=CH₂;
R' ausgewählt ist aus der Gruppe, bestehend aus
-CHO, -CO₂H, -CO₂MEM, 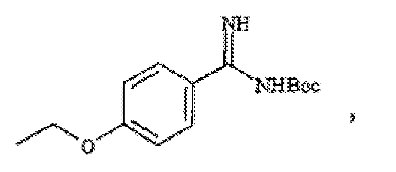 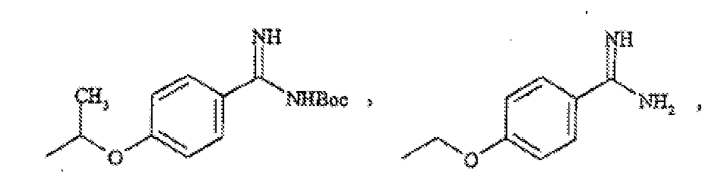 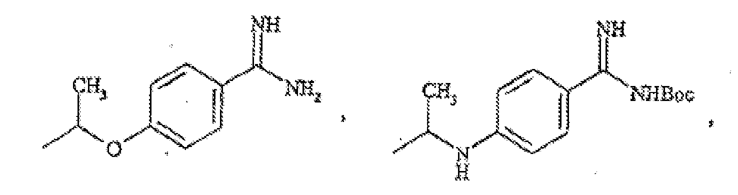 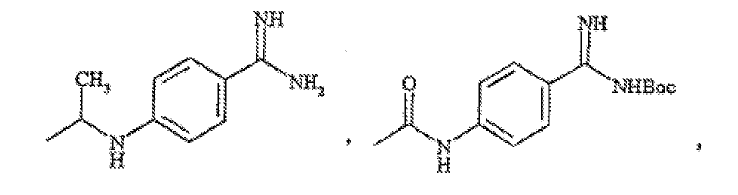 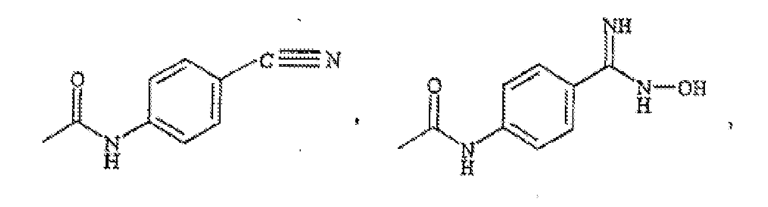 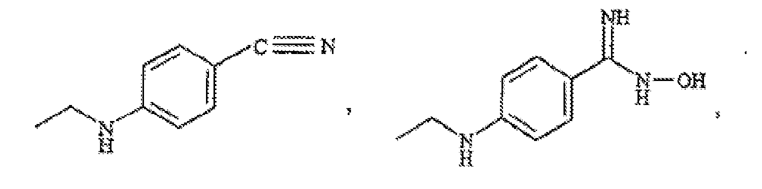 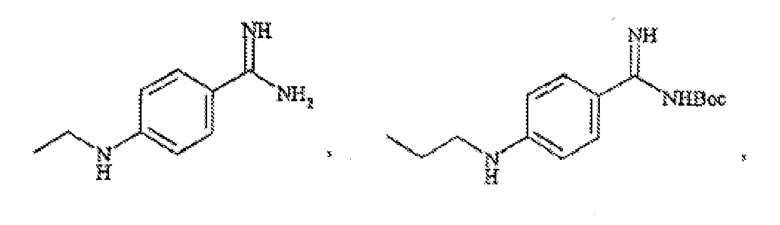 und 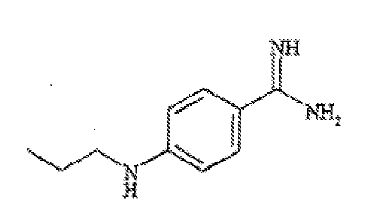
und R" ausgewählt ist aus der Gruppe, bestehend aus -H, -CH₃ und -Bn; und pharmazeutisch verträgliche Salze derselben, worin MEM eine Methoxyethoxymethylgruppe bezeichnet.

14. Verbindung, wiedergegeben durch die Struktur 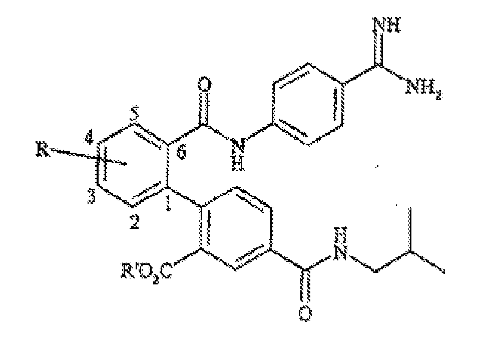 worin wenigstens ein R ausgewählt ist aus der Gruppe, bestehend aus
-CH=CH₂, -OSO₂CF₃, -OCH₂CO₂C₂H₅, -OCH₂CONH₂, 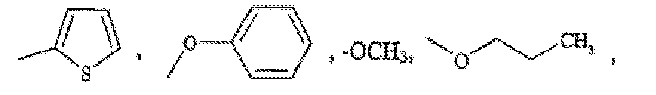 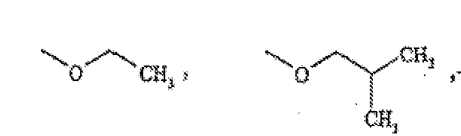 -O-CH₂-CH₂-OAc, -OH,
-OCH₂CO₂H, -O-CH₂-CH₂-OH, -CH(OH)CH₂OH, -CH₂OH, -CO₂H, 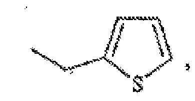 -OBn, -OH, -OCH₃, -OBn, -OH, -OC₂H₅, -OBn, -OCH₃, und -CH(OH)CH₃; und R' ausgewählt ist aus der Gruppe, bestehend aus -CH₃, -CH₂C₆H₅, -Bn, -H; und pharmazeutisch verträgliche Salze derselben.

15. Verbindung, wiedergegeben durch die Struktur 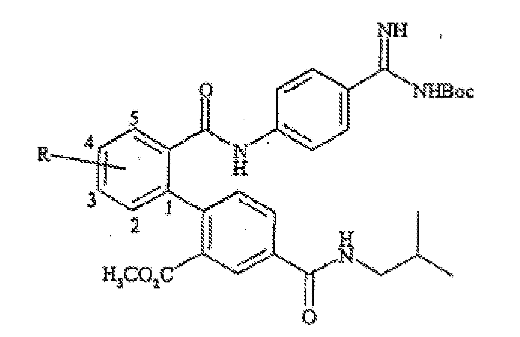 worin wenigstens ein R ausgewählt ist aus der Gruppe, bestehend aus -CH=CH₂, -CH(OH)CH₂OH, -CH=O, CH₂OH, -CO₂H, -OCH₃, -CH=CH₂; und pharma-zeutisch verträgliche Salze derselben.

16. Verbindung, wiedergegeben durch die Struktur 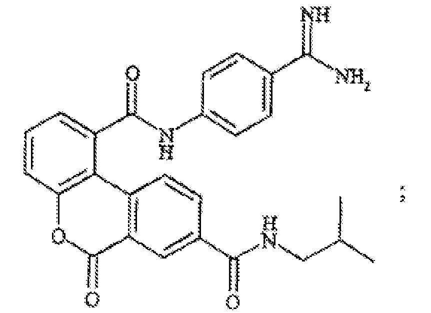 und pharmazeutisch verträgliche Salze derselben.

17. Verbindung, wiedergegeben durch die Struktur 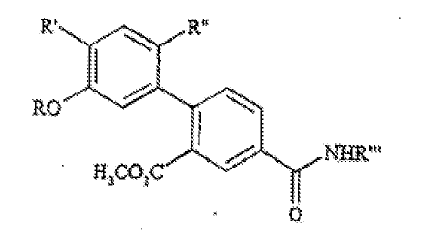 worin R ausgewählt ist aus der Gruppe, bestehend aus
-CH₃, -C₂H₅, -CH(CH₃)₂, 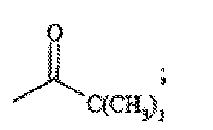
R' ausgewählt ist aus der Gruppe, bestehend aus -OBn, -OH, OSO₂CF₃ und -CH=CH₂;
R" ausgewählt ist aus der Gruppe, bestehend aus -CO₂H, -CO₂MEM, oder -CHO;
und R"' ausgewählt ist aus der Gruppe, bestehend aus 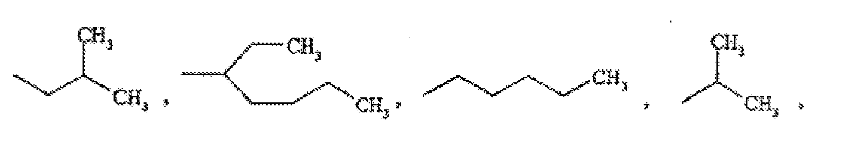 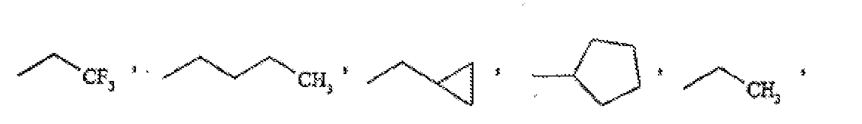 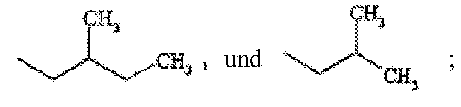
und pharmazeutisch verträgliche Salze derselben; worin MEM eine Methoxyethoxymethylgruppe bezeichnet.

18. Verbindung, wiedergegeben durch die Struktur 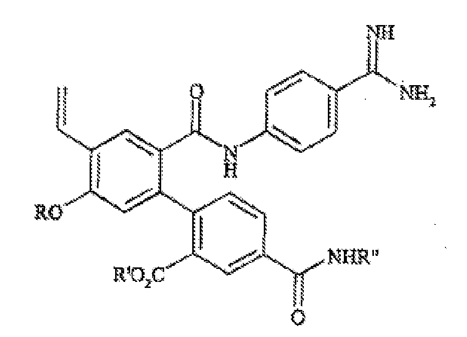 worin R ausgewählt ist aus der Gruppe, bestehend aus
-CH₃, -C₂H₅, -CH(CH₃)₂, 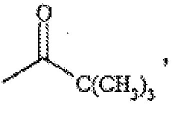 und H;
R' H oder Alkyl ist; und R" ausgewählt ist aus der Gruppe, bestehend aus 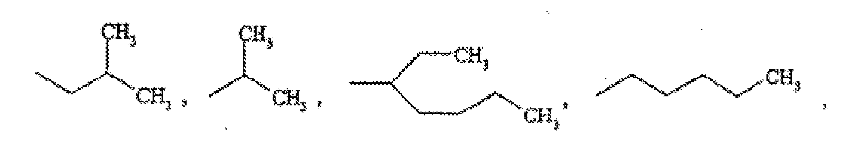 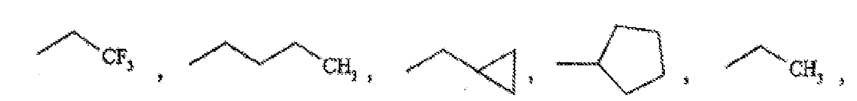 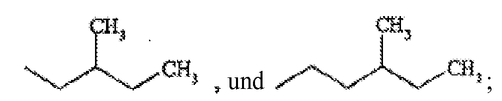 und pharmazeutisch verträgliche Salze derselben.

19. Verbindung nach Anspruch 18, worin das Alkyl -CH₃ ist.

20. Verbindung, wiedergegeben durch die Struktur 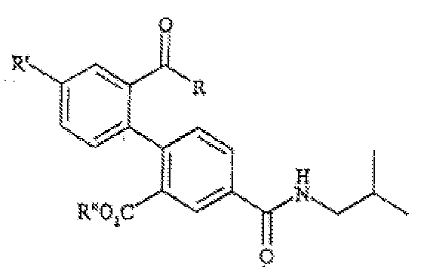 worin R ausgewählt ist aus der Gruppe, bestehend aus 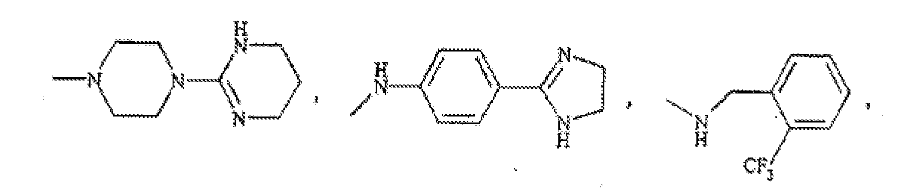 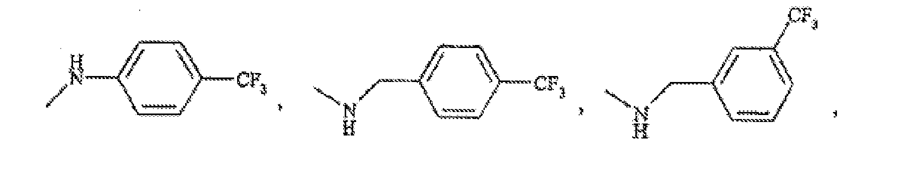 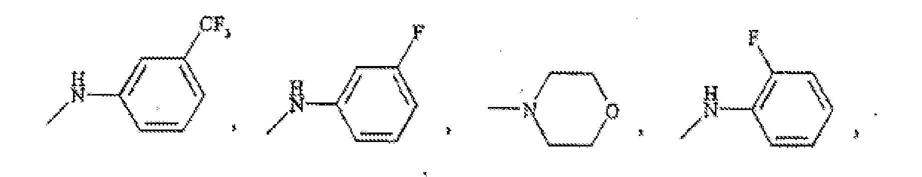 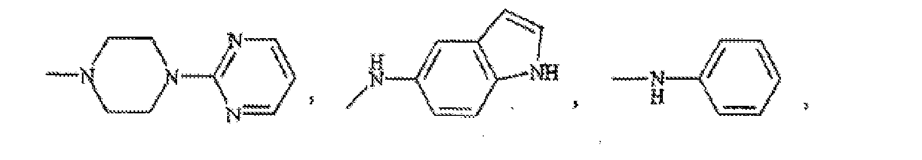 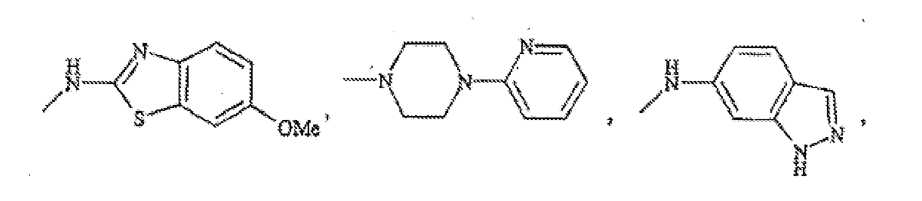 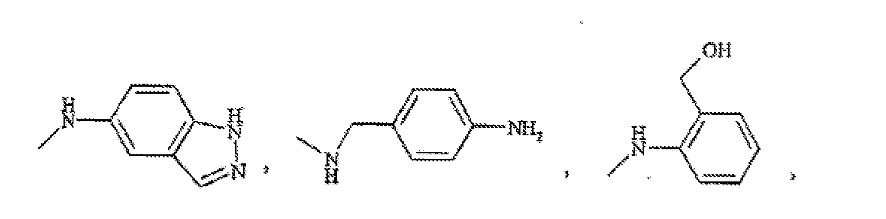 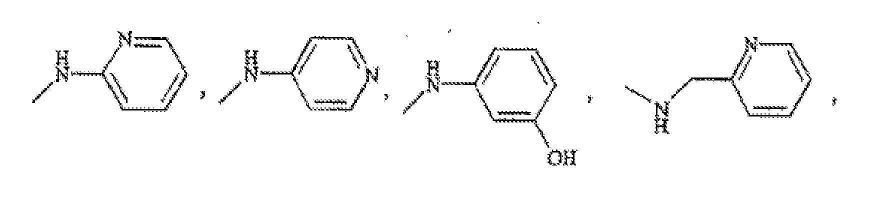 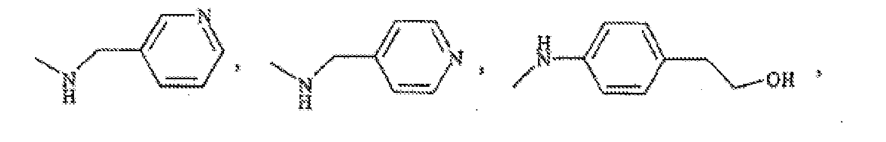 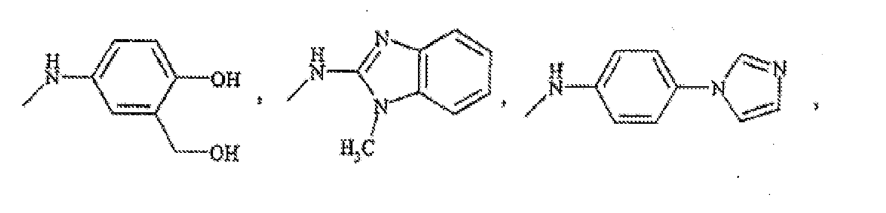 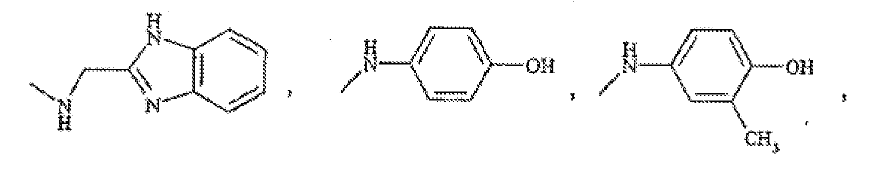 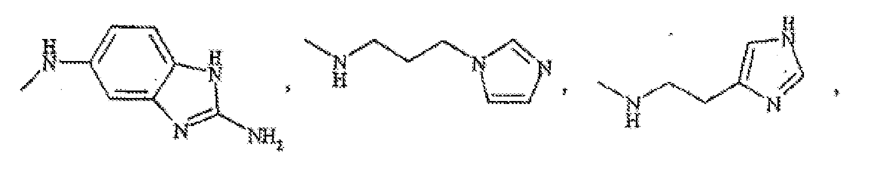 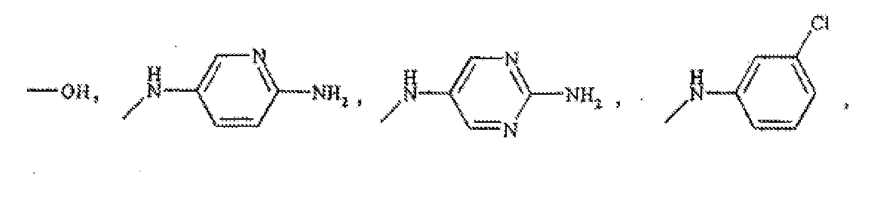 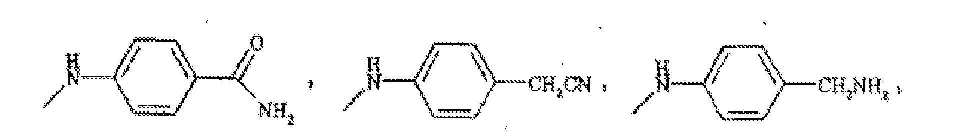 und 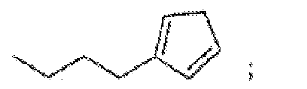
R' -H, -CH=CH₂ ist; und R" -H oder Alkyl ist; und pharmazeutisch verträgliche Salze derselben.

21. Verbindung nach Anspruch 20, wobei das Alkyl -CH₃ ist.

22. Verbindung, wiedergegeben durch die Struktur 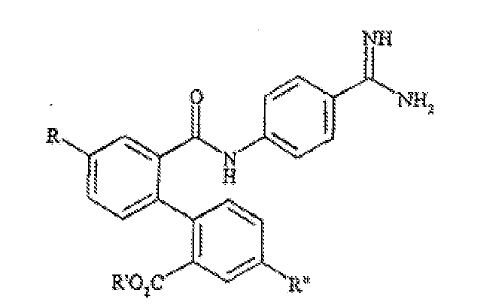 worin R ausgewählt ist aus der Gruppe, bestehend aus 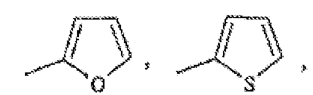 -CH=CH₂, und -H; R' -H oder Alkyl ist; und R" ausgewählt ist aus der Gruppe, bestehend aus 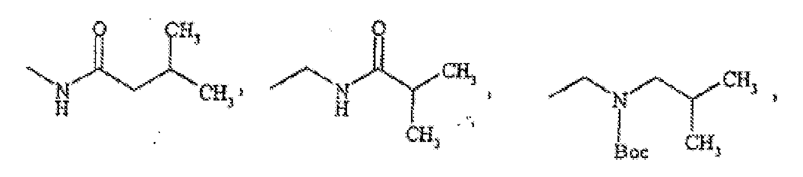 und 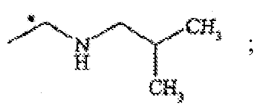 und pharmazeutisch verträgliche Salze derselben.

23. Verbindung nach Anspruch 22, worin das Alkyl -CH₃ ist.

24. Verbindung, wiedergegeben durch die Struktur 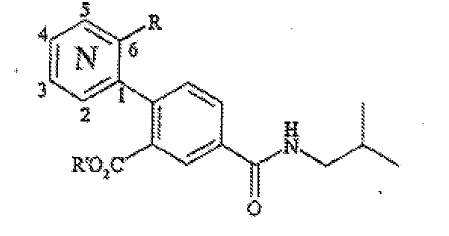 worin sich N in Position 3 oder 4 in dem Phenylring befindet; R ausgewählt ist aus der Gruppe, besehend aus -CHO, -CO₂H, und 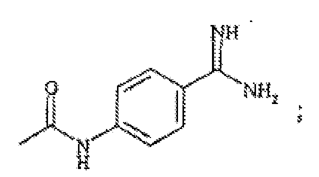 und R'H oder Alkyl ist; und pharmazeutisch verträgliche Salze derselben.

25. Verbindung nach Anspruch 24, worin das Alkyl -CH₃ ist.

26. Verbindung, wiedergegeben durch die Struktur 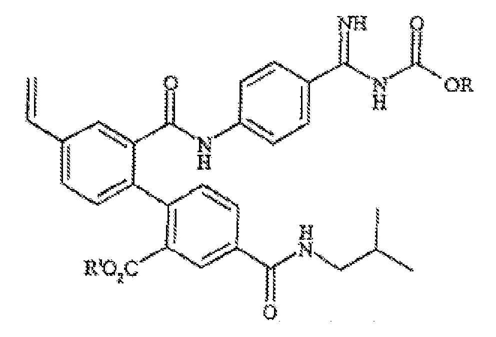 worin R ausgewählt ist aus der Gruppe, bestehend aus
-CH₃, -C₂H₅, -CH₂C₆H₅, -C(CH₃)₃, -CH₂-CCl₃, 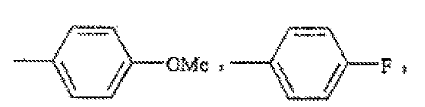 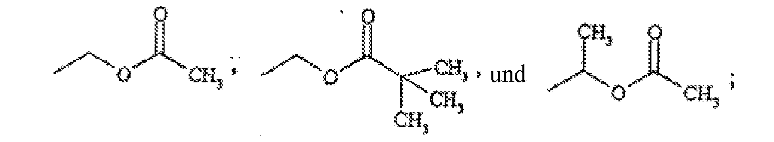 und R' -H oder Alkyl ist; und pharmazeutisch verträgliche Salze derselben.

27. Verbindung nach Anspruch 26, wobei das Alkyl -CH₃ ist.

28. Verbindung, wiedergegeben durch die Struktur 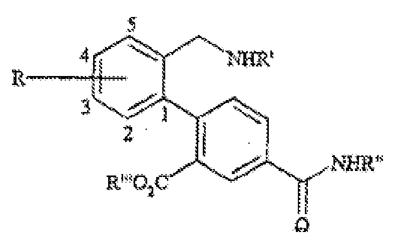 worin wenigstens ein R ausgewählt ist aus der Gruppe, bestehend aus -CH=CH₂, -OCH₃, -OBn, -OH und -H; R' ist 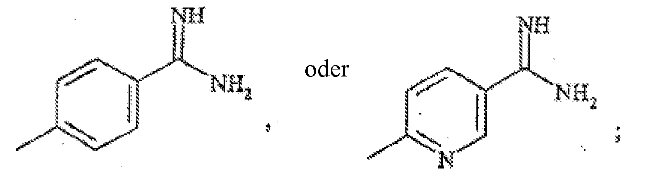
R" ist ausgewählt aus der Gruppe, bestehend aus 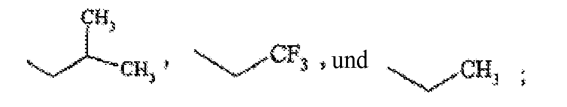
und R"' ist -H; und pharmazeutisch verträgliche Salze derselben.

29. Verbindung, wiedergegeben durch die Struktur 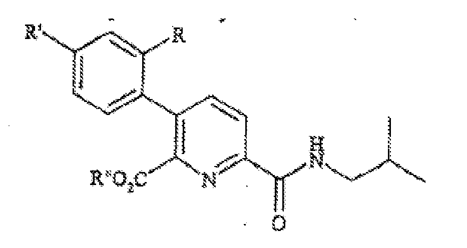 worin R' ausgewählt ist aus der Gruppe, bestehend aus - CHO, -CO₂H oder -CO₂MEM, 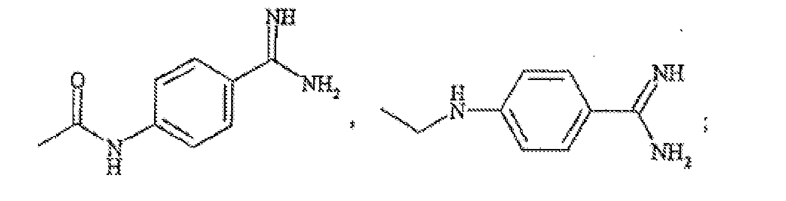
R' ausgewählt ist aus der Gruppe, bestehend aus -OBn, -OH, -OSO₂CF₃ und -CH=CH₂;
und R" -H oder Alkyl ist; und pharmazeutisch verträgliche Salze derselben.

30. Verbindung nach Anspruch 29, worin das Alkyl -CH₃ ist.

31. Verbindung, wiedergegeben durch die Struktur 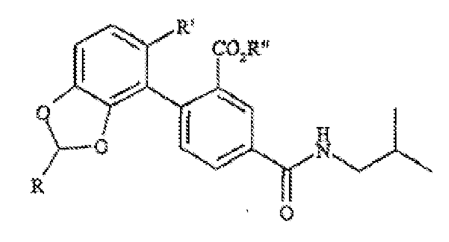 worin R -H oder CO₂H ist;
R' ausgewählt ist aus der Gruppe, bestehend aus - CHO, -CO₂H und 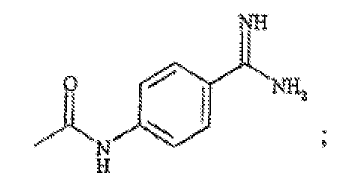
und R" -H oder Alkyl ist; und pharmazeutisch verträgliche Salze derselben.

32. Verbindung nach Anspruch 31, worin das Alkyl -CH₃ ist.

33. Verbindung, wiedergegeben durch die Struktur 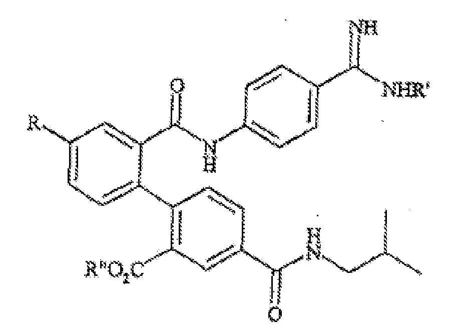 worin R ausgewählt ist aus der Gruppe, bestehend aus -CH(OH)CH₂OH, -CHO und - CH(OH)-CH=CH₂;
R'-Boc oder -H ist; und R" -H oder Alkyl ist; und pharmazeutisch verträgliche Salze derselben.

34. Verbindung nach Anspruch 33, worin das Alkyl -CH₃ ist.

35. Verbindung, wiedergegeben durch die Struktur 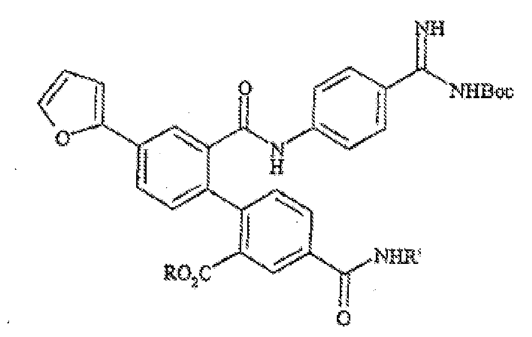 worin R Alkyl ist und R' ausgewählt ist aus der Gruppe, bestehend aus 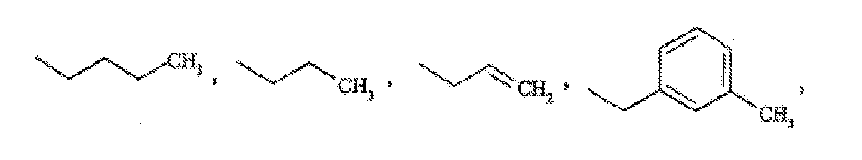 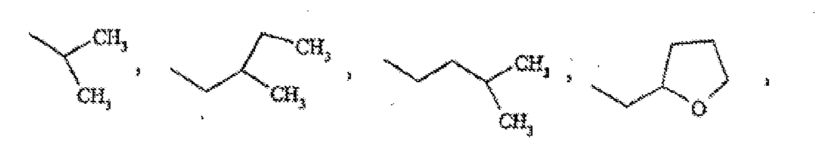 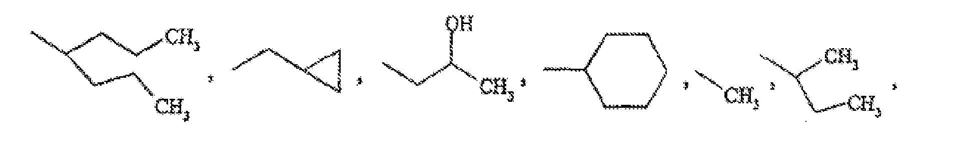 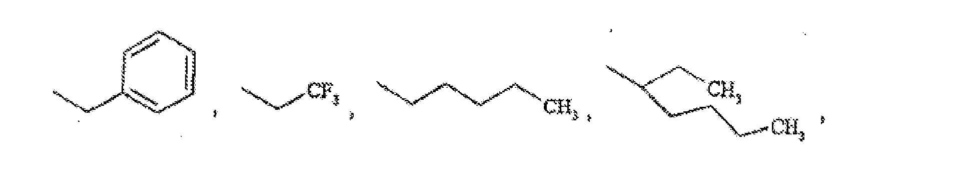 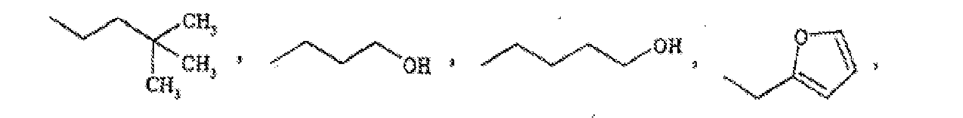 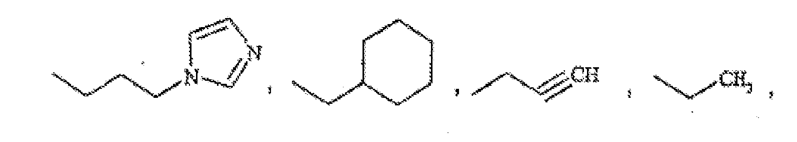 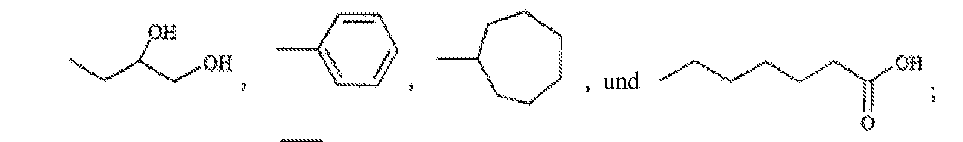 oder 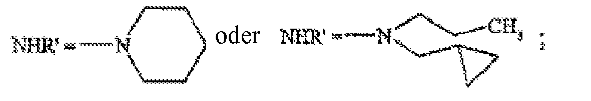 und pharmazeutisch verträgliche Salze derselben.

## Revendications

1. Composé représenté par la structure 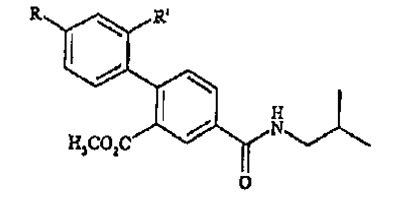 dans laquelle R est choisi dans le groupe consistant en
-OBn, -OH, -OSO₂CH₃, 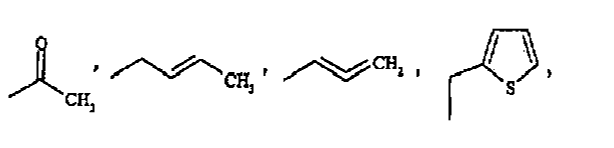 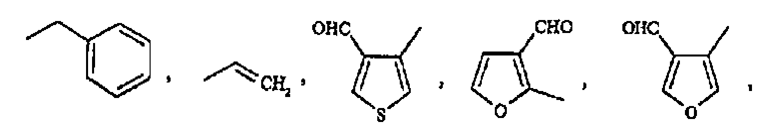 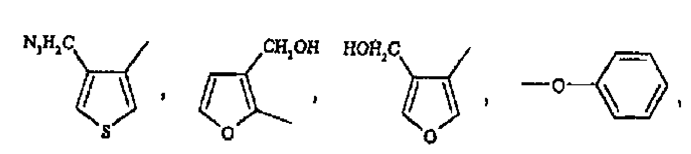 et -OCH₃ ;
et R' est choisi dans le groupe consistant en -CHO, -CO₂H et -CO₂MEM ; et ses sels pharmaceutiquement acceptables ; où MEM désigne un groupe méthoxyéthoxyméthyle.

2. Composé représenté par la structure 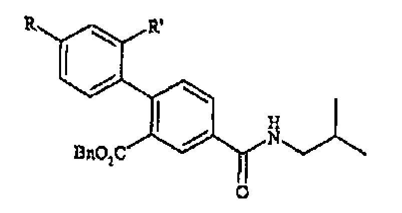 dans laquelle R est choisi dans le groupe consistant en
-OBn, -OH, -OSO₂CH₃, 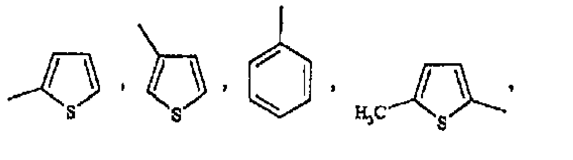 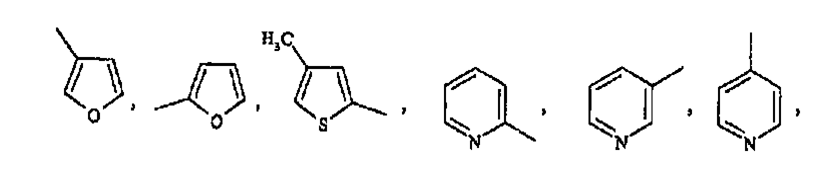 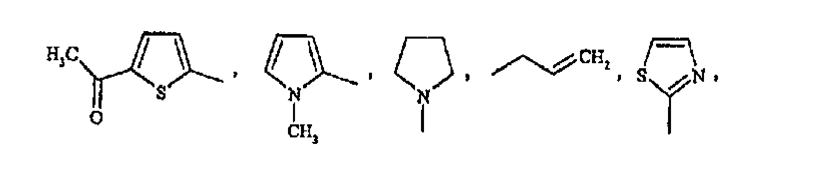 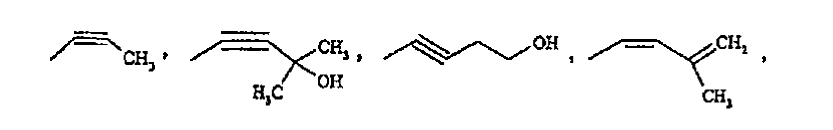 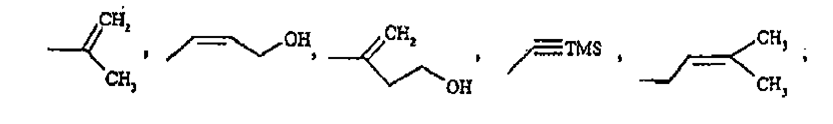 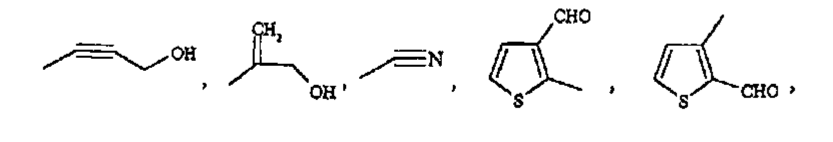 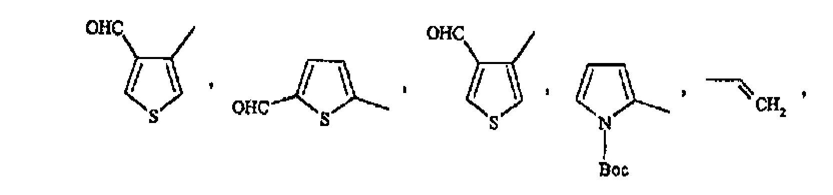 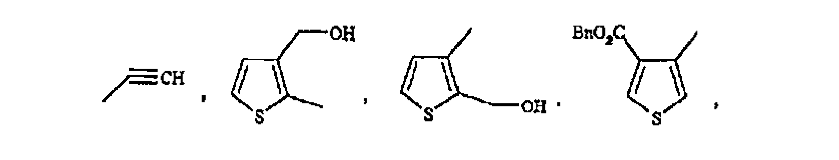 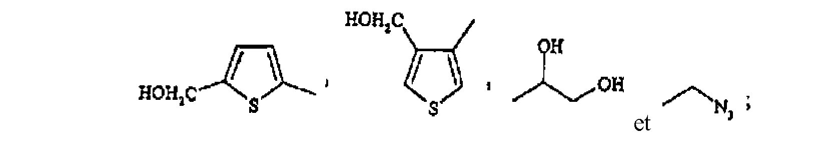 et R' est choisi dans le groupe consistant en -CHO, -CO₂H et -CO₂MEM ; et ses sels pharmaceutiquement acceptables ; où MEM désigne un groupe méthoxyéthoxyméthyle.

3. Composé représenté par la structure 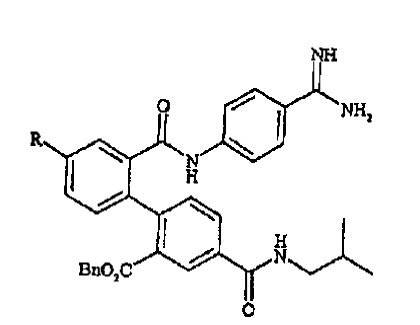 dans laquelle R est choisi dans le groupe consistant en 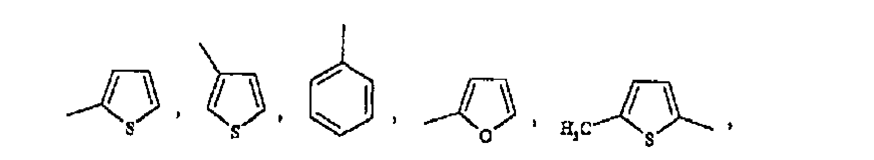 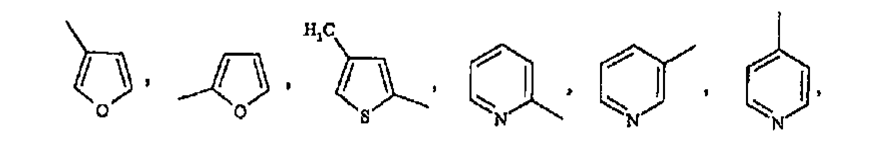 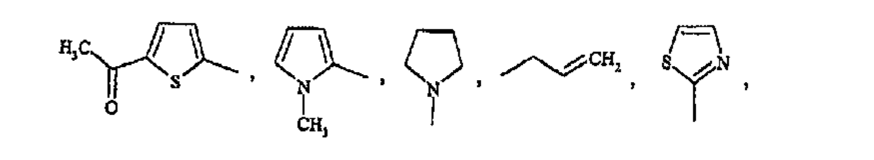 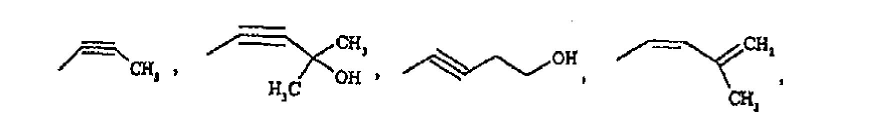 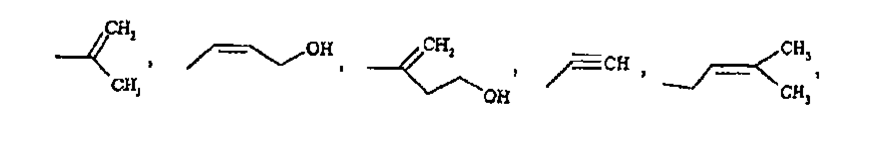 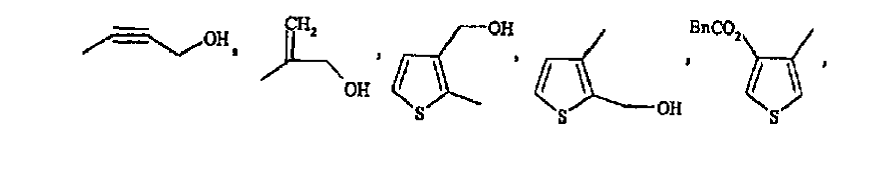 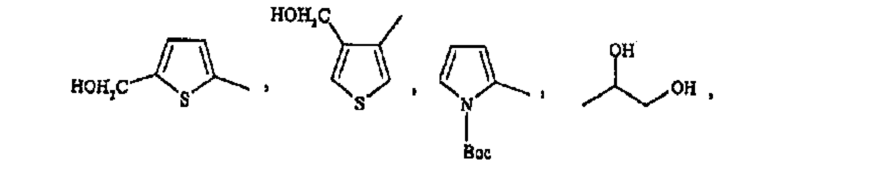 et 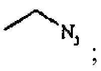 et ses sels pharmaceutiquement acceptables.

4. Composé représenté par la structure 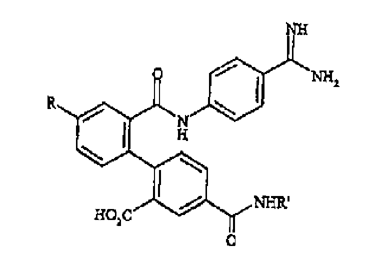 dans laquelle R est choisi dans le groupe consistant en 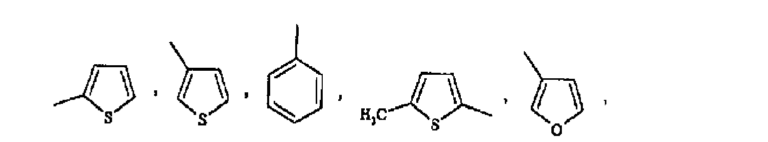 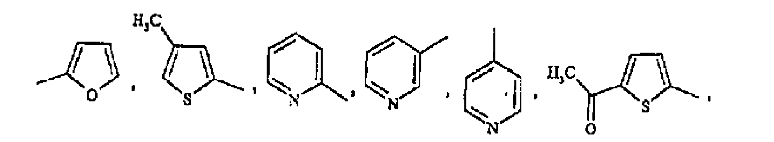 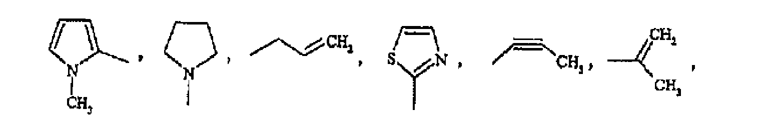 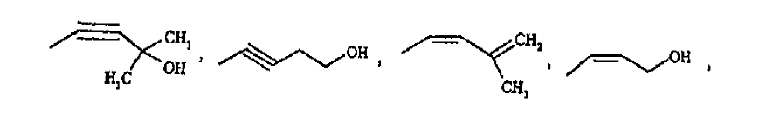 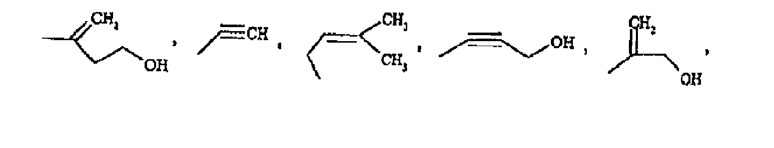 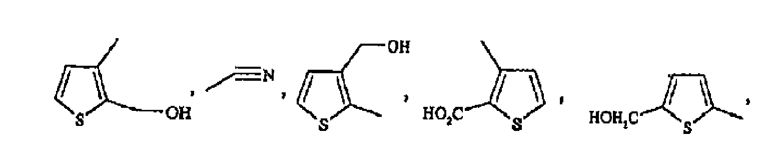 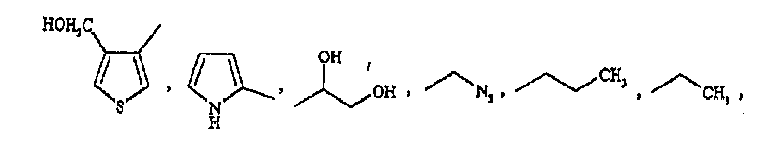 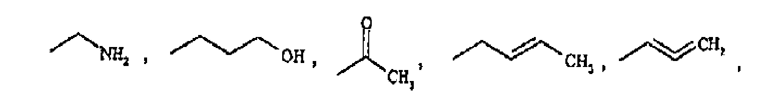 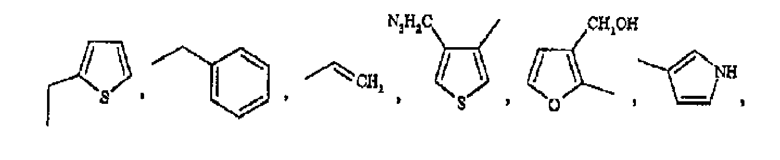 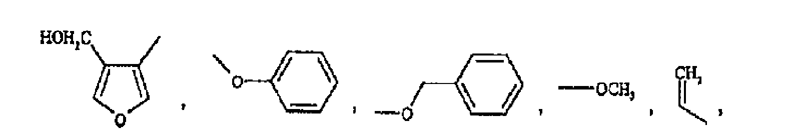 et 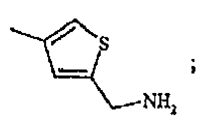 et R' est choisi dans le groupe consistant en 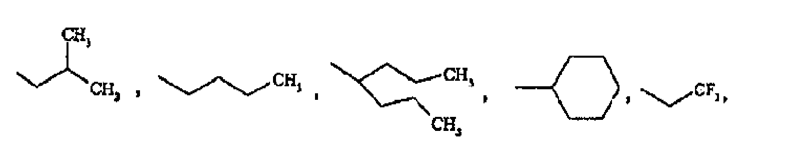 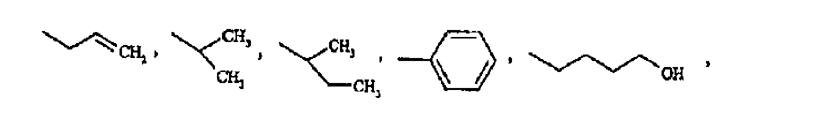 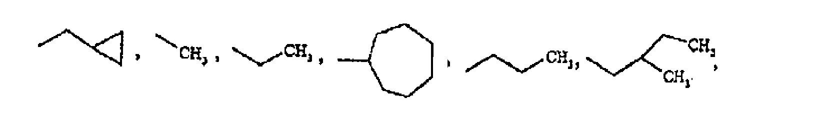 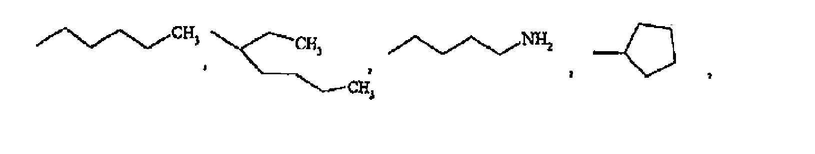 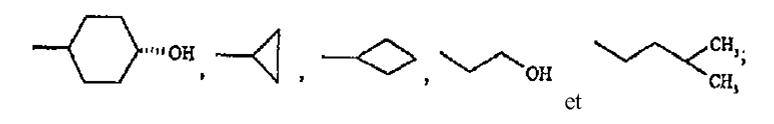 et ses sels pharmaceutiquement acceptables.

5. Composé représenté par la structure 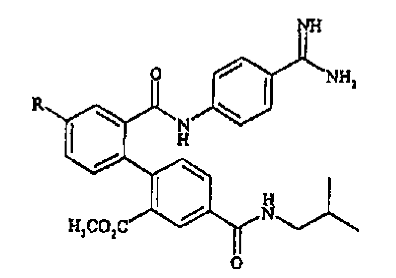 dans laquelle R est choisi dans le groupe consistant en 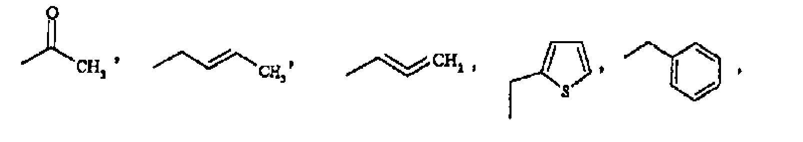 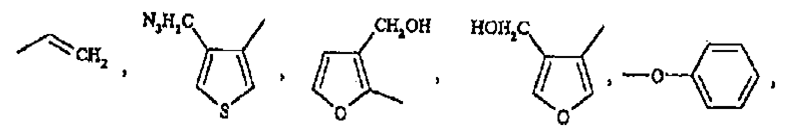 -OBn, -OCH₃, et 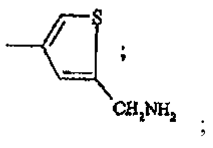 et ses sels pharmaceutiquement acceptables.

6. Composé représenté par la structure 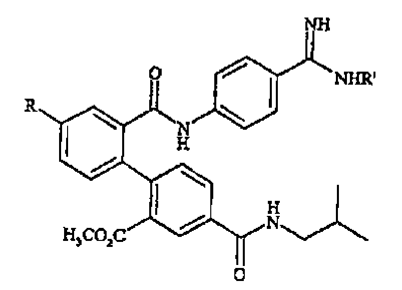 dans laquelle R est choisi dans le groupe consistant en -OSO₂CF₃, 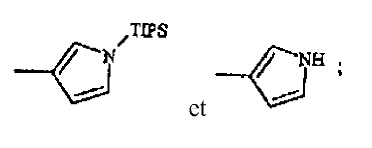 et R' est -H ou 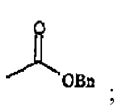 et ses sels pharmaceutiquement acceptables.

7. Composé représenté par la structure 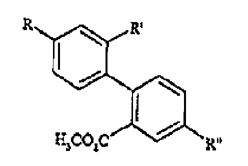 dans laquelle R est choisi dans le groupe consistant en
-OBn, -OH, -OSO₂CF₃, 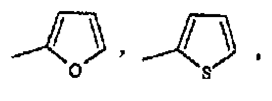 -CH=CH₂ et -H ;
R' est choisi dans le groupe consistant en -CHO, -CO₂H et -CO₂MEM ;
et R'' est choisi dans le groupe consistant en
-CO₂MEM, 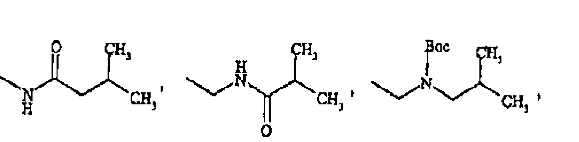 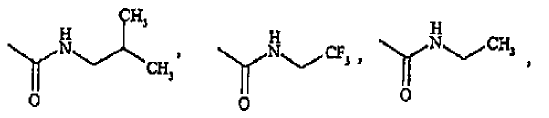 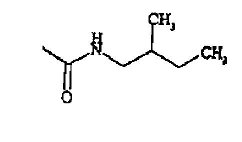 et CO₂H ;
et ses sels pharmaceutiquement acceptables.

8. Composé représenté par la structure 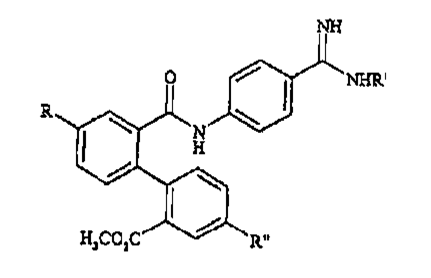 dans laquelle R est choisi dans le groupe consistant en
-OBn, -OH, -OSO₂CF₃, 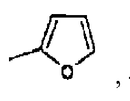 -CH=CH₂ ;
R' est -H ou -Boc ; et R" est -CO₂MEM ou -CO₂H ; et ses sels pharmaceutiquement acceptables;
où MEM désigne un groupe méthoxyéthoxyméthyle.

9. Composé représenté par la structure 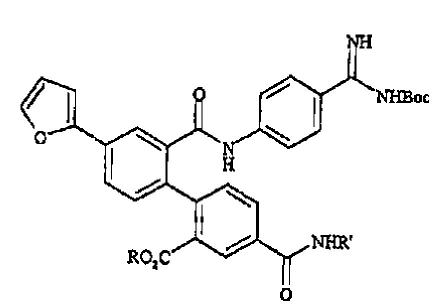 dans laquelle R est -CH₃ et R' est choisi dans le groupe consistant en 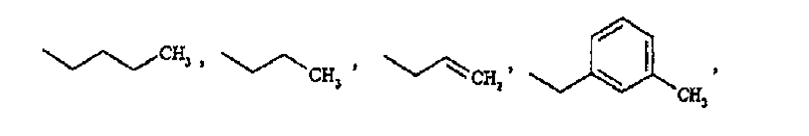 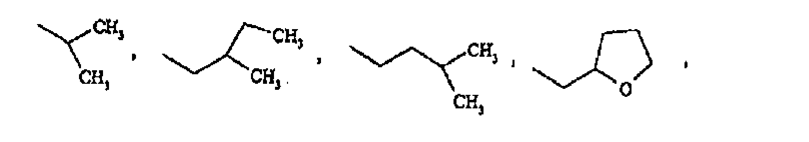 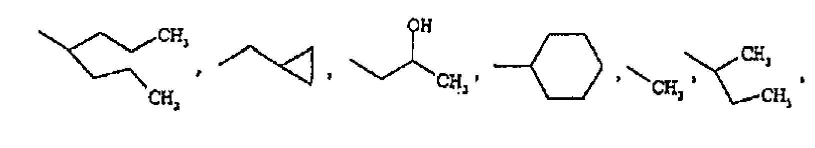 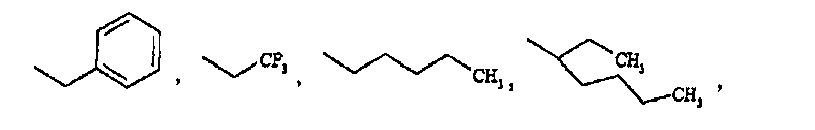 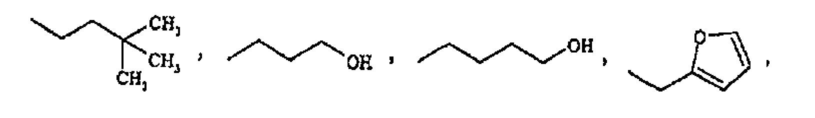 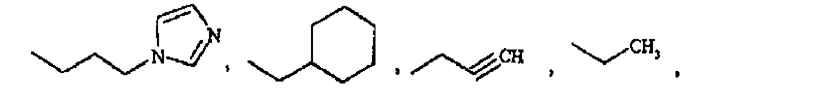 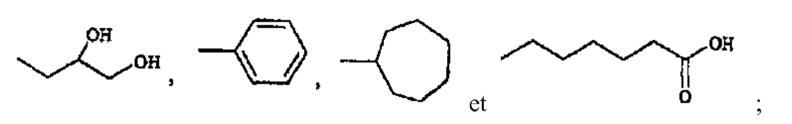 ou 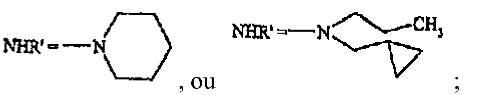 et ses sels pharmaceutiquement acceptables.

10. Composé représenté par la structure 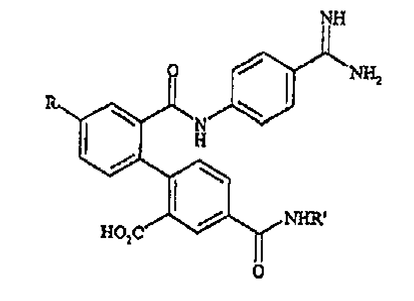 dans laquelle R est 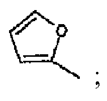 et R' est choisi dans le groupe consistant en 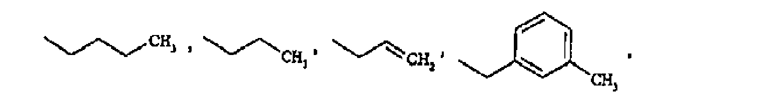 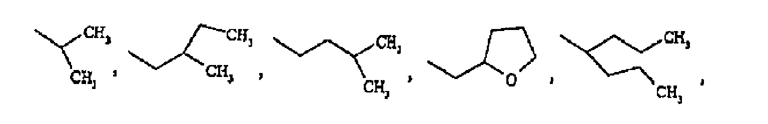 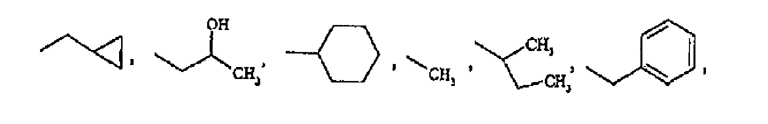 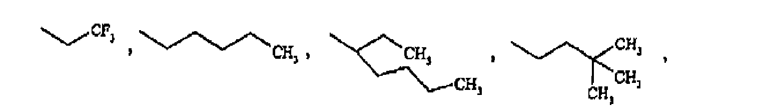 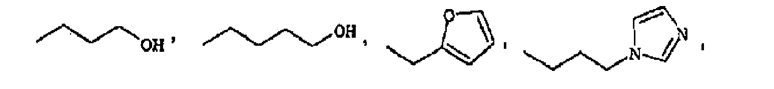 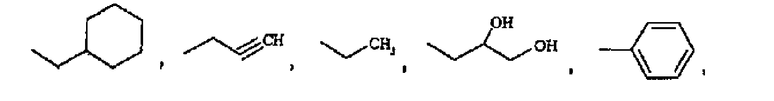 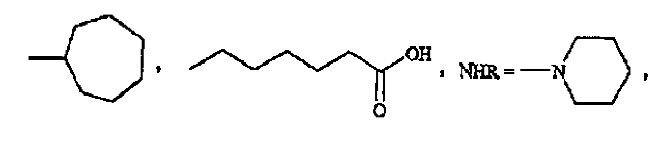 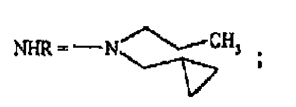 et ses sels pharmaceutiquement acceptables.

11. Composé selon la revendication 1 représenté par la structure 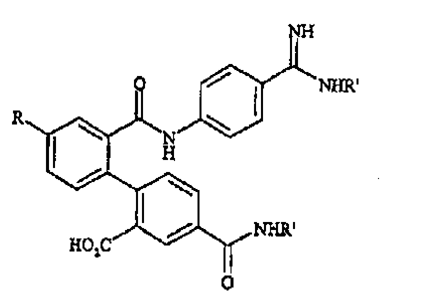 dans laquelle R est 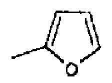 et -CH=CH₂ et R' est choisi dans le groupe consistant en 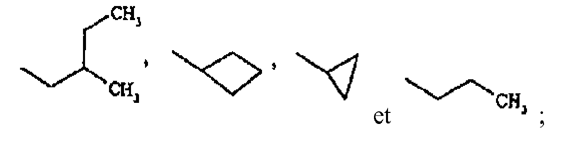 et ses sels pharmaceutiquement acceptables.

12. Composé représenté par la structure 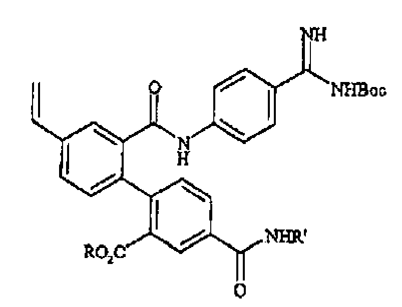 dans laquelle R est -CH₃ et R' est choisi dans le groupe consistant en 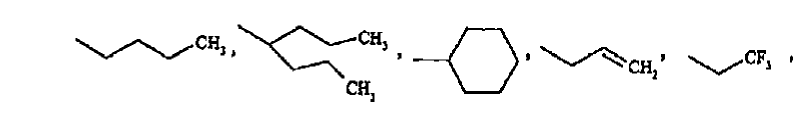 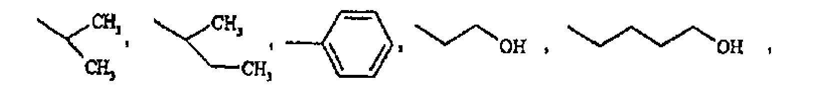 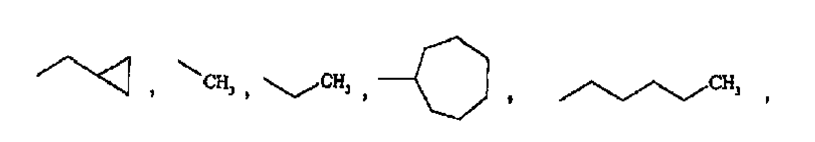 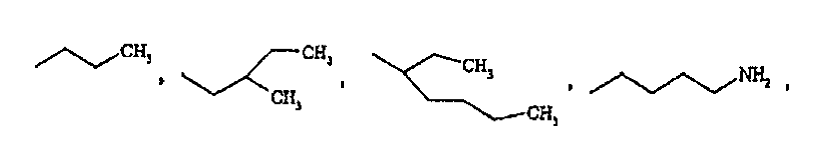 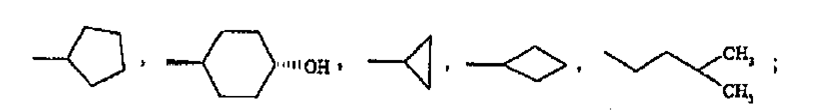 et ses sels pharmaceutiquement acceptables.

13. Composé représenté par la structure 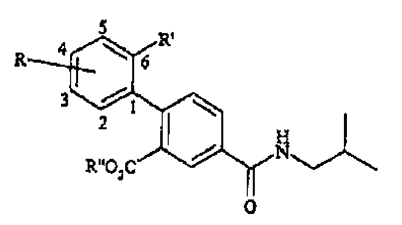 dans laquelle au moins un R est choisi dans le groupe consistant en -OCH₃, -OH, -OSO₂CF₃, -CH=CH₂, -OCH₂CO₂C₂H₅,
-OCH₂CON₂, 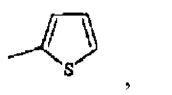 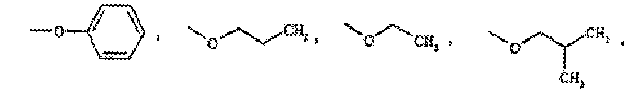 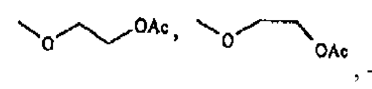 -OBn, -OH, -OSO₂CF₃, 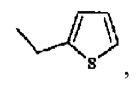 -OCH₃, -OBn, -H, -OBn et -CH=CH₂;
R' est choisi dans le groupe consistant en -CHO, -CO₂H, -CO₂MEM, 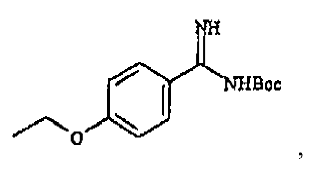 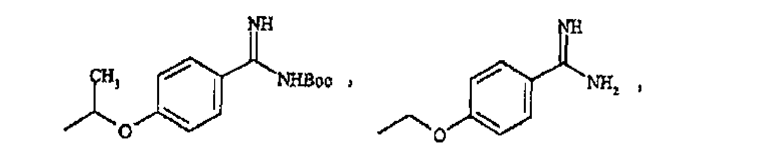 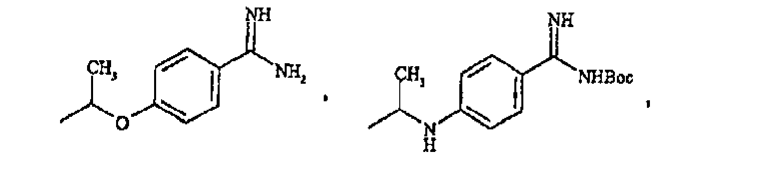 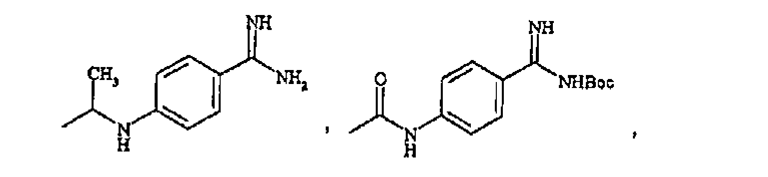 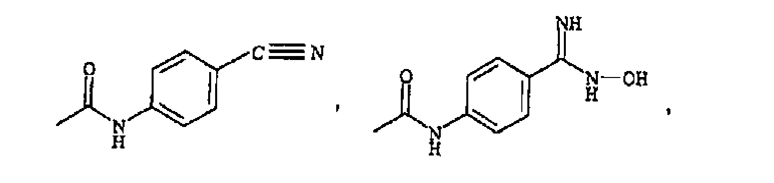 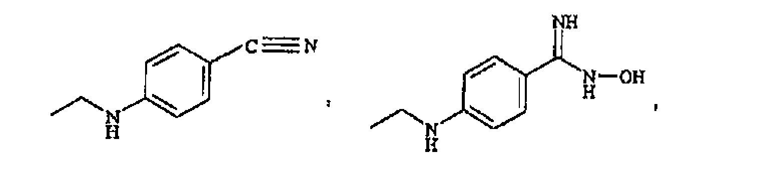 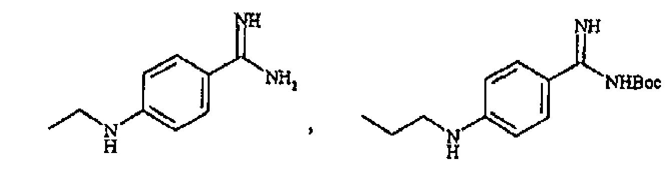 et 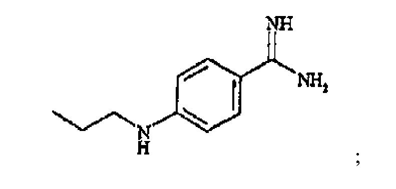
et R" est choisi dans le groupe consistant en -H, -CH₃ et -Bn ; et ses sels pharmaceutiquement acceptables ;
où MEM désigne un groupe méthoxyéthoxyméthyle.

14. Composé représenté par la structure 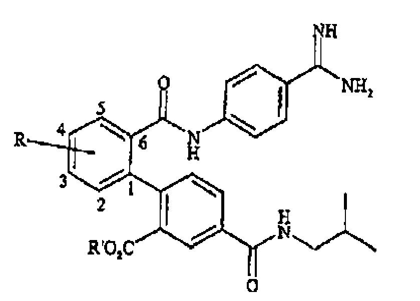 dans laquelle au moins un R est choisi dans le groupe consistant en -CH=CH₂, -OSO₂CF₃, -OCH₂CO₂C₂H₅, -OCH₂CONH₂, 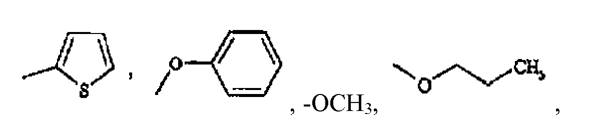 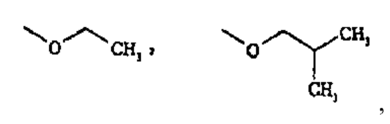 -O-CH₂-CH₂-OAc, -OH,
-OCH₂CO₂H, -O-CH₂-CH₂-OH, -CH(OH)CH₂OH, -CH₂OH, -CO₂H, 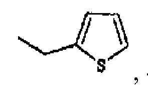 -OBn, -OH, -OCH₃, -OBn, -OH, -OC₂H₅,
-OBn, -OCH₃ et -CH(OH)CH₃ ; et R' est choisi dans le groupe consistant en -CH₃, - CH₂C₆H₅, -Bn, -H ; et ses sels pharmaceutiquement acceptables.

15. Composé représenté par la structure 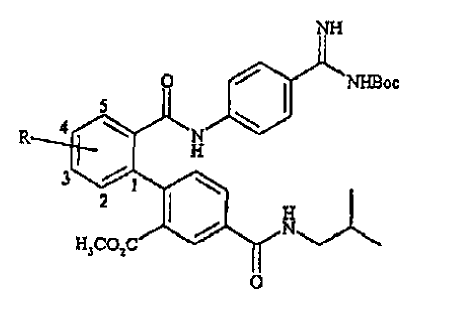 dans laquelle au moins un R est choisi dans le groupe consistant en -CH=CH₂, - CH(OH)CH₂OH, -CH=O, -CH₂OH, -CO₂H, -OCH₃, -CH=CH₂ ; et ses sels pharmaceutiquement acceptables.

16. Composé représenté par la structure 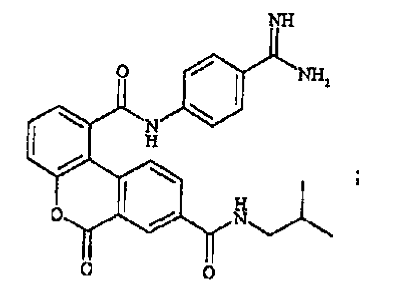 et ses sels pharmaceutiquement acceptables.

17. Composé représenté par la structure 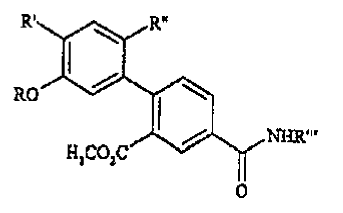 dans laquelle R est choisi dans le groupe consistant en
-CH₃, -C₂H₅, -CH(CH₃)₂, 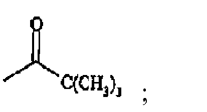
R' est choisi dans le groupe consistant en -OBn, -OH, -OSO₂CF₃ et -CH=CH₂ ;
R'' est choisi dans le groupe consistant en -CO₂H, -CO₂MEM ou -CHO ;
et R"' est choisi dans le groupe consistant en 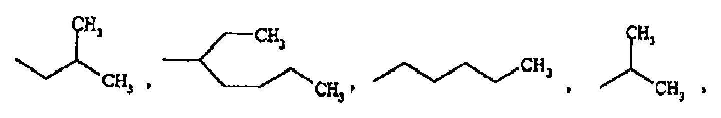 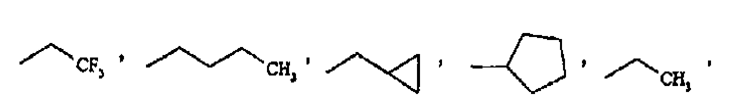 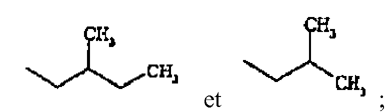 et ses sels pharmaceutiquement acceptables ; où MEM
désigne un groupe méthoxyéthoxyméthyle.

18. Composé représenté par la structure 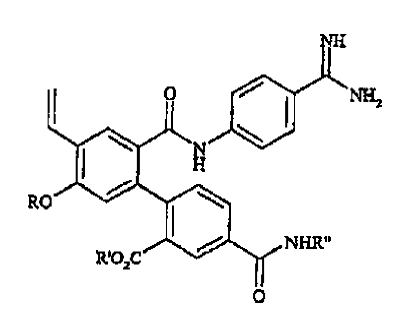 dans laquelle R est choisi dans le groupe consistant en
-CH₃, -C₂H₅, -CH(CH₃)₂, 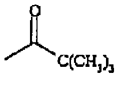 et -H ;
R' est -H ou un groupe alkyle ; et R" est choisi dans le groupe consistant en 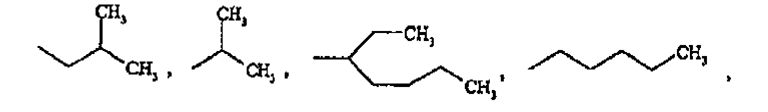 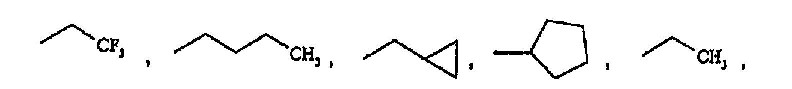 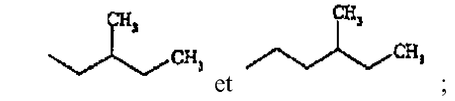 et ses sels pharmaceutiquement acceptables.

19. Composé selon la revendication 18, dans lequel ledit groupe alkyle est -CH₃.

20. Composé représenté par la structure 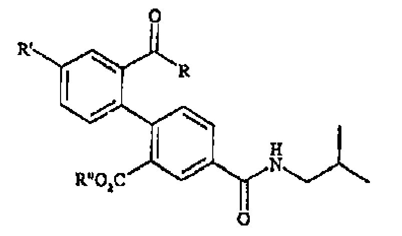 dans laquelle R est choisi dans le groupe consistant en 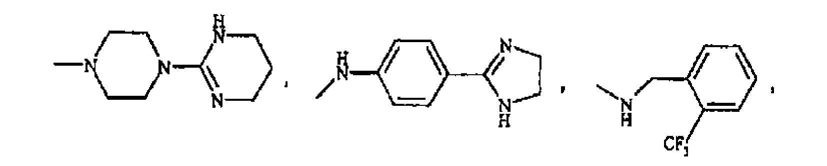 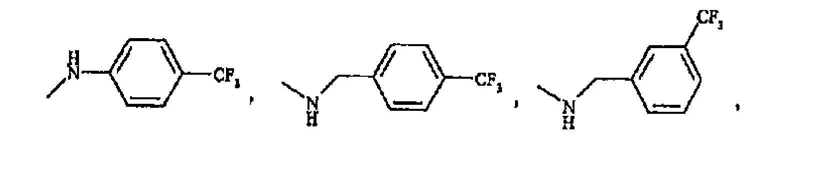 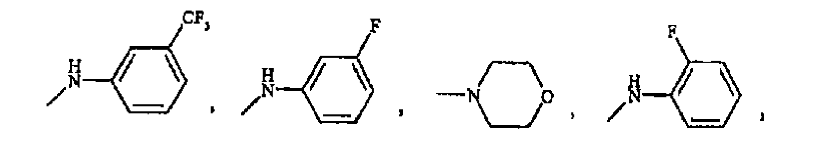 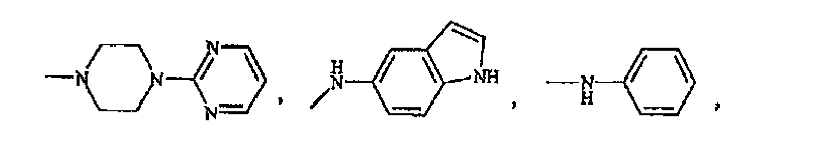 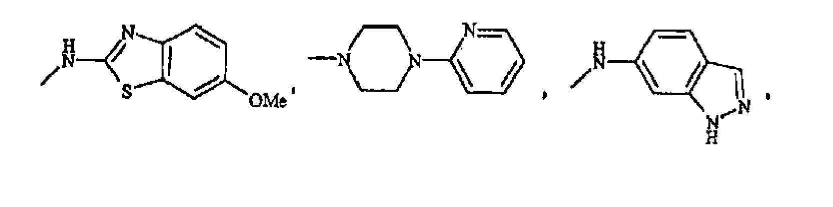 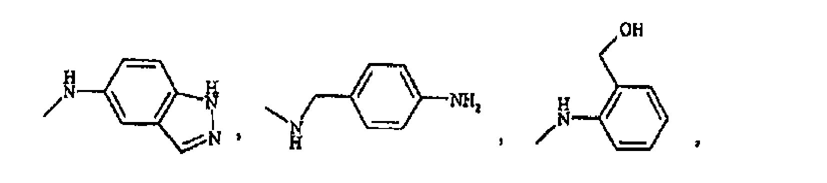 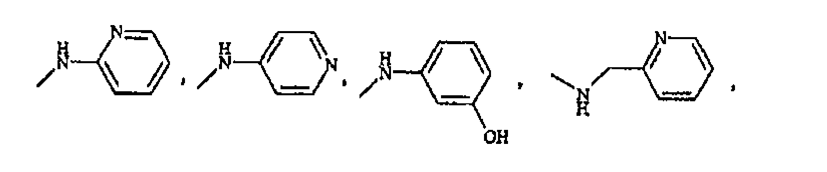 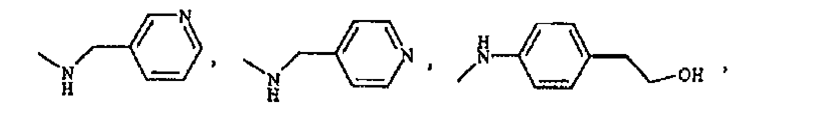 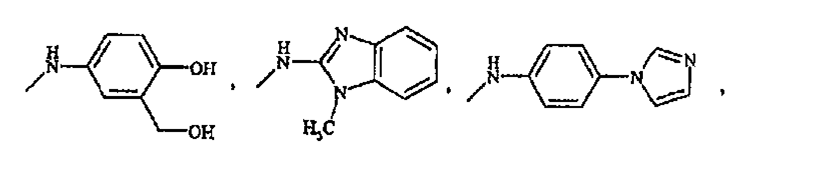 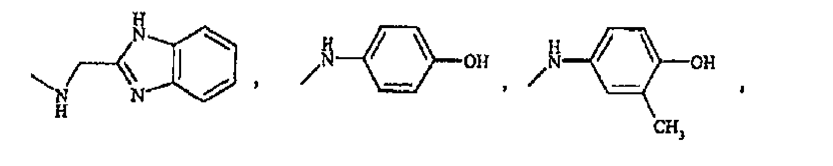 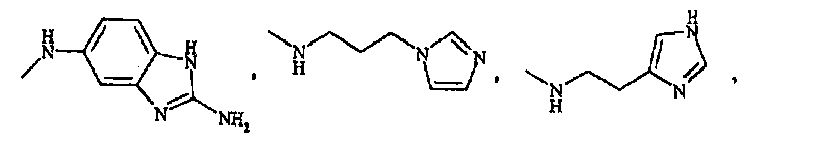 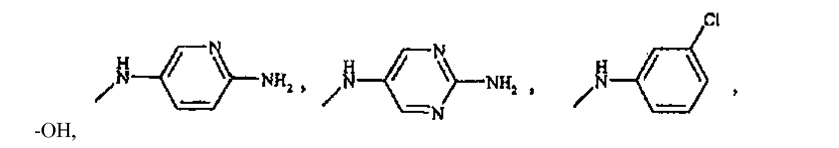 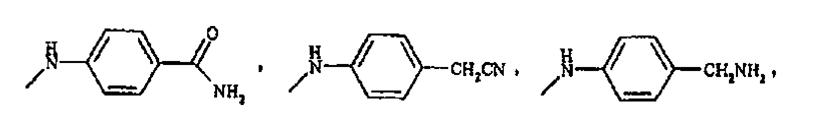 et 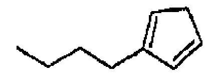
R' est -H, -CH=CH₂ ; et R" est -H ou un groupe alkyle ; et ses sels pharmaceutiquement acceptables.

21. Composé selon la revendication 20, dans lequel ledit groupe alkyle est -CH₃.

22. Composé représenté par la structure 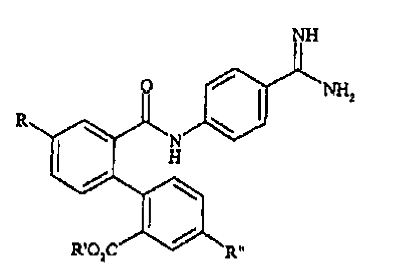 dans laquelle R est choisi dans le groupe consistant en 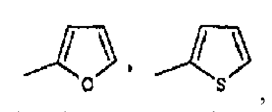 -CH=CH₂ et -H ; R' est -H ou un groupe alkyle ; et R" est choisi dans le groupe consistant en 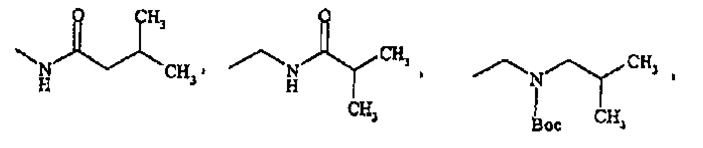 et 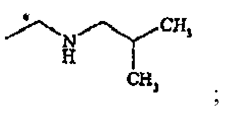 et ses sels pharmaceutiquement acceptables.

23. Composé selon la revendication 22, dans lequel ledit groupe alkyle est -CH₃.

24. Composé représenté par la structure 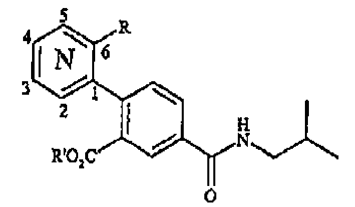 dans laquelle N est situé en position 3 ou 4 dans le cycle phényle ; R est choisi dans le groupe consistant en -CHO, -CO₂H et 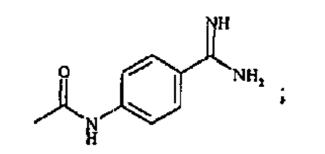 et R' est -H ou un groupe alkyle ; et ses sels pharmaceutiquement acceptables.

25. Composé selon la revendication 24, dans lequel ledit groupe alkyle est -CH₃.

26. Composé représenté par la structure 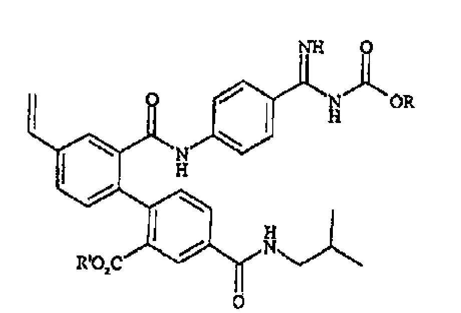 dans laquelle R est choisi dans le groupe consistant en
-CH₃, -C₂H₅, -CH₂C₆H₅, -C(CH₃)₃, -CH₂-CCl₃, 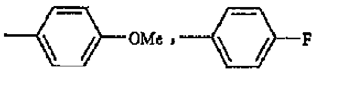 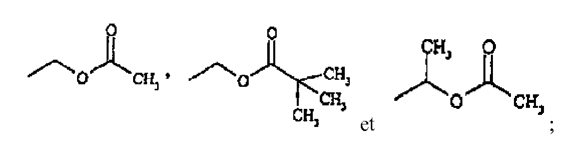 et R' est -H ou un groupe alkyle ; et ses sels pharmaceutiquement acceptables.

27. Composé selon la revendication 26, dans lequel le groupe alkyle est CH₃.

28. Composé représenté par la structure 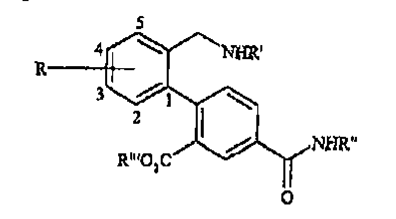 dans laquelle au moins un R est choisi dans le groupe consistant en -CH=CH₂, -OCH₃, - OBn, -OH et -H ; R' est 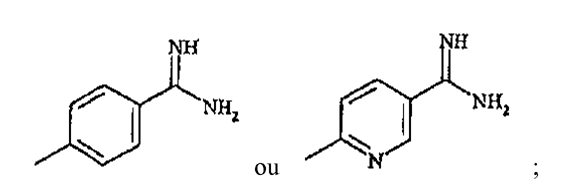
R'' est choisi dans le groupe consistant en 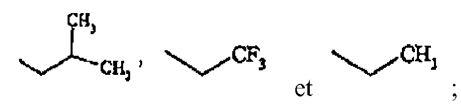 et R"' est -H ; et ses sels pharmaceutiquement acceptables.

29. Composé représenté par la structure 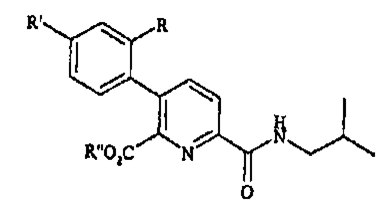 dans laquelle R est choisi dans le groupe consistant en -CHO, -CO₂H, -CO₂MEM, 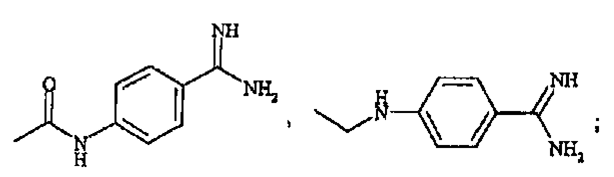
R' est choisi dans le groupe consistant en -OBn, -OH, -OSO₂CF₃ et -CH=CH₂ ;
et R'' est -H ou un groupe alkyle ; et ses sels pharmaceutiquement acceptables.

30. Composé selon la revendication 29, dans lequel ledit groupe alkyle est -CH₃.

31. Composé représenté par la structure 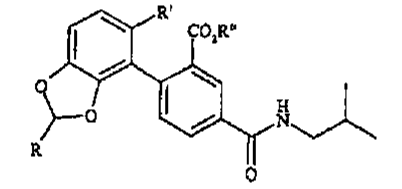 dans laquelle R est -H ou -CO₂H ;
R' est choisi dans le groupe consistant en -CHO, -CO₂H et 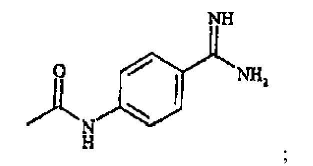
et R'' est -H ou un groupe alkyle ; et ses sels pharmaceutiquement acceptables.

32. Composé selon la revendication 31, dans lequel ledit groupe alkyle est -CH₃.

33. Composé représenté par la structure 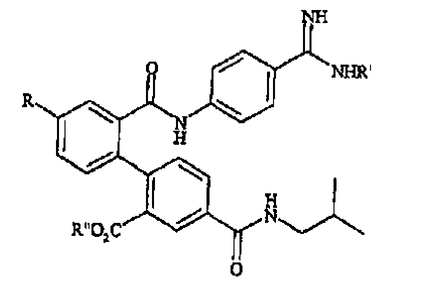 dans laquelle R est choisi dans le groupe consistant en -CH(OH)-CH₂OH, -CHO et - CH(OH)-CH=CH₂ ;
R' est -Boc ou -H ; et R" est -H ou un groupe alkyle ; et ses sels pharmaceutiquement acceptables.

34. Composé selon la revendication 33, dans lequel ledit groupe alkyle est -CH₃.

35. Composé représenté par la structure 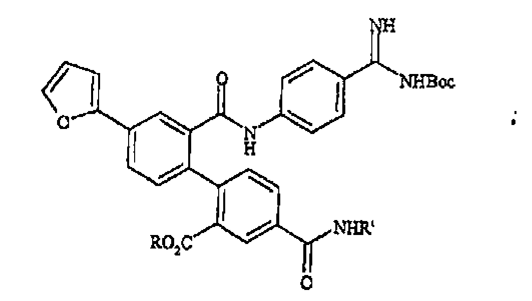 dans laquelle R est un groupe alkyle et R' est choisi dans le groupe consistant en 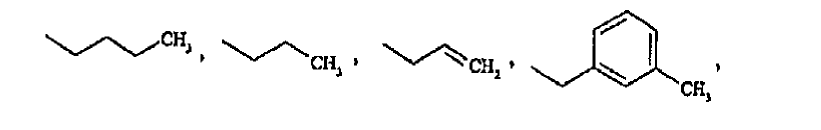 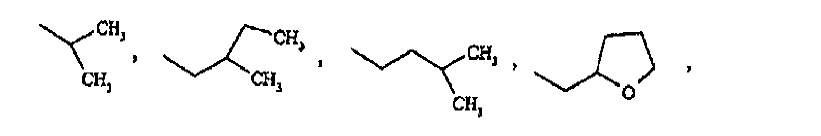 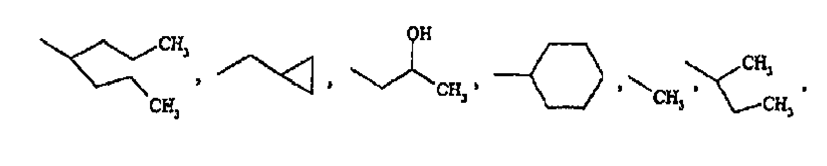 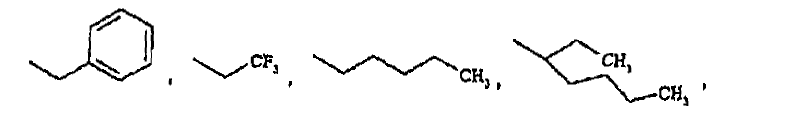 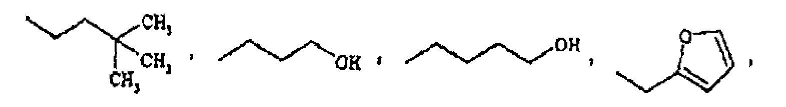 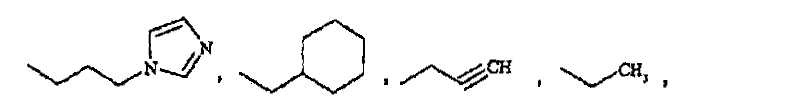 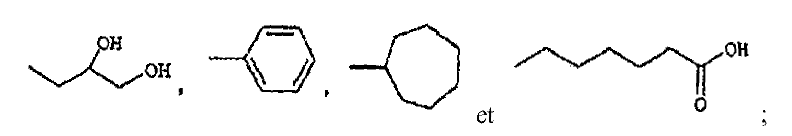 ou 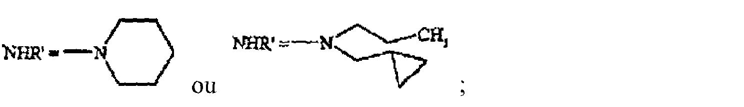 et ses sels pharmaceutiquement acceptables.
